# EUROPÄISCHE PATENTSCHRIFT

(11) **EP 0 422 485 B1**
(45) Veröffentlichungstag und Bekanntmachung des Hinweises auf die Patenterteilung: **03.04.1996**
(21) Anmeldenummer: 90118898.7
(22) Anmeldetag: 03.10.1990
(51) Int. Cl.: C07D 215/56, C07D 401/04, C07D 401/14, A61K 31/47

(54) **Chinoloncarbonsäurederivate, Verfahren zu ihrer Herstellung sowie ihre Verwendung als antivirale Mittel**
Quinolone carboxylic acid derivatives, process for their preparation and their use
Dérivés d'acide quinolone carboxylique, procédé pour leur préparation et leur utilisation

(30) Priorität: 12.10.1989 DE 3934082
(43) Veröffentlichungstag der Anmeldung: 17.04.1991
(73) Patentinhaber: BAYER AG, D-51368 Leverkusen (DE)
(72) Erfinder: Grohe, Klaus, Dr., W-5068 Odenthal (DE); Dummer, Wolfgang, Dr., W-5000 Köln 80 (DE); Rossen, Kai, Dr., W-5090 Leverkusen 1 (DE); Paessens, Arnold, Dr., W-5657 Haan (DE)

(56) Entgegenhaltungen:
- EP-A- 0 131 839
- EP-A- 0 154 780
- FR-A- 2 614 620
- CHEMICAL ABSTRACTS, Band 107, Nr. 19, 1987, Columbus, OH (US); Seite 711, Nr. 175900y
- CHEMICAL ABSTRACTS, Band 112, Nr. 23, 1990, Columbus, OH (US); S. RADL et al., Seite 760, Nr. 178881g
- CHEMICAL ABSTRACTS, Band 114, Nr. 4, 1991, Columbus, OH (US); L. ELROD, Jr. et al., Seite 397, Nr. 30231r
- PROCEEDINGS OF THE NATL. ACADEMY OF SCIENCES USA, Band 86, 1989, Washington, DC (US); J.L. DAVIES et al., Seiten 414-418
- VACCINES, 1986, F. Brown et al. (eds.), New York, NY (US); S. WAIN-HOBSON et al., Seiten 339-343
- NATURE, Band 330, 1987; M. GOUD et al., Seiten 388-391
- NATURE, Band 329, 1987; J. KAHON et al., Seiten 654-656

## Beschreibung

Die vorliegende Erfindung betrifft neue Chinoloncarbonsäurederivate, Verfahren zu ihrer Herstellung sowie ihre Verwendung als Arzneimittel, insbesondere als antivirale Mittel.

Es ist bekannt, daß manche Chinoloncarbonsäurederivate ausgezeichnete antibakterielle Wirksamkeit besitzen. Wenig ist bisher bekannt geworden über antivirale Wirkung von Chinolonen. So beschrieben E. Ferrazzi et al. die antivirale Wirkung von Norfloxacin, Coumermycin A₁ und Nalidixinsäure gegen BK-Viren, die zu den Papova-Viren gehören (Biochemical Pharmacology, Vol. 37, No. 9, pp. 1885-1886, 1986).

Auf der 5. AIDS-Konferenz in Montreal, Kanada, berichteten L. Gürtler et al. über die Inhibition der HIV-Reversen Transcriptase durch Ofloxacin, Ciprofloxacin und Norfloxacin (Abstract C. 624).

Aus Chemical Abstracts, Band 107, Nr. 19, 1987, Seite 711, Nr.175900y, Radl, S. et al., Collect. Czech. Chem. Commun., Band 54, 1989, Seiten 2181-2189 und EP-A-0 154 780 sind bereits1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolin-carbonsäure,1-(4-Aminophenyl)-6-fluor-7-(4-methylpiperazinyl)-1,4-dihydro-4-oxo-3-chinolincarbonsäure und 1-(4-Aminophenyl)-6,8-difluor-7-morpholinyl-1,4-dihydro-4-oxo-3-chinolincarbonsäure bekannt und als antimikrobiell wirksame Substanzen vorgestellt worden.

Es wurde nun gefunden, daß die Verbindungen der allgemeinen Formel (I) 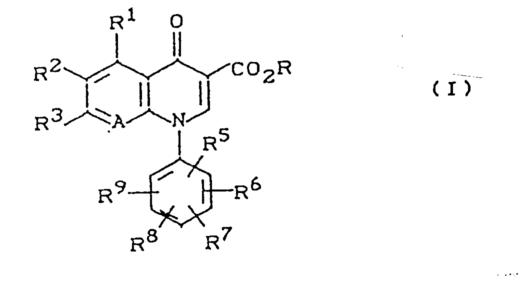
in der
- R: für Wasserstoff oder Alkyl mit 1 - 4 Kohlenstoffatomen,
- R¹: für Wasserstoff, Amino, Alkylamino mit 1 - 4 Kohlenstoffatomen, Dialkylamino mit 1 - 3 Kohlenstoffatomen je Alkylgruppe, Hydroxy, Alkoxy mit 1 - 4 Kohlenstoffatomen, Mercapto, Alkylthio mit 1 - 4 Kohlenstoffatomen, Arylthio, Halogen, Cyano oder Nitro,
- A: für N oder CR₄ steht, wobei
- R⁴: für Wasserstoff, Halogen, Methyl, Cyano, Nitro, Methoxy oder Amin steht oder auch für eine über O, S oder CH₂ Brücke, die mit der ortho-Position des Phenylrings an N-1 verbunden ist, steht.
- R²: für Wasserstoff, Nitro oder Halogen steht,
- R³ und R⁵: gleich oder verschieden sind und für NR²⁵R²⁶ stehen, worin R²⁵ und R²⁶ gleich oder verschieden sind und für Wasserstoff, C₁-C₂₀-Alkyl, gegebenenfalls substituiert durch ein oder mehrere Halogene, OH, O-Alkyl (C₁-C₆), Alkyl (C₁-C₄), CN, COO Alkyl (C₁-C₆),
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl stehen, oder R³ und R⁵ für eine Gruppe mit der Formel (II) 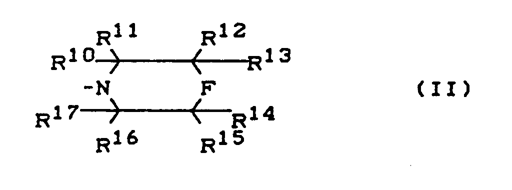 stehen, wobei
   F O, S, NR¹⁸ oder CR¹⁹R²⁰ bedeutet und R⁶, R⁷, R⁸ und R⁹ gleich oder verschieden sind und für
- Wasserstoff,
- Methyl, Ethyl, Nitro, Amino, Monoalkylamino mit 1-3 C-Atomen, Dialkylamino mit 1-4 C-Atomen, Methoxy, Ethoxy, Thiomethyl, Thioethyl, Trifluormethyl, Cyano, Halogen stehen,
- R¹⁸ für: - H, (C₁-C₁₀)-Alkyl oder (C₃-C₆)-Cycloalkyl, gegebenenfalls substituiert mit einem oder mehreren Substituenten Hal, OH, CN, COOR, OR mit R = (C₁-C₇)-Alkyl,
- Aryl(C₆-C₁₂), gegebenenfalls substituiert durch Halogen, -O-Alkyl(C₁-C₂) oder (C₁-C₄)-Alkyl,
- CN,
- COOAlkyl(C₁-C₄),
- C₁-C₄-Acyl oder
- (C₁-C₄)-Sulfonyl steht,
R¹⁰, R¹¹, R¹², R¹³, R¹⁴, R¹⁵, R¹⁶, R¹⁷, R¹⁹ und R²⁰ gleich oder verschieden sind und stehen für
- Wasserstoff,
- C₁-C₃ Alkyl oder C₃-C₆-Cycloalkyl, gegebenenfalls substituiert durch ein oder mehrere Halogene, OH, O-Alkyl (C₁-C₆), Alkyl (C₁-C₄), CN, COO Alkyl (C₁-C₆),
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl.

R³ und R⁵ können ferner für einen Rest der Struktur (IV) stehen, 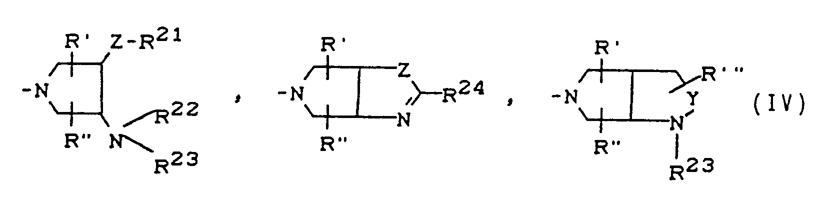
wobei
- R²¹: für H, C₁-C₄-Alkyl, Aryl, C₁-C₄-Acyl,
- R²²: für H, C₁-C₄-Alkyl, OH, OCH₃, wobei R²¹ und R²² gemeinsam auch eine gegebenenfalls durch Methyl ein- oder zweifach substituierte C₁-C₃-Alkylenbrücke bedeuten kann,
- R²³: für H, C₁-C₄-Alkyl, Aryl, Heteroaryl, Benzyl, C₁-C₄-Alkoxycarbonyl, C₁-C₄-Acyl oder (5-Methyl-2-oxo-1,3-dioxol-4-yl)-methyl,
- R²⁴: für H oder C₁-C₄-Alkyl,
- R': für H, CH₃ oder Phenyl,
- R'': für H, CH₃ oder Phenyl,
- R''': für H oder CH₃,
- Y: für O, CH₂, CH₂CH₂ oder CH₂-O stehen kann, wobei die Verknüpfung der CH₂-O-Gruppe zum Stickstoff sowohl über O als auch über CH₂ erfolgen kann,
- Z: für O oder S stehen kann,
- ZR²¹: für Wasserstoff stehen kann,
R⁶, R⁷, R⁸ und R⁹ sind gleich oder verschieden und stehen für
- Wasserstoff,
- Alkyl mit 1 bis 5-Kohlenstoffatomen, Nitro, Amino, Monoalkylamino mit 1-3 C-Atomen, Dialkylamino mit 1-6 C-Atomen,
   O-Alkyl mit 1-5 Kohlenstoffatomen, S-Alkyl mit 1-5 Kohlenstoffatomen, Trifluormethyl, Cyano, Halogen,
mit Ausnahme von 1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure, 1-(4-Aminophenyl)-6-fluor-7-(4-methylpiperazinyl)-1,4-dihydro-4-oxo-3-chinolincarbonsäure und 1-(4-Aminophenyl)-6,8-difluor-7-morpholinyl-1,4-dihydro-4-oxo-3-chinolincarbonsäure.

Bevorzugt sind hierbei Verbindungen der allgemeinen Formel (I) 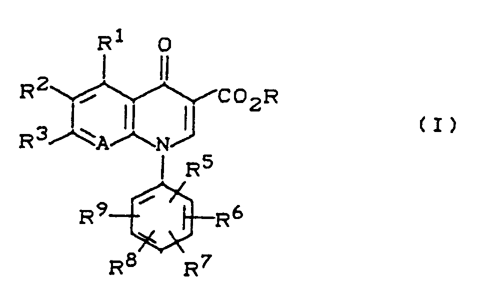
in der
- R: für Wasserstoff, Methyl oder Ethyl steht,
- R¹: für Wasserstoff, Amino, Nitro, Methoxy, Methylthio, Cyano oder Halogen,
- R²: für Wasserstoff, Nitro oder Halogen steht,
- A: für N oder CR₄ steht, wobei
- R⁴: für Wasserstoff, Halogen, Methyl, Cyano, Nitro, Methoxy oder Amin steht oder auch für eine über O, S oder CH₂ Brücke, die mit der ortho-Position des Phenylrings an N-1 verbunden ist, steht, und
- R³ und R⁵: gleich oder verschieden sind und für NR²⁵R²⁶ stehen, worin R²⁵ und R²⁶ gleich oder verschieden sind und für Wasserstoff: C₁-C₂₀-Alkyl, gegebenenfalls substituiert durch ein oder mehrere Halogene, O-Alkyl (C₁-C₆), Alkyl (C₁-C₄), je Alkylgruppe oder gegebenenfalls durch Halogen substituiertes Phenyl stehen, oder
- R³ und R⁵: für eine Gruppe mit der Formel (II) 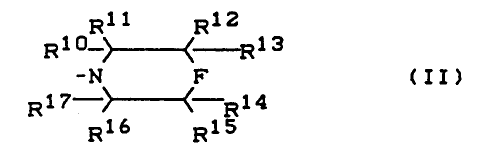 stehten, wobei
- F: Schwefel, Sauerstoff, NR¹⁸ oder CR¹⁹R²⁰ bedeutet
und
- R¹⁸: für
- H, (C₁-C₁₀)-Alkyl oder (C₃-C₆)-Cycloalkyl, gegebenenfalls substituiert mit einem oder mehreren Substituenten Hal, OH, CN, COOR, OR mit R = (C₁-C₇)-Alkyl,
- Aryl(C₆-C₁₂), gegebenenfalls substituiert durch Halogen, -O-Alkyl(C₁-C₂) oder (C₁-C₄)-Alkyl,
- CN,
- COOAlkyl(C₁-C₄)
- C₁-C₄-Acyl oder
- C₁-C₄-Sulfonyl steht.

R¹⁰, R¹¹, R¹², R¹³, R¹⁴, R¹⁵, R¹⁶, R¹⁷, R¹⁹, R²⁰ sind gleich oder verschieden und stehen für
- Wasserstoff,
- C₁-C₃ Alkyl oder C₃-C₆ Cycloalkyl, gegebenenfalls substituiert durch ein oder mehrere Halogene, OH, Methoxy, Ethoxy, Methyl, Ethyl, Cyano, CO₂Me,
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituierter Phenyl.

R³ und R⁵ können ferner für einen Rest der Strukturen 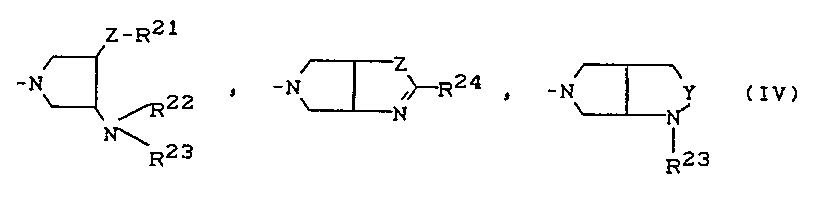
wobei
- R²¹: für H, C₁-C₄-Alkyl, Aryl, C₁-C₄-Acyl,
- R²²: für H, C₁-C₄-Alkyl, OH, OCH₃, wobei R²¹ und R²² gemeinsam auch eine gegebenenfalls durch Methyl ein- oder zweifach substituierte C₁-C₃-Alkylenbrücke bedeuten kann,
- R²³: für H, C₁-C₄-Alkyl, Aryl, Heteroaryl, Benzyl, C₁-C₄-Alkoxycarbonyl, C₁-C₄-Acyl
- R²⁴: für H oder C₁-C₄-Alkyl,
- Y: für O, CH₂, CH₂CH₂ oder CH₂-O stehen kann, wobei die Verknüpfung der CH₂-O-Gruppe zum Stickstoff sowohl über O als auch über CH₂ erfolgen kann,
- Z: für O oder S stehen kann,
- ZR²¹: für Wasserstoff stehen kann, wobei
R⁶, R⁷, R⁸ und R⁹ gleich oder verschieden sind und für
- Wasserstoff,
- Methyl, Ethyl, Nitro, Amino, Monoalkylamino mit 1-3 C-Atomen, Dialkylamino mit 1-4 C-Atomen, Methoxy, Ethoxy, Thiomethyl, Thioethyl, Trifluormethyl, Cyano, Halogen stehen, mit Ausnahme von 1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure, 1-(4-Aminophenyl)-6-fluor-7(4-methylpiperazinyl)-1,4-dihydro-4-oxo-3-chinolincarbonsäure und 1-(4-Aminophenyl)-6,8-difluor-7-morpholinyl-1,4-dihydro-4-oxo-3-chinolincarbonsäure.

Besonders bevorzugt sind hierbei Verbindungen der allgemeinen Formel (I) 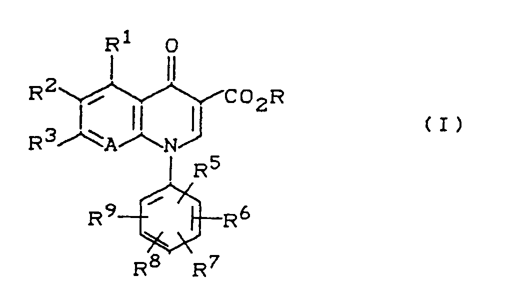
in der
- R: für Wasserstoff, Methyl oder Ethyl steht,
- R¹: für Wasserstoff, Halogen,
- R²: für Fluor, Nitro oder Wasserstoff steht,
- R³ und R⁵: gleich oder verschieden sind und für NR²⁵R²⁶ stehen, worin R²⁵ und R²⁶ gleich oder verschieden sind und für Wasserstoff, C₁-C₁₀-Alkyl, gegebenenfalls substituiert durch ein oder mehrere Halogene, O-Alkyl (C₁-C₆), Alkyl (C₁-C₄) je Alkylgruppe oder gegebenenfalls durch Halogen substituiertes Phenyl stehen, oder
R³ und R⁵ für eine Gruppe mit der Formel (II) 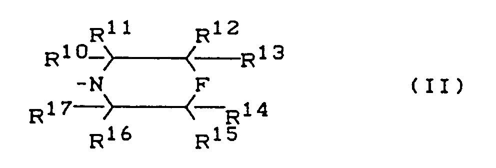
stehten, wobei
- F: Schwefel, Sauerstoff, NR¹⁸ oder CR¹⁹R²⁰ bedeutet
und
- R¹⁸: für
- Wasserstoff,
- Methyl, Ethyl, Propyl,
- (C₃-C₆)-Cycloalkyl,
- Phenyl, gegebenenfalls substituiert durch Halogen,
- CN,
- COO Alkyl (C₁-C₄),
- C₁-C₄ Acyl oder
- (C₁-C₄)-Sulfonyl steht, und
   R¹⁰, R¹¹, R¹⁷, R¹⁶ für Wasserstoff stehen, R¹², R¹³, R¹⁴, R¹⁵, R¹⁹, R²⁰ gleich oder verschieden sind und für
- Wasserstoff,
- C₁-C₃ Alkyl oder C₃-C₆ Cycloalkyl, gegebenenfalls substituiert durch ein oder mehrere Halogene, OH, Methoxy, Ethoxy, Methyl, Ethyl, Cyano, CO₂Me
- Hydroxyl,
- oder Amino, Alkylamino mit 1-4 Kohlenstoffatomen, Dialkylamino mit 1-3 Kohlenstoffatomen stehen, oder
R³ und R⁵ sind gleich oder verschieden und können ebenfalls für eine Gruppe der Formel 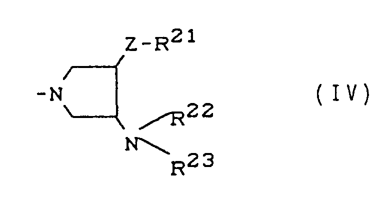
stehen,
wobei
- R²¹: für Wasserstoff, Methyl, Ethyl, Phenyl, C₁-C₄ Acyl,
- R²²: für Wasserstoff, Methyl, Ethyl, OH, OCH₃, wobei R²¹ und R²² gemeinsam auch durch eine durch Methyl ein- oder zweifach substituierte C₁-C₃-Alkylenbrücke bedeuten kann,
- R²³: für H, Methyl, Ethyl, Aryl, Benzyl, C₁-C₄ Alkoxycarbonyl, C₁-C₄ Acyl steht,
- Z: für O oder S stehen kann,
- ZR²¹: für Wasserstoff stehen kann,
- A: für N oder CR₄ steht, wobei
- R⁴: für Wasserstoff oder Halogen steht,
mit Ausnahme von 1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure, 1-(4-Aminophenyl)-6-fluor-7-(4-methylpiperazinyl)-1,4-dihydro-4-oxo-3-chinolincarbonsäure und 1-(4-Aminophenyl)-6,8-difluor-7-morpholinyl-1,4-dihydro-4-oxo-3-chinolincarbonsäure.

Weiterhin wurde gefunden, daß man erfindungsgemäße Verbindungen der Formel (I), in denen R³ und R⁵ gleich sind, erhält, wenn man Chinolone der allgemeinen Formel (III) 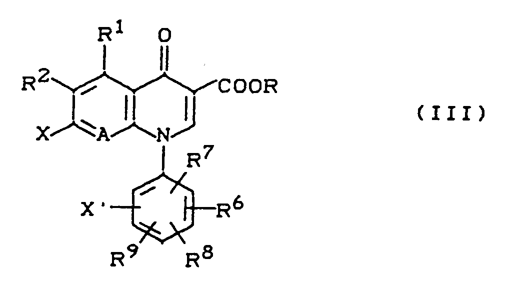
in der
A, R, R¹, R², R⁶, R⁷, R⁸ und R⁹ die oben angegebene Bedeutung haben und X und X' gleich oder verschieden sind und für ein Halogenatom stehen, mit Aminen der allgemeinen Formel (II) und (IV) 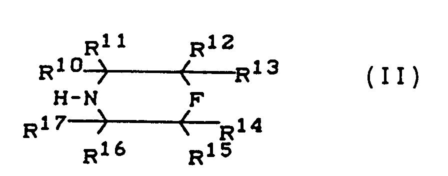 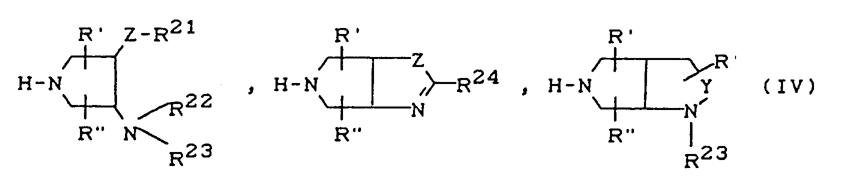
wobei
R¹⁰ - R¹⁷, F, R', R'', R''', Y, Z, R²¹ - R²⁴ die oben angegebene Bedeutung haben, umsetzt.

Verwendet man beispielsweise 1-(2,4-Difluorphenyl)-6-fluor-7-chlor-1,4-dihydro-4-oxo-3-chinolincarbonsäure und 2-Methylpiperazin als Ausgangsstoffe, so kann der Reaktionsablauf durch das folgende Formelschema wiedergegeben werden; 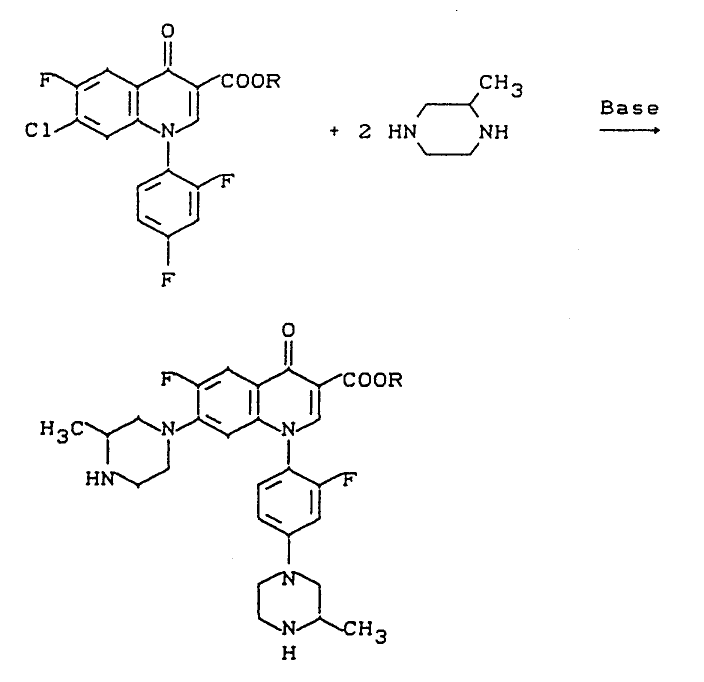
Ausgangsstoffe der allgemeinen Formel (III) sind bekannt oder können nach bekannten Verfahren hergestellt werden (Europäische Patentanmeldungen 131 839, 154 780, 302 372, Belgische Patentanmeldung 904 086).

### Beispiele sind:

1-(2,4-Difluorphenyl)-6-fluor-7-chlor-1,4-dihydro-4-oxo-3-chinolincarbonsäure,
1-(2,4-Difluorphenyl)-6,7-difluor-1,4-dihydro-4-oxo-3-chinolincarbonsäure,
1-(2,4-Difluorphenyl)-5,6,7-trifluor-1,4-dihydro-4-oxo-3-chinolincarbonsäure,
1-(2,4-Difluorphenyl)-6,7,8-trifluor-1,4-dihydro-4-oxo-3-chinolincarbonsäure,
1-(2,4-Difluorphenyl)-6,7-difluor-8-chlor-1,4-dihydro-4-oxo-3-chinolincarbonsäure,
1-(3,4-Difluorphenyl)-6,7-difluor-1,4-dihydro-4-oxo-3-chinolincarbonsäure,
1-(2-Chlor-4-fluorphenyl)-6,7-difluor-1,4-dihydro-4-oxo-3-chinolincarbonsäure,
1-(3-Chlor-4-fluorphenyl)-6,7-difluor-1,4-dihydro-4-oxo-3-chinolincarbonsäure,
1-(2,4-Difluorphenyl)-6-fluor-7-chlor-1,4-dihydro-4-oxo-3-chinolincarbonsäureethylester,
1-(2,4-Difluorphenyl)-6,7-difluor-1,4-dihydro-4-oxo-3-chinolincarbonsäureethylester,
1-(2,4-Difluorphenyl)-6,7-difluor-1,4-dihydro-4-oxo-3-chinolincarbonsäuremethylester,
7-Chlor-6-fluor-1-(2,4-difluorphenyl)-1,4-dihydro-4-oxo-1,8-naphthyridin-3-carbonsäure,
1-(2,3,4-Trifluorphenyl)-6-fluor-7-chlor-1,4-dihydro-4-oxo-3-chinolincarbonsäure,
1-(2,3,4-Trifluorphenyl)-6,7-difluor-1,4-dihydro-4-oxo-3-chinolincarbonsäure,
1-(2,3,4-Trifluorphenyl)-6,7,8-trifluor-1,4-dihydro-4-oxo-3-chinolincarbonsäure,
1-(3-Nitro-4-fluorphenyl)-6,7-difluor-1,4-dihydro-4-oxo-3-chinolincarbonsäure,
1-(3-Nitro-4-fluorphenyl)-6,7,8-trifluor-1,4-dihydro-4-oxo-3-chinolincarbonsäure,
1-(3-Nitro-4-fluorphenyl)-6,7-difluor-8-chlor-1,4-dihydro-4-oxo-3-chinolincarbonsäure,
7-Chlor-6-fluor-1-(2,4-difluorphenyl)-1,4-dihydro-4-oxo-1,8-naphthyridin-3-carbonsäureethylester,
1-(4-Fluorphenyl)-6-fluor-7-chlor-1,4-dihydro-4-oxo-3-chinolincarbonsäure,
1-(4-Fluorphenyl)-6,7-difluor-1,4-dihydro-4-oxo-3-chinolincarbonsäure,
1-(4-Fluorphenyl)-6,7-difluor-8-chlor-1,4-dihydro-4-oxo-3-chinolincarbonsäure,
1-(2-Fluorphenyl)-6-fluor-7-chlor-1,4-dihydro-4-oxo-chinolincarbonsäure,
1-(2-Fluorphenyl)-6,7-difluor-1,4-dihydro-4-oxo-chinolincarbonsäure,
1-(2,4-Difluorphenyl)-7-fluor-1,4-dihydro-4-oxo-chinolincarbonsäure,
1-(4-Fluorphenyl)-7-fluor-1,4-dihydro-4-oxo-chinolincarbonsäure,
1-(2-Fluorphenyl)-7-fluor-1,4-dihydro-4-oxo-chinolincarbonsäure,
7-Chlor-6-fluor-1-(4-fluorphenyl)-1,4-dihydro-4-oxo-1,8-naphthyridin-3-carbonsäure.

Die erfindungsgemäß verwendeten Amine sind bekannt oder können nach bekannten Verfahren hergestellt werden (Beilsteins Handbuch der Organischen Chemie, Bände 23 und 27, DE-OS 39 10 663).
Beispiele derartiger Amine sind: Piperazin, 1-Methylpiperazin, 1-Ethylpiperazin, 1-Propylpiperazin, 1-Isopropylpiperazin, 1-Cyclopropylpiperazin, 2-Methylpiperazin, 3-Ethylpiperazin, 2-Cyclohexylpiperazin, 1,2-Dimethylpiperazin, 2,2-Dimethylpiperazin, 2,8-Dimethylpiperazin, 1-(2-Hydroxyethyl)piperazin, 1-Phenylpiperazin, 2-Phenylpiperazin, Morpholin, Thiomorpholin, 2-Methylmorpholin, 3-Methylmorpholin, Piperidin, 2-Methylpiperidin, 3-Methylpiperidin, 4-Methylpiperidin, Pyrrolidin, 3-(tert.-Butoxycarbonylamino)-pyrrolidin, 3-Dimethylaminopyrrolidin,
4-Amino-3-hydroxypyrrolidin,
3-Hydroxy-4-methylaminopyrrolidin,
4-Dimethylamino-3-hydroxypyrrolidin,
4-Ethylamino-3-hydroxypyrrolidin,
3-Amino-4-methoxypyrrolidin,
4-Methoxy-3-methylaminopyrrolidin,
3-Dimethylamino-4-methoxypyrrolidin,
3-Ethylamino-4-methoxypyrrolidin,
3-Amino-4-ethoxypyrrolidin,
4-Ethoxy-3-methylaminopyrrolidin,
3-Dimethylamino-4-ethoxypyrrolidin,
4-Ethoxy-3-ethylaminopyrrolidin,
3-Hydroxy-4-hydroxyaminopyrrolidin,
3-Hydroxy-4-methoxyaminopyrrolidin,
3-Hydroxyamino-4-methoxypyrrolidin,
4-Methoxy-3-methoxyaminopyrrolidin,
3-Benzylamino-4-methoxypyrrolidin,
4-Methoxy-3-(5-methyl-2-oxo-1,3-dioxol-4-yl)-methylamino)-pyrrolidin,
3-Amino-4-methylmercaptopyrrolidin,
3-Acetoxy-4-dimethylaminopyrrolidin,
3-Acetamido-4-methoxypyrrolidin,
4-Methoxy-3-methoxycarbonylaminopyrrolidin,
3-Formamido-4-methoxypyrrolidin,
3-Amino-4-methoxy-2-methylpyrrolidin,
3-Amino-4-methoxy-5-methylpyrrolidin,
4-Methoxy-2-methyl-3-methylaminopyrrolidin,
4-Methoxy-5-methyl-3-methylaminopyrrolidin,
3-Amino-4-methoxy-2-phenylpyrrolidin,
4-Methoxy-3-methylamino-5-phenylpyrrolidin,
3-Methyl-2,7-diazabicyclo[3.3.0]octan,
4-Methyl-2,7-diazabicyclo[3.3.0]octan,
5-Methyl-2,7-diazabicyclo[3.3.0]octan,
3,5-Dimethyl-2,7-diazabicyclo[3.3.0]octan,
1,5-Dimethyl-2,7-diazabicyclo[3.3.0]octan,
2-Oxa-4,7-diazabicyclo[3.3.0]octan,
3,3-Dimethyl-2-oxa-4,7-diazabicyclo[3.3.0]octan,
3-Oxa-2,7-diazabicyclo[3.3.0]octan,
1,2-Dimethyl-3-oxa-2,7-diazabicyclo[3.3.0]octan,
2,5-Dimethyl-3-oxa-2,7-diazabicyclo[3.3.0]octan,
2,8-Dimethyl-3-oxa-2,7-diazabicyclo[3.3.0]octan,
5-Methyl-3-oxa-2,7-diazabicyclo[3.3.0]octan,
2-Oxa-4,7-diazabicyclo[3.3.0]oct-3-en,
3-Methyl-2-oxa-4,7-diazabicyclo[3.3.0]oct-3-en,
3-Phenyl-2-oxa-4,7-diazabicyclo[3.3.0]-3-en,
6-Methyl-2-oxa-4,7-diazabicyclo[3.3.0]oct-3-en,
8-Methyl-2-oxa-4,7-diazabicyclo[3.3.0]oct-3-en,
3-Methyl-2,8-diazabicyclo[4.3.0]nonan,
4-Methyl-2,8-diazabicyclo[4.3.0]nonan,
5-Methyl-2,8-diazabicyclo[4.3.0]nonan,
6-Methyl-2,8-diazabicyclo[4.3.0]nonan,
3-Methyl-2-oxa-5,8-diazabicyclo[4.3.0]nonan,
4-Methyl-2-oxa-5,8-diazabicyclo[4.3.0]nonan,
1-Methyl-2-oxa-5,8-diazabicyclo[4.3.0]nonan,
3,5-Dimethyl-2-oxa-5,8-diazabicyclo[4.3.0]nonan,
2-Thia-5,8-diazabicyclo[4.3.0]nonan,
5-Methyl-2-thia-5,8-diazabicyclo[4.3.0]nonan,
3,5-Dimethyl-2-thia-5,8-diazabicyclo[4.3.0]nonan,
3-Oxa-2,8-diazabiyclo[4.3.0]nonan,
2-Methyl-9-oxa-2,8-diazabicyclo[4.3.0]nonan,
4-Methyl-3-oxa-2,8-diazabicyclo[4.3.0]nonan,
2,5-Dimethyl-3-oxa-2,8-diazabicyclo[4.3.0]nonan,
3-Oxa-5,8-diazabicyclo[4.3.0]nonan,
5-Methyl-3-oxa-5,8-diazabicyclo[4.3.0]nonan,
1,5-Dimethyl-3-oxa-5,8-diazabicyclo[4.3.0]nonan,
Die Amine können als Diastereomerengemische, in diastereomerenreiner Form, als Racemat oder als teilweise oder vollständig angereicherte Enantiomere verwendet werden.

Die Umsetzung von (III) mit (IV) gemäß Methode A, bei der die Verbindungen (IV) auch in Form ihrer Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden, Hierzu gehören vorzugsweise die Alkalihydroxide, Alkalicarbonate, organische Amine und Amidine.

Als besonders geeignet seien im einzelnen genannt: Triethylamin, 1,4-Diazabicyclo[2.2,2]octan (DABCO), 1,8-Diazabicyclo[5,4,0]undec-7-en (DBU) oder überschüssiges Amin (IV).

Die Reaktionstemperatur wird zwischen 50°C und 250°C gehalten, vorzugsweise zwischen 100°C und 180°C,

Die Reaktion kann bei Normaldruck, aber auch bei erhöhtem Druck durchgeführt werden, Im allgemeinen arbeitet man bei Drucken zwischen 1 und 100 bar, vorzugsweise bei 1 bis 10 bar.

Bei der Durchführung des erfindungsgemäßen Verfahrens setzt man auf 1 mol einer Verbindung der allgemeinen Formel (III) 2 bis 10 Mol, vorzugsweise 3 bis 6 Mol einer Verbindung der allgemeinen Formel (IV) ein.

Ferner erhält man die erfindungsgemäßen Verbindungen, indem man Verbindungen der allgemeinen Formel (V) 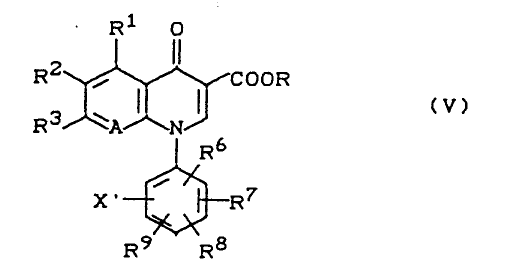
in der A, R, R¹ bis R⁹ die oben angegebene Bedeutung haben und X' für ein Halogenatom steht, mit Aminen der allgemeinen Formeln (II) und (IV), gegebenenfalls in Gegenwart von Verdünnungsmitteln und gegebenenfalls in Gegenwart von Säurebindern umsetzt (Methode B).

Verwendet man beispielsweise 1-(2,4-Difluorphenyl)-6-fluor-7-(3-methylpiperazin-1-yl)-1,4-dihydro-4-oxo-3-chinolincarbonsäure und 1-Methylpiperazin als Ausgangsstoffe, so kann der Reaktionsablauf durch das folgende Formelschema wiedergegeben werden:

### Reaktionsschema II:

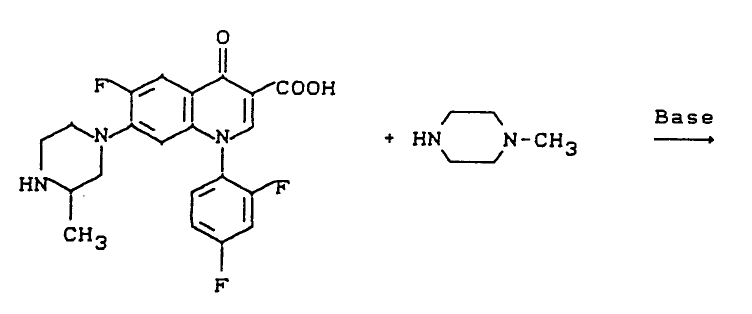

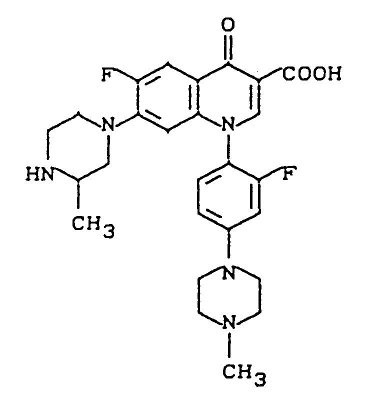

Die Verbindungen der allgemeinen Formel (V) sind bekannt oder nach bekannten Methoden herstellbar (Europäische Patentanmeldungen 131 839, 154 780, 302 372).

### Als Beispiele seien genannt:

Verbindungen der allgemeinen Formel (V) mit folgenden Resten (R⁷, R⁸, R⁹ sind Wasserstoff)

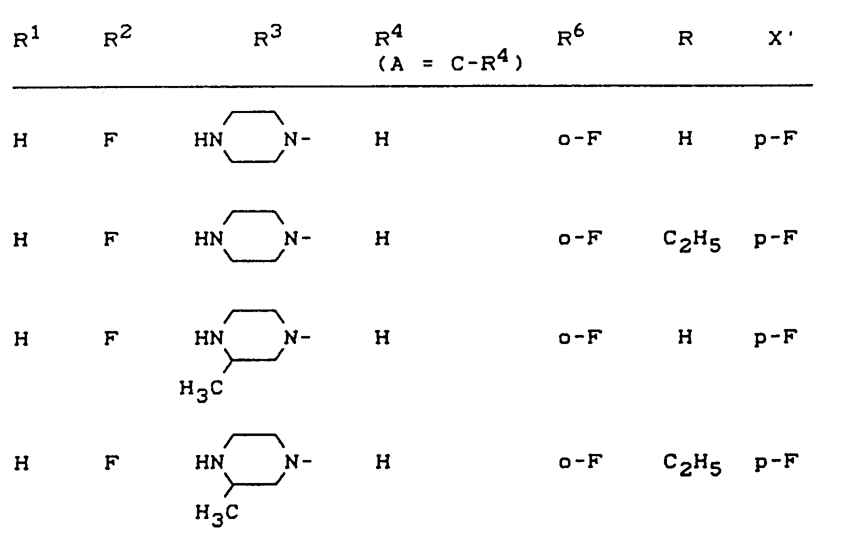

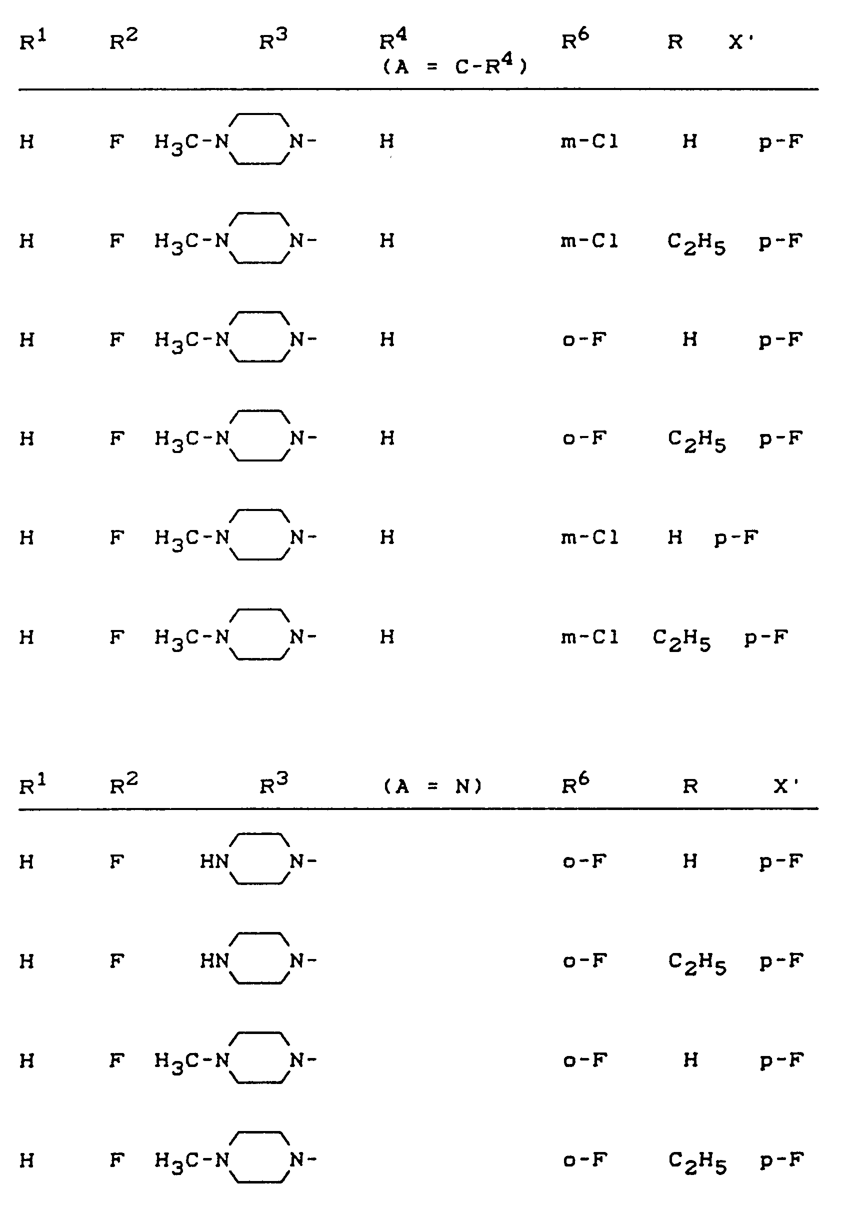

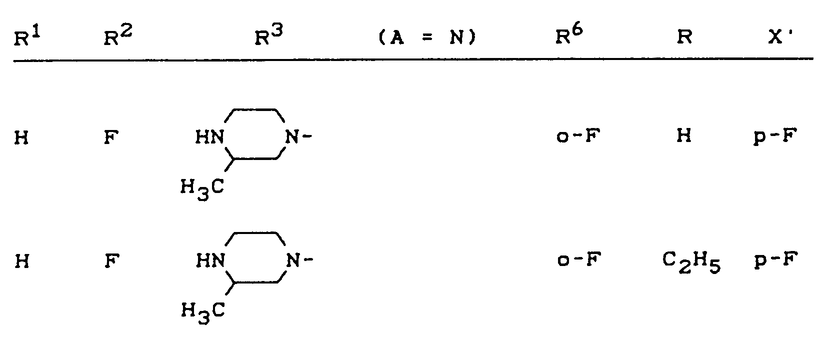

Die erfindungsgemäßen Verbindungen werden außerdem erhalten, wenn man Chinolone der allgemeinen Formel (VI) 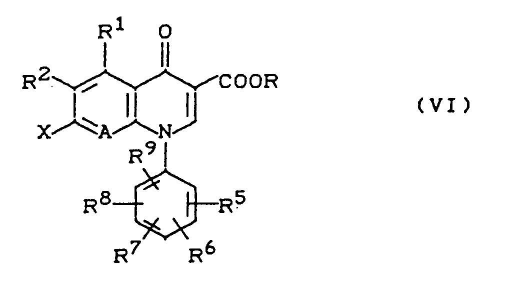
in der A, R, R¹ bis R⁹ und X die oben angegebene Bedeutung haben, mit Aminen der allgemeinen Formeln (II) und (IV), gegebenenfalls in Gegenwart eines Verdünnungsmittels und gegebenenfalls eines Säurefängers umsetzt (Methode C).

Verwendet man beispielsweise 1-[3-Chlor-4-(4-methylpiperazinyl)-phenyl]-7-chlor-6-fluor-1,4-dihydro-4-oxo-3-chinolincarbonsäure und 2-Methylpiperazin als Ausgangsstoffe, so kann der Reaktionsablauf durch das folgende Formelschema wiedergegeben werden.

### Reaktionsschema III:

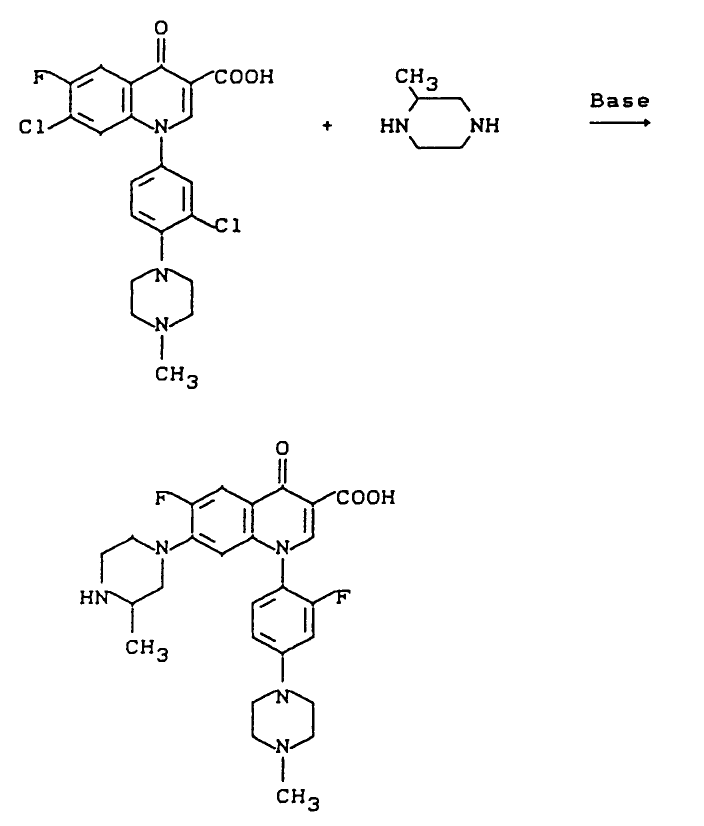

Verbindungen des Typs (VI) sind bekannt oder nach bekannten Methoden herstellbar. Als Beispiele seien genannt:

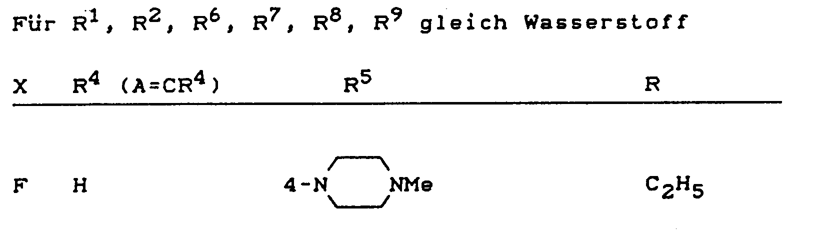

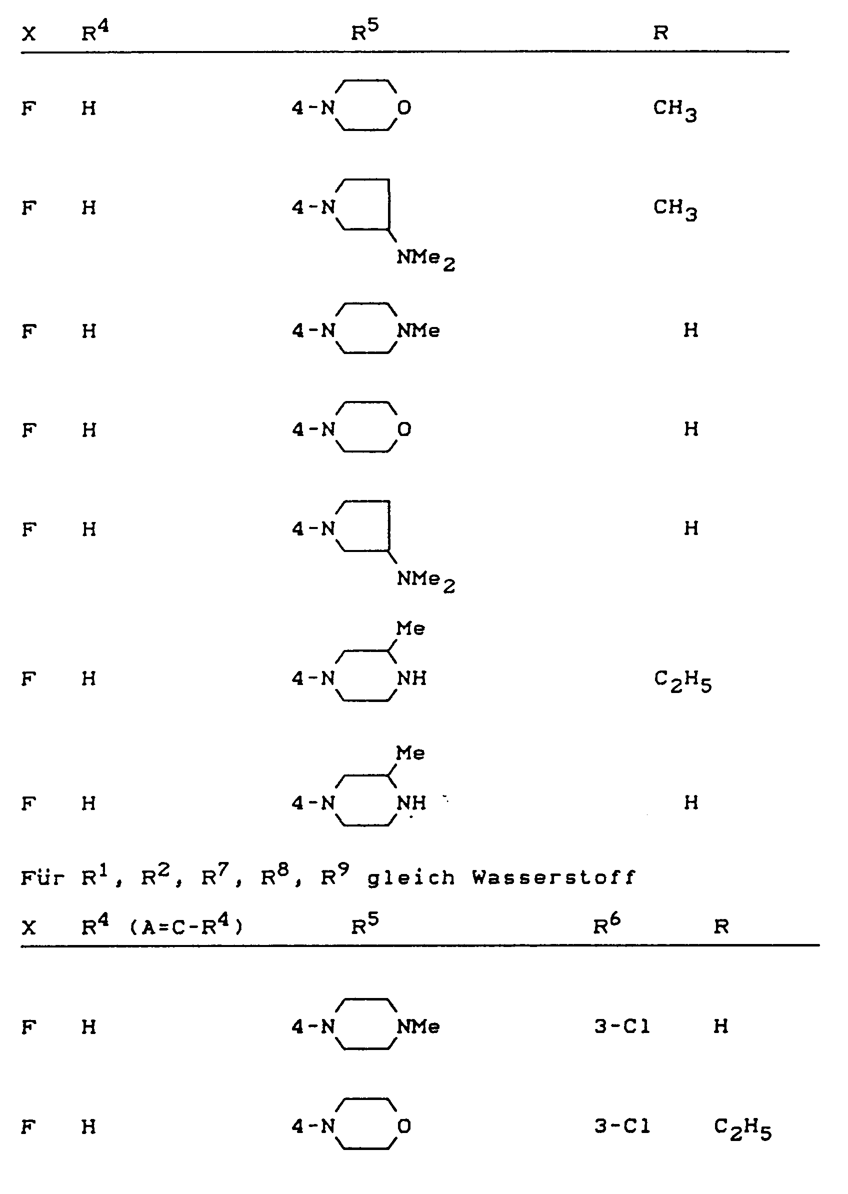

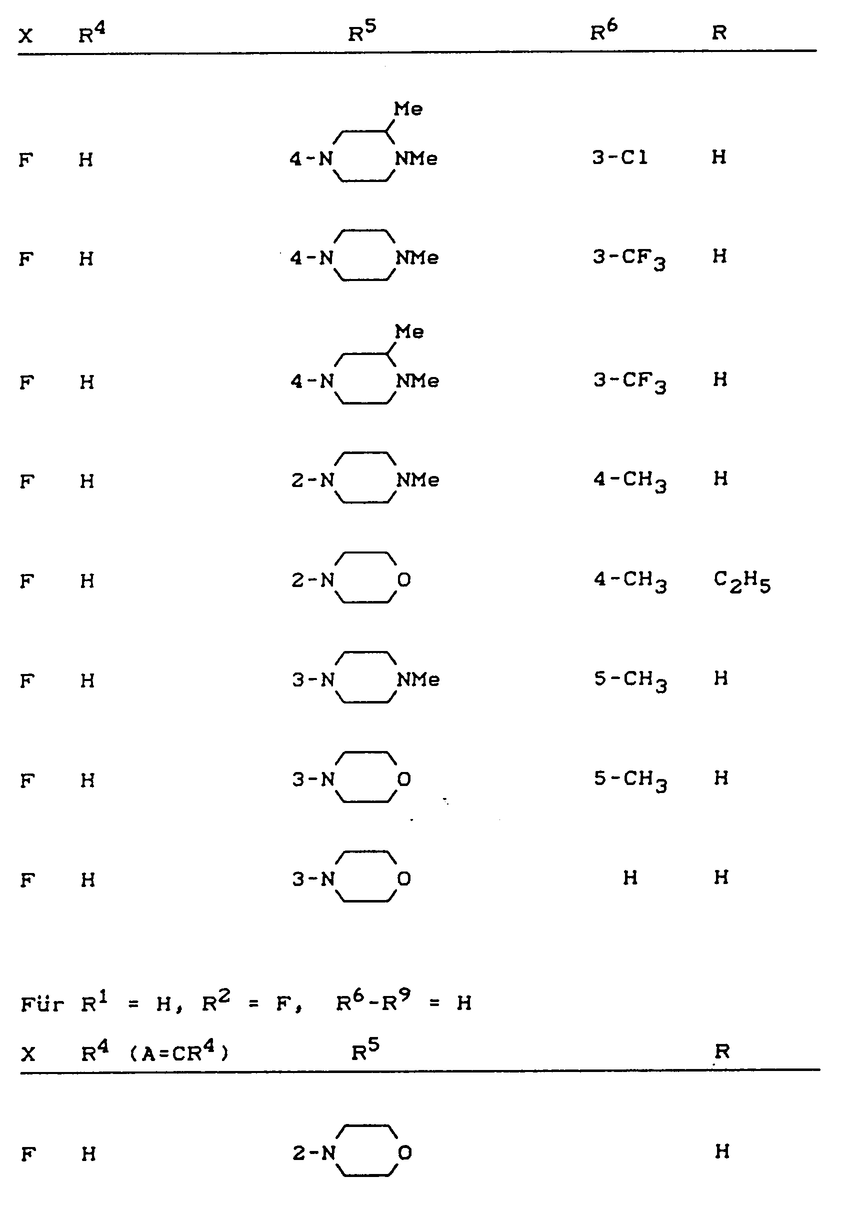

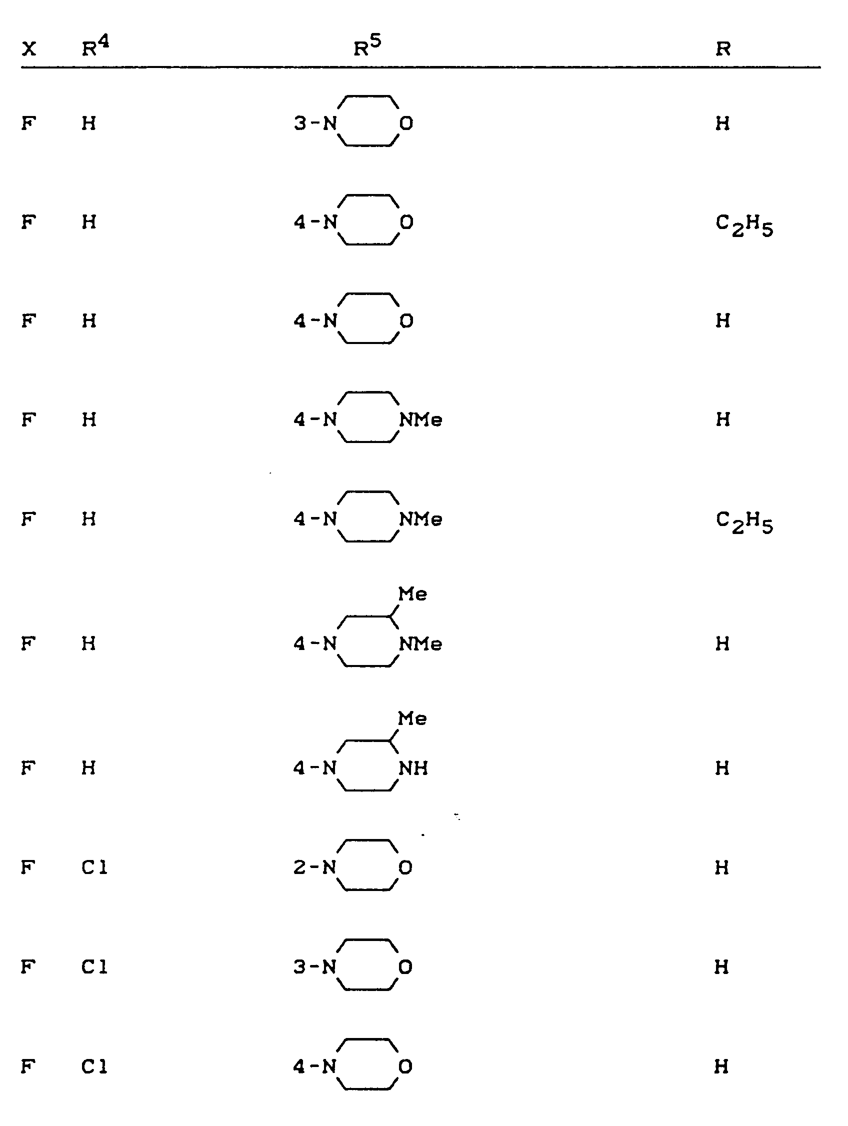

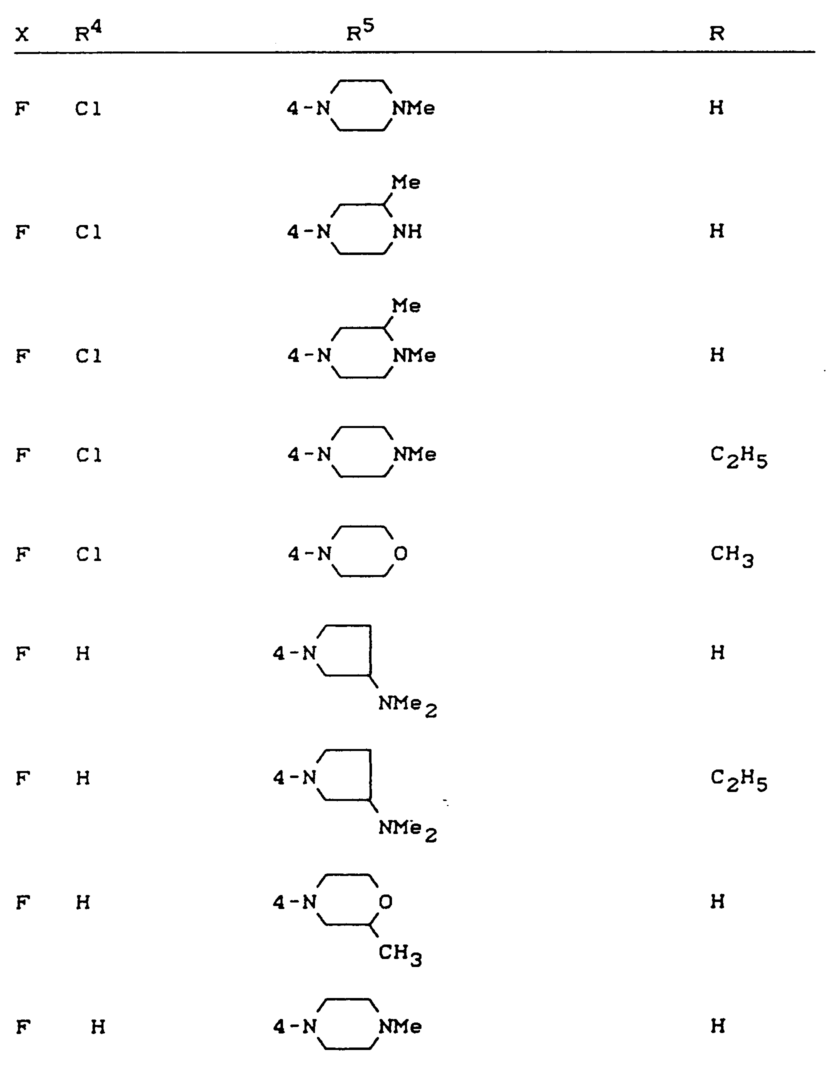

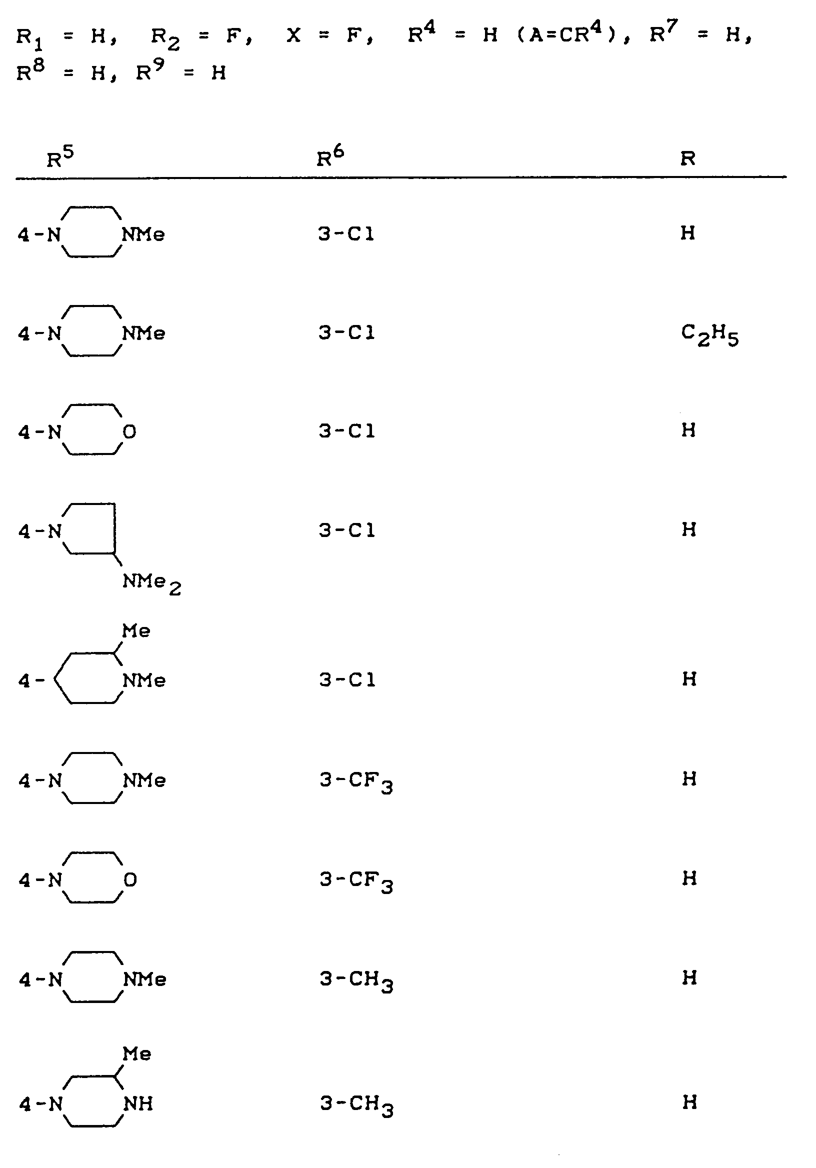

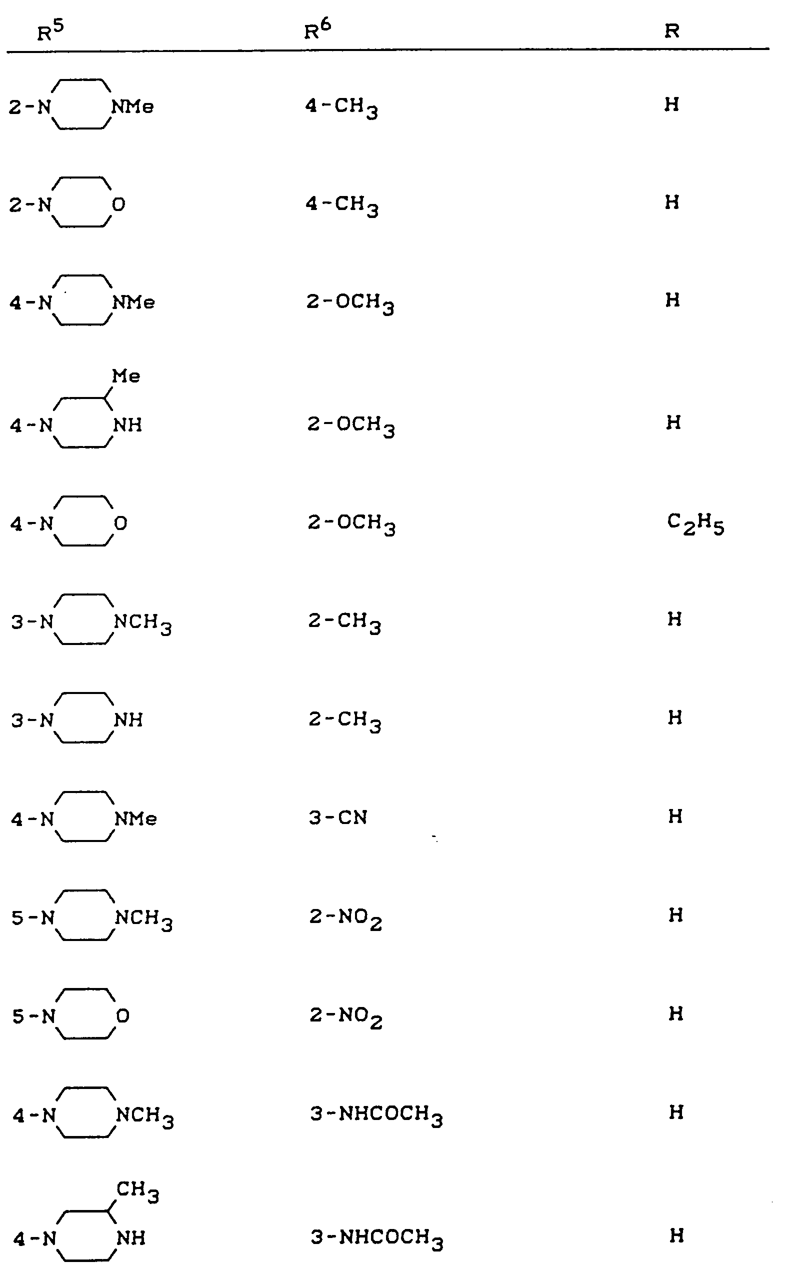

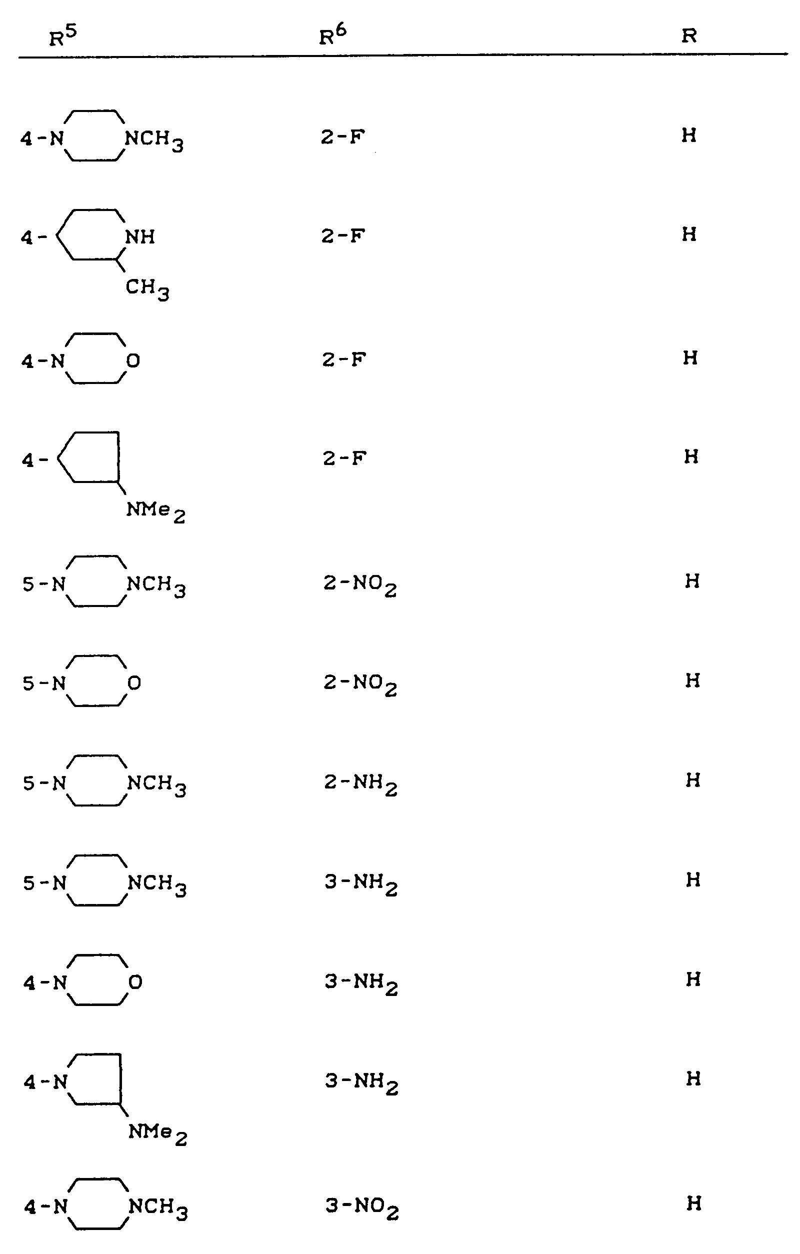

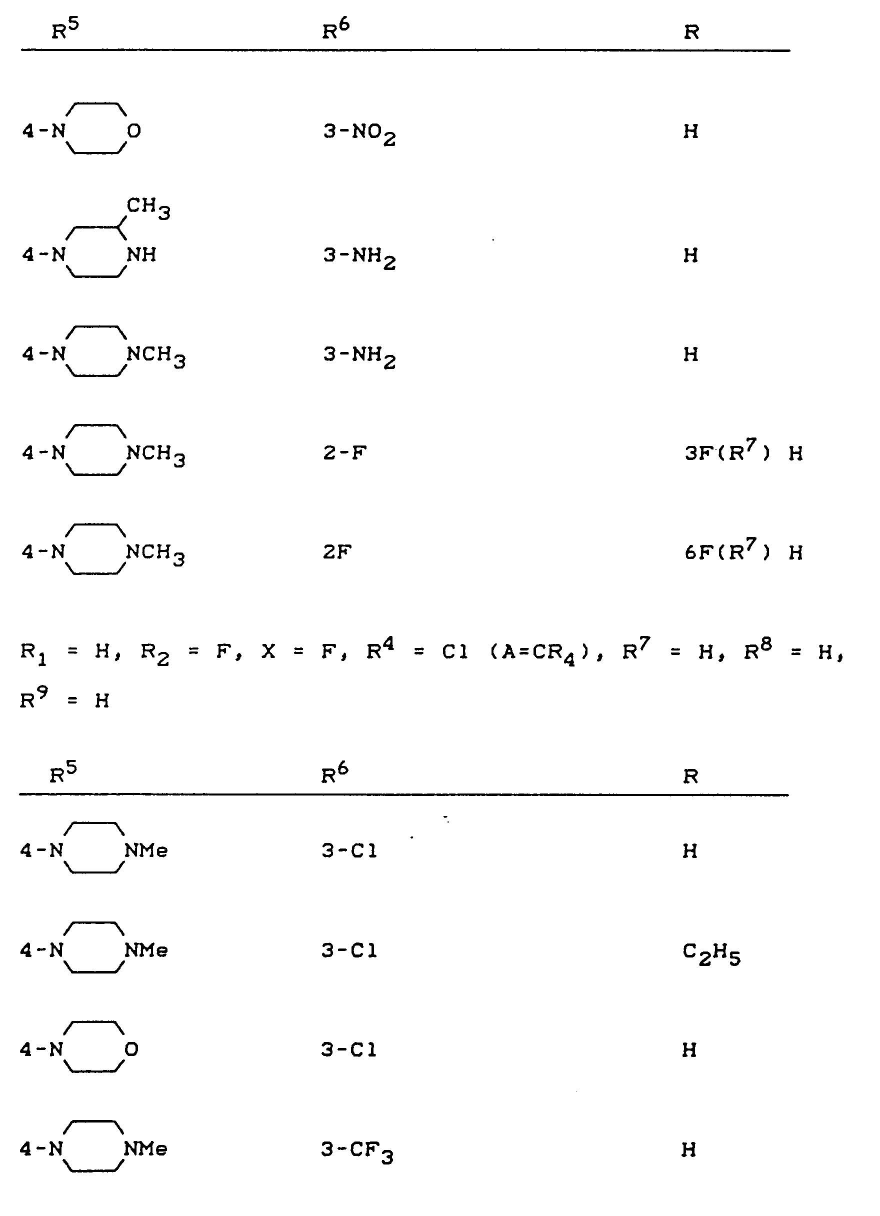

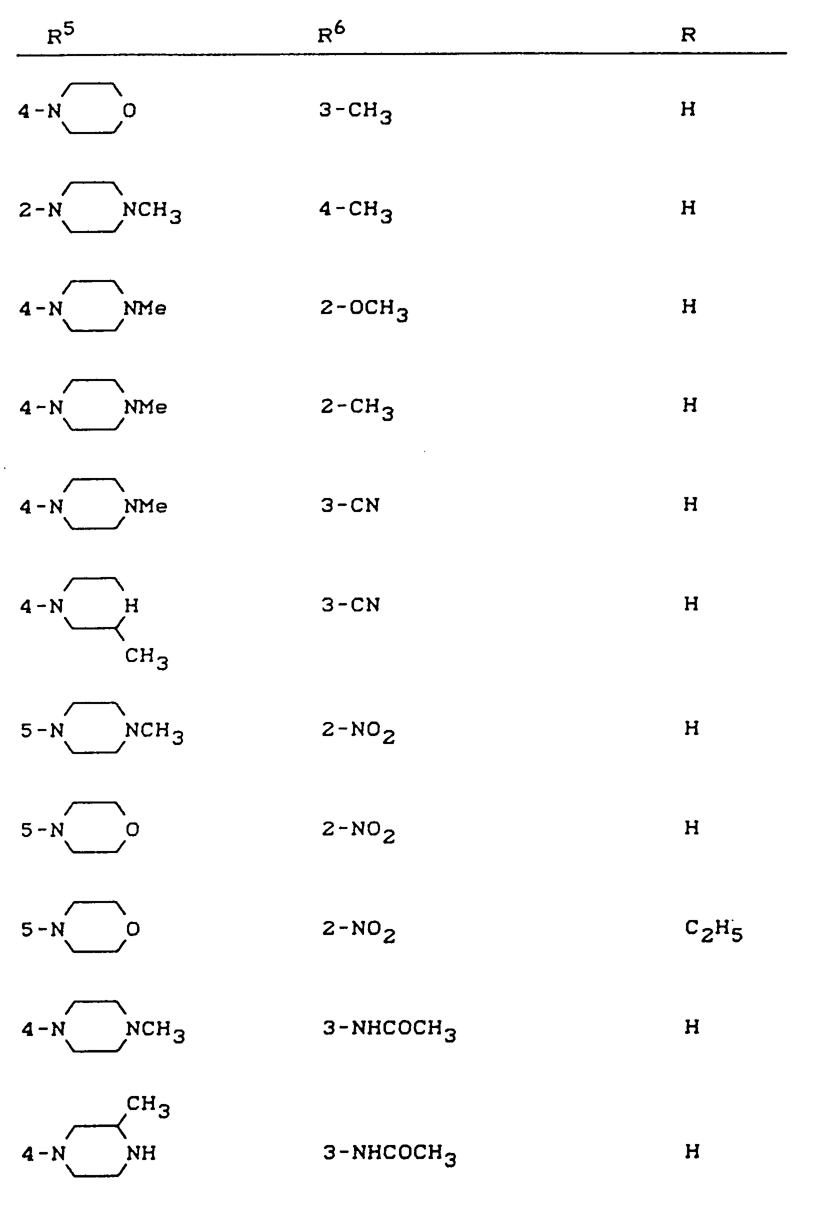

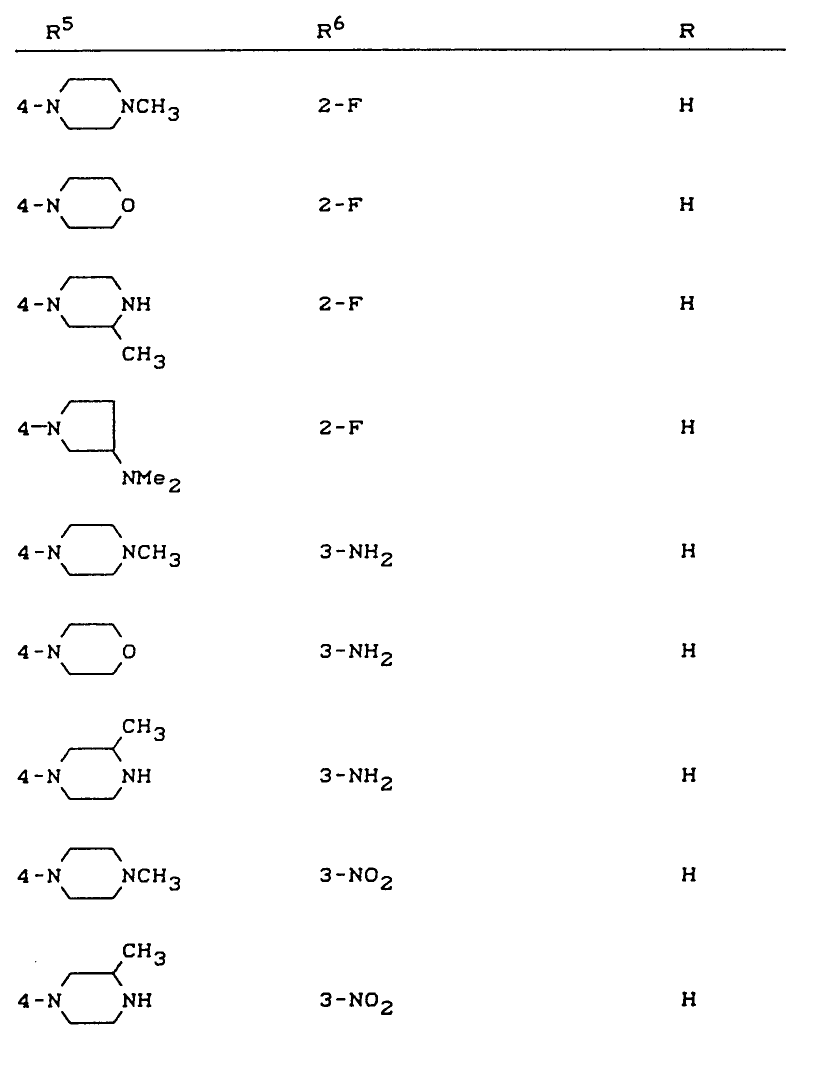

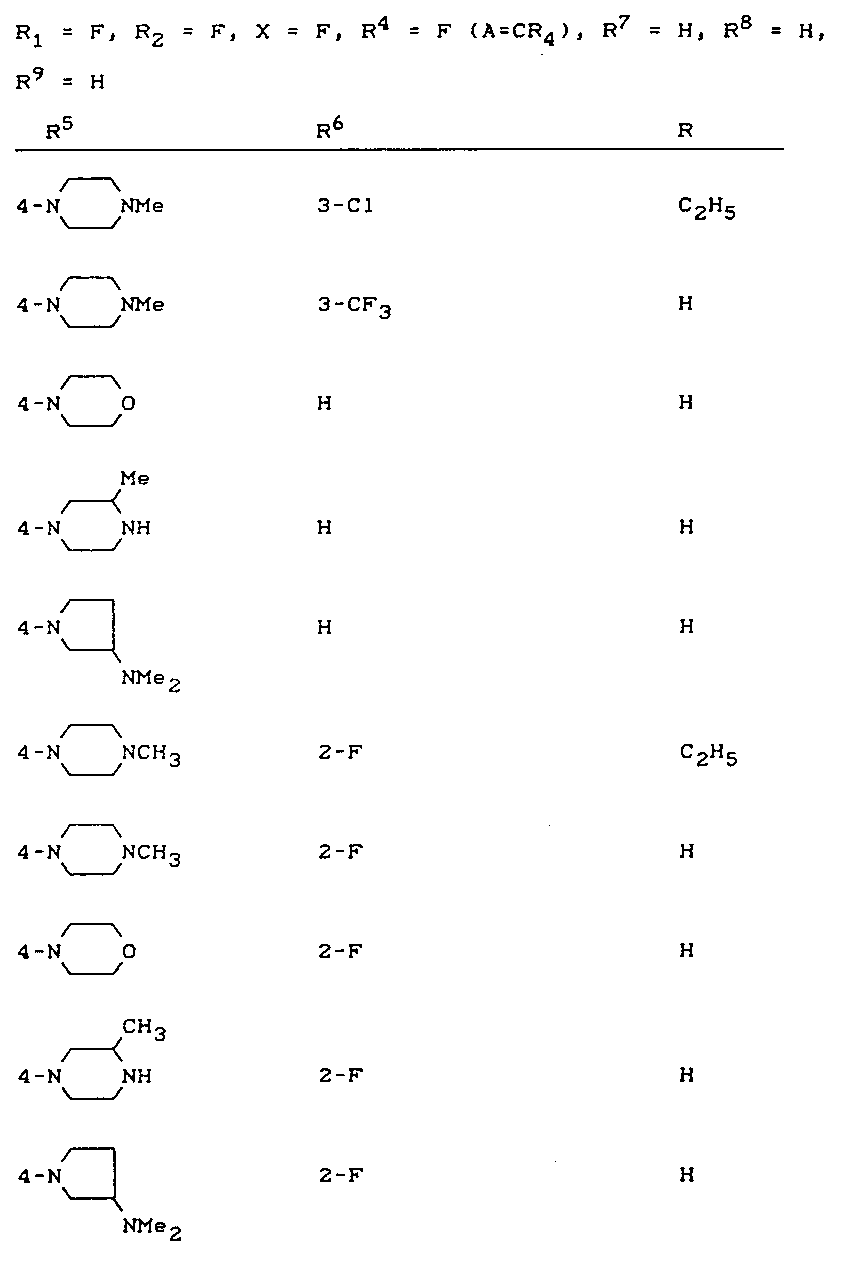

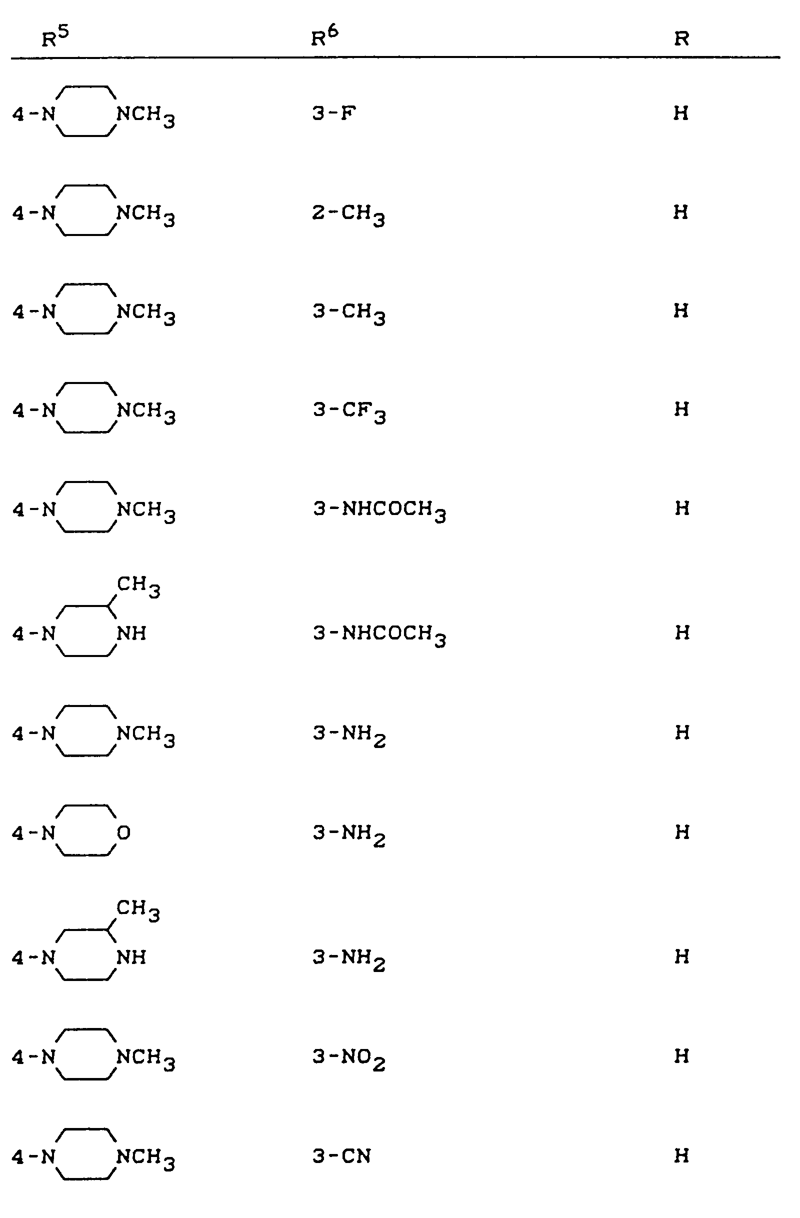

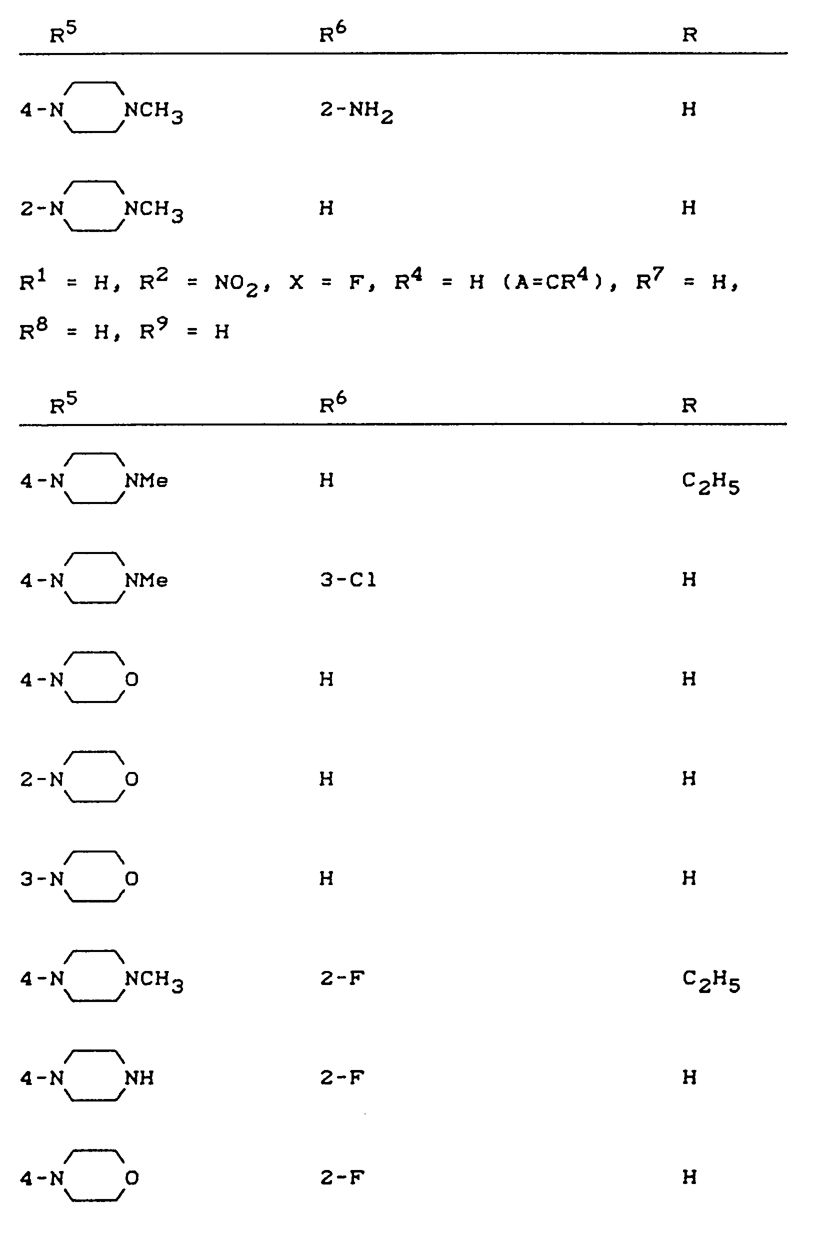

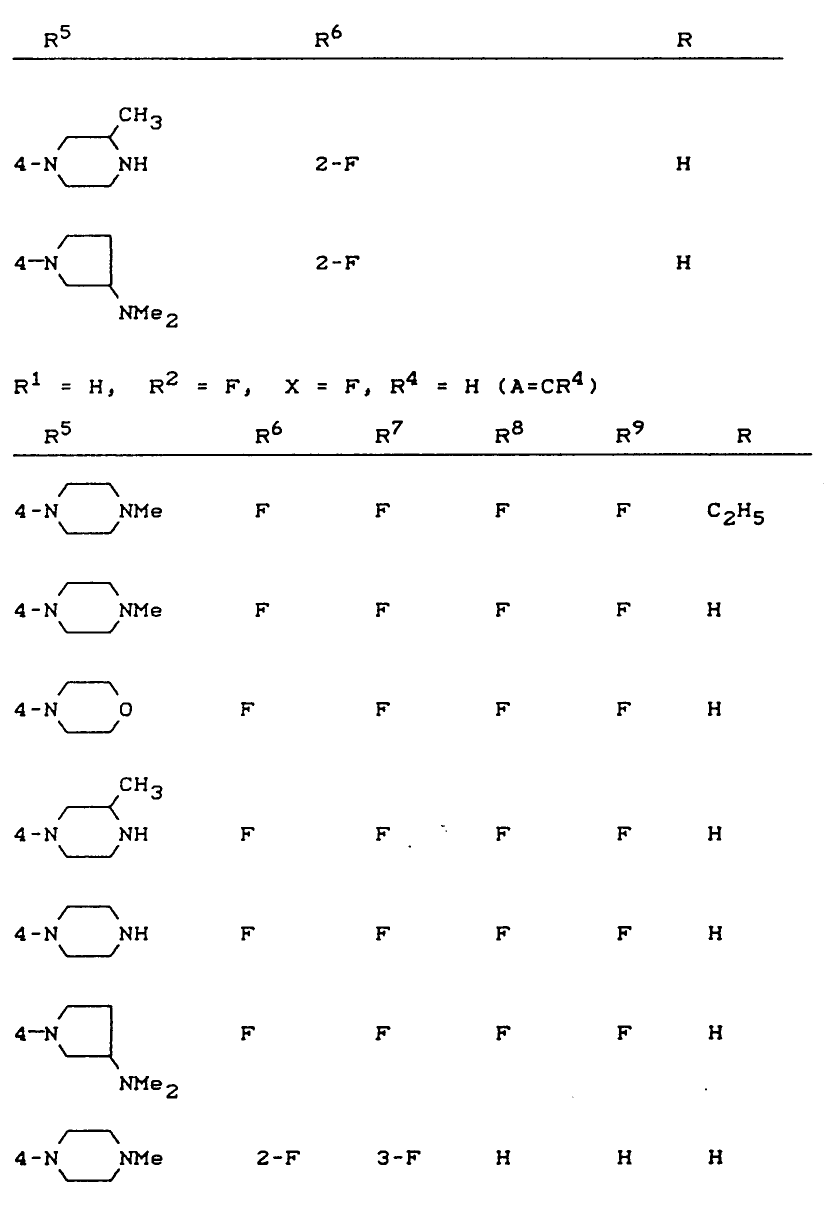

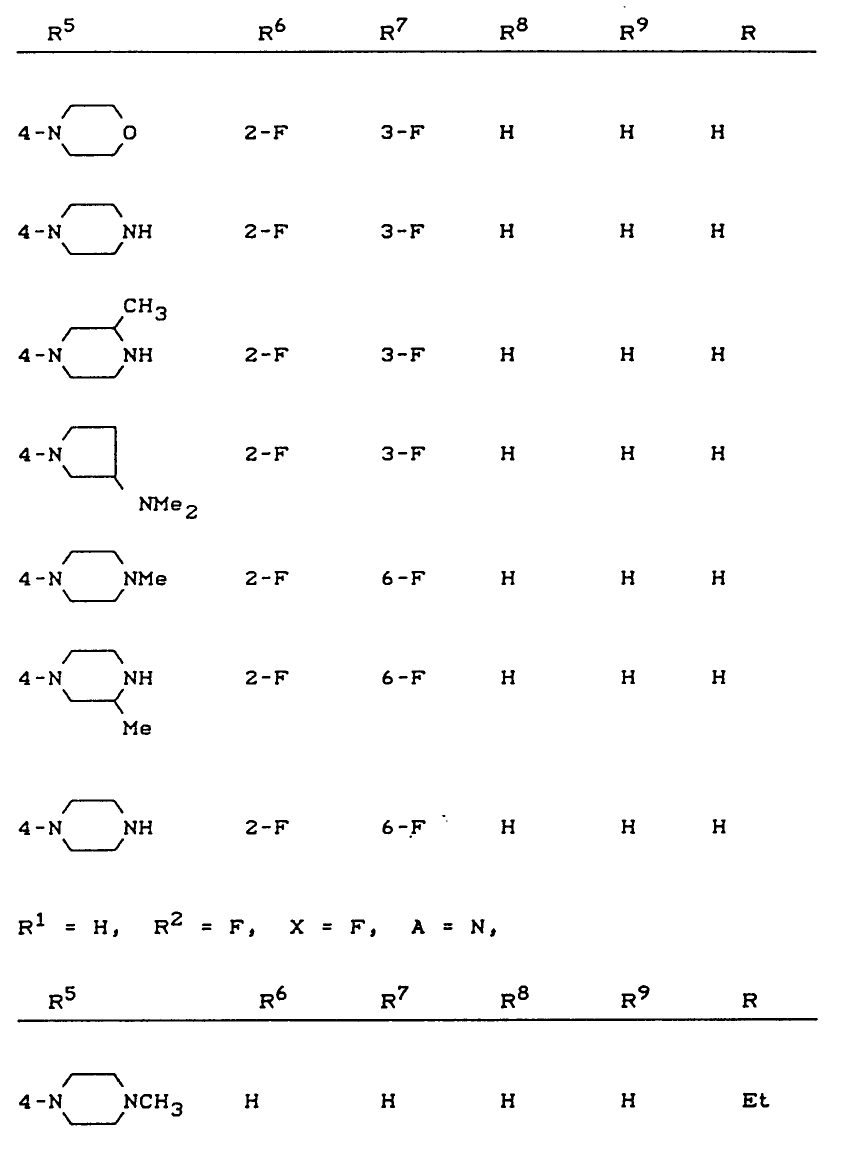

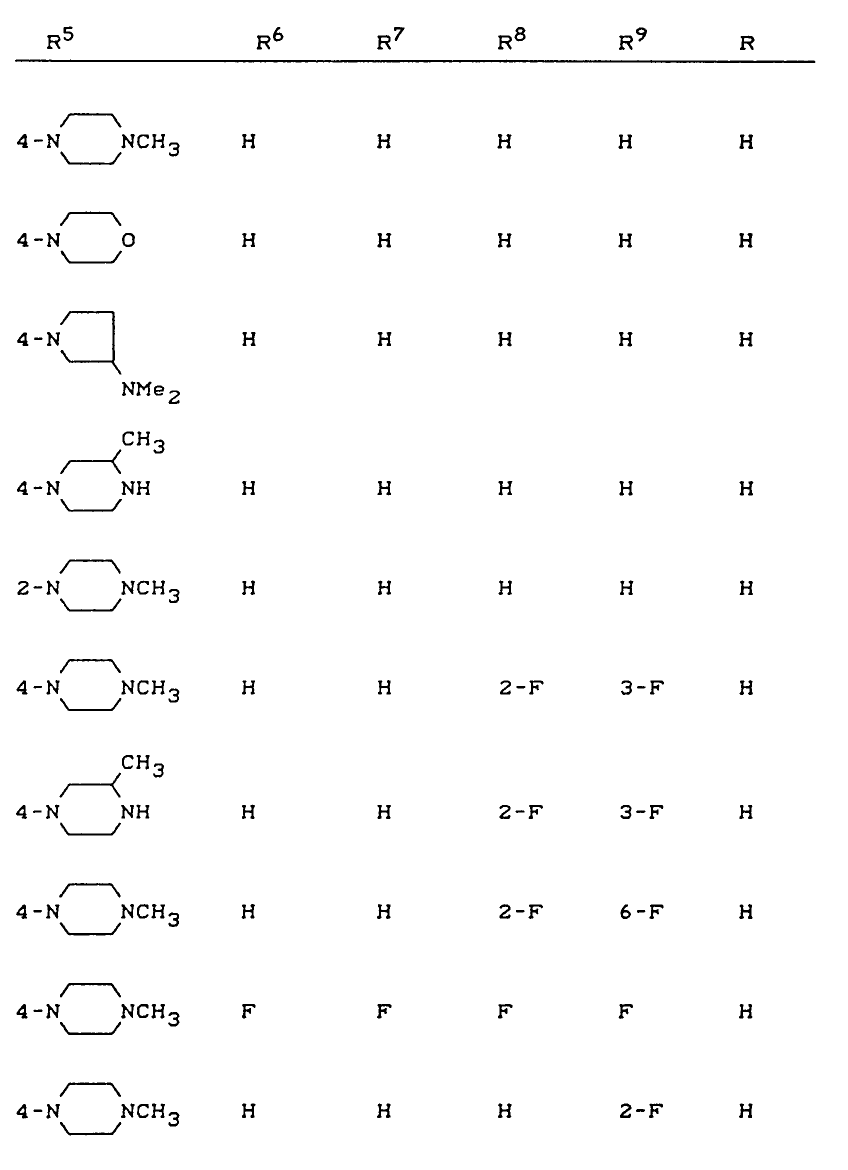

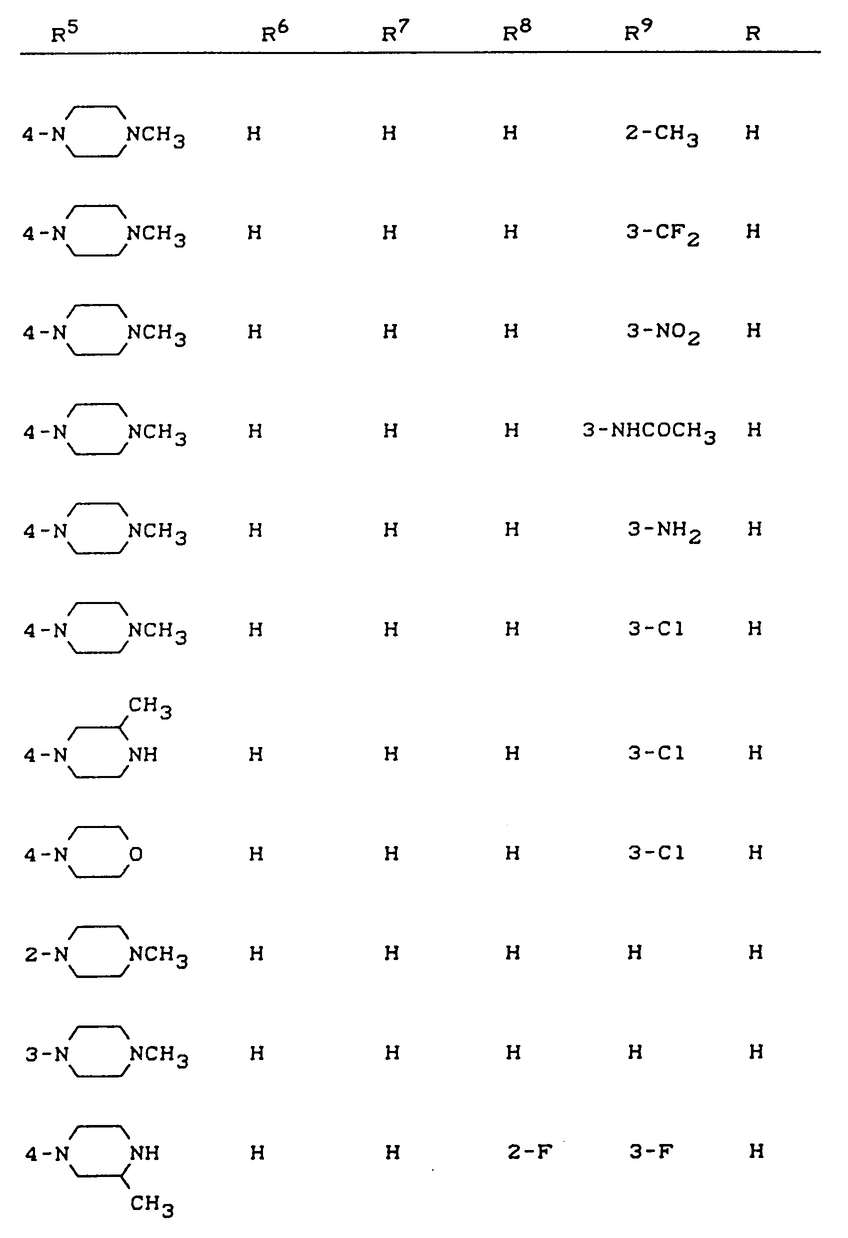

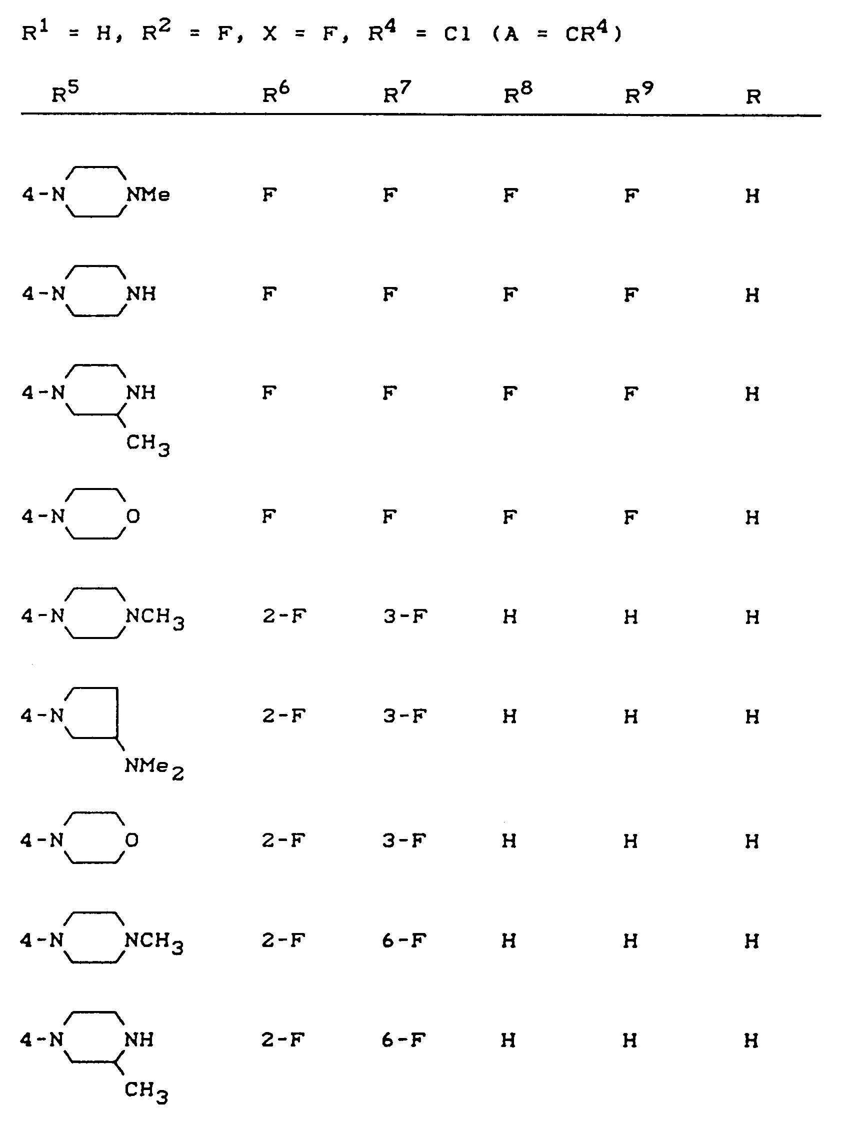

Einige der erfindungsgemäßen Verbindungen erhält man, indem man von Verbindungen der allgemeinen Formel 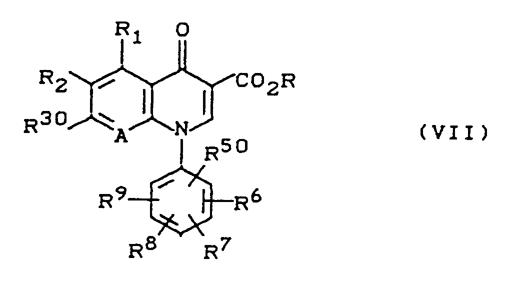
wobei R, R¹, R², A, R⁶ - R⁹, die oben angegebene Bedeutung haben und die Reste R³⁰ und R⁵⁰ gleich oder verschieden sind, ausgeht.

Die allgemeine Formel (VII) zeichnet sich dadurch aus, daß in R³⁰ und in R⁵⁰, nur in R³⁰ oder nur in R⁵⁰ sekundäre oder primäre Amine vorhanden sind. Die Strukturen von R³⁰ und R⁵⁰ sind durch die Vertreter der oben definierten Reste R³ bzw. R⁵ bestimmt, auf die dieses Kriterium zutrifft.

Diese werden mit Acylierungsmitteln oder Alkylierungsmitteln der allgemeinen Formel (VIII)

V - W (VIII)

in der
- W =: - CN
- (C₂-C₄)-Acyl, gegebenenfalls substituiert
- (C₁-C₄)-Sulfonyl
- (C₁-C₄)-Alkoxycarbonyl
- (C₁-C₅)-Alkyl
und V eine Abgangsgruppe darstellt, wie
- V =: - Halogen
- Trifluormethansulfonat
- Mesylat
- Tosylat
- Acetat
- Trihaloacetat
gegebenenfalls in Gegenwart von Verdünnungsmitteln und gegebenenfalls in Gegenwart von Säurebindern umgesetzt (Methode D).

Verwendet man beispielsweise 1-(2-Fluor-4-piperazinylphenyl)-6-fluor-7-piperazinyl-1,4-dihydro-4-oxo-3-chinolincarbonsäure und Methansulfonylchlorid als Ausgangsstoffe, so kann der Reaktionsablauf durch das folgende Formelschema wiedergegeben werden.

### Reaktionsschema IV:

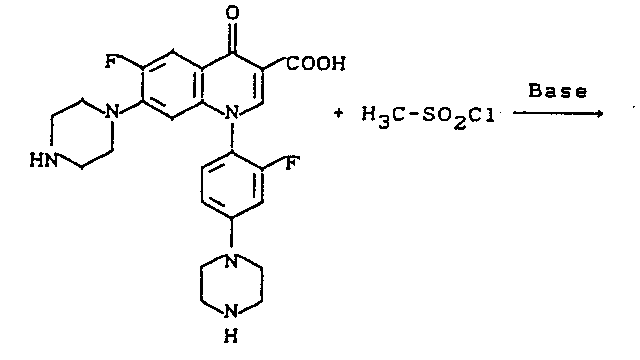

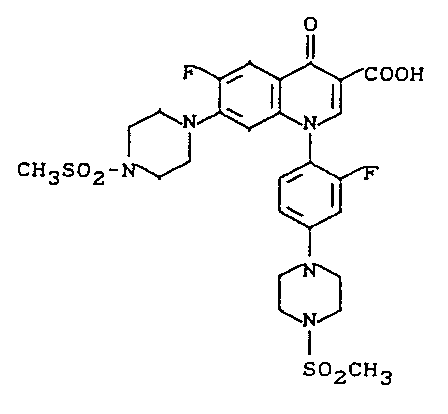

Verbindungen des Typs (VII) sind nach den Methoden A, B und C herstellbar,

Verbindungen des Typs (VIII) sind bekannt (Beilsteins Handbuch der Organischen Chemie, Bände 2 und 3). Als Beispiele seien genannt: Bromcyan, Acetylchlorid, Propionylchlorid, Buttersäurechlorid, Methansulfonylchlorid. Anstelle von organischen Säurechloriden sind auch die entsprechenden Anhydride (z.B. Acetanhydrid) verwendbar.

Als Verdünnungsmittel bei der Umsetzung von (VII) mit (VIII) werden vorzugsweise Dimethylsulfoxid, N,N-Dimethylformamid, Acetonitril, Pyridin oder Gemische dieser Verdünnungsmittel verwendet, Als Säurebinder können alle üblichen anorganischen und organischen Säurebindungsmittel verwendet werden. Hierzu gehören vorzugsweise die Alkalihydroxide, Alkalicarbonate, organische Amine und Amidine, Als besonders geeignet seien im einzelnen genannt: Triethylamin, 1,4-Diazabicyclo[2.2.2]octan (DABCO), 1,8-Diazabicyclo[5,4,0]undec-7-en (DBU).

Die Umsetzung wird im Temperaturbereich zwischen -100°C und 200°C, vorzugsweise zwischen -20°C und 100°C durchgeführt.

Die Reaktion kann bei Normaldruck, aber auch bei erhöhtem Druck durchgeführt werden. Im allgemeinen arbeitet man bei Drucken zwischen 1 und 100 bar, vorzugsweise bei 1 bis 10 bar.

Bei der Durchführung des erfindungsgemäßen Verfahrens setzt man auf 1 Mol einer Verbindung der allgemeinen Formel (VII) 1 bis 10 Mol, vorzugsweise 2 bis 6 Mol einer Verbindung der allgemeinen Formel (VIII) ein.

Weitere erfindungsgemäße Verbindungen erhält man, wenn man Verbindungen der allgemeinen Formel (VII) mit Verbindungen der allgemeinen Formel (IX),

CH₂=CH-U (IX)

in der U eine elektronenanziehende Gruppe wie COOB, COB, CONH₂, CN, SO₂B, SO₃B bedeutet und B für C₁-C₄-Alkyl, das gegebenenfalls substituiert ist, steht,
gegebenenfalls in Gegenwart von Verdünnungsmitteln umsetzt (Methode E)

Verwendet man beispielsweise 1-(2-Fluor-4-piperazinylphenyl)-6-fluor-7-piperazinyl-1,4-dihydro-4-oxo-3-chinolincarbonsäure und Acrylsäureethylester als Ausgangsstoffe, so kann der Reaktionsablauf durch das folgende Formelschema (V) wiedergegeben werden:

### Reaktionsschema V:

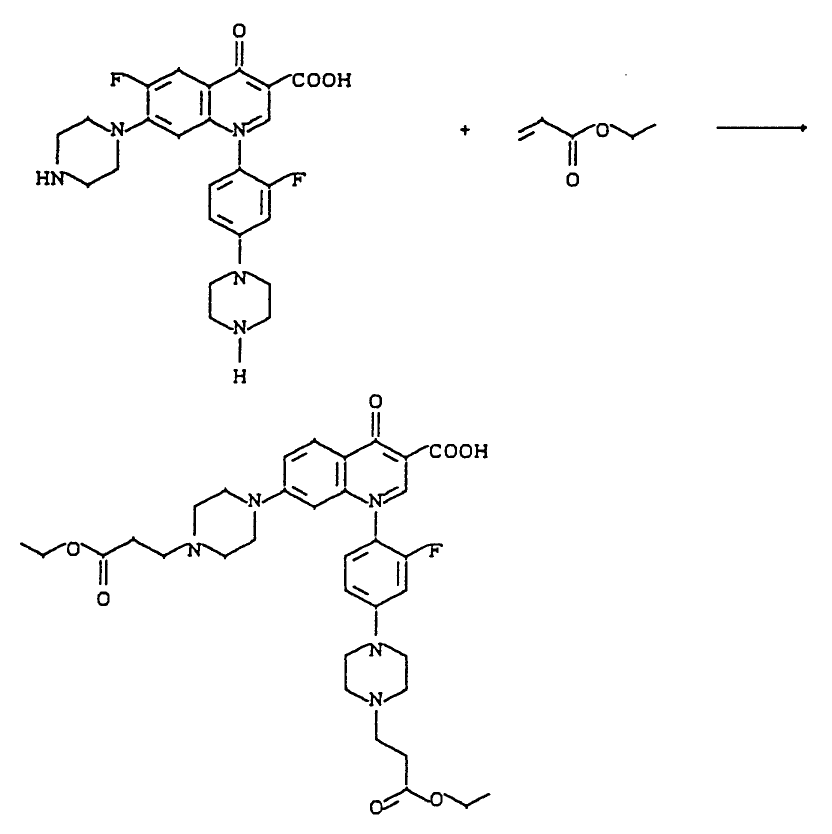

Die Umsetzung von (VII) mit (IX) gemäß Methode E wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Die Reaktionstemperatur liegt im Bereich zwischen -100°C und 200°C, vorzugsweise zwischen -20°C und 80°C.

Die Reaktion kann bei Normaldruck, aber auch bei erhöhtem Druck durchgeführt werden. Im allgemeinen arbeitet man bei Drucken zwischen 1 und 100 bar, vorzugsweise bei 1 bis 10 bar.

Bei der Durchfürung des erfindungsgemäßen Verfahrens setzt man auf ein Mol einer Verbindung der allgemeinen Formel (VII) 1 bis 10 Mol, vorzugsweise 2 bis 6 Mol einer Verbindung der allgemeinen Formel (IX) ein.

Erfindungsgemäße Verbindungen erhält man auch, wenn man Verbindungen der allgemeinen Formel (VII) mit Carbonsäuren der allgemeinen Formel (X),

D-COOH (X)

in der D für H oder ggf. substituiertes (C₁-C₄)-Alkyl steht,
gegebenenfalls in Gegenwart von Verdünnungsmitteln und gegebenenfalls in Gegenwart wasserentziehender Reagenzien und gegebenenfalls in Gegenwart eines Katalysators umsetzt (Methode F).

Verwendet man beispielsweise 1-(2-Fluor-4-piperazinylphenyl)-6-fluor-7-piperazinyl-1,4-dihydro-4-oxo-3-chinolincarbonsäure und Ameisensäure als Ausgangsstoffe, so kann der Reaktionsablauf durch das folgende Formelschema (VI) wiedergegeben werden.

### Reaktionsschema VI:

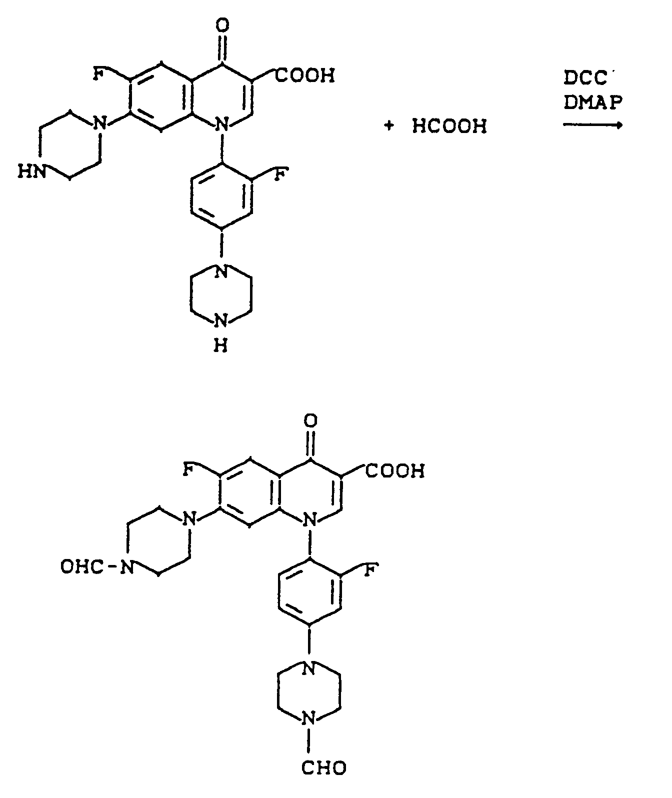

Die Reaktion kann bei Normaldruck, aber auch bei erhöhtem Druck durchgeführt werden. Im allgemeinen arbeitet man bei Drucken zwischen 1 und 100 bar, vorzugsweise bei 1 bis 10 bar.

Bei der Durchführung des erfindungsgemäßen Verfahrens setzt man auf 1 Mol einer Verbindung der allgemeinen Formel (VII) 1 bis 10 Mol, vorzugsweise 2 bis 6 Mol einer Verbindung der allgemeinen Formel (X) ein.

Verbindungen des Typs (X) sind bekannt (Beilstein, Bände 2 und 3). Als Beispiele seien genannt: Ameisensäure, Essigsäure, Propionsäure, Isobuttersäure.

Die Umsetzung von (VII) nach (X) wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Methylenchlorid, Benzol, Toluol oder Pyridin vorgenommen. Ebenso können Gemische dieser Verdünnungsmittel verwendet werden.

Als wasserentziehende Mittel kommen vorzugsweise Molekularsieb, Lösemittel wie Methylenchlorid, Benzol oder Toluol, die mit Wasser Azeotrope bilden, aber nicht mit Wasser mischbar sind, oder Verbindungen, die mit Wasser, nicht aber mit den Reaktionspartnern reagieren, wie zum Beispiel Dicyclohexylcarbodiimid (DCC) in Frage. Als Katalysator kann zum Beispiel 4-N,N-Dimethylaminopyridin (DMAP) dienen.

Die Reaktionstemperatur liegt im Bereich von -100°C bis 200°C, vorzugsweise im Bereich von 0°C bis 150°C.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Abfiltrieren von ungelöstem Betain und Eindampfen des Filtrats bis zur Trockne erhaltene. Pharmazeutisch geeignete Salze sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können ebenfalls die in Tabelle 1 beispielhaft aufgeführten Verbindungen hergestellt werden, wobei diese Verbindungen sowohl als Diastereomerengemische als auch als diastereomerenreine oder enantiomerenreine Verbindungen vorliegen können.

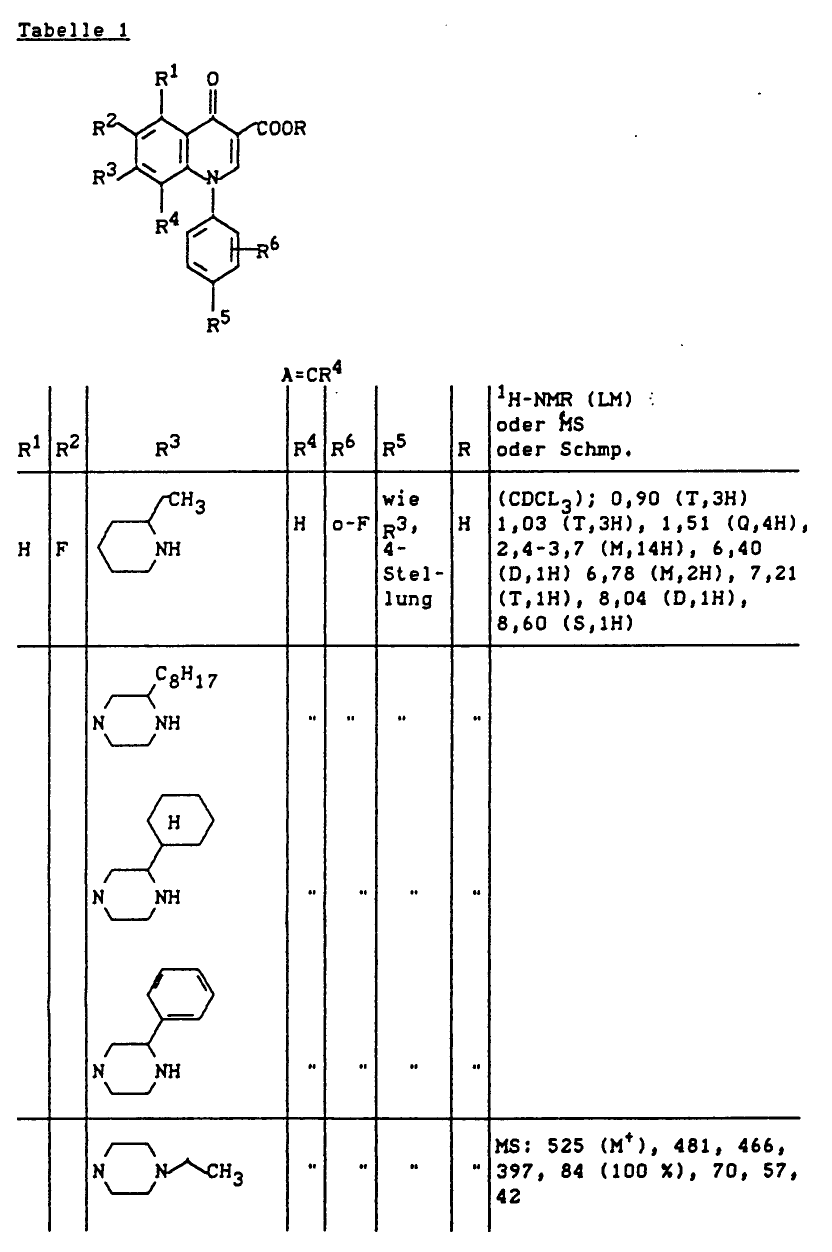

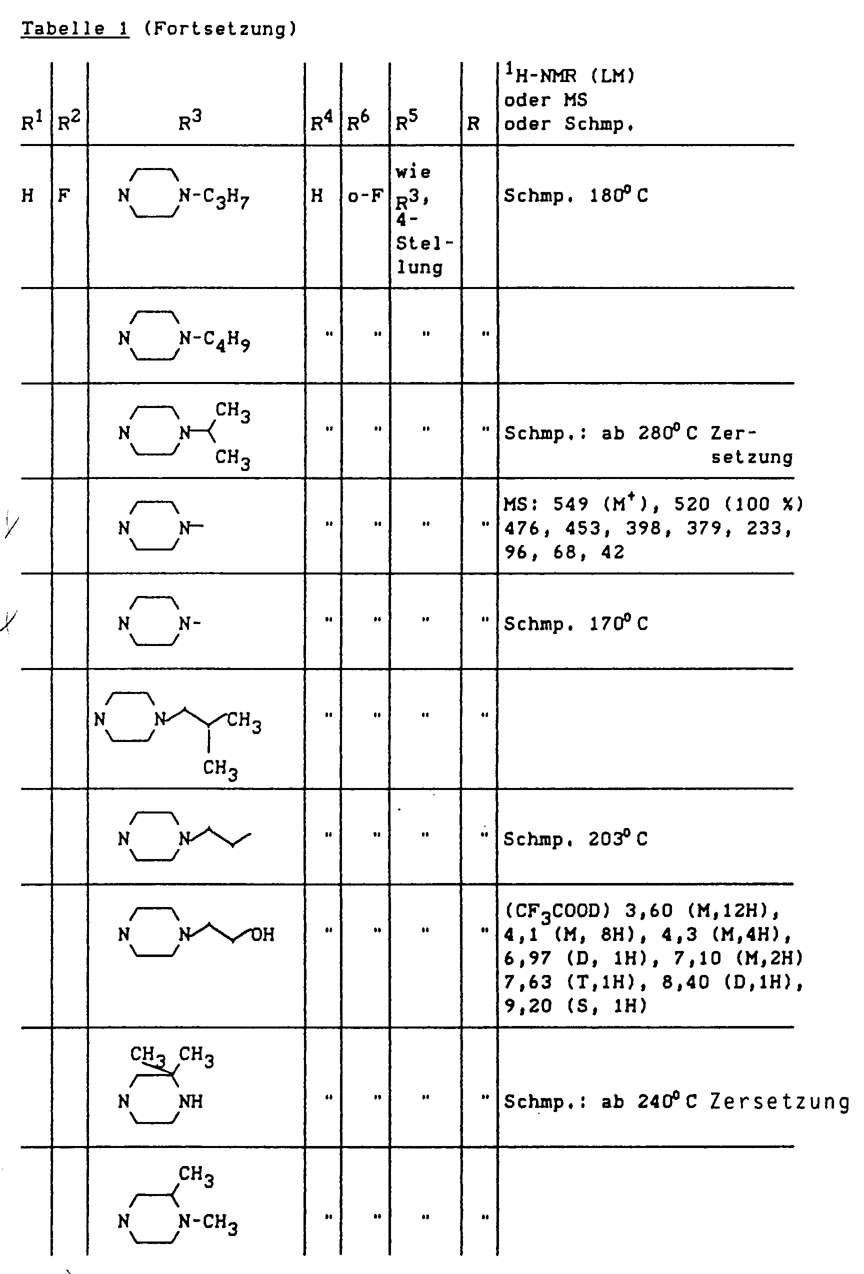

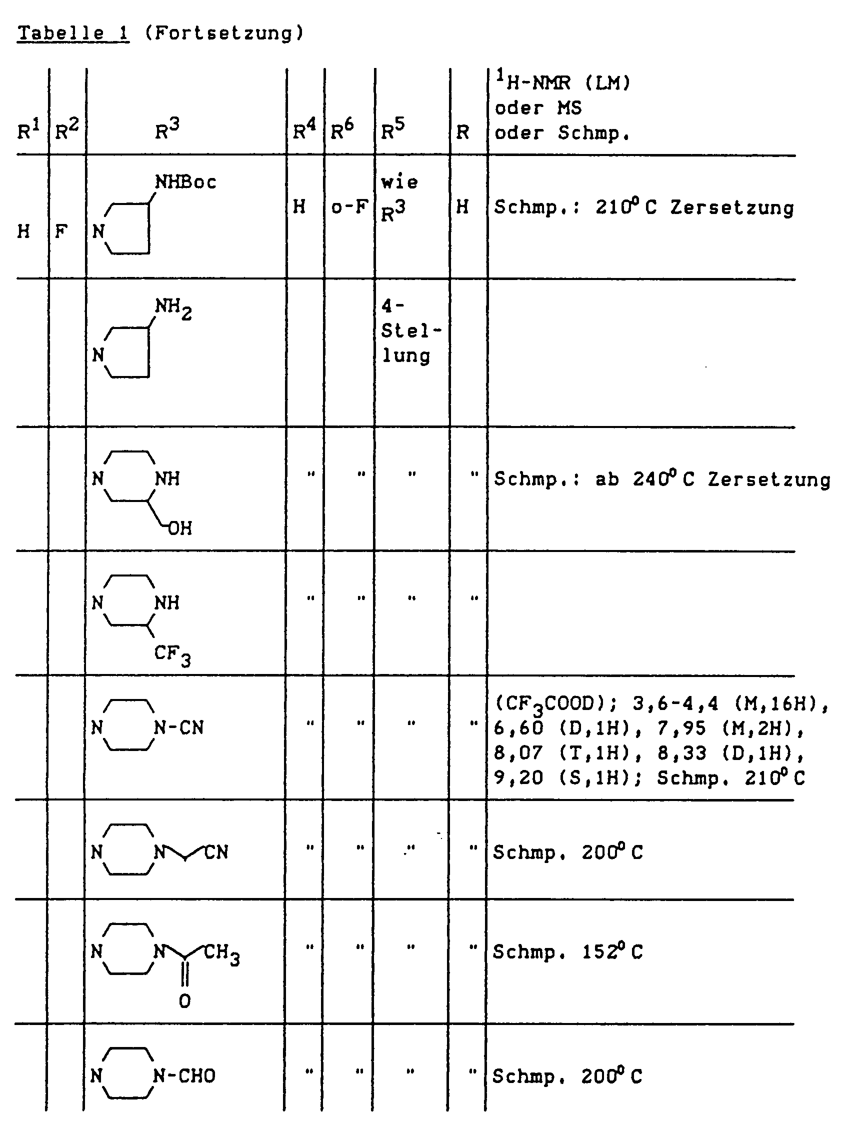

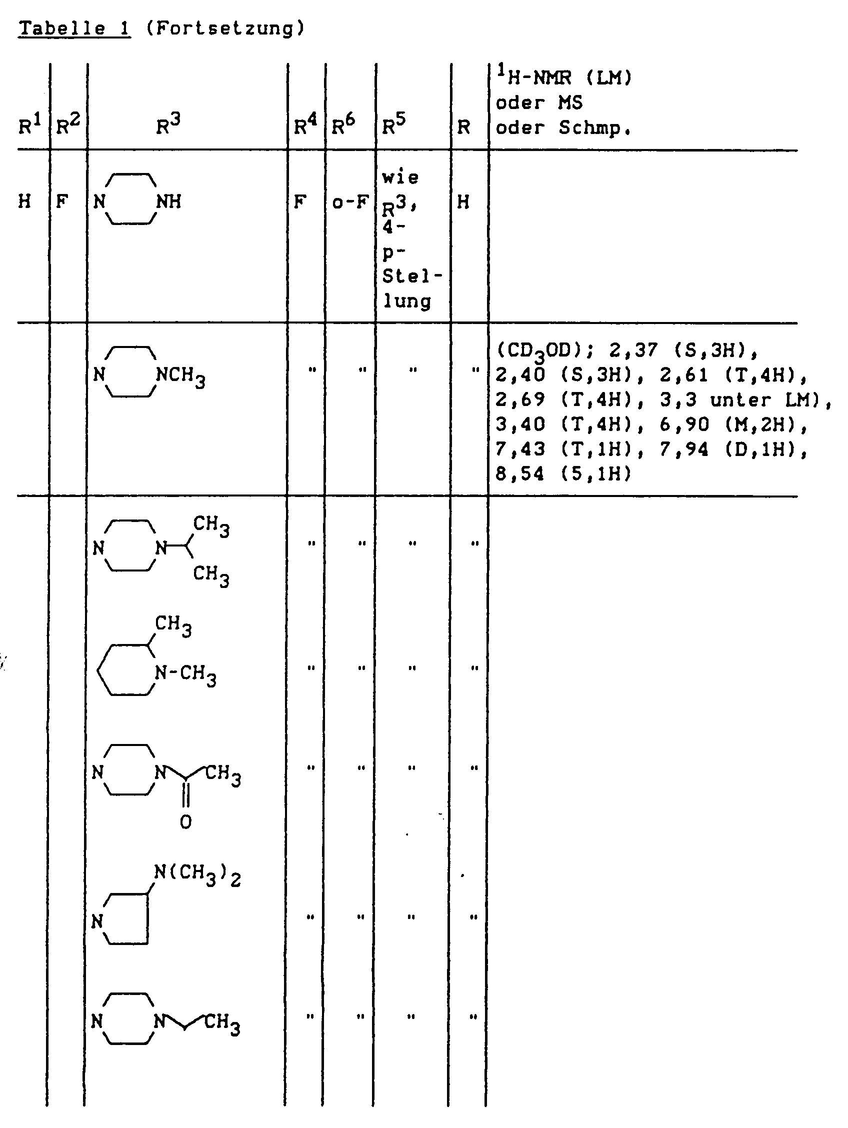

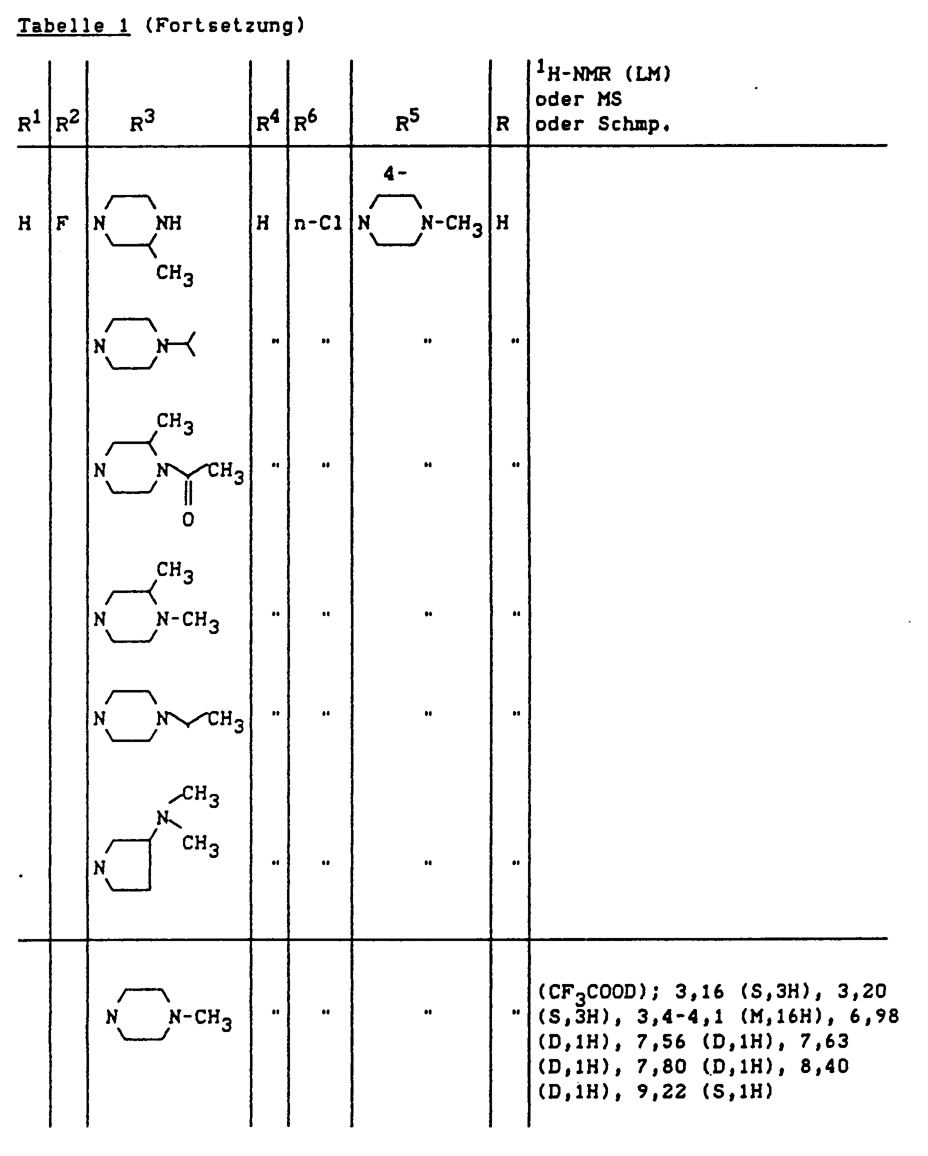

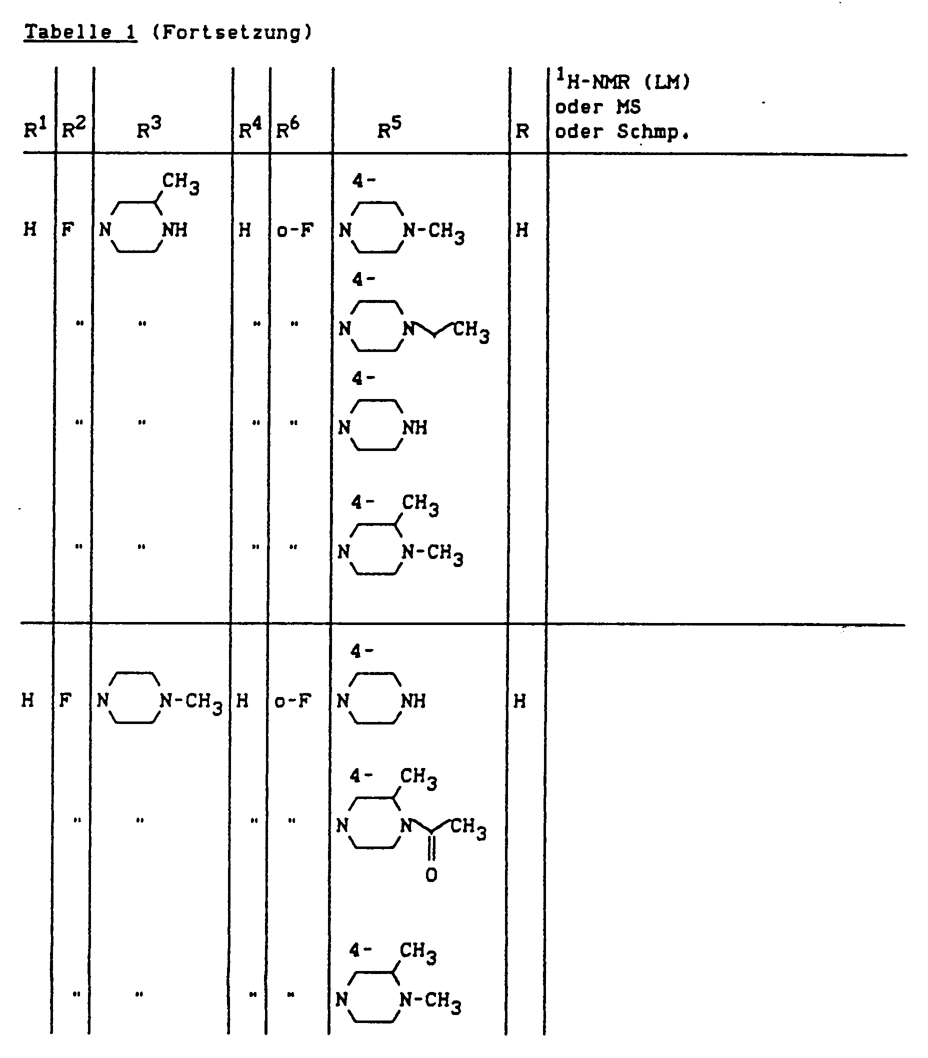

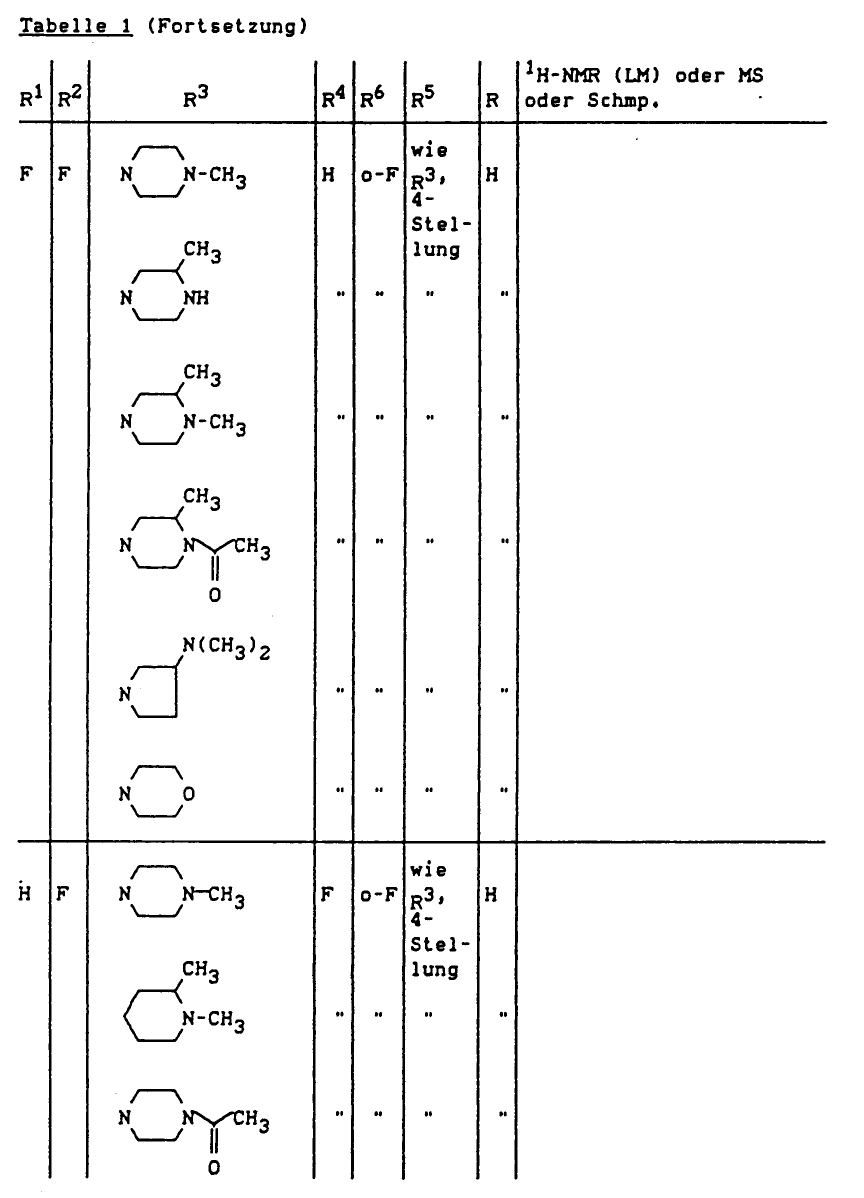

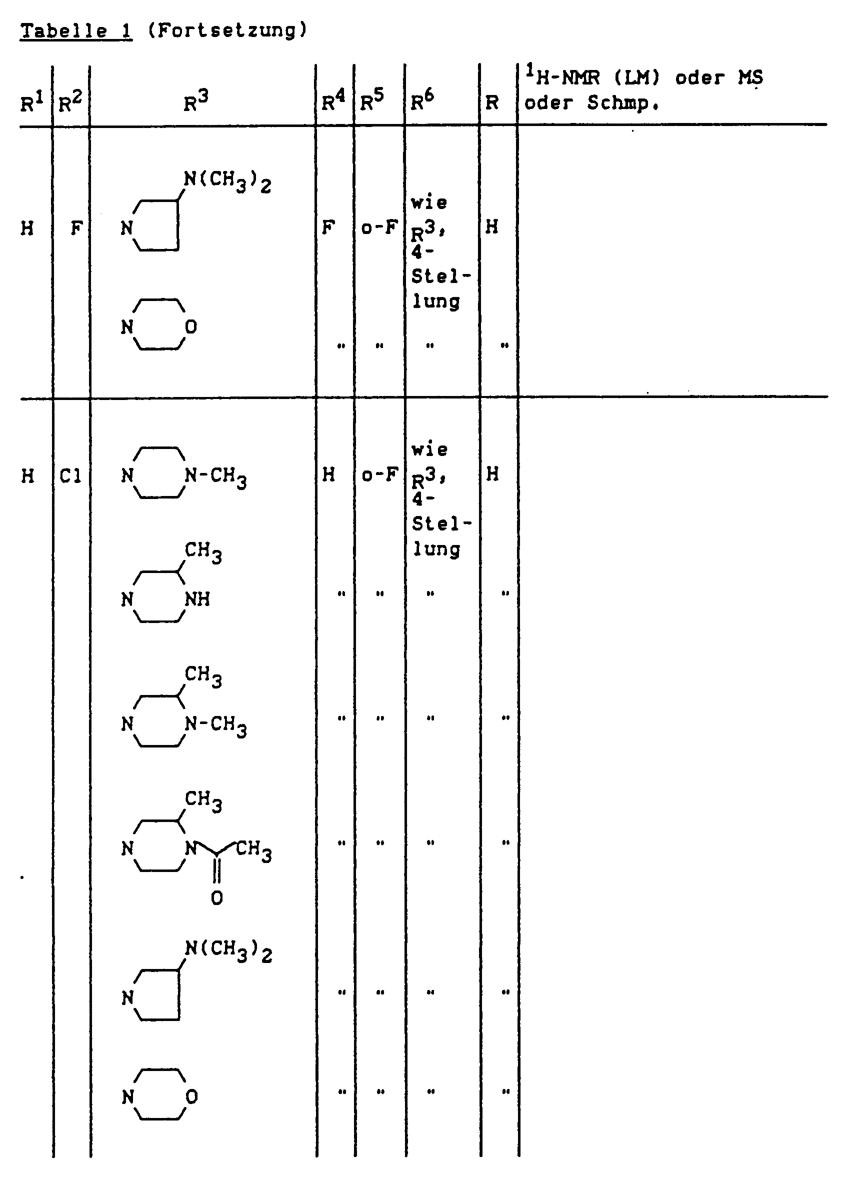

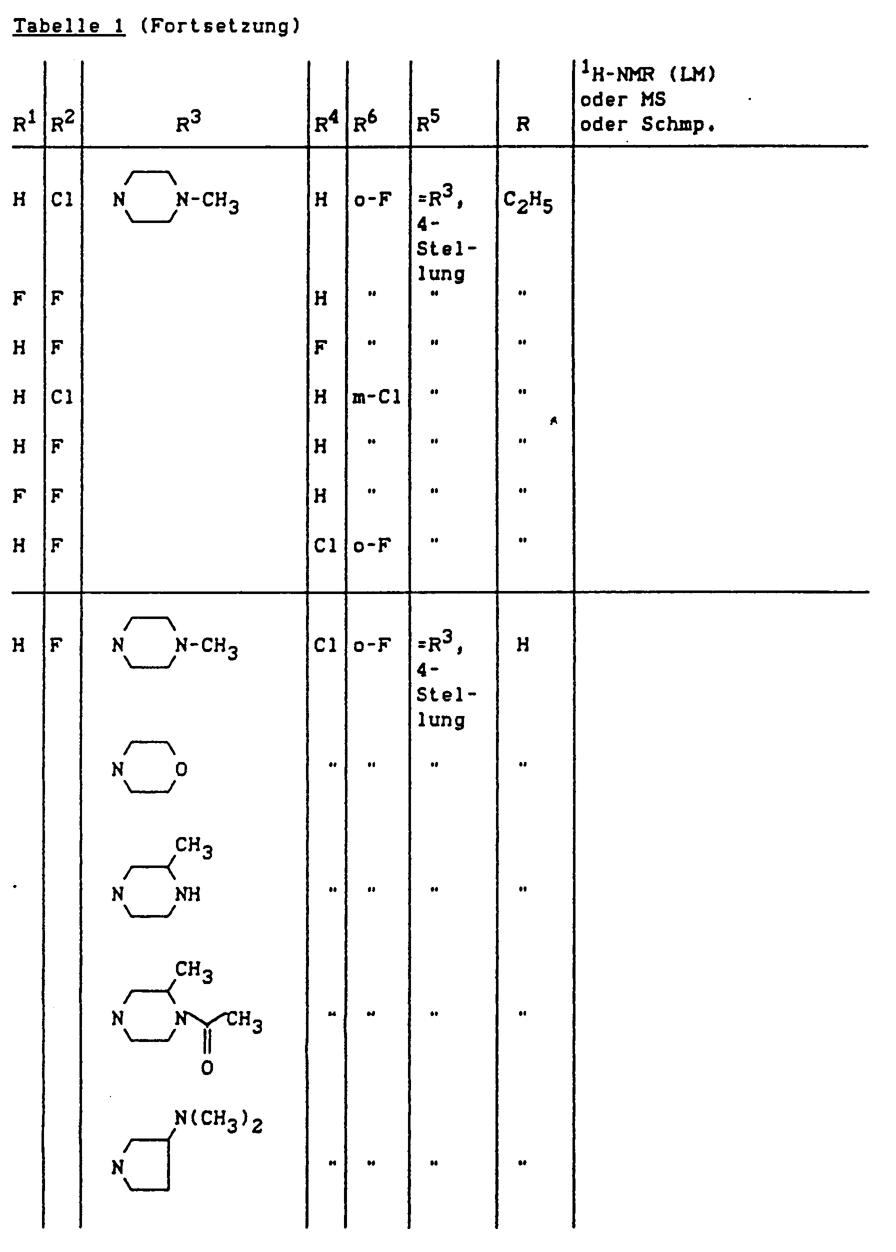

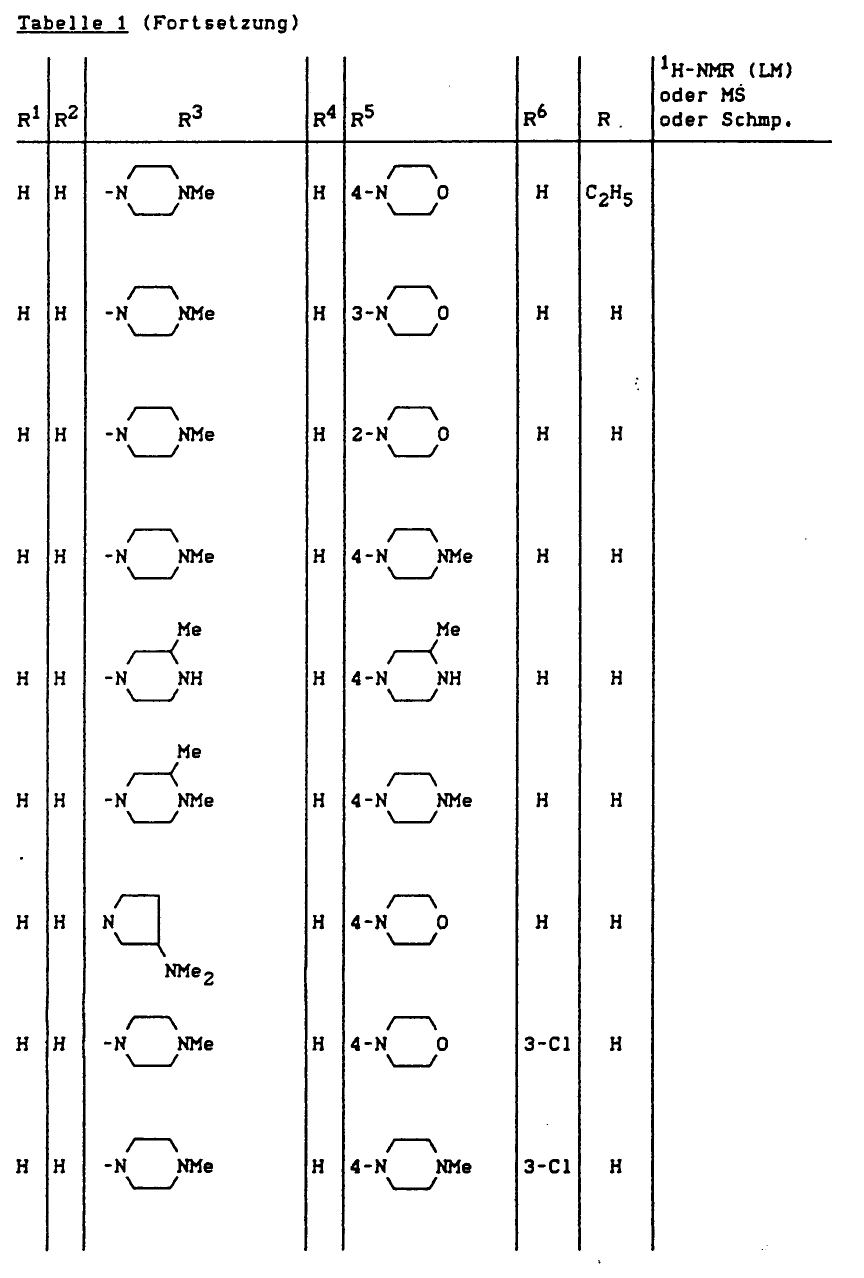

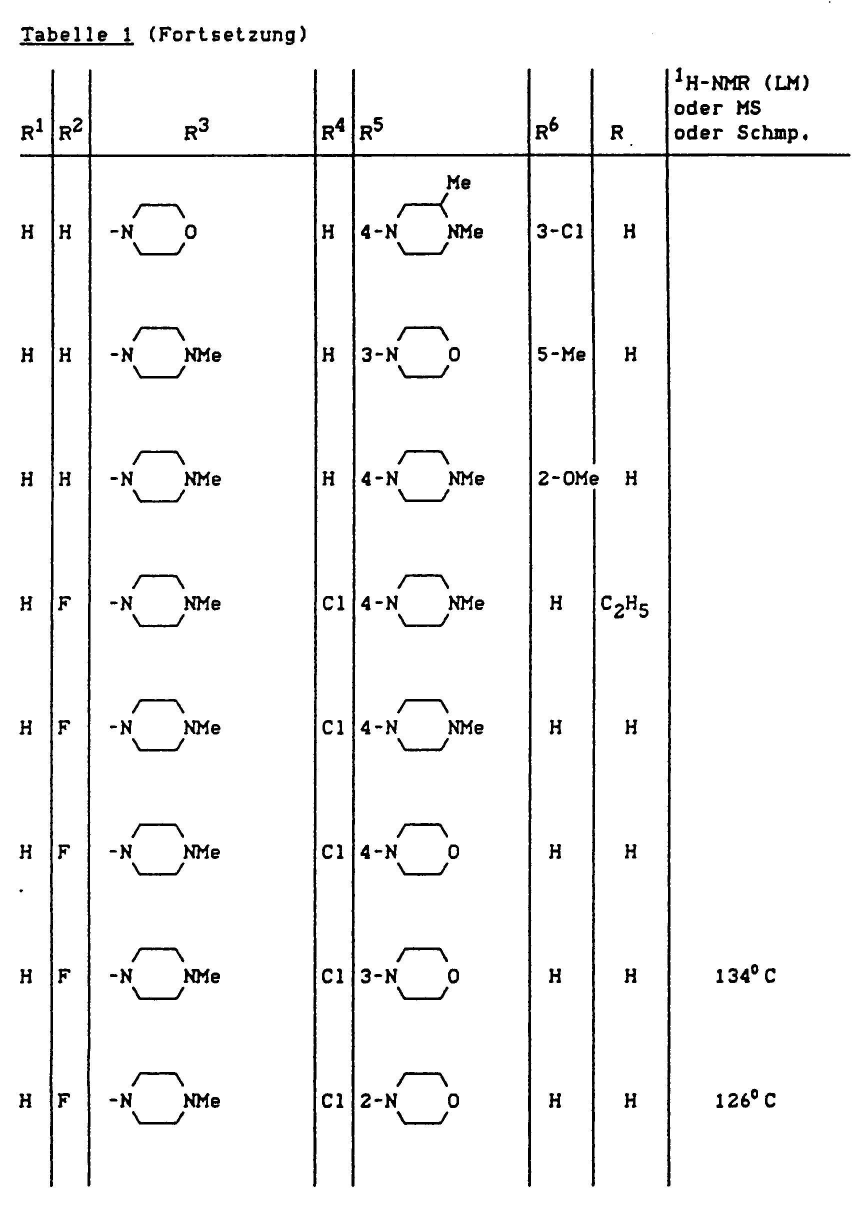

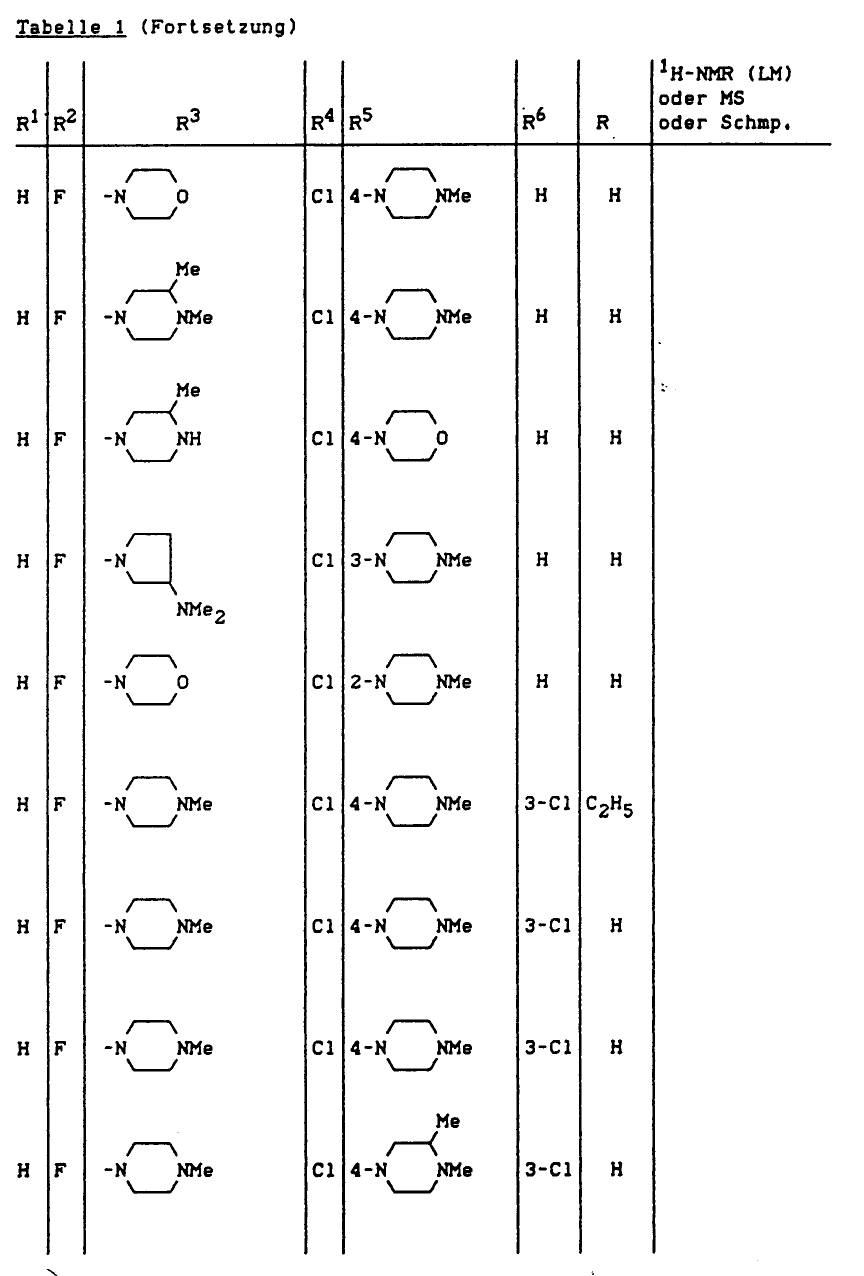

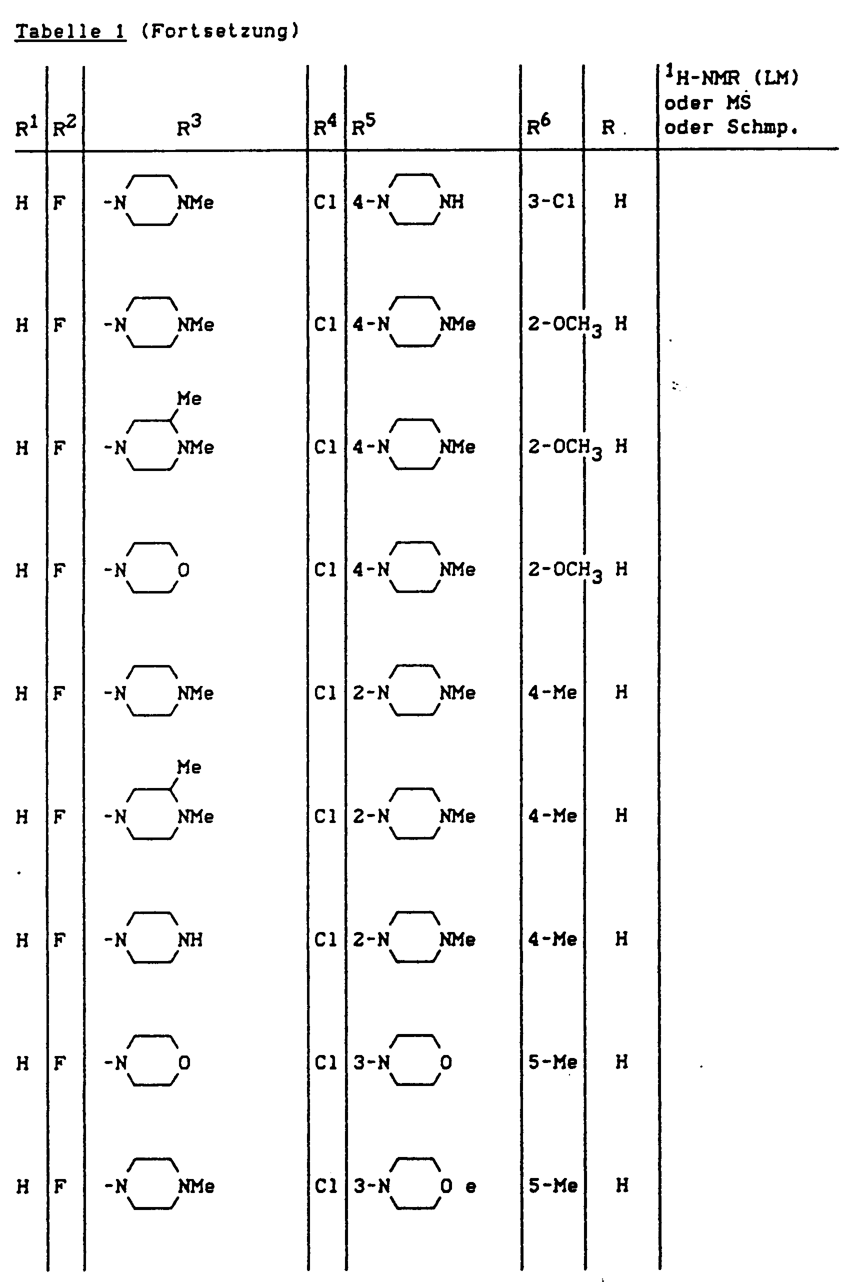

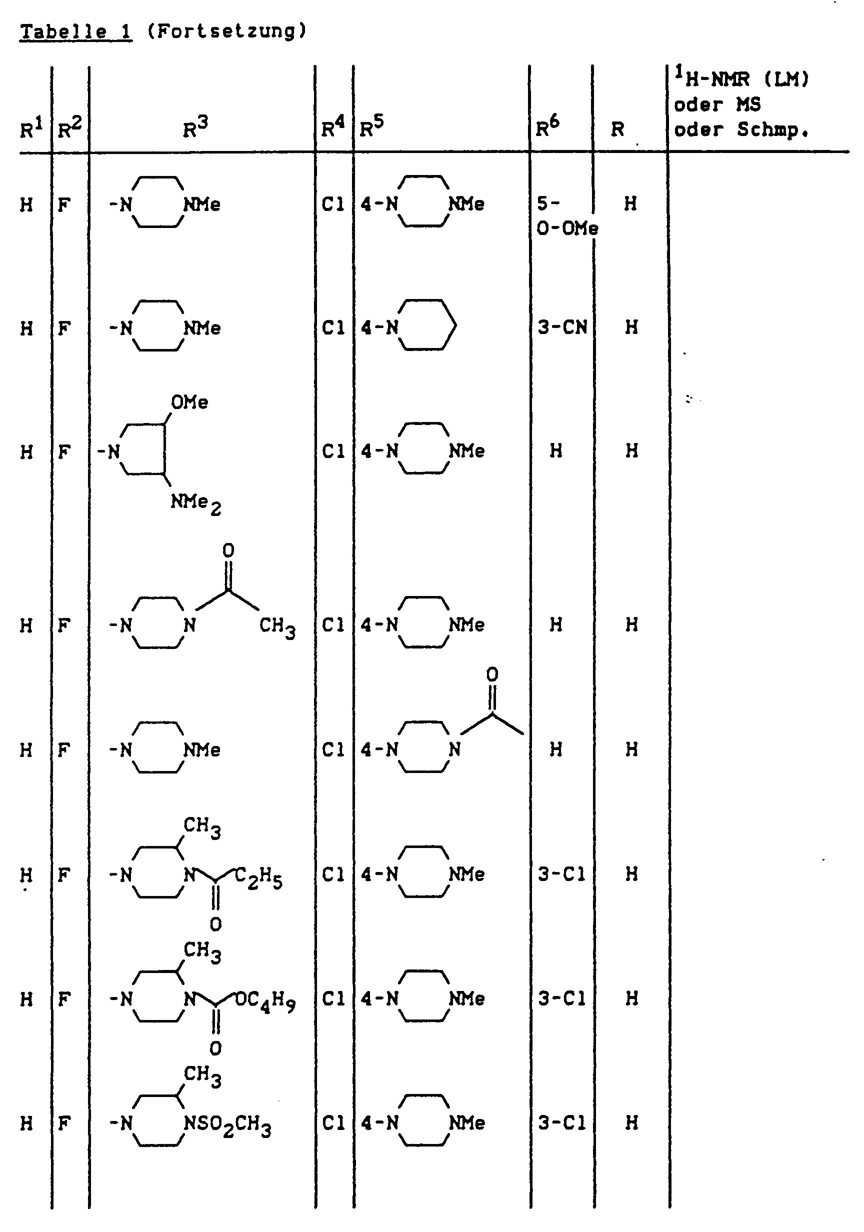

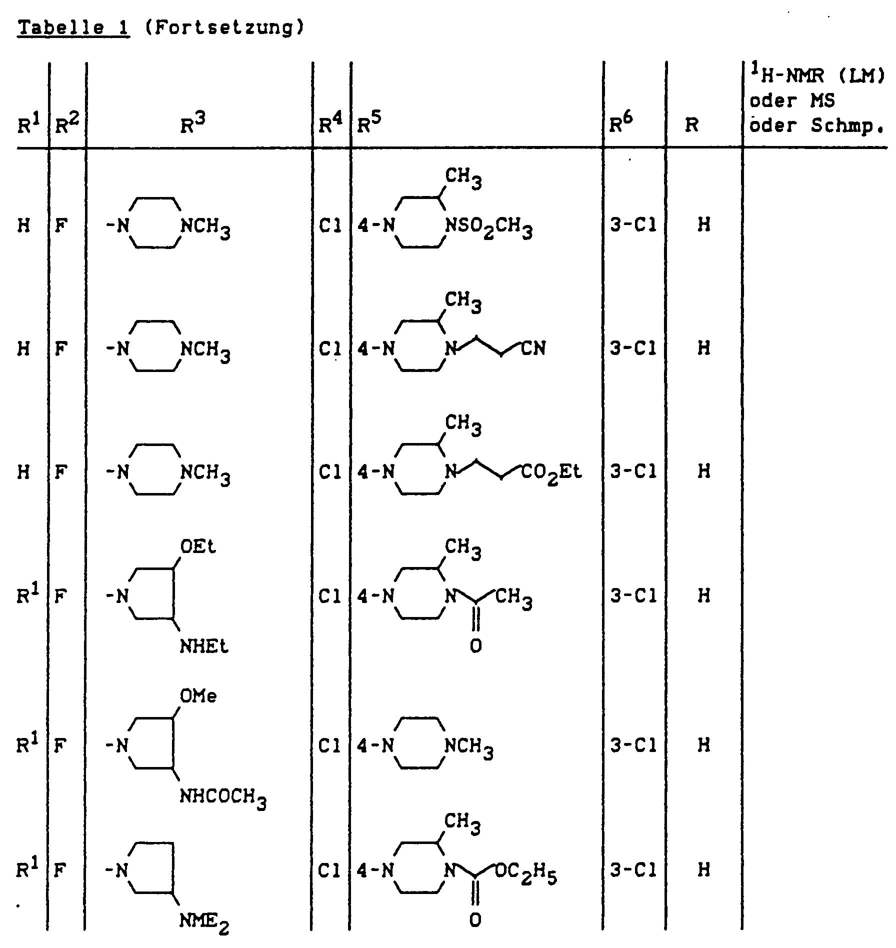

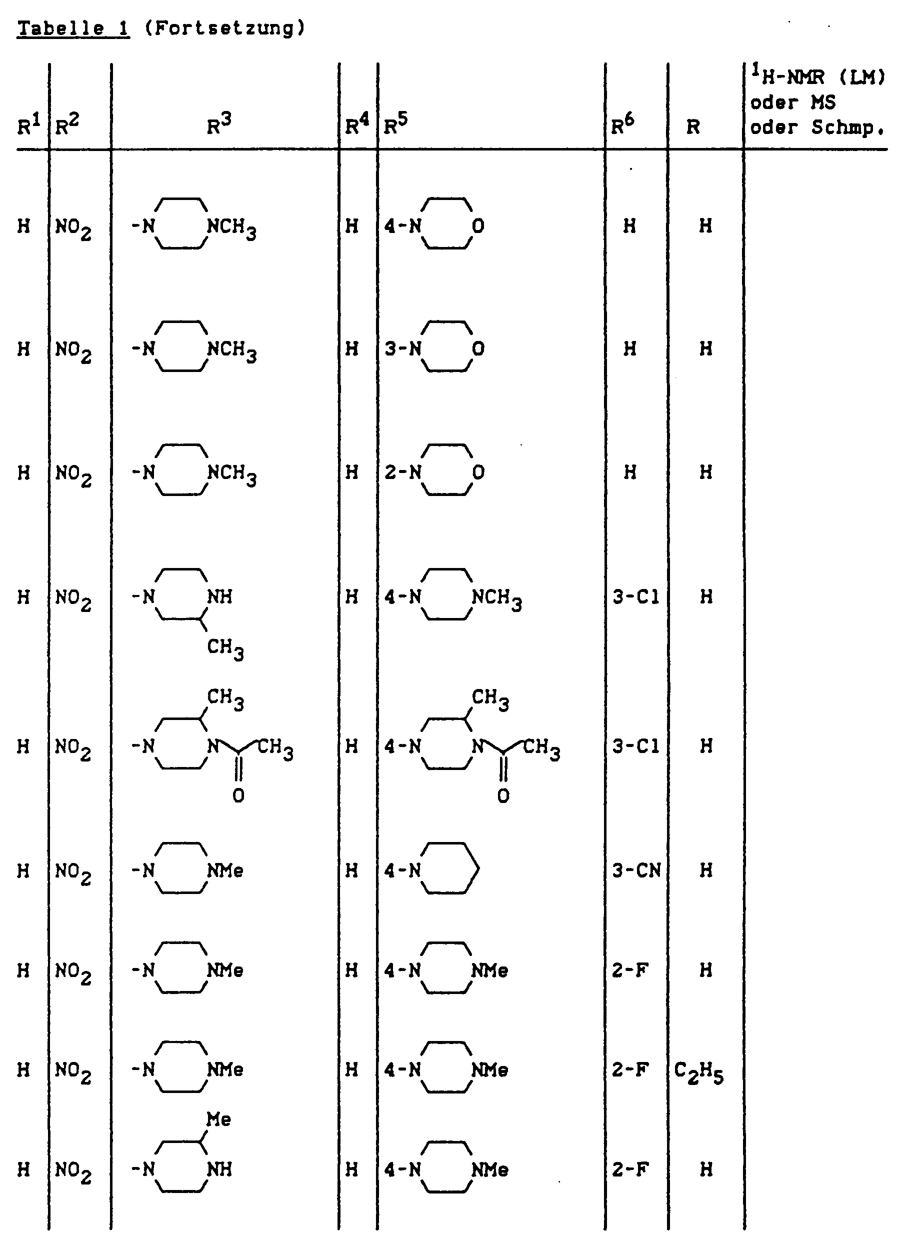

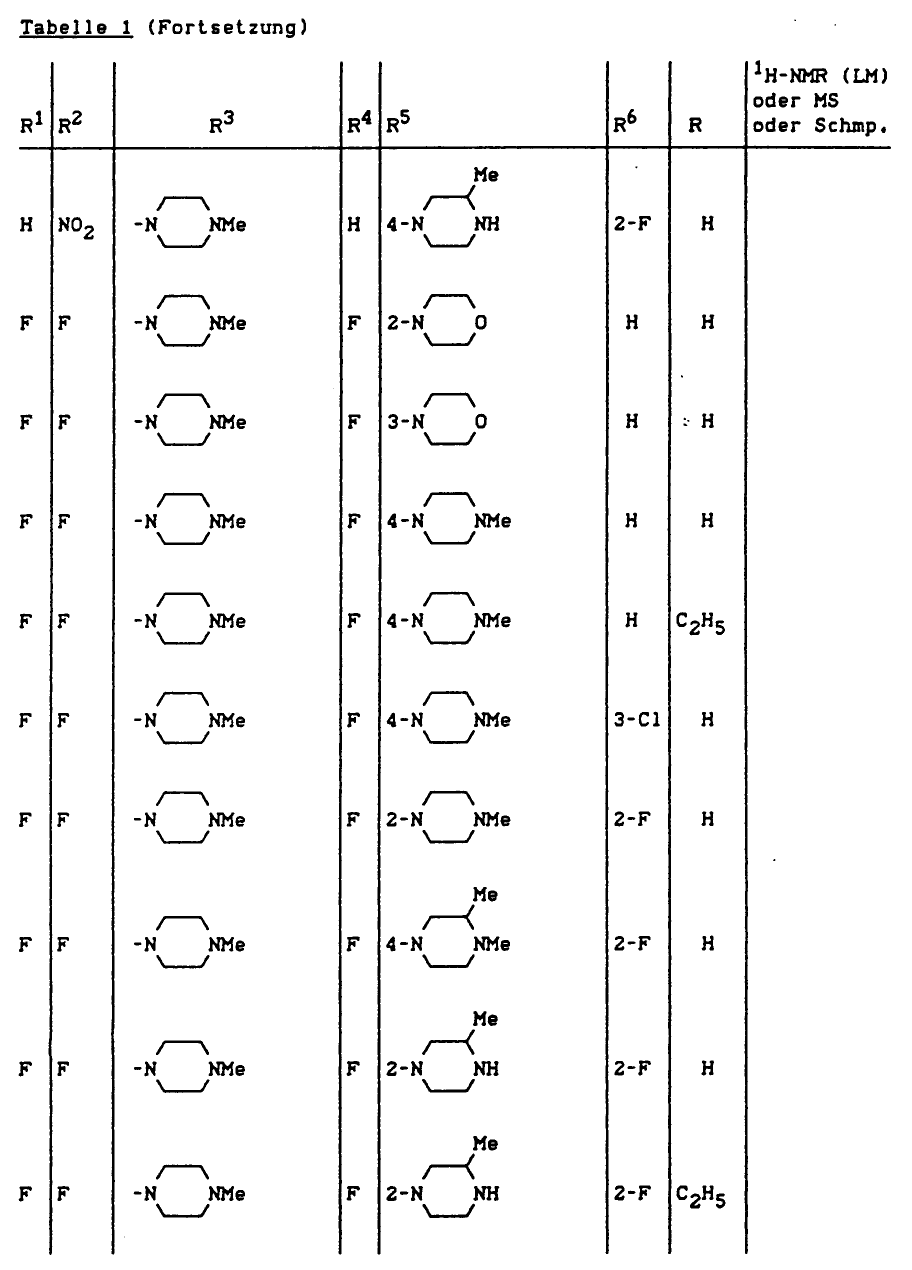

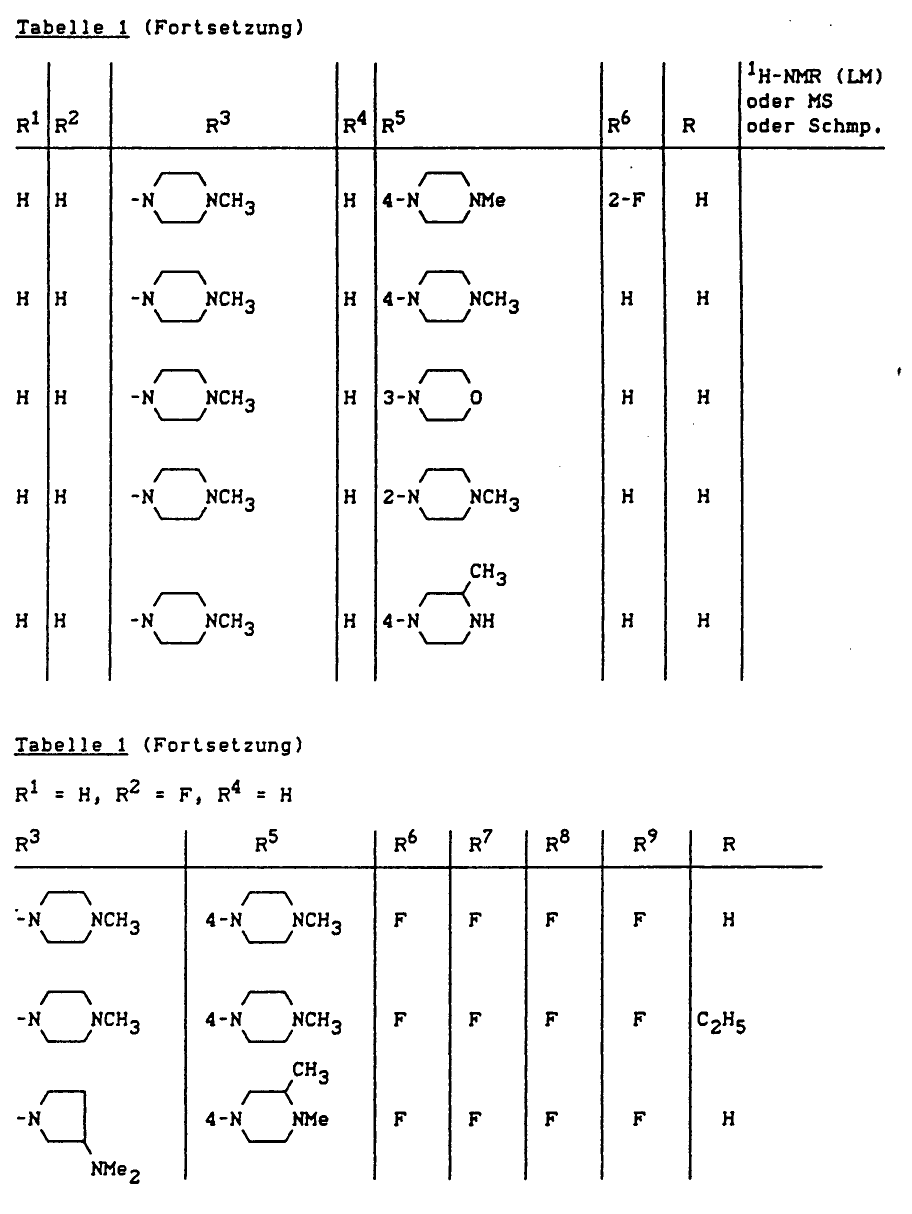

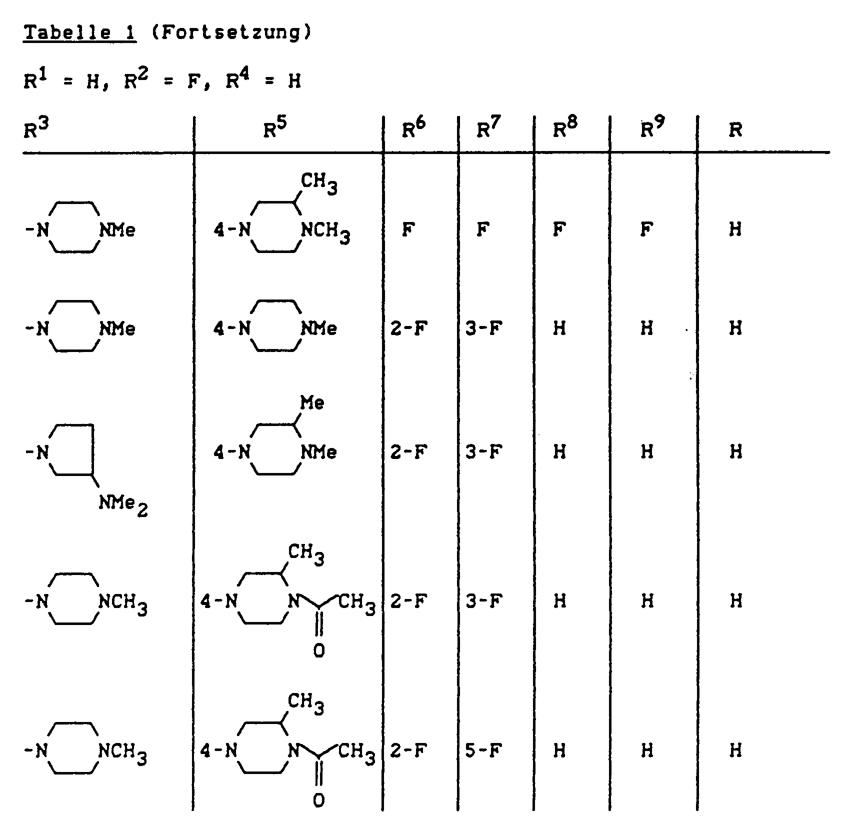

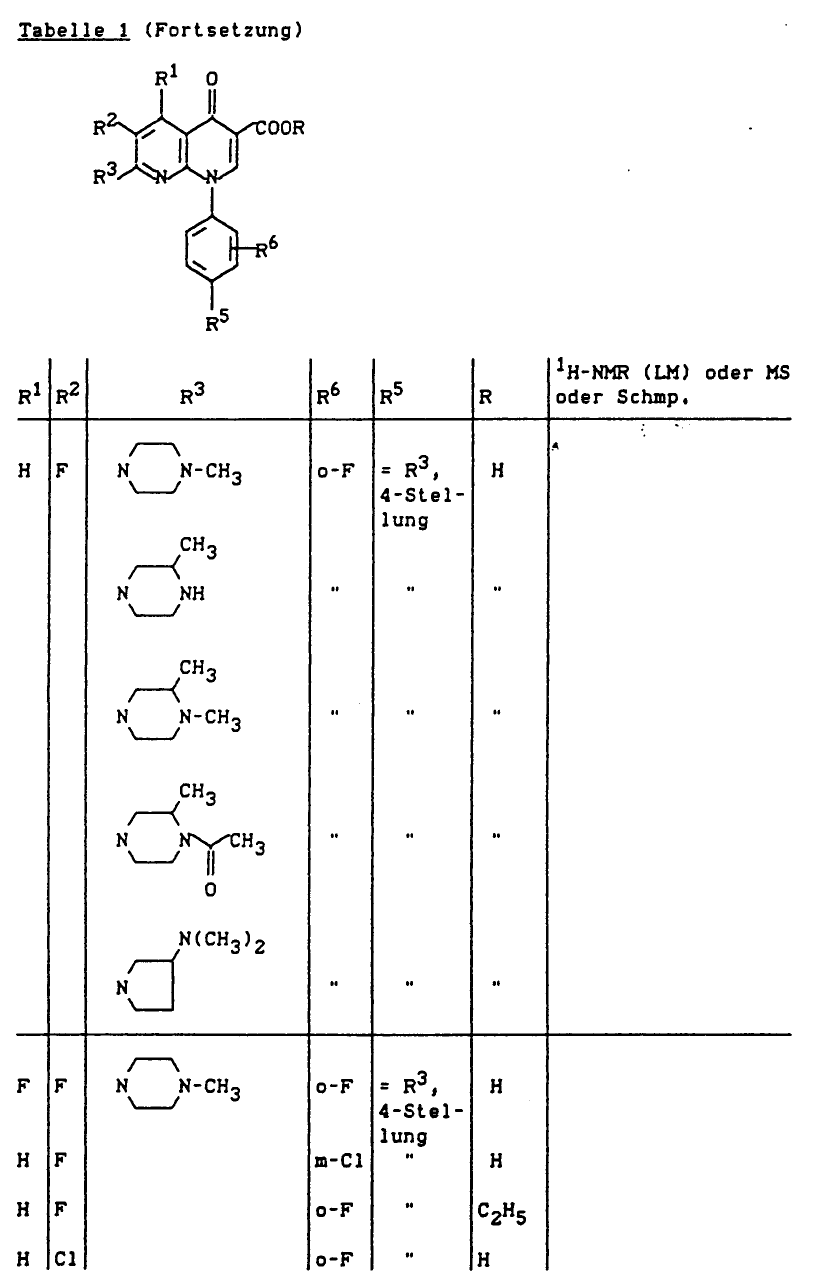

Die erfindungsgemäß zu verwendenden Verbindungen stellen wertvolle Wirkstoffe zur Behandlung oder Prophylaxe von Erkrankungen, hervorgerufen durch Viren, in der Human- und Tiermedizin dar.

Als Indikationsgebiete in der Humanmedizin können beispielsweise genannt werden:
1. Die Behandlung oder Prophylaxe von menschlichen Virus- und Retrovirusinfektionen.
2. Für die Behandlung oder Prophylaxe von HIV (Virus der humanen Immundefiziens; früher HTLV III/LAV genannt) verursachten Erkrankungen (AIDS) und den damit assoziierten Stadien wie ARC (AIDS related complex) and LAS (Lymphadenopathie-Syndrom) sowie der durch dieses Virus verursachten Immunschwäche und Encephalopathie.
3. Für die Behandlung oder die Prophylaxe einer HTLV I- oder HTLV II-Infektion.
4. Für die Behandlung oder die Prophylaxe des AIDS-carrier Zustandes (AIDS-Überträger-Zustand).

Als Indikationen in der Tiermedizin können beispielsweise angeführt werden;

Infektionen mit
a) Maedi-Visna (bei Schafen und Ziegen)
b) progressives Pneumonievirus (PPV) (bei Schafen und Ziegen)
c) caprine arthritis encephalitis Virus (bei Schafen und Ziegen)
d) Zwoegerziekte Virus (bei Schafen)
e) infektiöses Virus der Anämie (des Pferdes)
f) Infektionen verursacht durch das Katzenleukämievirus.

Die erfindungsgemäßen Wirkstoffe können auch zur Therapie und Prophylaxe von durch Influenza-Viren verursachten Krankheiten eingesetzt werden.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie
(a) Füll- und Streckmittel, z.B. Stärken, Milchzucker, Rohrzucker, Glukose, Mannit und Kieselsäure,
(b) Bindemittel, z.B. Carboxymethylcellulose, Alginate, Gelatine, Polyvinylpyrrolidon,
(c) Feuchthaltemittel, z.B. Glycerin,
(d) Sprengmittel, z.B. Agar-Agar, Calciumcarbonat und Natriumcarbonat,
(e) Lösungsverzögerer, z.B. Paraffin und
(f) Resorptionsbeschleuniger, z.B. quarternäre Ammoniumverbindungen,
(g) Netzmittel, z.B. Cetylalkohol, Glycerinmonostearat,
(h) Adsorptionsmittel, z.B. Kaolin und Bentonit und
(i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C₁₄-Alkohol mit C₁₆-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionslösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden, Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden, Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die Wirkstoffe der Formel (I) in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäß zu verwendenden Verbindungen können in den üblichen Konzentrationen und Zubereitungen zusammen mit dem Futter bzw. mit Futterzubereitungen oder mit dem Trinkwasser gegeben werden.

Die folgenden Beispiele erläutern die Erfindung.

### Beispiel 1

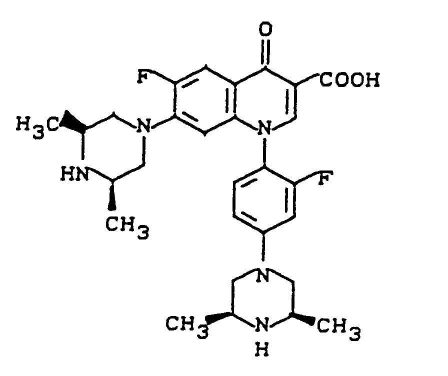

500 mg (1,4 mmol 1-(2,4-Difluorphenyl)-6-fluor-7-chlor-1,4-dihydro-4-oxo-3-chinolincarbonsäure, 4 ml Dimethylsulfoxid und 640 mg (5,6 mmol) cis-2,6-Dimethylpiperazin werden 2 Stunden auf 140°C erhitzt. Die Mischung wird eingedampft, der Rückstand in Wasser gelöst und einmal mit Methylenchlorid extrahiert. Die wäßrige Phase wird eingedampft, der Rückstand mit Ethanol verrieben, abgesaugt und getrocknet.

Ausbeute:
60 mg (8 % der Theorie) 1-(2-Fluor-3-(cis-3,5-dimethylpiperazinyl)-phenyl)-6-fluor-7-(cis-3,5-dimethylpiperazinyl)-1,4-dihydro-4-oxo-3-chinolincarbonsäure
Schmelzpunkt: 180 - 182°C (unter Zersetzung)

### Beispiel 2

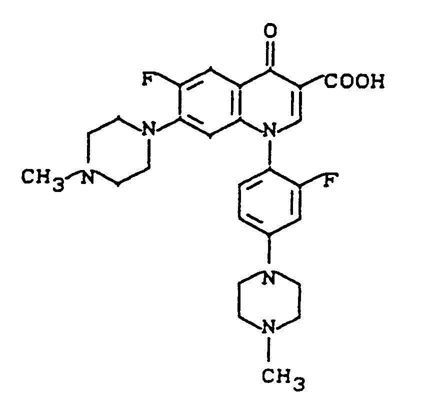

Analog Beispiel 1 setzt man mit 1-Methylpiperazin um, nach Eindampfen des Reaktionsgemisches wird der Rückstand in Methylenchlorid gelöst und zweimal mit Wasser gewaschen, die organische Phase wird eingedampft und an 40 g Kieselgel chromatographiert. (Laufmittel: Acetonitril : Wasser : Eisessig = 5 : 1 : 1 (v : v : v)).

Ausbeute:
190 mg (54 % der Theorie) 1-(2-Fluor-4-(4-methylpiperazinyl)-phenyl)-6-fluor-7-(4-methylpiperazinyl)-1,4-dihydro-4-oxo-3-chinolincarbonsäure
- ¹H-NMR-Spektrum (CDCl₃):: 2,34 (S, 3H); 2,40 (S, 3H); 2,54 (T, 4H); 2,61 (T, 4H); 6,40 (D, 1H); 6,80 (M, 2H); 7,24 (T, 1H); 8,07 (D, 1H); 8,60 (S, 1H) ppm

### Beispiel 3

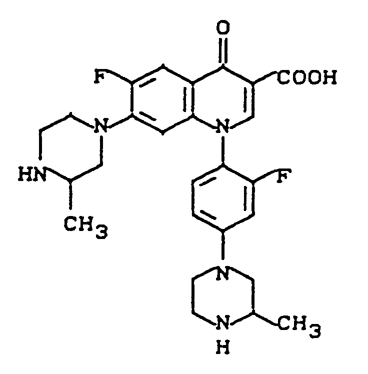

Analog Beispiel 1 setzt man mit 2-Methylpiperazin um.

Ausbeute:
291 mg (41 % der Theorie) 1-(2-Fluor-4-(3-methylpiperazinyl)-phenyl)-6-fluor-7-(3-methylpiperazinyl)-1,4-dihydro-4-oxo-3-chinolincarbonsäure
Schmelzpunkt: 273 - 281°C (unter Zersetzung)

### Beispiel 4

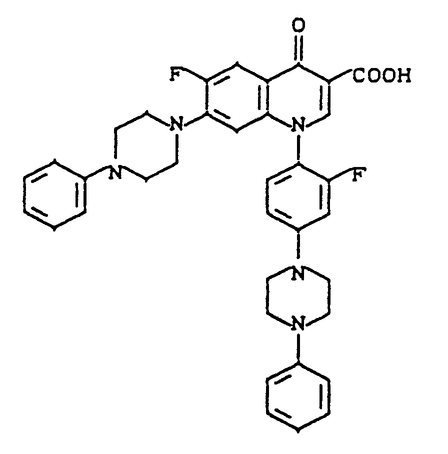

Analog Beispiel 1 setzt man mit 1-Phenylpiperazin um. Nach Eindampfen des Reaktionsgemisches wird der Rückstand mit Toluol ausgekocht und filtriert, der Filterrückstand wird in Methylenchlorid gelöst, zweimal mit Wasser gewaschen, eingeengt und getrocknet.

Ausbeute:
507 mg (58 % der Theorie) 1-(2-Fluor-4-(4-phenylpiperazinyl)-phenyl)-6-fluor-7-(4-phenylpiperazinyl)-1,4-dihydro-4-oxo-3-chinolincarbonsäure
Schmelzpunkt: 138 - 140°C

### Beispiel 5

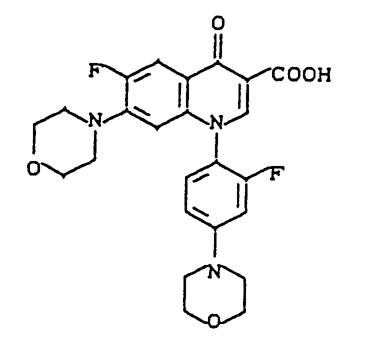

Analog Beispiel 1 wird mit Morpholin umgesetzt.

Ausbeute:
402 mg (60 % der Theorie) 1-(2-Fluor-4-morpholinylphenyl)-6-fluor-7-morpholinyl-1,4-dihydro-4-oxo-3-chinolincarbonsäure
Schmelzpunkt: 273°C (Methylenchlorid, n-Hexan) (unter Zersetzung)

### Beispiel 6

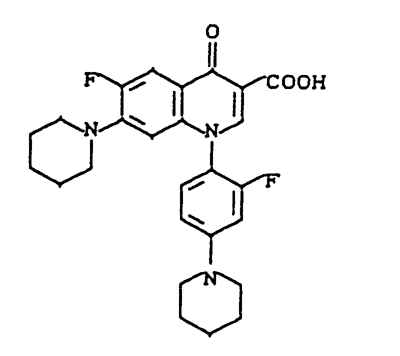

Analog Beispiel 1 wird mit Piperidin umgesetzt.

Ausbeute:
404 mg (61 % der Theorie) 1-(2-Fluor-4-piperidinylphenyl)-6-fluor-7-piperidinyl-1,4-dihydro-4-oxo-3-chinolincarbonsäure
Schmelzpunkt: 223°C (Methylenchlorid, n-Hexan) (unter Zersetzung)

### Beispiel 7

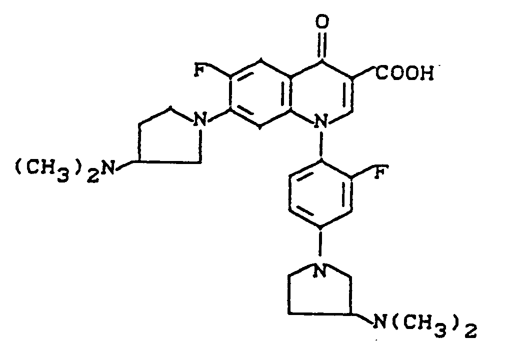

Analog Beispiel 1 wird mit 3-Dimethylaminopyrrolidin umgesetzt.

Nach Eindampfen des Reaktionsgemisches wird der Rückstand mit Toluol ausgekocht, die vereinigte Toluolphase dreimal mit Wasser gewaschen, eingedampft, in Methylenchlorid gelöst und durch Zugabe von n-Hexan gefällt.

Ausbeute:
304 mg (41 % der Theorie 1-(2-Fluor-4-(3-(dimethylamino)pyrrolidinyl)-phenyl)-6-fluor-7-(3-(dimethylamino)pyrrolidinyl)-1,4-dihydro-4-oxo-3-chinolincarbonsäure
Schmelzpunkt: 190°C

### Beispiel 8

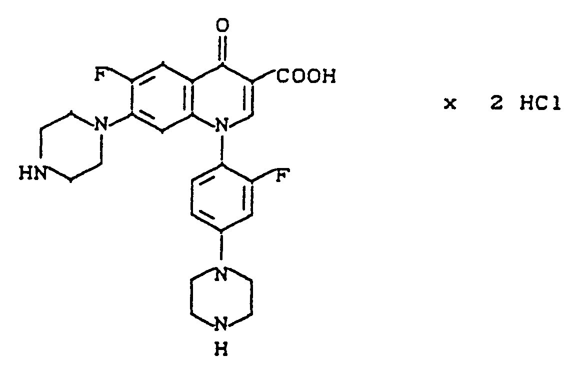

A) 150 g (43,5 mmol) 1-(2,4-Difluorphenyl)-6-fluor-7-chlor-1,4-dihydro-4-oxo-3-chinolinsäure, 100 ml Dimethylsulfoxid und 14,6 g (170 mmol) wasserfreier Piperazin werden zwei Stunden auf 140°C erhitzt. Anschließend fällt innerhalb von drei Tagen bei Raumtemperatur das Produkt aus, das abgesaugt und mit eiskaltem Wasser gewaschen wird.
   Ausbeute:
   9,9 g (50 % der Theorie) 1-(2-Fluor-4-piperazinylphenyl)-6-fluor-7-piperazinyl-1,4-dihydro-4-oxo-3-chinolincarbonsäure.
   Schmelzpunkt: 207 - 209°C.
B) 500 mg (1,1 mol) des Produktes aus Stufe A werden in 5 ml 3N-Salzsäure gelöst, eingedampft, mit Ethanol verrieben, abgesaugt und getrocknet.
   Ausbeute; 325 mg (60 % der Theorie) 1-(2-Fluor-4-piperazinyl-phenyl)-6-fluor-7-piperazinyl-1,4-dihydro-4-oxo-3-chinolincarbonsäure-Dihydrochlorid.
   Schmelzpunkt: > 250°C (unter Zersetzung)

### Beispiel 9

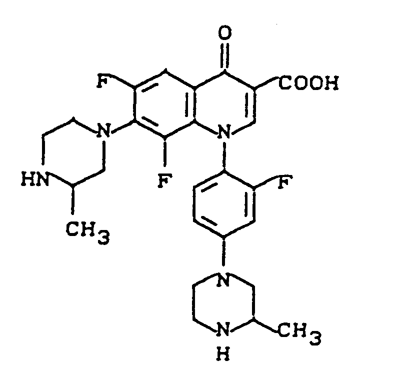

1,0 g (2,9 mmol) 1-(2,4-Difluorphenyl)-6,7,8-trifluor1,4-dihydro-4-oxo-3-chinolinsäure, 12 ml Dimethylsulfoxid, 1,2 g (11,8 mmol) 2-Methylpiperazin werden zwei Stunden auf 140°C erhitzt, eingedampft und der Rückstand von 80 g Kieselgel chromatographiert (Laufmittel; Methylenchlorid : Methanol ; 20%ige wäßrige Ammoniaklösung = 4 : 8 : 1), produkthaltige Fraktionen werden eingedampft, der Rückstand in Methanol gelöst und mit n-Hexan gefällt.

Ausbeute:
104 mg (7 % der Theorie) 1-(2-Fluor-4-(3-methylpiperazinyl)-phenyl)-6,8-difluor-7-(3-methylpiperazinyl)-1,4-dihydro-4-oxo-3-chinolincarbonsäure
Schmelzpunkt; 250°C (unter Zersetzung)

### Beispiel 10

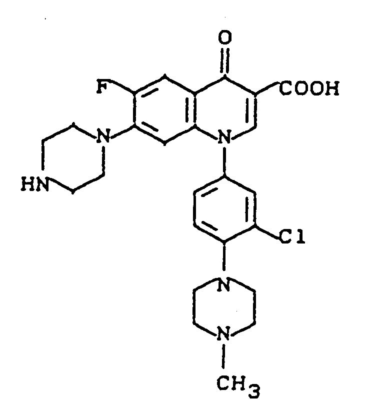

450 mg (1,0 mmol) 1-(3-Chlor-4-(4-methylpiperazinyl)phenyl-6-fluor-7-chlor-1,4-dihydro-4-oxo-3-chinolinsäure, 4 ml Acetonitril, 2 ml N,N-Dimethylformamid, 172 mg (2,0 mmol) Piperazin und 336 mg (3,0 mmol) DABCO werden 24 Stunden auf 120°C erwärmt und anschließend eingedampft. Der Rückstand wird mit Toluol ausgekocht, die Toluolphase eingedampft, in Methylenchlorid gelöst und zweimal mit Wasser gewaschen. Die Methylenchloridphase wird eingedampft und der Rückstand an 500 g Kieselgel chromatographiert (Laufmittel: Acetonitril : Wasser : Eisessig = 5 : 1 : 1 (v : v : v)).

Ausbeute:
123 mg (25 % der Theorie) 1-(3-Chlor-4-(4-methylpiperazinyl)-phenyl)-6-fluor-7-piperazinyl)-1,4-dihydro-4-oxo-3-chinolincarbonsäure
Schmelzpunkt; 230°C (unter Zersetzung)

### Beispiel 11

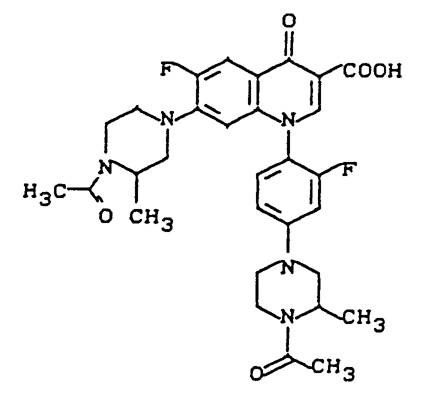

248 mg (0,5 mmol) 1-(2-Fluor-4-(3-methylpiperazinyl)phenyl-6-fluor-7-(3-methylpiperazinyl)-1,4-dihydro-4-oxo-3-chinolinsäure, 204 mg (2,0 mmol) Acetanhydrid und 15 ml Pyridin läßt man zwei Stunden bei 0°C reagieren, erwärmt auf Raumtemperatur und dampft ein. Der Rückstand wird zweimal mit Toluol versetzt und eingedampft, anschließend in Methylenchlorid gelöst, zweimal mit Wasser gewaschen, eingedampft und getrocknet.

Ausbeute:
277 mg (95 % der Theorie) 1-(2-Fluor-4-(3-methyl-4-acetylpiperazinyl)-phenyl)-6-fluor-7-(3-methyl-4-acetylpiperazinyl)-1,4-dihydro-4-oxo-3-chinolincarbonsäure
R_{F}; 0,23 (Kieselgel; Acetonitril : Wasser : Eisessig = 150 : 10 : 1)

### Beispiel 12

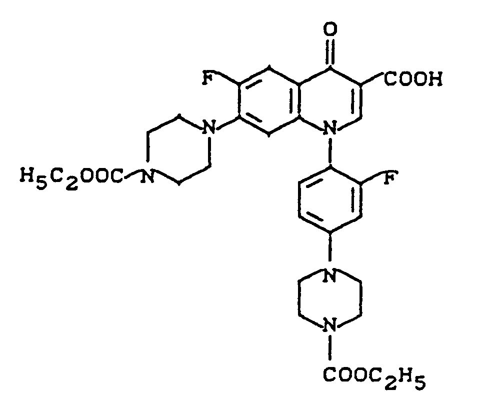

Analog Beispiel 11 werden 234 mg 1-(2-Fluor-4-piperazinyl-phenyl)-6-fluor-7-piperazinyl-1,4-dihydro-4-oxo-3-chinolinsäure mit 141 mg (1,3 mmol) Chlorameisensäure umgesetzt.

Ausbeute:
286 mg (89 % der Theorie) 1-(2-Fluor-4-(4-carbethoxypiperazinyl)-phenyl)-6-fluor-7-(4-carbethoxypiperazinyl)1,4-dihydro-4-oxo-3-chinolincarbonsäureethylester
- Massenspektrum:: m/e = 641 (M⁺), 569 (100 %, M⁺-CO₂C₂H₅), 467, 56, 29

### Beispiel 13

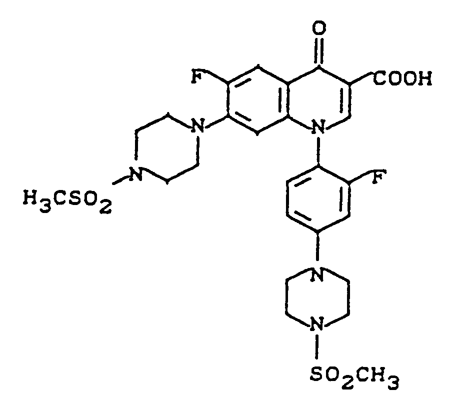

Analog Beispiel 11 werden 234 mg (0,5 mmol) 1-(2-Fluor-4-piperazinyl-phenyl)-6-fluor-7-piperazinyl-1,4-dihydro-4-oxo-3-chinolinsäure mit 286 mg (2,5 mmol) Methansulfonylchlorid umgesetzt.

Ausbeute:
114 mg (36 % der Theorie) 1-(2-Fluor-4-(4-methylsulfonylpiperazinyl)-phenyl)-6-fluor-7-(4-methylsulfonylpiperazinyl)-1,4-dihydro-4-oxo-3-chinolincarbonsäure
Schmelzpunkt: 178°C (unter Zersetzung)

### Beispiel 14

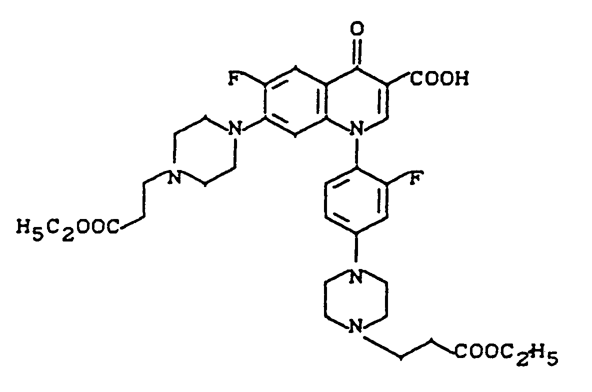

469 mg (1,0 mmol) 1-(2-Fluor-4-piperazinyl-phenyl)-6-fluor-7-piperazinyl-1,4-dihydro-4-oxo-3-chinolincarbonsäure, 9 ml Wasser, 4,5 ml Ethanol, 1,0 g (10 mmol) Acrylsäureethylester werden 3 Tage bei 25°C verrührt, eingedampft, der rückstand in Methylenchlorid gelöst, zweimal mit Wasser gewaschen. Die organische Phase wird eingedampft und getrocknet.

Ausbeute:
445 mg (67 % der Theorie) 1-(2-Fluor-4-(4-carboxyeth-2-yl-piperazinyl)-phenyl)-6-fluor-7-(4-carboxyeth-2-yl-piperazinyl)-1,4-dihydro-4-oxo-3-chinolincarbonsäure
- ¹H-NMR-Spektrum (CF₃COOD);: 1,37 (2T,3H); 3,16 (2T,4H); 3,16 (2T,4H); 3,56 (2T,8H); 3,77 (2T,4H); 4,06 (M, 8H); 4,37 (2Q,4H); 6,94 (D,1H); 7,10 (M,2H); 7,60 (T,1H); 8,40 (D,1H); 9,20 (S,1H) ppm

### Beispiel 15

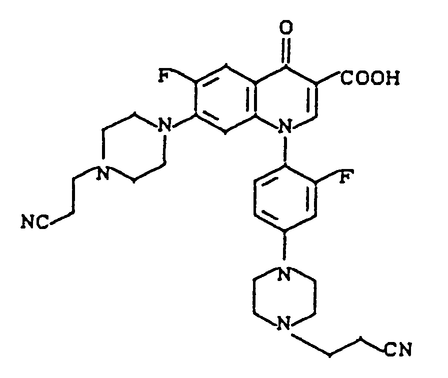

Analog Beispiel 14 wird mit Acrylnitril umgesetzt.

Ausbeute:
396 mg (69 % der Theorie) 1-(2-Fluor-4-(4-cyanoeth-2-yl-piperazinyl)-phenyl)-6-fluor-7-(4-cyanoeth-2-yl-piperazinyl)-1,4-dihydro-4-oxo-3-chinolincarbonsäure
- ¹H-NMR-Spektrum (CF₃COOD);: 3,30 (2T,3H); 3,60 (M,8H); 3,87 (2T,4H); 4,10 (M,8H); 6,97 (D,1H); 7,10 (M,2H); 7,60 (T,1H); 8,40 (D,1H); 9,20 (S,1H) ppm

### Beispiel 16

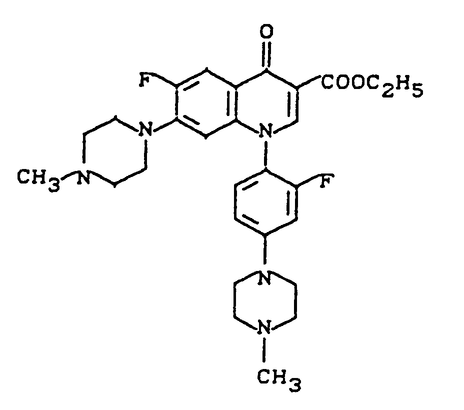

560 mg (1,4 mmol) 1-(2,4-Difluorphenyl)-6,7-difluor-1,4-dihydro-4-oxo-3-chinolincarbonsäureethylester, 6 ml Dimethylsulfoxid, 550 mg (5,5 mmol) N-Methylpiperazin werden zwei Stunden auf 140°C erhitzt. Das Gemisch wird eingedampft, der Rückstand in Methylenchlorid gelöst und zweimal mit Wasser gewaschen. Nach Eindampfen wird an 100 g Kieselgel chromatographiert (Laufmittel zunächst Toluol : Ethanol = 1 : 1, dann Ethanol : Toluol : Eisessig = 100 : 100 : 1). Die produkthaltigen Fraktionen wurden eingeengt und getrocknet.

Ausbeute:
300 mg (42 % der Theorie) 1-(2-Fluor-4-(methylpiperazinyl)-phenyl)-6-fluor-7-(4-methylpiperazinyl)-1,4-dihydro-4-oxo-3-chinolincarbonsäureethylester, Essigsäuresalz
Schmelzpunkt; 58°C

### Anwendungsbeispiele

Es wurde im Rahmen von Untersuchungen, die zu der vorliegenden Erfindung führten, überraschend gefunden, daß die erfindungsgemäß zu verwendende Verbindung der allgemeinen Formel (I) eine außerordentlich starke Wirkung gegen Retroviren besitzt. Dies wird durch die weiter unten angegebenen Versuchsdaten beispielhaft für die Verbindung am Visna-Virus in Zellkulturen belegt. Das Visna-Virus und das HIV-Virus (Virus der humanen Immundefiziens) gehören beide zu der Retrovirus-Subfamilie der Lentiviren [Haase A.T. Nature (1986) 322, 130-136]. Beide Viren weisen eine ähnliche Genomorganisation und ein gegenüber den andern Retroviren komplexes Transkriptionsmuster auf [Sojijo P. et al., Cell (1985), 42, 369 - 382; Davis J. L. et al., Journal of Virology (1987) 61, (5) 1325 - 1331].

Darüber hinaus wurde gezeigt [Frank, K.B., et al., Anti-microbial Agents and Chemotherapy (1987) 31 (9), S. 1369 - 1374], daß bekannte Inhibitoren des HIV auch das Visnavirus in vitro in vergleichbaren Konzentrationen hemmen; d.h., dieses Modell eignet sich für die Prüfung und Auffindung von Hemmstoffen des HIV.

In Zellkulturen, die mit Visna-Virus infiziert sind, treten 5 bis 10 Tage nach der Infektion ausgeprägte virusinduzierte, zytopathische Effekte auf. Durch Behandlung der infizierten Zellkulturen mit der erfindungsgemäßen Verbindung konnte das Auftreten dieser zytopathischen Effekte verhindert werden.

Der Visna-Virus-Test wurde nach der Methode von O. Narayan et al., Journal of Infectious Diseases 135, 5, 1977, 800 - 806 durchgeführt. Dazu wurde die erfindungsgemäße Verbindung im Kulturmedium in nicht zytotoxischen Konzentrationen in 96er Microtiterplatten verdünnt. Anschließend wurden Fibroblastenzellen vom Schaf (5 x 10⁴ Zellen pro Napf) in Produktionsmedium zu jedem Näpfchen zugeführt. Jedes Näpfchen erhielt dann 50 µl einer Visna-Viruslösung mit einer Titer von ca. 2,5 x 10⁴ TCID₅₀ (TCID = tissue culture infectious dosis). Diese Virusdosis entspricht einer MOI (Multiplizität der Infektion) von ca. 0,05.

Unter diesen Infektionsbedingungen resultierte zwischen Tag 5 und Tag 10 in einer Infektionskontrolle ohne Substanz ein virusinduzierter, zytopathischer Effekt. Die infizierten und behandelten Zellen und die Kontrollzellen wurden 7 Tage bei 37°C 5 % CO₂ inkubiert.

Bei Auftreten des virusinduzierten zytopathogenen Effektes in der unbehandelten Viruskontrolle wurden die Kulturen mit Formalin fixiert und anschließend mit einer Giemsa-Lösung gefärbt. Die inhibitorische Konzentration (IC₅₀) wurde mikroskopisch als die Konzentration ermittelt, bei der der zytopathische Effekt um 50 % gehemmt wurde im Vergleich zur unbehandelten Viruskontrolle, die 100 % Zellzerstörung aufwies.

Es wurde gefunden, daß mit den erfindungsgemäßen Verbindungen die Visna-Virus infizierten Zellen vor der virusinduzierten Zellzerstörung schützen. Dies wird anhand der Daten der folgenden Verbindungen exemplarisch gezeigt;

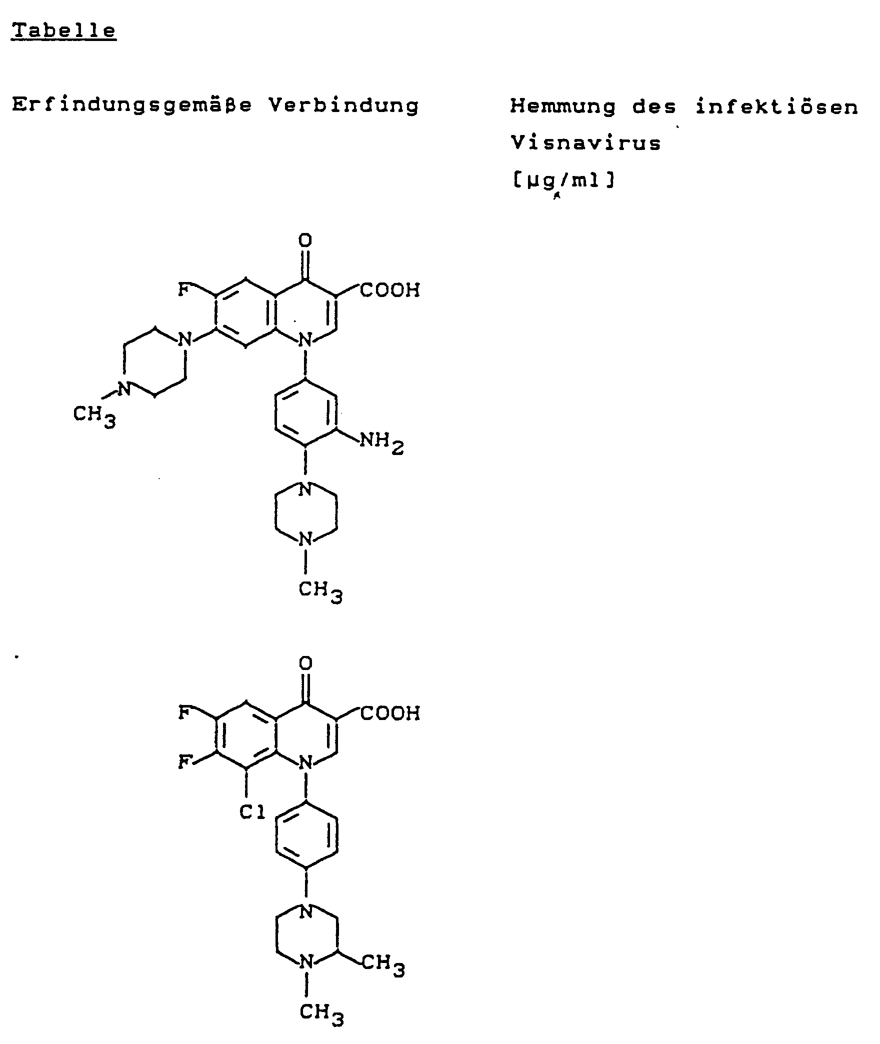

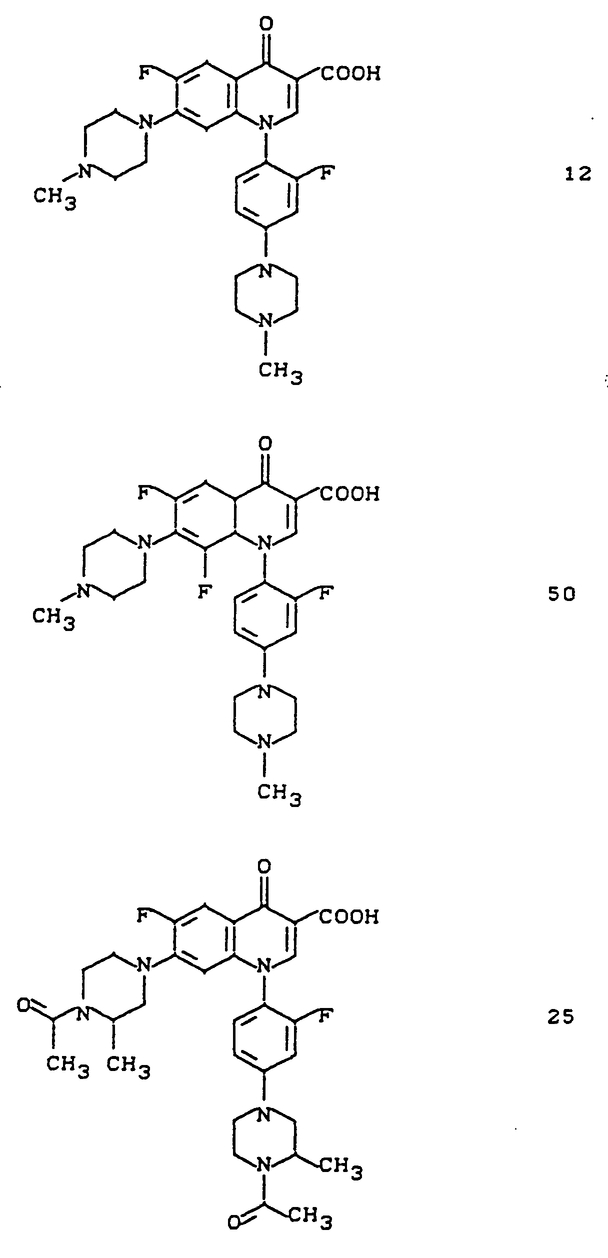

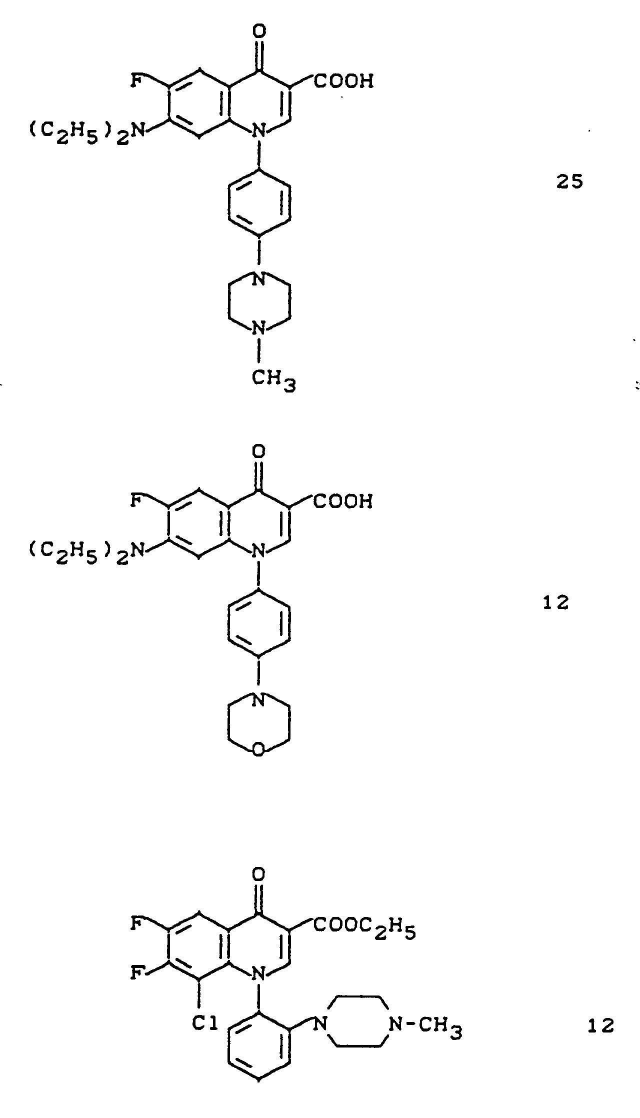

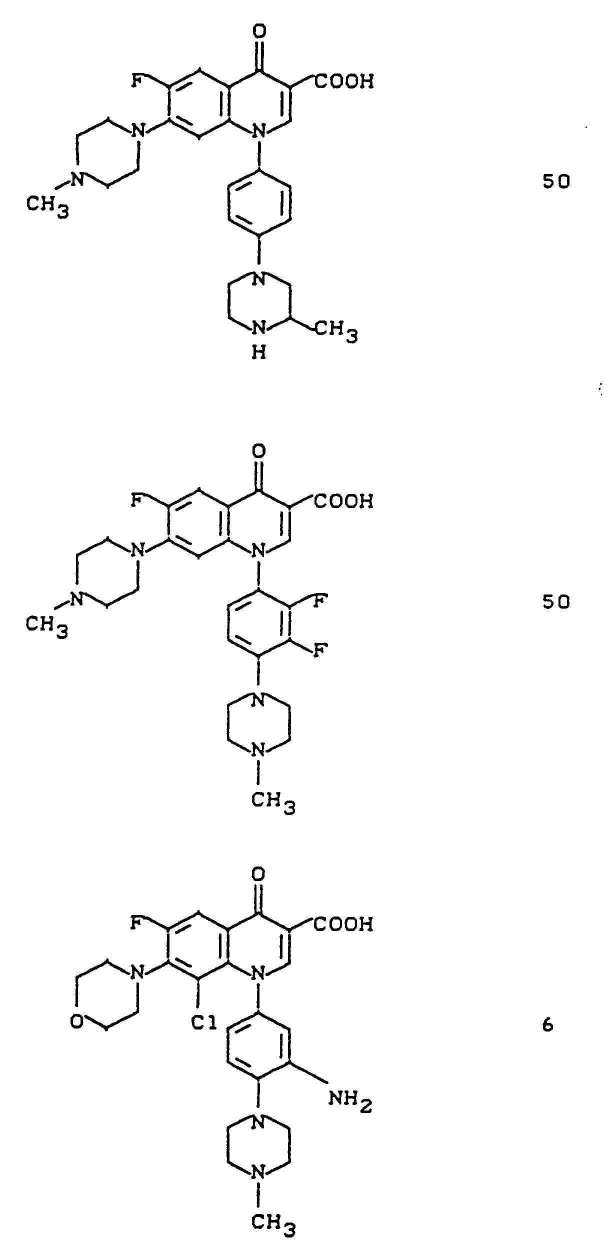

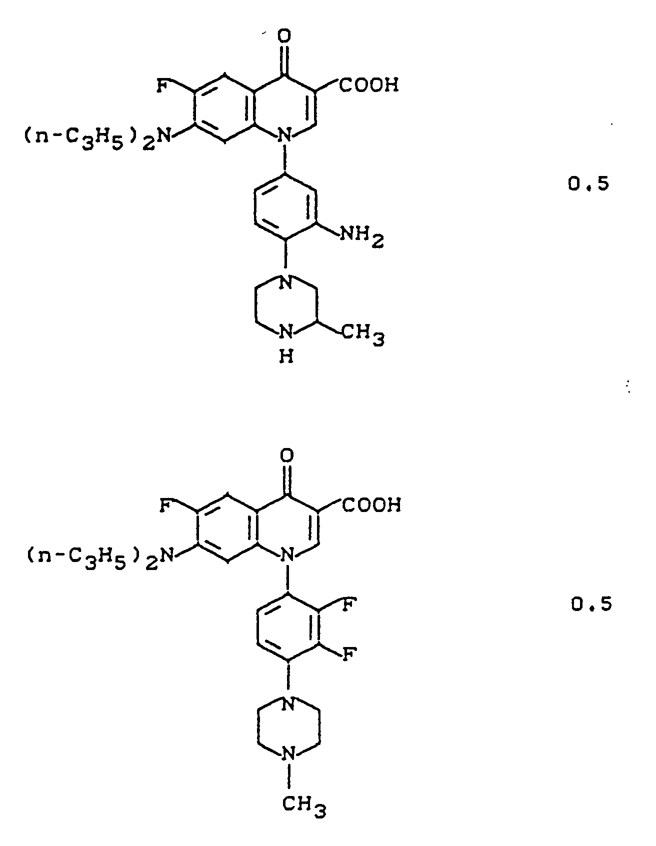

## Patentansprüche (Patentansprüche für folgende(n) Vertragsstaat(en): AT, BE, CH, DE, DK, FR, GB, IT, LI, LU, NL, SE)

1. Verbindungen der allgemeinen Formel (I) 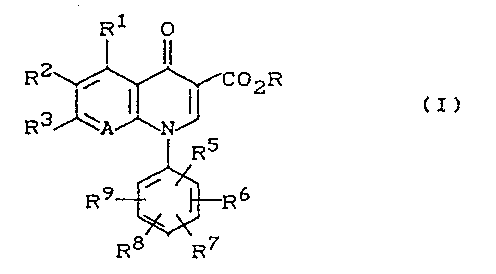 in der
R für Wasserstoff oder Alkyl mit 1-4 Kohlenstoffatomen,
R¹ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen, Cyano oder Nitro,
R² für Wasserstoff, Nitro oder Halogen steht,
A für N oder CR₄ steht, wobei
R⁴ für Wasserstoff, Halogen, Methyl, Cyano, Nitro, Methoxy oder Amin steht oder auch für eine über O, S oder CH₂ Brücke, die mit der ortho-Position des Phenylrings an N-1 verbunden ist, steht,
R³ und R⁵ gleich oder verschieden sind und für NR²⁵R²⁶ stehen, worin R²⁵ und R²⁶ gleich oder verschieden sind und für Wasserstoff, C₁-C₂₀-Alkyl, gegebenenfalls substituiert durch ein oder mehrere Halogene, OH, O-Alkyl (C₁-C₆), Alkyl (C₁-C₄), CN, COO Alkyl (C₁-C₆),
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl stehen, oder
R³ und R⁵ für eine Gruppe mit der Formel (II) 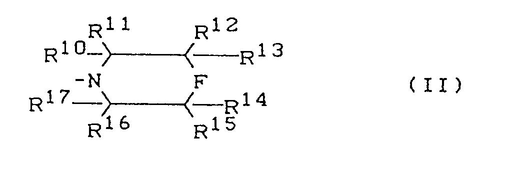 stehen, wobei
F O, S, NR¹⁸ oder CR¹⁹R²⁰ bedeutet und R⁶, R⁷, R⁸ und R⁹ gleich oder verschieden sind und für
- Wasserstoff,
- Methyl, Ethyl, Nitro, Amino, Monoalkylamino mit 1-3 C-Atomen, Dialkylamino mit 1-4 C-Ato men, Methoxy, Ethoxy, Thiomethyl, Thioethyl, Trifluormethyl, Cyano, Halogen stehen,
R¹⁸ für
- H, (C₁-C₁₀)-Alkyl oder (C₃-C₆)-Cycloalkyl, gegebenenfalls substituiert mit einem oder mehreren Substituenten Hal, OH, CN, COOR, OR mit R = (C₁-C₇)-Alkyl,
- Aryl(C₆-C₁₂), gegebenenfalls substituiert durch Halogen, -O-Alykl(C₁-C₂) oder (C₁-C₄)-Alkyl,
- CN
- COOAlkyl(C₁-C₄)
- C₁-C₄-Acyl oder
- (C₁-C₄)-Sulfonyl steht,
R¹⁰, R¹¹, R¹², R¹³, R¹⁴, R¹⁵, R¹⁶, R¹⁷, R¹⁹ und R²⁰ gleich oder verschieden sind und für
- Wasserstoff,
- C₁-C₃ Alkyl oder C₃-C₆-Cycloalkyl, gegebenenfalls substituiert durch ein oder mehrere Halogene, OH, O-Alkyl (C₁-C₆), Alkyl (C₁-C₄), CN, COO Alkyl (C₁-C₆),
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl stehen, oder
R³ und R⁵ für einen Rest der Struktur (IV) stehen, 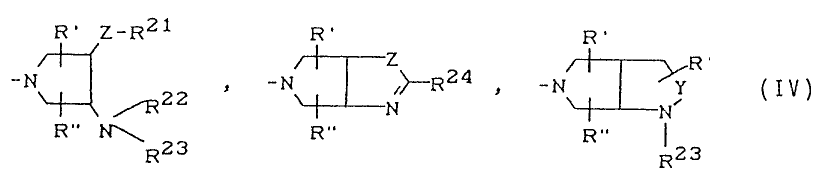 wobei
R²¹ für H, C₁-C₄-Alkyl, Aryl, C₁-C₄-Acyl,
R²² für H, C₁-C₄-Alkyl, OH, OCH₃, wobei R²¹ und R²² gemeinsam auch eine gegebenenfalls durch Methyl ein- oder zweifach substituierte C₁-C₃-Alkylenbrücke bedeuten kann,
R²³ für H, C₁-C₄-Alkyl, Aryl, Heteroaryl, Benzyl, C₁-C₄-Alkoxycarbonyl, C₁-C₄-Acyl oder (5-Methyl-2-oxo-1,3-dioxol-4-yl)methyl,
R²⁴ für H oder C₁-C₄-Alkyl,
R' für H, CH₃ oder Phenyl,
R'' für H, CH₃ oder Phenyl,
R''' für H oder CH₃,
Y für O, CH₂, CH₂CH₂ oder CH₂-O stehen kann, wobei die Verknüpfung der CH₂-O-Gruppe zum Stickstoff sowohl über O als auch über CH₂ erfolgen kann,
Z für O oder S stehen kann,
ZR²¹ für Wasserstoff stehen kann, wobei
R⁶, R⁷, R⁸ und R⁹ gleich oder verschieden sind und für
- Wasserstoff,
- Alkyl mit 1 bis 5-Kohlenstoffatomen, Nitro, Amino, Monoalkylamino mit 1-3 C-Atomen, Dialkylamino mit 1-6 C-Atomen, O-Alkyl mit 1-5 Kohlenstoffatomen, S-Alkyl mit 1-5 Kohlenstoffatomen, Trifluormethyl, Cyano, Halogen stehen,
mit Ausnahme von 1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und 1-(4-Aminophenyl)-6,8-difluor-7-morpholinyl-1,4-dihydro-4-oxo-3-chinolincarbonsäure und die pharmazeutisch vertretbaren Derivate dieser Verbindungen.

2. Verbindungen der allgemeinen Formel (I) gemäß Anspruch 1, 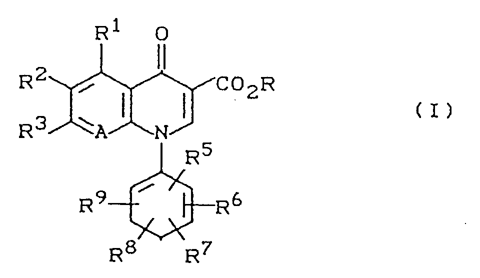 in der
R für Wasserstoff, Methyl oder Ethyl steht,
R¹ für Wasserstoff, Amino, Nitro, Methoxy, Methylthio, Cyano oder Halogen,
R² für Wasserstoff, Nitro oder Halogen steht,
A für N oder CR₄ steht, wobei
R⁴ für Wasserstoff, Halogen, Methyl, Cyano, Nitro, Methoxy oder Amin steht oder auch für eine über O, S oder CH₂ Brücke, die mit der ortho-Position des Phenylrings an N-1 verbunden ist, steht,
R³ und R⁵ gleich oder verschieden sind und für NR²⁵R²⁶ stehen, worin R²⁵ und R²⁶ gleich oder verschieden sind und für Wasserstoff, C₁-C₂₀-Alkyl, gegebenenfalls substituiert durch ein oder mehrere Halogene, O-Alkyl (C₁-C₆), Alkyl (C₁-C₄) je Alkylgruppe oder gegebenenfalls durch Halogen substituiertes Phenyl stehen, oder
R³ und R⁵ für eine Gruppe mit der Formel (II) 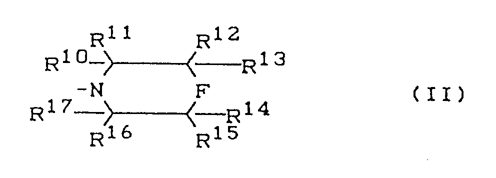 stehen, wobei
F Schwefel, Sauerstoff, NR¹⁸ oder CR¹⁹R²⁰ bedeutet
und
R¹⁸ für
- H, (C₁-C₁₀)-Alkyl oder (C₃-C₆)-Cycloalkyl, gegebenenfalls substituiert mit einem oder mehreren Substituenten Hal, OH, CN, COOR, OR mit R = (C₁-C₇)-Alkyl,
- Aryl (C₆-C₁₂), gegebenenfalls substituiert durch Halogen, -O-Alkyl(C₁-C₂) oder (C₁-C₄)-Alkyl,
- CN,
- COOAlkyl(C₁-C₄)
- C₁-C₄-Acyl oder
- C₁-C₄-Sulfonyl steht,
R¹⁰, R¹¹, R¹², R¹³, R¹⁴, R¹⁵, R¹⁶, R¹⁷, R¹⁹, R²⁰ gleich oder verschieden sind und für
- Wasserstoff,
- C₁-C₃ Alkyl oder C₃-C₆ Cycloalkyl, gegebenenfalls substituiert durch ein oder mehrere Halogene, OH, Methoxy, Ethoxy, Methyl, Ethyl, Cyano, CO₂Me,
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl, stehen, oder
R³ und R⁵ für einen Rest der Strukturen 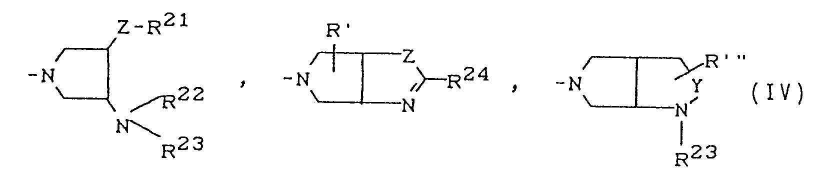 stehen,
wobei
R²¹ für H, C₁-C₄-Alkyl, Aryl, C₁-C₄-Acyl,
R²² für H, C₁-C₄-Alkyl, OH, OCH₃, wobei R²¹ und R²² gemeinsam auch eine gegebenenfalls durch Methyl ein- oder zweifach substituierte C₁-C₃-Alkylenbrücke bedeuten kann,
R²³ für H, C₁-C₄-Alkyl, Aryl, Heteroaryl, Benzyl, C₁-C₄-Alkoxycarbonyl, C₁-C₄-Acyl
R²⁴ für H oder C₁-C₄-Alkyl,
Y für O, CH₂, CH₂CH₂ oder CH₂-O stehen kann, wobei die Verknüpfung der CH₂-O-Gruppe zum Stickstoff sowohl über O als auch über CH₂ erfolgen kann,
Z für O oder S stehen kann,
ZR²¹ für Wasserstoff stehen kann, wobei
R⁶, R⁷, R⁸ und R⁹ gleich oder verschieden sind und für
- Wasserstoff,
- Methyl, Ethyl, Nitro, Amino, Monoalkylamino mit 1 - 3 C-Atomen, Dialkylamino mit 1 - 4 C-Atomen, Methoxy, Ethoxy, Thiomethyl, Thioethyl, Trifluormethyl, Cyano, Halogen stehen,
mit Ausnahme von 1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und 1-(4-Aminophenyl)-6,8-difluor-7-morpholinyl-1,4-dihydro-4-oxo-3-chinolincarbonsäure.
und die pharmazeutisch vertretbaren Derivate dieser Verbindungen.

3. Verbindungen der allgemeinen Formel (I) gemäß Anspruch 2, 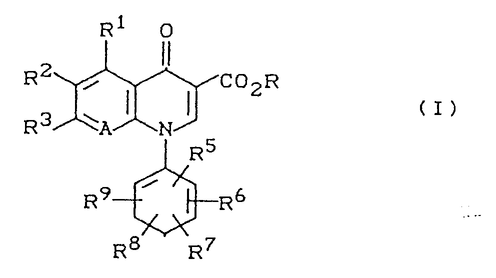 in der
R für Wasserstoff, Methyl oder Ethyl steht,
R¹ für Wasserstoff, Halogen,
R² für Fluor, Nitro oder Wasserstoff steht,
A für N oder CR₄ steht, wobei
R⁴ für Wasserstoff oder Halogen steht,
R³ und R⁵ gleich oder verschieden sind und für NR²⁵R²⁶ stehen, worin R²⁵ und R²⁶ gleich oder verschieden sind und für Wasserstoff, C₁-C₁₀-Alkyl, gegebenenfalls substituiert durch ein oder mehrere Halogene, O-Alkyl (C₁-C₆), Alkyl (C₁-C₄) je Alkylgruppe oder gegebenenfalls durch Halogen substituiertes Phenyl stehen, oder
R³ und R⁵ für eine Gruppe mit der Formel (II) 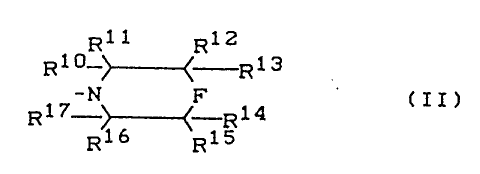 stehen, wobei
F Schwefel, Sauerstoff, NR¹⁸ oder CR¹⁹R²⁰ bedeutet und
R¹⁸ für
- Wasserstoff,
- Methyl, Ethyl, Propyl,
- (C₃-C₆) Cycloalkyl,
- Phenyl, gegebenenfalls substituiert durch Halogen,
- CN,
- COO-Alkyl (C₁-C₄)
- C₁-C₄-Acyl
- (C₁-C₄)-Sulfonyl steht und
R¹⁰, R¹¹, R¹⁷, R¹⁶ für Wasserstoff stehen, R¹², R¹³, R¹⁴, R¹⁵, R¹⁹, R²⁰ gleich oder verschieden sind und für
- Wasserstoff,
- C₁-C₃ Alkyl oder C₃-C₆ Cycloalkyl, gegebenenfalls substituiert durch ein oder mehrere Halogene, OH, Methoxy, Ethoxy, Methyl, Ethyl, Cyano, CO₂Me
- Hydroxyl,
- oder Amino, Alkylamino mit 1-4 Kohlenstoffatomen, Dialkylamino mit 1-3 Kohlenstoffatomen stehen,
R³ und R⁵ gleich oder verschieden sind und für eine Gruppe der Formel 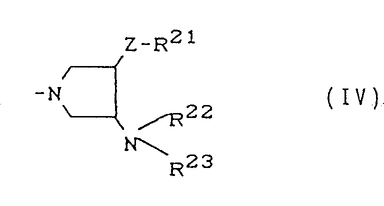 stehen, wobei
R²¹ für Wasserstoff, Methyl, Ethyl, Phenyl, C₁-C₄ Acyl,
R²² für Wasserstoff, Methyl, Ethyl, OH, OCH₃, wobei R²¹ und R²² gemeinsam auch durch eine durch Methyl ein- oder zweifach substituierte C₁-C₃-Alkylenbrücke bedeuten kann,
R²³ für H, Methyl, Ethyl, Aryl, Benzyl, C₁-C₄ Alkoxycarbonyl, C₁-C₄ Acyl stehen,
Z für O oder S stehen kann,
ZR²¹ für Wasserstoff stehen kann,
mit Ausnahme von 1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und 1-(4-Aminophenyl)-6,8-difluor-7-morpholinyl-1,4-dihydro-4-oxo-3-chinolincarbonsäure.
und die pharmazeutisch vertretbaren Derivate dieser Verbindungen.

4. Verfahren zur Herstellung von Verbindungen der Formel (I) gemäß Anspruch 1, in der R³ und R⁵ gleich sind, mit Ausnahme von
1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und
1-(4-Aminophenyl)-6,8-difluor-7-morpholinyl-1,4-dihydro-4-oxo-3-chinolincarbonsäure, dadurch gekennzeichnet, daß man Chinoline der allgemeinen Formel (III) 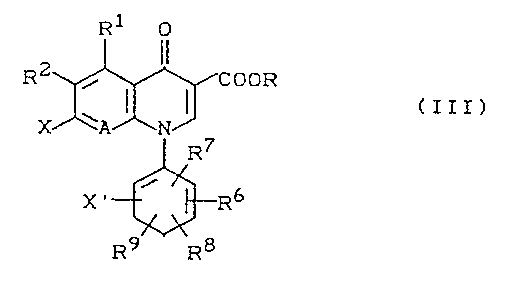 in der
A, R, R¹, R², R⁶, R⁷, R⁸ und R⁹ die in Anspruch 1 angegebene Bedeutung haben und X und X' gleich oder verschieden sind und für ein Halogenatom stehen, mit Aminen der allgemeinen Formeln (II) und (IV) 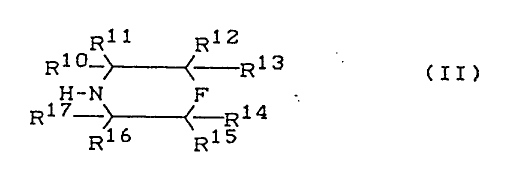 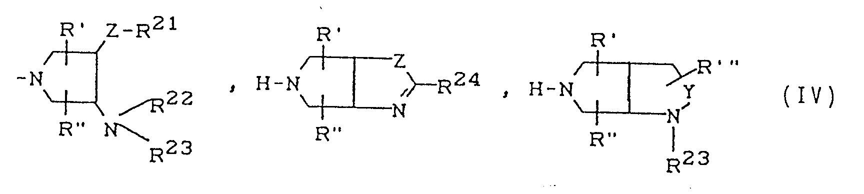 in denen
R¹⁰ bis R¹⁷, R²¹ bis R²⁴, Y, Z, R', R'', R''' und F die in Anspruch 1 angegebene Bedeutung haben, gegebenenfalls in Gegenwart von Verdünnungsmitteln und gegebenenfalls in Gegenwart von Säurebindern umsetzt,

5. Verfahren zur Herstellung der Verbindungen der Formel (I) gemäß Anspruch 1, mit Ausnahme von 1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und
1-(4-Aminophenyl)-6,8-difluor-7-morpholinyl-1,4-dihydro-4-oxo-3-chinolincarbonsäure, dadurch gekennzeichnet, daß man Verbindungen der allgemeinen Formel (V) 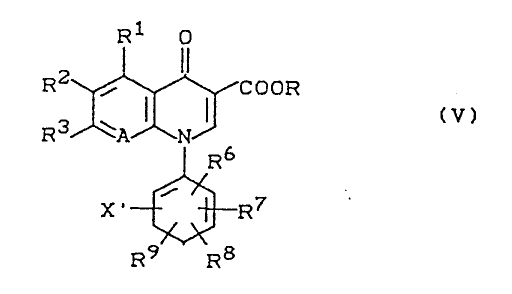 in der A, R, R¹ bis R⁹ die in Anspruch 1 angegebene Bedeutung haben und X' für ein Halogenatom steht, mit Aminen der allgemeinen Formeln (II) und (IV), 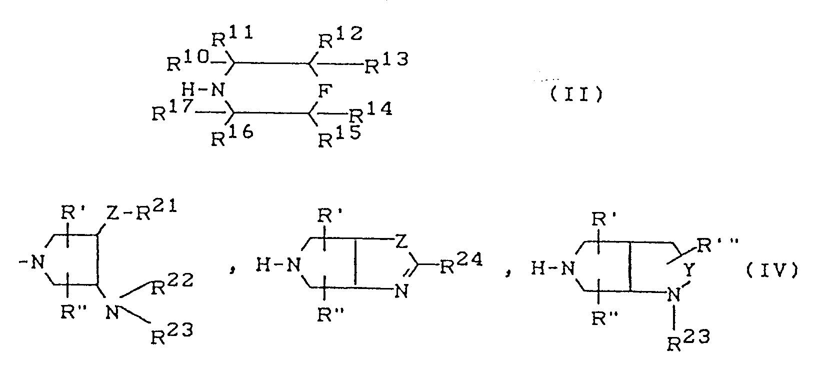 in denen
R¹⁰ bis R¹⁷, R²¹ bis R²⁴, R', R'' und R''', Y, Z und F die in Anspruch 1 angegebene Bedeutung haben, gegebenenfalls in Gegenwart von Verdünnungsmitteln und gegebenenfalls in Gegenwart von Säurebindern umsetzt.

6. Verfahren zur Herstellung der Verbindungen der Formel (I) gemäß Anspruch 1, mit Ausnahme von 1-(3-Methylamino-4-fluorphenyl)-6-fluor-7-(3-methylpiperazinyl)-1,4-dihydro-4-oxo-3-chinoiincarbonsäure, 1-(4-Aninophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und
1-(4-Aminophenyl)-6,8-difluor-7-morpholinyl-1,4-dihydro-4-oxo-3-chinolincarbonsäure, dadurch gekennzeichnet,
daß man Chinoline der allgemeinen Formel (VI) 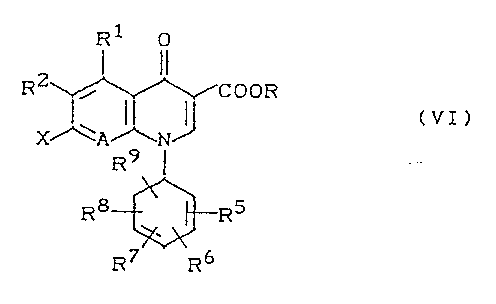 in der A, R, R¹, R², R⁵, R⁶, R⁷, R⁸, R⁹ die in Anspruch 1 angegebene Bedeutung haben und X für Halogen steht, mit Aminen der allgemeinen Formeln (II) und (IV), 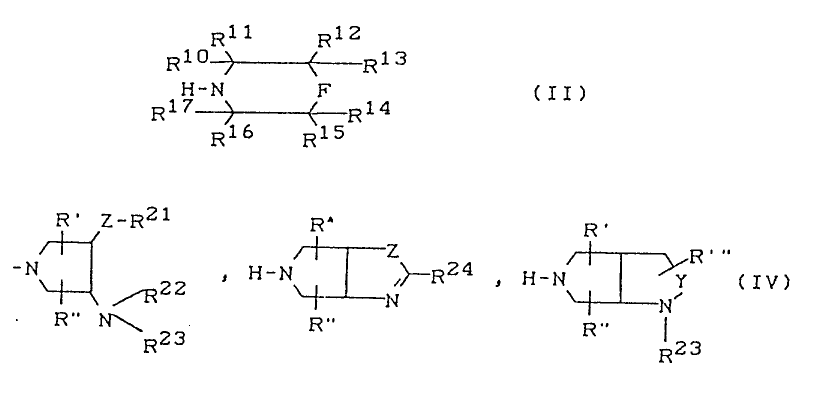 in denen
R¹⁰ bis R¹⁷, R²¹ bis R²⁴, R', R'' und R''', Y, Z und F die in Anspruch 1 angegebene Bedeutung haben,
gegebenenfalls in Gegenwart von Verdünnungsmitteln und gegebenenfalls in Gegenwart von Säurebindern umsetzt.

7. Verfahren zur Herstellung der Verbindungen der Formel (I) gemäß Anspruch 1, mit Ausnahme von 1-(3-Methylamino-4-fluorphenyl)-6-fluor-7-(3-methylpiperazinyl)-1,4-dihydro-4-oxo-3-chinolincarbonsäure und 1-(4-Aminophenyl)-6-fluor-7-(4-methylpiperazinyl)-1,4-dihydro-4-oxo-3-chinolincarbonsäure, dadurch gekennzeichnet, daß man Verbindungen der allgemeinen Formel (VII) 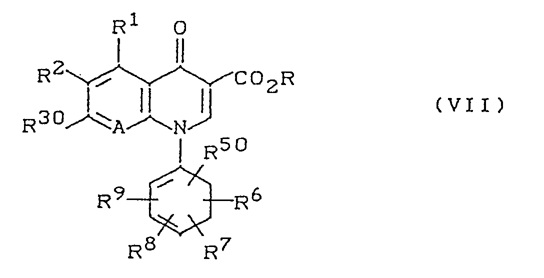 wobei R, R¹, R², A, R⁶ - R⁹ die in Anspruch 1 angegebene Bedeutung haben, die Reste R³⁰ und R⁵⁰ gleich oder verschieden sind, wobei in R³⁰ und R⁵⁰ oder nur in R³⁰ oder nur in R⁵⁰ sekundäre oder primäre Amine vorhanden sind und die Strukturen von R³⁰ und R⁵⁰ durch die Vertreter der Reste R³ bzw. R⁵ bestimmt sind,
mit Acylierungsmitteln oder Alkylierungsmitteln der allgemeinen Formel (VIII)
V - W (VIII)
in der
W = - CN
- (C₂-C₄)-Acyl, gegebenenfalls substi
tuiert - (C₁-C₄)-Sulfonyl
- (C₁-C₄)-Alkoxycarbonyl
- (C₁-C₅)-Alkyl
und V eine Abgangsgruppe darstellt, wie
V = - Halogen
- Trifluormethansulfonat
- Mesylat
- Tosylat
- Acetat
- Trihaloacetat
gegebenenfalls in Gegenwart von Verdünnungsmitteln und gegebenenfalls in Gegenwart von Säurebindern umgesetzt,

8. Verfahren zur Herstellung der Verbindungen der allgemeinen Formel (I) gemäß Anspruch 1, mit Ausnahme von 1-(3-Methylamino-4-fluorphenyl)-6-fluor-7-(3-methyl-piperazinyl)-1,4-dihydro-4-oxo-3-chinolincarbonsäure, dadurch gekennzeichnet, daß man Verbindungen der allgemeinen Formel (VII) nach Anspruch 7 mit Verbindungen der allgemeinen Formel (IX),
CH₂=CH-U (IX)
in der U eine elektronenanziehende Gruppe wie COOB, COB, CONH₂, CN, SO₂B, SO₃B bedeutet und B für (C₁-C₄)-Alkyl, das gegebenenfalls substituiert ist, steht,
gegebenenfalls in Gegenwart von Verdünnungsmitteln umsetzt.

9. Verfahren zur Herstellung der Verbindungen der Formel (I) gemäß Anspruch 1, mit Ausnahme von 1-(3-Methylamino-4-fluorphenyl)-6-fluor-7-(3-methylpiperazinyl)-1,4-dihydro-4-oxo-3-chinolincarbonsäure, dadurch gekennzeichnet, daß man Verbindungen der allgemeinen Formel (VII) nach Anspruch 7 mit Carbonsäuren der allgemeinen Formel (X)
D-COOH (X)
in der D für H oder gegebenenfalls substituiertes (C₁-C₄)-Alkyl steht, gegebenenfalls in Gegenwart von Verdünnungsmitteln und gegebenenfalls in Gegenwart wasserentziehender Reagenzien und gegebenenfalls in Gegenwart eines Katalysators umsetzt.

10. Arzneimittel, enthaltend Verbindungen gemäß Ansprüchen 1 bis 3.

11. Antivirale Mittel, enthaltend Verbindungen gemäß Ansprüchen 1 bis 3.

12. Verwendung von Verbindungen der allgemeinen Formel (I) 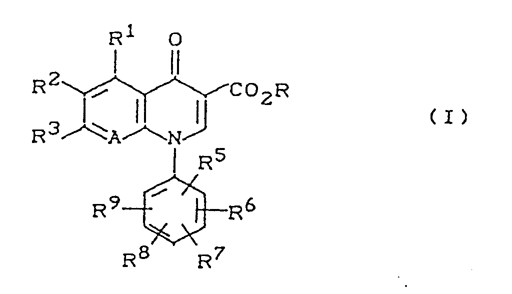 in der
R für Wasserstoff oder Alkyl mit 1-4 Kohlenstoffatomen,
R¹ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen, Cyano oder Nitro,
R² für Wasserstoff, Nitro oder Halogen steht,
A für N oder CR₄ steht, wobei
R⁴ für Wasserstoff, Halogen, Methyl, Cyano, Nitro, Methoxy oder Amin steht oder auch für eine über O, S oder CH₂ Brücke, die mit der ortho-Position des Phenylrings an N-1 verbunden ist, steht,
R³ und R⁵ gleich oder verschieden sind und für NR²⁵R²⁶ stehen, worin R²⁵ und R²⁶ gleich oder verschieden sind und für Wasserstoff, C₁-C₂₀-Alkyl, gegebenenfalls substituiert durch ein oder mehrere Halogene, OH, O-Alkyl (C₁-C₆), Alkyl (C₁-C₄), CN, COO Alkyl (C₁-C₆),
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl stehen, oder
R³ und R⁵ für eine Gruppe mit der Formel (II) 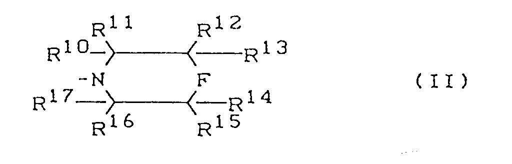 stehen, wobei
F O, S, NR¹⁸ oder CR¹⁹R²⁰ bedeutet und
R⁶, R⁷, R⁸ und R⁹ gleich oder verschieden sind und für
- Wasserstoff,
- Methyl, Ethyl, Nitro, Amino, Monoalkylamino mit 1-3 C-Atomen, Dialkylamino mit 1-4 C-Ato men, Methoxy, Ethoxy, Thiomethyl, Thioethyl, Trifluormethyl, Cyano, Halogen stehen,
R¹⁸ für
- H, (C₁-C₁₀)-Alkyl oder (C₃-C₆)-Cycloalkyl, gegebenenfalls substituiert mit einem oder mehreren Substituenten Hal, OH, CN, COOR, OR mit R = (C₁-C₇)-Alkyl,
- Aryl(C₆-C₁₂), gegebenenfalls substituiert durch Halogen, -O-Alykl(C₁-C₂) oder (C₁-C₄)-Alkyl,
- CN
- COOAlkyl(C₁-C₄)
- C₁-C₄-Acyl oder
- (C₁-C₄)-Sulfonyl steht,
R¹⁰, R¹¹, R¹², R¹³, R¹⁴, R¹⁵, R¹⁶, R¹⁷, R¹⁹ und R²⁰ gleich oder verschieden sind und für
- Wasserstoff,
- C₁-C₃ Alkyl oder C₃-C₆-Cycloalkyl, gegebenenfalls substituiert durch ein oder mehrere Halogene, OH, O-Alkyl (C₁-C₆), Alkyl (C₁-C₄), CN, COO Alkyl (C₁-C₆),
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl, oder
R³ und R⁵ für einen Rest der Struktur (IV) stehen, 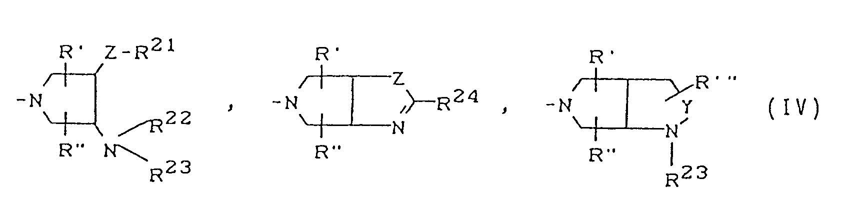 wobei
R²¹ für H, C₁-C₄-Alkyl, Aryl, C₁-C₄-Acyl,
R²² für H, C₁-C₄-Alkyl, OH, OCH₃, wobei R²¹ und R²² gemeinsam auch eine gegebenenfalls durch Methyl ein- oder zweifach substituierte C₁-C₃-Alkylenbrücke bedeuten kann,
R²³ für H, C₁-C₄-Alkyl, Aryl, Heteroaryl, Benzyl, C₁-C₄-Alkoxycarbonyl, C₁-C₄-Acyl oder (5-Methyl-2-oxo-1,3-dioxol-4-yl)methyl,
R²⁴ für H oder C₁-C₄-Alkyl,
R' für H, CH₃ oder Phenyl,
R'' für H, CH₃ oder Phenyl,
R''' für H oder CH₃,
Y für O, CH₂, CH₂CH₂ oder CH₂-O stehen kann, wobei die Verknüpfung der CH₂-O-Gruppe zum Stickstoff sowohl über O als auch über CH₂ erfolgen kann,
Z für O oder S stehen kann,
ZR²¹ für Wasserstoff stehen kann, wobei
R⁶, R⁷, R⁸ und R⁹ gleich oder verschieden sind und für
- Wasserstoff,
- Alkyl mit 1 bis 5-Kohlenstoffatomen, Nitro, Amino, Monoalkylamino mit 1-3 C-Atomen, Dialkylamino mit 1-6 C-Atomen, O-Alkyl mit 1-5 Kohlenstoffatomen, S-Alkyl mit 1-5 Kohlenstoffatomen, Trifluormethyl, Cyano, Halogen stehen,
und die pharmazeutisch vertretbaren Derivate dieser Verbindungen zur Herstellung eines Arzneimittels für Anwendung in der Antiviralen Therapie des menschlichen oder tierischen Körpers.

13. Verwendung von Verbindungen gemäß Anspruch 12 der allgemeinen Formel (I) 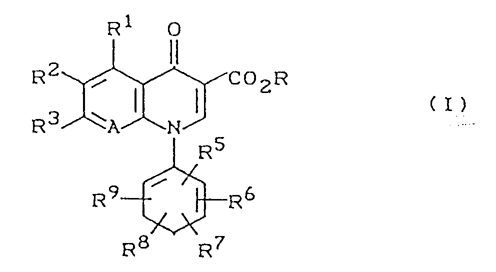 in der
R für Wasserstoff, Methyl oder Ethyl steht,
R¹ für Wasserstoff, Amino, Nitro, Methoxy, Methylthio, Cyano oder Halogen,
R² für Wasserstoff, Nitro oder Halogen steht,
A für N oder CR₄ steht, wobei
R⁴ für Wasserstoff, Halogen, Methyl, Cyano, Nitro, Methoxy oder Amin steht oder auch für eine über O, S oder CH₂ Brücke, die mit der ortho-Position des Phenylrings an N-1 verbunden ist, steht,
R³ und R⁵ gleich oder verschieden sind und für NR²⁵R²⁶ stehen, worin R²⁵ und R²⁶ gleich oder verschieden sind und Wasserstoff, C₁-C₂₀-Alkyl, gegebenenfalls substituiert durch ein oder mehrere Halogene, O-Alkyl (C₁-C₆), Alkyl (C₁-C₄) je Alkylgruppe oder gegebenenfalls durch Halogen substituiertes Phenyl stehen, oder
R³ und R⁵ für eine Gruppe mit der Formel (II) 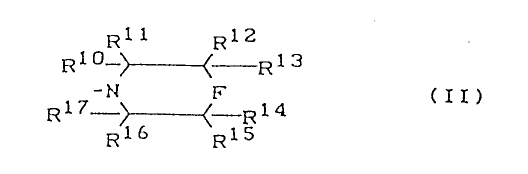 stehen, wobei
F Schwefel, Sauerstoff, NR¹⁸ oder CR¹⁹R²⁰ bedeutet
und
R¹⁸ für
- H, (C₁-C₁₀)-Alkyl oder (C₃-C₆)-Cycloalkyl, gegebenenfalls substituiert mit einem oder mehreren Substituenten Hal, OH, CN, COOR, OR mit R = (C₁-C₇)-Alkyl,
- Aryl(C₆-C₁₂), gegebenenfalls substituiert durch Halogen, -O-Alkyl(C₁-C₂) oder (C₁-C₄)-Alkyl,
- CN,
- COOAlkyl(C₁-C₄)
- C₁-C₄-Acyl oder
- C₁-C₄-Sulfonyl steht,
R¹⁰, R¹¹, R¹², R¹³, R¹⁴, R¹⁵, R¹⁶, R¹⁷, R¹⁹, R²⁰ gleich oder verschieden sind und für
- Wasserstoff,
- C₁-C₃ Alkyl oder C₃-C₆ Cycloalkyl, gegebenenfalls substituiert durch ein oder mehrere Halogene, OH, Methoxy, Ethoxy, Methyl, Ethyl, Cyano, CO₂Me,
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl,
stehen, oder
R³ und R⁵ für einen Rest der Strukturen 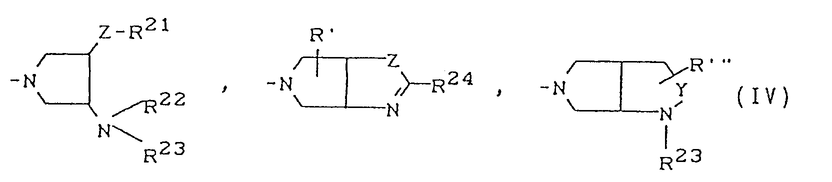 stehen,
wobei
R²¹ für H, C₁-C₄-Alkyl, Aryl, C₁-C₄-Acyl,
R²² für H, C₁-C₄-Alkyl, OH, OCH₃, wobei R²¹ und R²² gemeinsam auch eine gegebenenfalls durch Methyl ein- oder zweifach substituierte C₁-C₃-Alkylenbrücke bedeuten kann,
R²³ für H, C₁-C₄-Alkyl, Aryl, Heteroaryl, Benzyl, C₁-C₄-Alkoxycarbonyl, C₁-C₄-Acyl
R²⁴ für H oder C₁-C₄-Alkyl,
Y für O, CH₂, CH₂CH₂ oder CH₂-O stehen kann, wobei die Verknüpfung der CH₂-O-Gruppe zum Stickstoff sowohl über O als auch über CH₂ erfolgen kann,
Z für O oder S stehen kann,
ZR²¹ für Wasserstoff stehen kann, wobei R⁶, R⁷, R⁸ und R⁹ gleich oder verschieden sind und für
- Wasserstoff,
- Methyl, Ethyl, Nitro, Amino, Monoalkylamino mit 1 - 3 C-Atomen, Dialkylamino mit 1 - 4 C-Atomen, Methoxy, Ethoxy, Thiomethyl, Thioethyl, Trifluormethyl, Cyano, Halogen stehen,
und die pharmazeutisch vertretbaren Derivate dieser Verbindungen zur Herstellung eines Arzneimittels für Anwendung in der antiviralen Therapie des menschlichen oder tierischen Körpers.

14. Verwendung von Verbindungen gemäß Anspruch 12 der allgemeinen Formel (I) 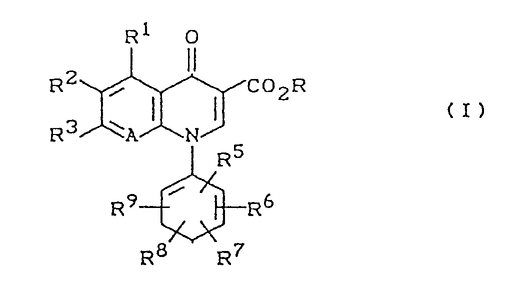 in der
R für Wasserstoff, Methyl oder Ethyl steht,
R¹ für Wasserstoff, Halogen,
R² für Fluor, Nitro oder Wasserstoff steht,
A für N oder CR₄ steht, wobei
R⁴ für Wasserstoff oder Halogen steht,
R³ und R⁵ gleich oder verschieden sind und für NR²⁵R²⁶ stehen, worin R²⁵ und R²⁶ gleich oder verschieden sind und für Wasserstoff, C₁-C₁₀-Alkyl, gegebenenfalls substituiert durch ein oder mehrere Halogene, O-Alkyl (C₁-C₆), Alkyl (C₁-C₄) je Alkylgruppe oder gegebenenfalls durch Halogen substituiertes Phenyl stehen, oder
R³ und R⁵ für eine Gruppe mit der Formel (II) 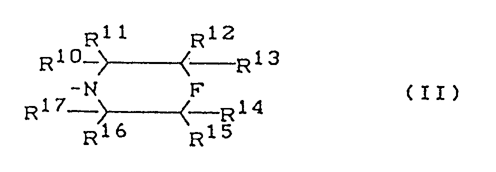 stehen, wobei
F Schwefel, Sauerstoff, NR¹⁸ oder CR¹⁹R²⁰ bedeutet
und
R¹⁸ für
- Wasserstoff,
- Methyl, Ethyl, Propyl,
- (C₃-C₆) Cycloalkyl,
- Phenyl, gegebenenfalls substituiert durch Halogen,
- CN,
- COO-Alkyl (C₁-C₄)
- C₁-C₄-Acyl
- (C₁-C₄)-Sulfonyl steht und
R¹⁰, R¹¹, R¹⁷, R¹⁶ für Wasserstoff stehen, R¹², R¹³, R¹⁴, R¹⁵, R¹⁹, R²⁰ gleich oder verschieden sind und für
- Wasserstoff,
- C₁-C₃ Alkyl oder C₃-C₆ Cycloalkyl, gegebenenfalls substituiert durch ein oder mehrere Halogene, OH, Methoxy, Ethoxy, Methyl, Ethyl, Cyano, CO₂Me
- Hydroxyl,
- oder Amino, Alkylamino mit 1-4 Kohlenstoffatomen, Dialkylamino mit 1-3 Kohlenstoffatomen stehen,
R³ und R⁵ gleich oder verschieden sind und für eine Gruppe der Formel 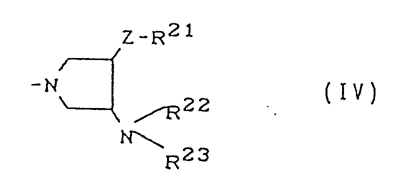 stehen,
wobei
R²¹ für Wasserstoff, Methyl, Ethyl, Phenyl, C₁-C₄ Acyl,
R²² für Wasserstoff, Methyl, Ethyl, OH, OCH₃, wobei R²¹ und R²² gemeinsam auch durch eine durch Methyl ein- oder zweifach substituierte C₁-C₃-Alkylenbrücke bedeuten kann,
R²³ für H, Methyl, Ethyl, Aryl, Benzyl, C₁-C₄ Alkoxycarbonyl, C₁-C₄ Acyl stehen,
Z für O oder S stehen kann,
ZR²¹ für Wasserstoff stehen kann,
und die pharmazeutisch vertretbaren Derivate dieser Verbindungen zur Herstellung eines Arzneimittels für Anwendung in der antiviralen Therapie des menschlichen oder tierischen Körpers.

## Patentansprüche (Patentansprüche für folgende(n) Vertragsstaat(en): ES)

1. Verfahren zur Herstellung von Verbindungen der allgemeinen Formel (I) 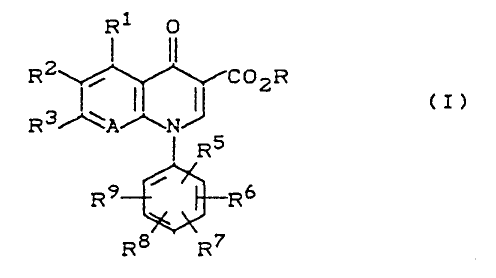 in der
R für Wasserstoff oder Alkyl mit 1-4 Kohlenstoffatomen,
R¹ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen, Cyano oder Nitro,
R² für Wasserstoff, Nitro oder Halogen steht,
A für N oder CR₄ steht, wobei
R⁴ für Wasserstoff, Halogen, Methyl, Cyano, Nitro, Methoxy oder Amin steht oder auch für eine über O, S oder CH₂ Brücke, die mit der ortho-Position des Phenylrings an N-1 verbunden ist, steht,
R³ und R⁵ gleich oder verschieden sind und für NR²⁵R²⁶ stehen, worin R²⁵ und R²⁶ gleich oder verschieden sind und für Wasserstoff, C₁-C₂₀-Alkyl, gegebenenfalls substituiert durch ein oder mehrere Halogene, OH, O-Alkyl (C₁-C₆), Alkyl (C₁-C₄), CN, COO Alkyl (C₁-C₆),
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl stehen, oder
R³ und R⁵ für eine Gruppe mit der Formel (II) 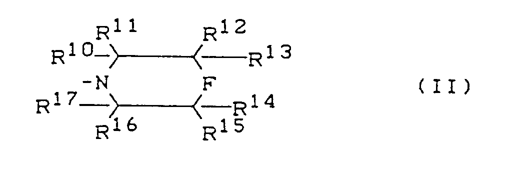 stehen, wobei
F O, S, NR¹⁸ oder CR¹⁹R²⁰ bedeutet und
R⁶, R⁷, R⁸ und R⁹ gleich oder verschieden sind und für
- Wasserstoff,
- Methyl, Ethyl, Nitro, Amino, Monoalkylamino mit 1-3 C-Atomen, Dialkylamino mit 1-4 C-Ato men, Methoxy, Ethoxy, Thiomethyl, Thioethyl, Trifluormethyl, Cyano, Halogen stehen,
R¹⁸ für
- H, (C₁-C₁₀)-Alkyl oder (C₃-C₆)-Cycloalkyl, gegebenenfalls substituiert mit einem oder mehreren Substituenten Hal, OH, CN, COOR, OR mit R = (C₁-C₇)-Alkyl,
- Aryl(C₆-C₁₂), gegebenenfalls substituiert durch Halogen, -O-Alykl(C₁-C₂) oder (C₁-C₄)-Alkyl,
- CN
- COOAlkyl(C₁-C₄)
- C₁-C₄-Acyl oder
- (C₁-C₄)-Sulfonyl steht,
R¹⁰, R¹¹, R¹², R¹³, R¹⁴, R¹⁵, R¹⁶, R¹⁷, R¹⁹ und R²⁰ gleich oder verschieden sind und für
- Wasserstoff,
- C₁-C₃ Alkyl oder C₃-C₆-Cycloalkyl, gegebenenfalls substituiert durch ein oder mehrere Halogene, OH, O-Alkyl (C₁-C₆), Alkyl (C₁-C₄), CN, COO Alkyl (C₁-C₆),
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl stehen, oder
R³ und R⁵ für einen Rest der Struktur (IV) stehen, 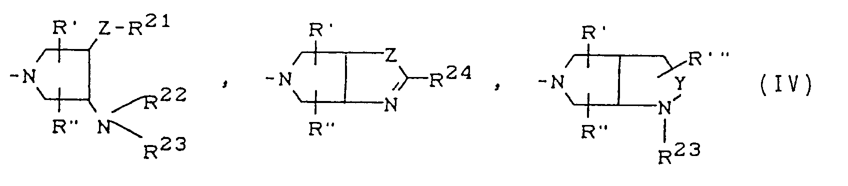 wobei
R²¹ für H, C₁-C₄-Alkyl, Aryl, C₁-C₄-Acyl,
R²² für H, C₁-C₄-Alkyl, OH, OCH₃, wobei R²¹ und R²² gemeinsam auch eine gegebenenfalls durch Methyl ein- oder zweifach substituierte C₁-C₃-Alkylenbrücke bedeuten kann,
R²³ für H, C₁-C₄-Alkyl, Aryl, Heteroaryl, Benzyl, C₁-C₄-Alkoxycarbonyl, C₁-C₄-Acyl oder (5-Methyl-2-oxo-1,3-dioxol-4-yl)-methyl,
R²⁴ für H oder C₁-C₄-Alkyl,
R' für H, CH₃ oder Phenyl,
R'' für H, CH₃ oder Phenyl,
R''' für H oder CH₃,
Y für O, CH₂, CH₂CH₂ oder CH₂-O stehen kann, wobei die Verknüpfung der CH₂-O-Gruppe zum Stickstoff sowohl über O als auch über CH₂ erfolgen kann,
Z für O oder S stehen kann,
ZR²¹ für Wasserstoff stehen kann, wobei
R⁶, R⁷, R⁸ und R⁹ gleich oder verschieden sind und für
- Wasserstoff,
- Alkyl mit 1 bis 5-Kohlenstoffatomen, Nitro, Amino, Monoalkylamino mit 1-3 C-Atomen, Dialkylamino mit 1-6 C-Atomen, O-Alkyl mit 1-5 Kohlenstoffatomen, S-Alkyl mit 1-5 Kohlenstoffatomen, Trifluormethyl, Cyano, Halogen stehen,
mit Ausnahme von 1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und
1-(4-Aminophenyl)-6,8-difluor-7-morpholinyl-1,4-dihydro-4-oxo-3-chinolincarbonsäure
und die pharmazeutisch vertretbaren Derivate dieser Verbindungen, dadurch gekennzeichnet, daß man Chinoline der allgemeinen Formel (VI) 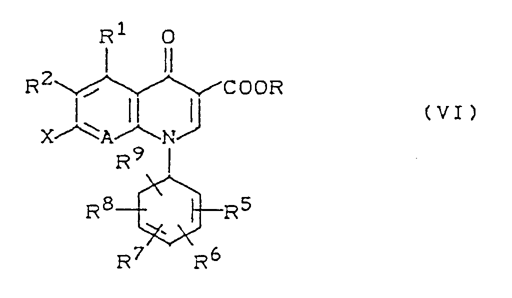 in der A, R, R¹, R², R⁵, R⁶, R⁷, R⁸, R⁹ die oben angegebene Bedeutung haben und X für Halogen steht, mit Aminen der allgemeinen Formeln (II) und (IV), 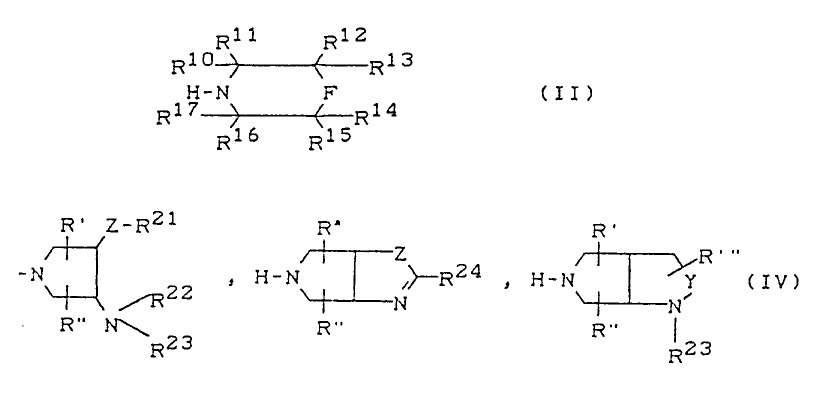 in denen
R¹⁰ bis R¹⁷, R²¹ bis R²⁴, R', R'' und R''', Y, Z und F die in Anspruch 1 angegebene Bedeutung haben,
gegebenenfalls in Gegenwart von Verdünnungsmitteln und gegebenenfalls in Gegenwart von Säurebindern umsetzt, und die so erhaltenen Verbindungen gegebenenfalls auf an sich bekannte Weise in pharmazeutisch vertretbare Derivate überführt.

2. Verfahren zur Herstellung der Verbindungen der Formel (I) gemäß Anspruch 1, mit Ausnahme von 1-(3-Methylamino-4-fluorphenyl)-6-fluor-7-(3-methylpiperazinyl)-1,4-dihydro-4-oxo-3-chinolincarbonsäure und 1-(4-Aminophenyl)-6-fluor-7-(4-methylpiperazinyl)-1,4-dihydro-4-oxo-3-chinolincarbonsäure, dadurch gekennzeichnet, daß man Verbindungen der allgemeinen Formel (VII) 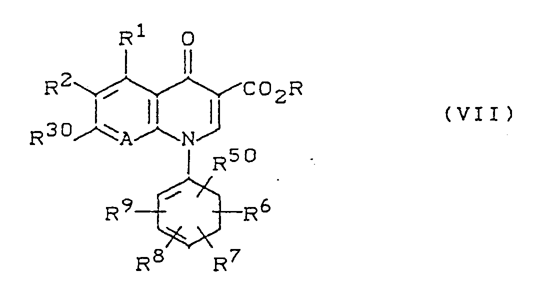 wobei R, R¹, R², A, R⁶ - R⁹ die in Anspruch 1 angegebene Bedeutung haben, die Reste R³⁰ und R⁵⁰ gleich oder verschieden sind, wobei in R³⁰ und R⁵⁰ oder nur in R³⁰ oder nur in R⁵⁰ sekundäre oder primäre Amine vorhanden sind und die Strukturen von R³⁰ und R⁵⁰ durch die Vertreter der Reste R³ bzw. R⁵ bestimmt sind,
mit Acylierungsmitteln oder Alkylierungsmitteln der allgemeinen Formel (VIII)
V - W (VIII)
in der
W = - CN
- (C₂-C₄)-Acyl, gegebenenfalls substituiert
- (C₁-C₄)-Sulfonyl
- (C₁-C₄)-Alkoxycarbonyl
- (C₁-C₅)-Alkyl
und V eine Abgangsgruppe darstellt, wie
V = - Halogen
- Trifluormethansulfonat
- Mesylat
- Tosylat
- Acetat
- Trihaloacetat
gegebenenfalls in Gegenwart von Verdünnungsmitteln und gegebenenfalls in Gegenwart von Säurebindern umgesetzt, und die so erhaltenen Verbindungen gegebenenfalls auf an sich bekannte Weise in pharmazeutisch vertretbare Derivate überführt.

3. Verfahren zur Herstellung der Verbindungen der allgemeinen Formel (I) gemäß Anspruch 1, mit Ausnahme von 1-(3-Methylamino-4-fluorphenyl)-6-fluor-7-(3-methyl-piperazinyl)-1,4-dihydro-4-oxo-3-chinolincarbonsäure, dadurch gekennzeichnet, daß man Verbindungen der allgemeinen Formel (VII) nach Anspruch 2 mit Verbindungen der allgemeinen Formel (IX),
CH₂=CH-U (IX)
in der U eine elektronenanziehende Gruppe wie COOB, COB, CONH₂, CN, SO₂B, SO₃B bedeutet und B für (C₁-C₄)-Alkyl, das gegebenenfalls substituiert ist, steht,
gegebenenfalls in Gegenwart von Verdünnungsmitteln umsetzt,

4. Verfahren zur Herstellung der Verbindungen der Formel (I) gemäß Anspruch 1, mit Ausnahme von 1-(3-Methylamino-4-fluorphenyl)-6-fluor-7-(3-methylpiperazinyl)-1,4-dihydro-4-oxo-3-chinolincarbonsäure, dadurch gekennzeichnet, daß man Verbindungen der allgemeinen Formel (VII) nach Anspruch 2 mit Carbonsäuren der allgemeinen Formel (X)
D-COOH (X)
in der D für H oder gegebenenfalls substituiertes (C₁-C₄)-Alkyl steht, gegebenenfalls in Gegenwart von Verdünnungsmitteln und gegebenenfalls in Gegenwart wasserentziehender Reagenzien und gegebenenfalls in Gegenwart eines Katalysators umsetzt.

## Patentansprüche (Patentansprüche für folgende(n) Vertragsstaat(en): GR)

1. Verbindungen der allgemeinen Formel (I) 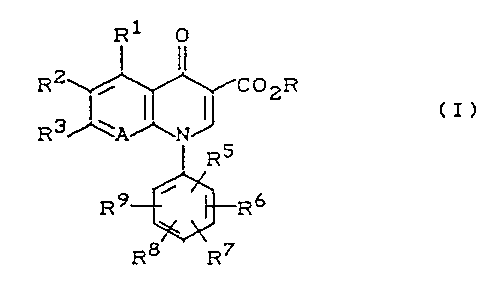 in der
R für Wasserstoff oder Alkyl mit 1-4 Kohlenstoffatomen,
R¹ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen, Cyano oder Nitro,
R² für Wasserstoff, Nitro oder Halogen steht,
A für N oder CR₄ steht, wobei
R⁴ für Wasserstoff, Halogen, Methyl, Cyano, Nitro, Methoxy oder Amin steht oder auch für eine über O, S oder CH₂ Brücke, die mit der ortho-Position des Phenylrings an N-1 verbunden ist, steht,
R³ und R⁵ gleich oder verschieden sind und für NR²⁵R²⁶ stehen, worin R²⁵ und R²⁶ gleich oder verschieden sind und für Wasserstoff, C₁-C₂₀-Alkyl, gegebenenfalls substituiert durch ein oder mehrere Halogene, OH, O-Alkyl (C₁-C₆), Alkyl (C₁-C₄), CN, COO Alkyl (C₁-C₆),
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl stehen, oder
R³ und R⁵ für eine Gruppe mit der Formel (II) 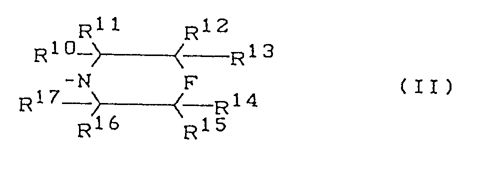 stehen, wobei
F O, S, NR¹⁸ oder CR¹⁹R²⁰ bedeutet und
R⁶, R⁷, R⁸ und R⁹ gleich oder verschieden sind und für
- Wasserstoff,
- Methyl, Ethyl, Nitro, Amino, Monoalkylamino mit 1-3 C-Atomen, Dialkylamino mit 1-4 C-Ato men, Methoxy, Ethoxy, Thiomethyl, Thioethyl, Trifluormethyl, Cyano, Halogen stehen,
R¹⁸ für
- H, (C₁-C₁₀)-Alkyl oder (C₃-C₆)-Cycloalkyl, gegebenenfalls substituiert mit einem oder mehreren Substituenten Hal, OH, CN, COOR, OR mit R = (C₁-C₇)-Alkyl,
- Aryl(C₆-C₁₂), gegebenenfalls substituiert durch Halogen, -O-Alykl(C₁-C₂) oder (C₁-C₄)-Alkyl,
- CN
- COOAlkyl(C₁-C₄)
- C₁-C₄-Acyl oder
- (C₁-C₄)-Sulfonyl steht,
R¹⁰, R¹¹, R¹², R¹³, R¹⁴, R¹⁵, R¹⁶, R¹⁷, R¹⁹ und R²⁰ gleich oder verschieden sind und für
- Wasserstoff,
- C₁-C₃ Alkyl oder C₃-C₆-Cycloalkyl, gegebenenfalls substituiert durch ein oder mehrere Halogene, OH, O-Alkyl (C₁-C₆), Alkyl (C₁-C₄), CN, COO Alkyl (C₁-C₆),
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl stehen, oder
R³ und R⁵ für einen Rest der Struktur (IV) stehen, 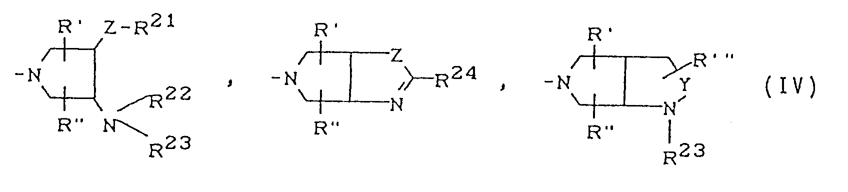 wobei
R²¹ für H, C₁-C₄-Alkyl, Aryl, C₁-C₄-Acyl,
R²² für H, C₁-C₄-Alkyl, OH, OCH₃, wobei R²¹ und R²² gemeinsam auch eine gegebenenfalls durch Methyl ein- oder zweifach substituierte C₁-C₃-Alkylenbrücke bedeuten kann,
R²³ für H, C₁-C₄-Alkyl, Aryl, Heteroaryl, Benzyl, C₁-C₄-Alkoxycarbonyl, C₁-C₄-Acyl oder (5-Methyl-2-oxo-1,3-dioxol-4-yl)methyl,
R²⁴ für H oder C₁-C₄-Alkyl,
R' für H, CH₃ oder Phenyl,
R'' für H, CH₃ oder Phenyl,
R''' für H oder CH₃,
Y für O, CH₂, CH₂CH₂ oder CH₂-O stehen kann, wobei die Verknüpfung der CH₂-O-Gruppe zum Stickstoff sowohl über O als auch über CH₂ erfolgen kann,
Z für O oder S stehen kann,
ZR²¹ für Wasserstoff stehen kann, wobei
R⁶, R⁷, R⁸ und R⁹ gleich oder verschieden sind und für
- Wasserstoff,
- Alkyl mit 1 bis 5-Kohlenstoffatomen, Nitro, Amino, Monoalkylamino mit 1-3 C-Atomen, Dialkylamino mit 1-6 C-Atomen, O-Alkyl mit 1-5 Kohlenstoffatomen, S-Alkyl mit 1-5 Kohlenstoffatomen, Trifluormethyl, Cyano, Halogen stehen,
mit Ausnahme von
1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und
1-(4-Aminophenyl)-6,8-difluor-7-morpholinyl-1,4-dihydro-4-oxo-3-chinolincarbonsäure
und die pharmazeutisch vertretbaren Derivate dieser Verbindungen.

2. Verbindungen der allgemeinen Formel (I) gemäß Anspruch 1, 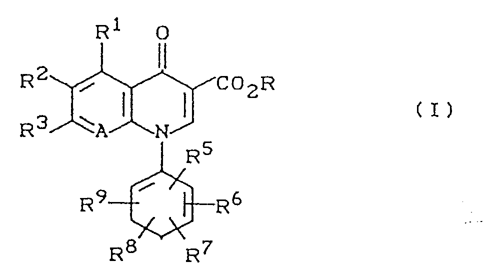 in der
R für Wasserstoff, Methyl oder Ethyl steht,
R¹ für Wasserstoff, Amino, Nitro, Methoxy, Methylthio, Cyano oder Halogen,
R² für Wasserstoff, Nitro oder Halogen steht,
A für N oder CR₄ steht, wobei
R⁴ für Wasserstoff, Halogen, Methyl, Cyano, Nitro, Methoxy oder Amin steht oder auch für eine über O, S oder CH₂ Brücke, die mit der ortho-Position des Phenylrings an N-1 verbunden ist, steht,
R³ und R⁵ gleich oder verschieden sind und für NR²⁵R²⁶ stehen, worin R²⁵ und R²⁶ gleich oder verschieden sind und für Wasserstoff, C₁-C₂₀-Alkyl, gegebenenfalls substituiert durch ein oder mehrere Halogene, O-Alkyl (C₁-C₆), Alkyl (C₁-C₄) je Alkylgruppe oder gegebenenfalls durch Halogen substituiertes Phenyl stehen, oder
R³ und R⁵ für eine Gruppe mit der Formel (II) 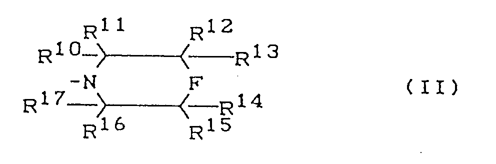 stehen, wobei
F Schwefel, Sauerstoff, NR¹⁸ oder CR¹⁹R²⁰ bedeutet
und
R¹⁸ für
- H, (C₁-C₁₀)-Alkyl oder (C₃-C₆)-Cycloalkyl, gegebenenfalls substituiert mit einem oder mehreren Substituenten Hal, OH, CN, COOR, OR mit R = (C₁-C₇)-Alkyl,
- Aryl(C₆-C₁₂), gegebenenfalls substituiert durch Halogen, -O-Alkyl(C₁-C₂) oder (C₁-C₄)-Alkyl,
- CN,
- COOAlkyl(C₁-C₄)
- C₁-C₄-Acyl oder
- C₁-C₄-Sulfonyl steht,
R¹⁰, R¹¹, R¹², R¹³, R¹⁴, R¹⁵, R¹⁶, R¹⁷, R¹⁹, R²⁰ gleich oder verschieden sind und für
- Wasserstoff,
- C₁-C₃ Alkyl oder C₃-C₆ Cycloalkyl, gegebenenfalls substituiert durch ein oder mehrere Halogene, OH, Methoxy, Ethoxy, Methyl, Ethyl, Cyano, CO₂Me,
- Hydroxyl,
- oder Amino, Alkylamino mit 1-4 Kohlenstoffatomen, Dialkylamino mit 1-3 Kohlenstoffatomen je Alkylgruppe oder gegebenenfalls durch Halogen substituiertes Phenyl,
stehen, oder
R³ und R⁵ für einen Rest der Strukturen 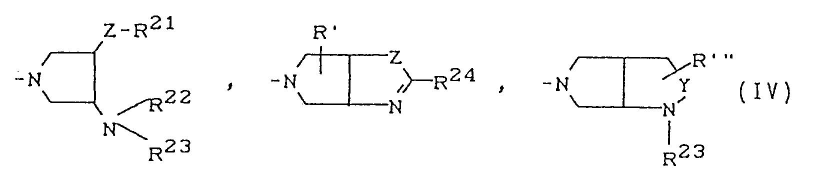 stehen,
wobei
R²¹ für H, C₁-C₄-Alkyl, Aryl, C₁-C₄-Acyl,
R²² für H, C₁-C₄-Alkyl, OH, OCH₃, wobei R²¹ und R²² gemeinsam auch eine gegebenenfalls durch Methyl ein- oder zweifach substituierte C₁-C₃-Alkylenbrücke bedeuten kann,
R²³ für H, C₁-C₄-Alkyl, Aryl, Heteroaryl, Benzyl, C₁-C₄-Alkoxycarbonyl, C₁-C₄-Acyl
R²⁴ für H oder C₁-C₄-Alkyl,
Y für O, CH₂, CH₂CH₂ oder CH₂-O stehen kann, wobei die Verknüpfung der CH₂-O-Gruppe zum Stickstoff sowohl über O als auch über CH₂ erfolgen kann,
Z für O oder S stehen kann,
ZR²¹ für Wasserstoff stehen kann, wobei
R⁶, R⁷, R⁸ und R⁹ gleich oder verschieden sind und für
- Wasserstoff,
- Methyl, Ethyl, Nitro, Amino, Monoalkylamino mit 1 - 3 C-Atomen, Dialkylamino mit 1 - 4 C-Atomen, Methoxy, Ethoxy, Thiomethyl, Thioethyl, Trifluormethyl, Cyano, Halogen stehen,
mit Ausnahme von
1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und
1-(4-Aminophenyl)-6,8-difluor-7-morpholinyl-1,4-dihydro-4-oxo-3-chinolincarbonsäure.
und die pharmazeutisch vertretbaren Derivate dieser Verbindungen.

3. Verbindungen der allgemeinen Formel (I) gemäß Anspruch 2, 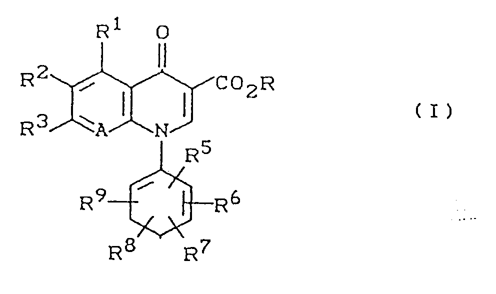 in der
R für Wasserstoff, Methyl oder Ethyl steht,
R¹ für Wasserstoff, Halogen,
R² für Fluor, Nitro oder Wasserstoff steht,
A für N oder CR₄ steht, wobei
R⁴ für Wasserstoff oder Halogen steht,
R³ und R⁵ gleich oder verschieden sind und für NR²⁵R²⁶ stehen, worin R²⁵ und R²⁶ gleich oder verschieden sind und für Wasserstoff, C₁-C₁₀-Alkyl, gegebenenfalls substituiert durch ein oder mehrere Halogene, O-Alkyl (C₁-C₆), Alkyl (C₁-C₄) je Alkylgruppe oder gegebenenfalls durch Halogen substituiertes Phenyl stehen, oder
R³ und R⁵ für eine Gruppe mit der Formel (II) 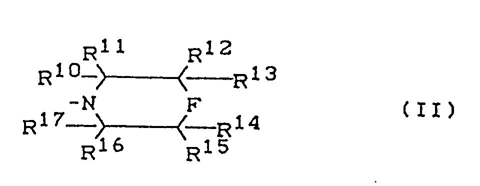 stehen, wobei
F Schwefel, Sauerstoff, NR¹⁸ oder CR¹⁹R²⁰ bedeutet
und
R¹⁸ für
- Wasserstoff,
- Methyl, Ethyl, Propyl,
- (C₃-C₆) Cycloalkyl,
- Phenyl, gegebenenfalls substituiert durch Halogen,
- CN,
- COO-Alkyl (C₁-C₄)
- C₁-C₄-Acyl
- (C₁-C₄)-Sulfonyl steht und
R¹⁰, R¹¹, R¹⁷, R¹⁶ für Wasserstoff stehen, R¹², R¹³, R¹⁴, R¹⁵, R¹⁹, R²⁰ gleich oder verschieden sind und für
- Wasserstoff,
- C₁-C₃ Alkyl oder C₃-C₆ Cycloalkyl, gegebenenfalls substituiert durch ein oder mehrere Halogene, OH, Methoxy, Ethoxy, Methyl, Ethyl, Cyano, CO₂Me
- Hydroxyl,
- oder Amino, Alkylamino mit 1-4 Kohlenstoffatomen, Dialkylamino mit 1-3 Kohlenstoffatomen stehen,
R³ und R⁵ gleich oder verschieden sind und für eine Gruppe der Formel 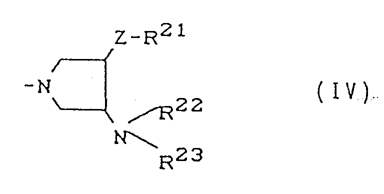 stehen, wobei
R²¹ für Wasserstoff, Methyl, Ethyl, Phenyl, C₁-C₄ Acyl,
R²² für Wasserstoff, Methyl, Ethyl, OH, OCH₃, wobei R²¹ und R²² gemeinsam auch durch eine durch Methyl ein- oder zweifach substituierte C₁-C₃-Alkylenbrücke bedeuten kann,
R²³ für H, Methyl, Ethyl, Aryl, Benzyl, C₁-C₄ Alkoxycarbonyl, C₁-C₄ Acyl stehen,
Z für O oder S stehen kann,
ZR²¹ für Wasserstoff stehen kann,
mit Ausnahme von
1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und
1-(4-Aminophenyl)-6,8-difluor-7-morpholinyl-1,4-dihydro-4-oxo-3-chinolincarbonsäure.
und die pharmazeutisch vertretbaren Derivate dieser Verbindungen.

4. Verfahren zur Herstellung von Verbindungen der Formel (I) gemäß Anspruch 1, in der R³ und R⁶ gleich sind, mit Ausnahme von
1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und
1-(4-Aminophenyl)-6,8-difluor-7-morpholinyl-1,4-dihydro-4-oxo-3-chinolincarbonsäure, dadurch gekennzeichnet, daß man Chinoline der allgemeinen Formel (III) 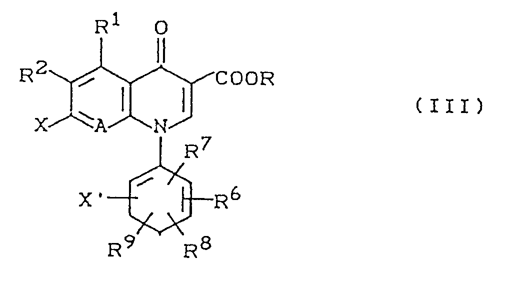 in der
A, R, R¹, R², R⁶, R⁷, R⁸ und R⁹ die in Anspruch 1 angegebene Bedeutung haben und X und X' gleich oder verschieden sind und für ein Halogenatom stehen, mit Aminen der allgemeinen Formeln (II) und (IV) 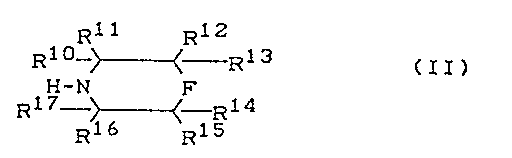 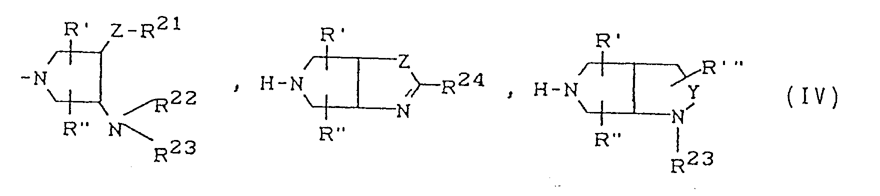 in denen
R¹⁰ bis R¹⁷, R²¹ bis R²⁴, Y, Z, R', R'', R''' und F die in Anspruch 1 angegebene Bedeutung haben, gegebenenfalls in Gegenwart von Verdünnungsmitteln und gegebenenfalls in Gegenwart von Säurebindern umsetzt.

5. Verfahren zur Herstellung der Verbindungen der Formel (I) gemäß Anspruch 1, mit Ausnahme von 1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und
1-(4-Aminophenyl)-6,8-difluor-7-morpholinyl-1,4-dihydro-4-oxo-3-chinolincarbonsäure, dadurch gekennzeichnet, daß man Verbindungen der allgemeinen Formel (V) 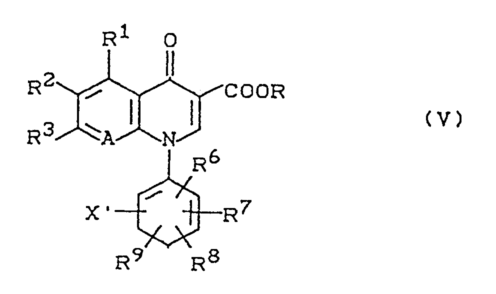 in der A, R, R¹ bis R⁹ die oben angegebene Bedeutung haben und X' für ein Halogenatom steht, mit Aminen der allgemeinen Formeln (II) und (IV), 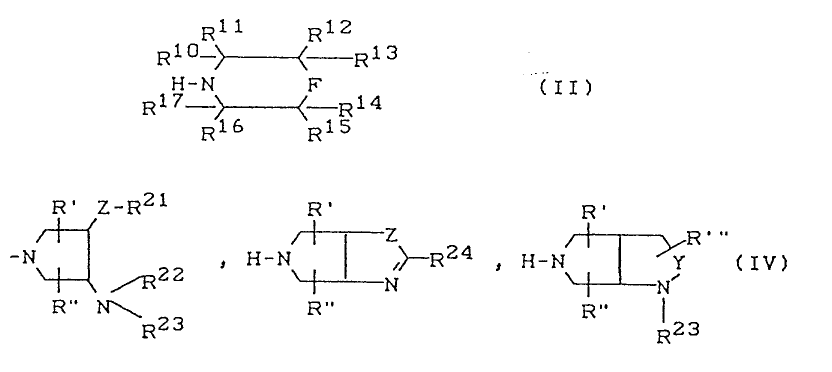 in denen
R¹⁰ bis R¹⁷, R²¹ bis R²⁴, R', R'' und R''', Y, Z, und F die in Anspruch 1 angegebene Bedeutung haben, gegebenenfalls in Gegenwart von Verdünnungsmitteln und gegebenenfalls in Gegenwart von Säurebindern umsetzt.

6. Verfahren zur Herstellung der Verbindungen der Formel (I) gemäß Anspruch 1, mit Ausnahme von 1-(3-Methylamino-4-fluorphenyl)-6-fluor-7-(3-methylpiperazinyl)-1,4-dihydro-4-oxo-3-chinolincarbonsäure, 1-(4-Aminophenyl)-6-fluor-7-piperazinyl-1,4-dihydro-4-oxo-3-chinolincarbonsäure,
1-(4-Aminophenyl)-6-fluor-7-(4-methylpiperazinyl)-1,4-dihydro-4-oxo-3-chinolincarbonsäure und
1-(4-Aminophenyl)-6,8-difluor-7-morpholinyl-1,4-dihydro-4-oxo-3-chinolincarbonsäure, dadurch gekennzeichnet, daß man Chinoline der allgemeinen Formel (VI) 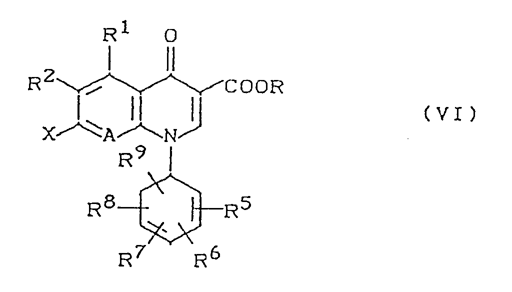 in der A, R, R¹, R², R⁴, R⁵, R⁶, R⁷, R⁸, R⁹ die in Anspruch 1 angegebene Bedeutung haben und X für Halogen steht, mit Aminen der allgemeinen Formeln (II) und (IV), 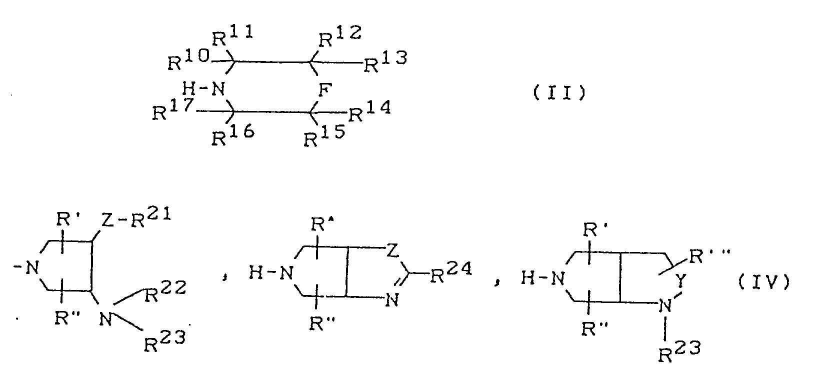 in denen
R¹⁰ bis R¹⁷, R²¹ bis R²⁴, R', R'' und R''', Y, Z, und F die in Anspruch 1 angegebene Bedeutung haben,
gegebenenfalls in Gegenwart von Verdünnungsmitteln und gegebenenfalls in Gegenwart von Säurebindern umsetzt.

7. Verfahren zur Herstellung der Verbindungen der Formel (I) gemäß Anspruch 1, mit Ausnahme von 1-(3-Methylamino-4-fluorphenyl)-6-fluor-7-(3-methylpiperazinyl)-1,4-dihydro-4-oxo-3-chinolincarbonsäure und 1-(4-Aminophenyl-6-fluor-7-(4-methylpiperazinyl)-1,4-dihydro-4-oxo-3-chinolincarbonsäure, dadurch gekennzeichnet, daß man Verbindungen der allgemeinen Formel (VII) 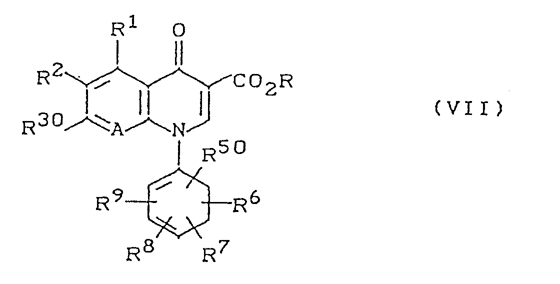 wobei R, R¹, R², A, R⁶ - R⁹ die in Anspruch 1 angegebene Bedeutung haben, die Reste R³⁰ und R⁵⁰ gleich oder verschieden sind, wobei in R³⁰ und R⁵⁰ oder nur in R³⁰ oder nur in R⁵⁰ sekundäre oder primäre Amine vorhanden sind und die Strukturen von R³⁰ und R⁵⁰ durch die Vertreter der Reste R³ bzw. R⁵ bestimmt sind,
mit Acylierungsmitteln oder Alkylierungsmitteln der allgemeinen Formel (VIII)
V - W (VIII)
in der
W = - CN
- (C₂-C₄)-Acyl, gegebenenfalls substituiert
- (C₁-C₄)-Sulfonyl
- (C₁-C₄)-Alkoxycarbonyl
- (C₁-C₅)-Alkyl
und V eine Abgangsgruppe darstellt, wie
V = - Halogen
- Trifluormethansulfonat
- Mesylat
- Tosylat
- Acetat
- Trihaloacetat
gegebenenfalls in Gegenwart von Verdünnungsmitteln und gegebenenfalls in Gegenwart von Säurebindern umgesetzt.

8. Verfahren zur Herstellung der Verbindungen der allgemeinen Formel (I) gemäß Anspruch 1, mit Ausnahme von 1-(3-Methylamino-4-fluorphenyl)-6-fluor-7-(3-methyl-piperazinyl)-1,4-dihydro-4-oxo-3-chinolincarbonsäure, dadurch gekennzeichnet, daß man Verbindungen der allgemeinen Formel (VII) nach Anspruch 7 mit Verbindungen der allgemeinen Formel (IX),
CH₂=CH-U (IX)
in der U eine elektronenanziehende Gruppe wie COOB, COB, CONH₂, CN, SO₂B, SO₃B bedeutet und B für (C₁-C₄)-Alkyl, das gegebenenfalls substituiert ist, steht,
gegebenenfalls in Gegenwart von Verdünnungsmitteln umsetzt.

9. Verfahren zur Herstellung der Verbindungen der Formel (I) gemäß Anspruch 1, mit Ausnahme von 1-(3-Methylamino-4-fluorphenyl)-6-fluor-7-(3-methylpiperazinyl)-1,4-dihydro-4-oxo-3-chinolincarbonsäure, dadurch gekennzeichnet, daß man Verbindungen der allgemeinen Formel (VII) nach Anspruch 7 mit Carbonsäuren der allgemeinen Formel (X)
D-COOH (X)
in der D für H oder gegebenenfalls substituiertes (C₁-C₄)-Alkyl steht, gegebenenfalls in Gegenwart von Verdünnungsmitteln und gegebenenfalls in Gegenwart wasserentziehender Reagenzien und gegebenenfalls in Gegenwart eines Katalysators umsetzt.

## Claims (Claims for the following Contracting State(s): AT, BE, CH, DE, DK, FR, GB, IT, LI, LU, NL, SE)

1. Compounds of the general formula (I) 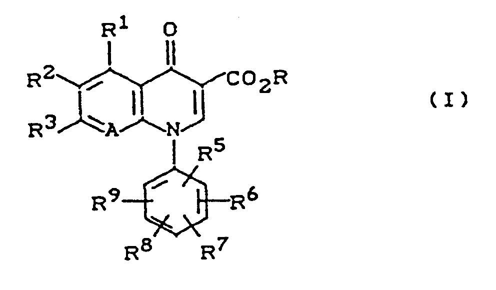 in which
R represents hydrogen or alkyl having 1-4 carbon atoms,
R¹ represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen, cyano or nitro,
R² represents hydrogen, nitro or halogen,
A represents N or CR⁴, where
R⁴ represents hydrogen, halogen, methyl, cyano, nitro, methoxy or amino or, alternatively, represents an O, S or CH₂ bridge which is connected to the ortho-position of the phenyl ring at N-1,
R³ and R⁵ are identical or different and represent NR²⁵R²⁶, in which R²⁵ and R²⁶ are identical or different and represent hydrogen, C₁-C₂₀-alkyl, optionally substituted by one or more halogens, OH, O-alkyl (C₁-C₆), alkyl (C₁-C₄), CN, or COOalkyl (C₁-C₆),
- hydroxyl,
- or amino, alkylamino having 1-4 carbon atoms, dialkylamino having 1-3 carbon atoms per alkyl group or phenyl which is optionally substituted by halogen, or
R³ and R⁵ represent a group having the formula (II) 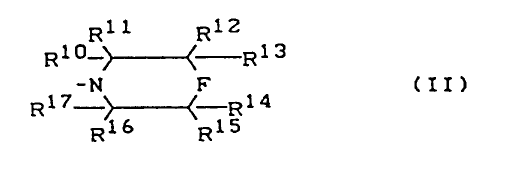 in which
F denotes O, S, NR¹⁸ or CR¹⁹R²⁰ and
R⁶, R⁷, R⁸ and R⁹ are identical or different and represent
- hydrogen,
- methyl, ethyl, nitro, amino, monoalkylamino having 1-3 C atoms, dialkylamino having 1-4 C atoms, methoxy, ethoxy, thiomethyl, thioethyl, trifluoromethyl, cyano or halogen,
R¹⁸ represents
- H, (C₁-C₁₀)-alkyl or (C₃-C₆)-cycloalkyl, optionally substituted by one or more substituents Hal, OH, CN, COOR or OR where R = (C₁-C₇)-alkyl,
- aryl (C₆-C₁₂), optionally substituted by halogen, -O-alkyl (C₁-C₂) or (C₁-C₄)-alkyl,
- CN,
- COOalkyl (C₁-C₄),
- C₁-C₄-acyl or
- (C₁-C₄)-sulphonyl,
R¹⁰, R¹¹ R¹², R¹³, R¹⁴, R¹⁵, R¹⁶, R¹⁷, R¹⁹ and R²⁰ are identical or different and represent
- hydrogen,
- C₁-C₃-alkyl or C₃-C₆-cycloalkyl, optionally substituted by one or more halogens, OH, O-alkyl (C₁-C₆), alkyl (C₁-C₄), CN or COO alkyl (C₁-C₆),
- hydroxyl,
- or amino, alkylamino having 1-4 carbon atoms, dialkylamino having 1-3 carbon atoms per alkyl group or phenyl which is optionally substituted by halogen, or
R³ and R⁵ represent a radical of the structure (IV) 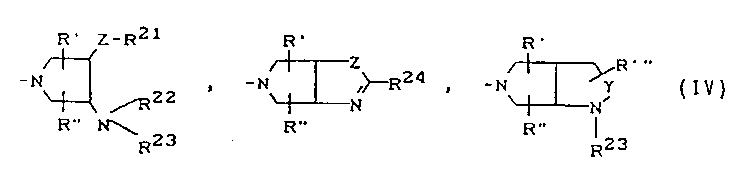 where
R²¹ can represent H, C₁-C₄-alkyl, aryl or C₁-C₄-acyl,
R²² can represent H, C₁-C₄-alkyl, OH or OCH₃, where R²¹ and R²² can together also denote a C₁-C₃-alkylene bridge which is optionally monosubstituted or disubstituted by methyl,
R²³ can represent H, C₁-C₄-alkyl, aryl, heteroaryl, benzyl, C₁-C₄-alkoxycarbonyl, C₁-C₄-acyl or (5-methyl-2-oxo-1,3-dioxol-4-yl)-methyl,
R²⁴ can represent H or C₁-C₄-alkyl,
R' can represent H, CH₃ or phenyl,
R'' can represent H, CH₃ or phenyl,
R''' can represent H or CH₃,
Y can represent O, CH₂, CH₂CH₂ or CH₂-O, where the linkage of the CH₂-O group to the nitrogen can take place both via O and via CH₂,
Z can represent O or S,
ZR²¹ can represent hydrogen, where
R⁶, R⁷, R⁸ and R⁹ are identical or different and represent
- hydrogen
- alkyl having 1 to 5 carbon atoms, nitro, amino, monoalkylamino having 1-3 C atoms, dialkylamino having 1-6 C atoms, O-alkyl having 1-5 carbon atoms, S-alkyl having 1-5 carbon atoms, trifluoromethyl, cyano or halogen,
with the exception of 1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid,
and the pharmaceutically acceptable derivatives of these compounds.

2. Compounds of the general formula (I) according to Claim 1, 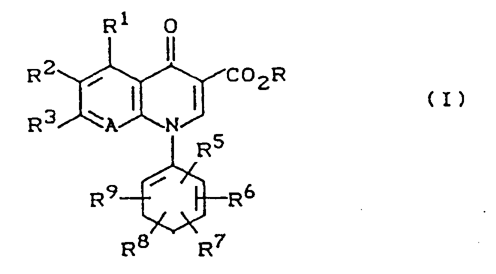 in which
R represents hydrogen, methyl or ethyl,
R¹ represents hydrogen, amino, nitro, methoxy, methylthio, cyano or halogen,
R² represents hydrogen, nitro or halogen,
A represents N or CR⁴, where
R⁴ represents hydrogen, halogen, methyl, cyano, nitro, methoxy or amino or, alternatively, represents an O, S or CH₂ bridge which is connected to the ortho-position of the phenyl ring at N-1,
R³ and R⁵ are identical or different and represent NR²⁵R²⁶, in which R²⁵ and R²⁶ are identical or different and represent hydrogen, C₁-C₂₀-alkyl, optionally substituted by one or more halogens, O-alkyl (C₁-C₆), alkyl (C₁-C₄) or phenyl which is optionally substituted by halogen, or
R³ and R⁵ represent a group having the formula (II) 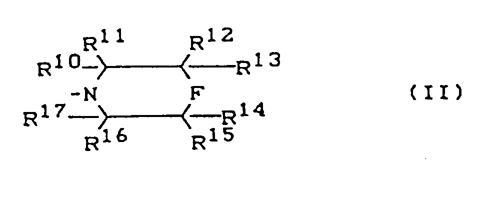 where
F denotes sulphur, oxygen, NR¹⁸ OR CR¹⁹R²⁰
and
R¹⁸ represents
- H, (C₁-C₁₀)-alkyl or (C₃-C₆)-cycloalkyl, optionally substituted by one or more substituents Hal, OH, CN, COOR or OR where R = (C₁-C₇)-alkyl,
- aryl (C₆-C₁₂), optionally substituted by halogen, -O-alkyl (C₁-C₂) or (C₁-C₄)-alkyl,
- CN,
- COOalkyl (C₁-C₄),
- C₁-C₄-acyl or
- (C₁-C₄)-sulphonyl,
R¹⁰, R¹¹ R¹², R¹³, R¹⁴, R¹⁵, R¹⁶, R¹⁷, R¹⁹ and R²⁰ are identical or different and represent
- hydrogen,
- C₁-C₃-alkyl or C₃-C₆-cycloalkyl, optionally substituted by one or more halogens, OH, methoxy, ethoxy, methyl, ethyl, cyano or CO₂Me,
- hydroxyl,
- or amino, alkylamino having 1-4 carbon atoms, dialkylamino having 1-3 carbon atoms per alkyl group or phenyl which is optionally substituted by halogen,
or
R³ and R⁵ represent a radical of the structures 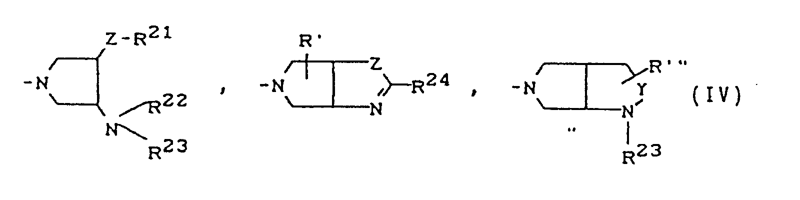 where
R²¹ can represent H, C₁-C₄-alkyl, aryl or C₁-C₄-acyl,
R²² can represent H, C₁-C₄-alkyl, OH or OCH₃, where R²¹ and R²² together can also denote a C₁-C₃-alkylene bridge which is optionally monosubstituted or disubstituted by methyl,
R²³ can represent H, C₁-C₄-alkyl, aryl, heteroaryl, benzyl, C₁-C₄-alkoxycarbonyl or C₁-C₄-acyl,
R²⁴ can represent H or C₁-C₄-alkyl,
Y can represent O, CH₂, CH₂CH₂ or CH₂-O, where the linkage of the CH₂-O group to the nitrogen can take place both via O and via CH₂,
Z can represent O or S,
ZR²¹ can represent hydrogen, and
R⁶, R⁷, R⁸ and R⁹ are identical or different and represent
- hydrogen
- methyl, ethyl, nitro, amino, monoalkylamino having 1-3 C atoms, dialkylamino having 1-4 C atoms, methoxy, ethoxy, thiomethyl, thioethyl, trifluoromethyl, cyano or halogen,
with the exception of 1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid,
and the pharmaceutically acceptable derivatives of these compounds.

3. Compounds of the general formula (I) according to Claim 1 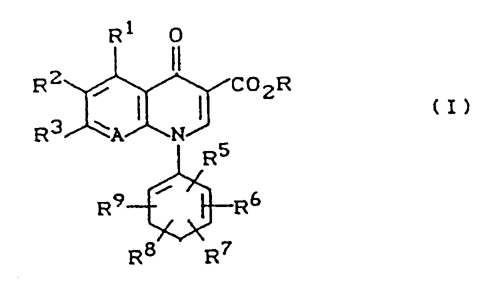 in which
R represents hydrogen, methyl or ethyl,
R¹ represents hydrogen or halogen,
R² represents fluorine, nitro or hydrogen,
A represents N or CR⁴, where
R⁴ represents hydrogen or halogen,
R³ and R⁵ are identical or different and represent NR²⁵R²⁶, in which R²⁵ and R²⁶ are identical or different and represent hydrogen, C₁-C₁₀-alkyl, optionally substituted by one or more halogens, O-alkyl (C₁-C₆), alkyl (C₁-C₄) or phenyl which is optionally substituted by halogen, or
R³ and R⁵ represent a group having the formula (II) 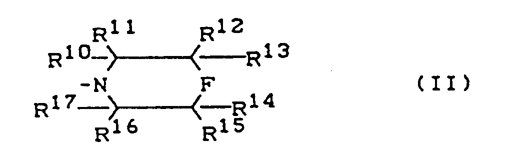 F denotes sulphur, oxygen, NR¹⁸ or CR¹⁹R²⁰
and
R¹⁸ represents
- hydrogen,
- methyl, ethyl, propyl,
- (C₃-C₆)-cycloalkyl,
- phenyl, optionally substituted by halogen,
- CN,
- COO-alkyl (C₁-C₄),
- C₁-C₄-acyl or
- (C₁-C₄)-sulphonyl, and
R¹⁰, R¹¹, R¹⁷ and R¹⁶ represent hydrogen, R¹², R¹³, R¹⁴, R¹⁵, R¹⁹ and R²⁰ are identical or different and represent,
- hydrogen,
- C₁-C₃-alkyl or C₃-C₆-cycloalkyl, optionally substituted by one or more halogens, OH, methoxy, ethoxy, methyl, ethyl, cyano, CO₂Me,
- hydroxyl,
- or amino, alkylamino having 1-4 carbon atoms or dialkylamino having 1-3 carbon atoms,
R³ and R⁵ are identical or different and represent a group of the formula 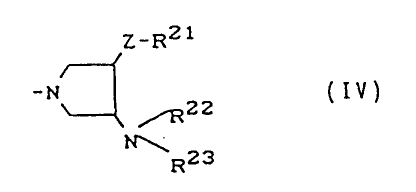 where
R²¹ can represent hydrogen, methyl, ethyl, phenyl or C₁-C₄-acyl,
R²² can represent hydrogen, methyl, ethyl, OH or OCH₃, where R²¹ and R²² may together also denote a C₁-C₃-alkylene bridge which is optionally monosubstituted or disubstituted by methyl,
R²³ can represent H, methyl, ethyl, aryl, benzyl, C₁-C₄-alkoxycarbonyl or C₁-C₄-acyl,
Z can represent O or S,
ZR²¹ can represent hydrogen,
with the exception of 1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid,
and the pharmaceutically acceptable derivatives of these compounds.

4. Process for the preparation of compounds of the formula (I) according to Claim 1, in which R³ and R⁵ are identical, with the exception of 1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid, characterized in that quinolines of the general formula (III) 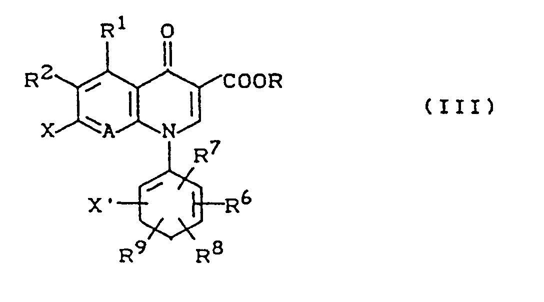 in which
A, R, R¹, R², R⁶, R⁷, R⁸ and R⁹ have the meaning intended in Claim 1 and X and X' are identical or different and represent a halogen atom,
are reacted with amines of the general formulae (II) and (IV) 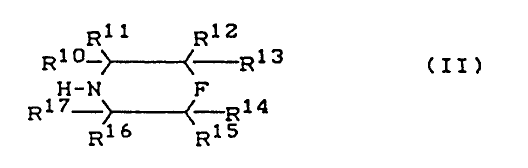 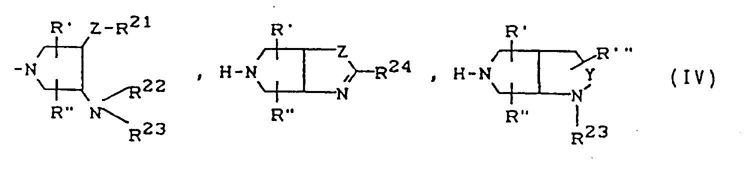 in which
R¹⁰ to R¹⁷, R²¹ to R²⁴, Y, Z, R', R'', R''' and F have the meaning indicated in Claim 1, if appropriate in the presence of diluents and if appropriate in the presence of acid binders.

5. Process for the preparation of the compounds of the formula (I) according to Claim 1, with the exception of
1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid, characterized in that compounds of the general formula (V) 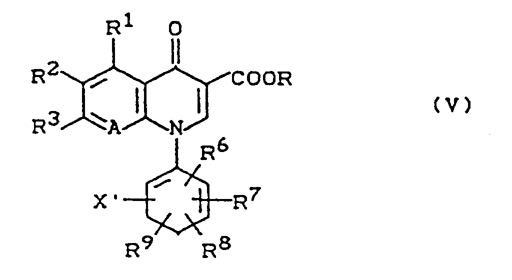 in which A, R, R¹ to R⁹ have the meaning indicated in Claim 1 and X' represents a halogen atom, are reacted with amines of the general formulae (II) and (IV) 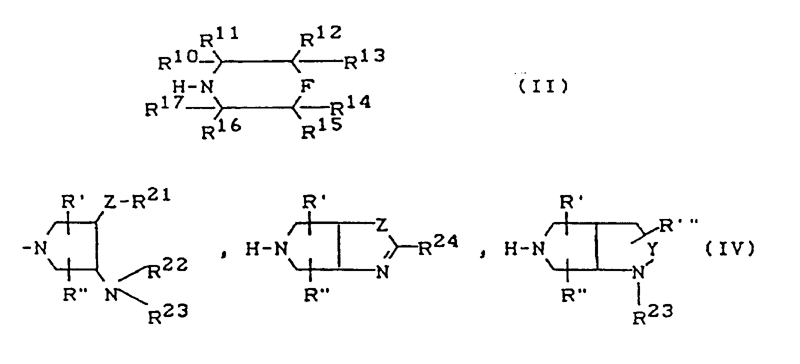 in which
R¹⁰ to R¹⁷, R²¹ to R²⁴, R', R'' and R''', Y, Z and F have the meaning indicated in Claim 1, if appropriate in the presence of diluents and if appropriate in the presence of acid binders.

6. Process for the preparation of the compounds of the formula (I) according to Claim 1, with the exception of
1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid, characterized in that quinolines of the general formula (VI) 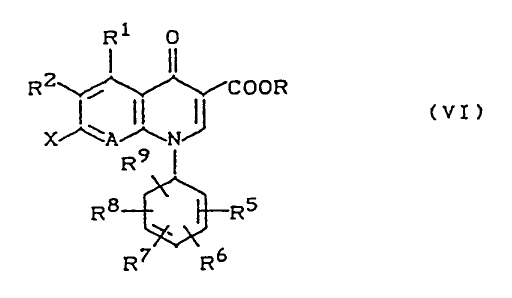 in which A, R, R¹, R², R⁵, R⁶, R⁷, R⁸ and R⁹ have the meaning indicated in Claim 1 and X represents halogen, are reacted with amines of the general formulae (II) and (IV) 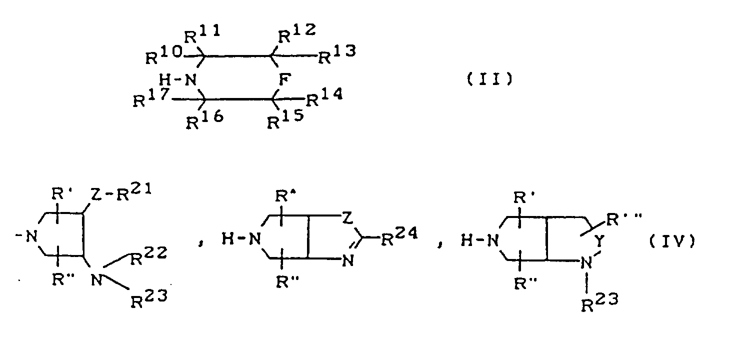 in which
R¹⁰ to R¹⁷, R²¹ to R²⁴, R', R'' and R''', Y, Z and F have the meaning indicated in Claim 1,
if appropriate in the presence of diluents and if appropriate in the presence of acid binders.

7. Process for the preparation of the compounds of the formula (I) according to Claim 1, with the exception of 1-(3-methylamino-4-fluorophenyl)-6-fluoro-7-(3-methylpiperazinyl)-1,4-dihydro-4-oxo-3-quinolinecarboxylic acid and 1-(4-aminophenyl)-6-fluoro-7-(4-methylpiperazinyl)-1,4-dihydro-4-oxo-3-quinolinecarboxylic acid, characterized in that compounds of the general formula (VII) 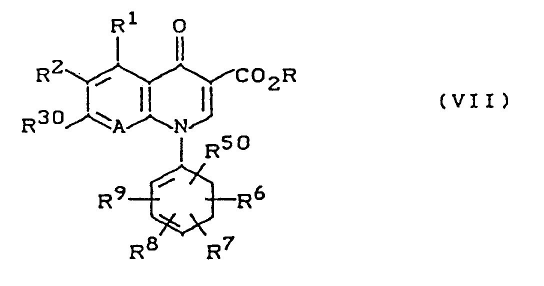 where R, R¹, R², A and R⁶-R⁹ have the meaning indicated in Claim 1, the radicals R³⁰ and R⁵⁰ are identical or different, where secondary or primary amines are present in R³⁰ and R⁵⁰ or only in R³⁰ or only in R⁵⁰ and the structures of R³⁰ and R⁵⁰ are determined by the substituents of the radicals R³ or R⁵,
are reacted with acylating agents or alkylating agents of the general formula (VIII)
V - W (VIII)
in which
W = - CN
- (C₂-C₄)-acyl, optionally substituted
- (C₁-C₄)-sulphonyl
- (C₁-C₄)-alkoxycarbonyl
- (C₁-C₅)-alkyl
and V represents a leaving group, such as
V = - halogen
- trifluoromethanesulphonate
- mesylate
- tosylate
- acetate
- trihaloacetate
if appropriate in the presence of diluents and if appropriate in the presence of acid binders.

8. Process for the preparation of the compounds of the general formula (I) according to Claim 1, with the exception of 1-(3-methylamino-4-fluorophenyl)-6-fluoro-7-(3-methylpiperazinyl)-1,4-dihydro-4-oxo-3-quinolinecarboxylic acid, characterized in that compounds of the general formula (VII) according to Claim 7 are reacted with compounds of the general formula (IX)
CH₂=CH-U (IX)
in which U denotes an electron-attracting group such as COOB, COB, CONH₂ CN, SO₂B or SO₃B and B represents (C₁-C₄)-alkyl which is optionally substituted,
if appropriate in the presence of diluents.

9. Process for the preparation of the compounds of the formula (I) according to Claim 1, with the exception of 1-(3-methylamino-4-fluorophenyl)-6-fluoro-7-(3-methylpiperazinyl)-1,4-dihydro-4-oxo-3-quinolinecarboxylic acid, characterized in that compounds of the general formula (VII) according to Claim 7 are reacted with carboxylic acids of the general formula (X)
D-COOH (X)
in which D represents H or optionally substituted (C₁-C₄)-alkyl, if appropriate in the presence of diluents and if appropriate in the presence of dehydrating reagents and if appropriate in the presence of a catalyst.

10. Medicaments, containing compounds according to Claims 1 to 3.

11. Antiviral agents, containing compounds according to claims 1 to 3.

12. Use of compounds of the general formula (I) 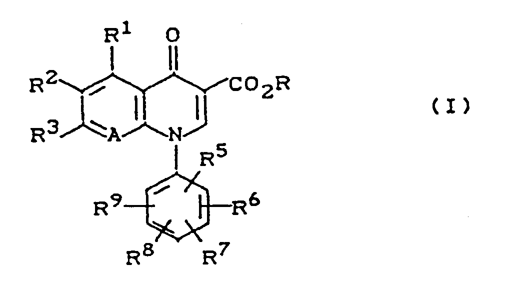 in which
R represents hydrogen or alkyl having 1-4 carbon atoms,
R¹ represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen,
cyano or nitro,
R² represents hydrogen, nitro or halogen,
A represents N or CR⁴, where
R⁴ represents hydrogen, halogen, methyl, cyano, nitro, methoxy or amino or, alternatively, represents an O, S or CH₂ bridge which is connected to the ortho-position of the phenyl ring at N-1,
R³ and R⁵ are identical or different and represent NR²⁵R²⁶, in which R²⁵ and R²⁶ are identical or different and represent hydrogen, C₁-C₂₀-alkyl, optionally substituted by one or more halogens, OH, O-alkyl (C₁-C₆), alkyl (C₁-C₄), CN, or COOalkyl (C₁-C₆),
- hydroxyl,
- or amino, alkylamino having 1-4 carbon atoms, dialkylamino having 1-3 carbon atoms per alkyl group or phenyl which is optionally substituted by halogen, or
R³ and R⁵ represent a group having the formula (II) 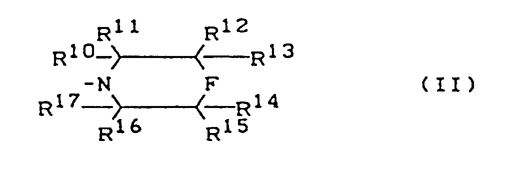 in which
F denotes O, S, NR¹⁸ or CR¹⁹R²⁰ and
R⁶, R⁷, R⁸ and R⁹ are identical or different and represent
- hydrogen,
- methyl, ethyl, nitro, amino, monoalkylamino having 1-3 C atoms, dialkylamino having 1-4 C atoms, methoxy, ethoxy, thiomethyl, thioethyl, trifluoromethyl, cyano or halogen,
R¹⁸ represents
- H, (C₁-C₁₀)-alkyl or (C₃-C₆)-cycloalkyl, optionally substituted by one or more substituents Hal, OH, CN, COOR or OR where R = (C₁-C₇)-alkyl,
- aryl (C₆-C₁₂), optionally substituted by halogen, -O-alkyl (C₁-C₂) or (C₁-C₄)-alkyl,
- CN,
- COOalkyl (C₁-C₄),
- C₁-C₄-acyl or
- (C₁-C₄)-sulphonyl,
R¹⁰, R¹¹, R¹², R¹³, R¹⁴, R¹⁵, R¹⁶, R¹⁷, R¹⁹ and R²⁰ are identical or different and represent
- hydrogen,
- C₁-C₃-alkyl or C₃-C₆-cycloalkyl, optionally substituted by one or more halogens, OH, O-alkyl (C₁-C₆), alkyl (C₁-C₄), CN or COO alkyl (C₁-C₆),
- hydroxyl,
- or amino, alkylamino having 1-4 carbon atoms, dialkylamino having 1-3 carbon atoms per alkyl group or phenyl which is optionally substituted by halogen, or
R³ and R⁵ represent a radical of the structure (IV) 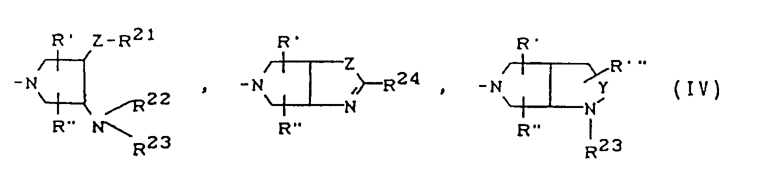 where
R²¹ can represent H, C₁-C₄-alkyl, aryl or C₁-C₄-acyl,
R²² can represent H, C₁-C₄-alkyl, OH or OCH₃, where R²¹ and R²² can together also denote a C₁-C₃-alkylene bridge which is optionally monosubstituted or disubstituted by methyl,
R²³ can represent H, C₁-C₄-alkyl, aryl, heteroaryl, benzyl, C₁-C₄-alkoxycarbonyl, C₁-C₄-acyl or (5-methyl-2-oxo-1,3-dioxol-4-yl)-methyl,
R²⁴ can represent H or C₁-C₄-alkyl,
R' can represent H, CH₃ or phenyl,
R'' can represent H, CH₃ or phenyl,
R''' can represent H or CH₃,
Y can represent O, CH₂, CH₂CH₂ or CH₂-O, where the linkage of the CH₂-O group to the nitrogen can take place both via O and via CH₂,
Z can represent O or S,
ZR²¹ can represent hydrogen, where
R⁶, R⁷, R⁸ and R⁹ are identical or different and represent
- hydrogen
- alkyl having 1 to 5 carbon atoms, nitro, amino, monoalkylamino having 1-3 C atoms, dialkylamino having 1-6 C atoms, O-alkyl having 1-5 carbon atoms, S-alkyl having 1-5 carbon atoms, trifluoromethyl, cyano or halogen,
and the pharmaceutically acceptable derivatives of these compounds for the production of a medicament for use in the antiviral therapy of the human or animal body.

13. Use of compounds according to Claim 12 of the general formula (I) 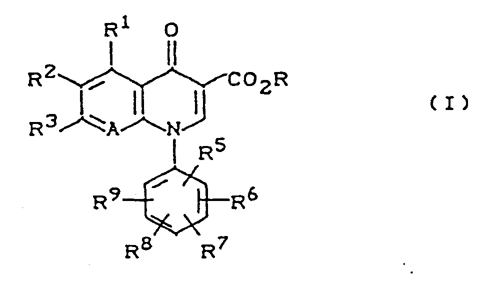 in which
R represents hydrogen, methyl or ethyl,
R¹ represents hydrogen, amino, nitro, methoxy, methylthio, cyano or halogen,
R² represents hydrogen, nitro or halogen,
A represents N or CR⁴, where
R⁴ represents hydrogen, halogen, methyl, cyano, nitro, methoxy or amino or, alternatively, represents an O, S or CH₂ bridge which is connected to the ortho-position of the phenyl ring at N-1,
R³ and R⁵ are identical or different and represent NR²⁵R²⁶, in which R²⁵ and R²⁶ are identical or different and represent hydrogen, C₁-C₂₀-alkyl, optionally substituted by one or more halogens, O-alkyl (C₁-C₆), alkyl (C₁-C₄) or phenyl which is optionally substituted by halogen, or
R³ and R⁵ represent a group having the formula (II) 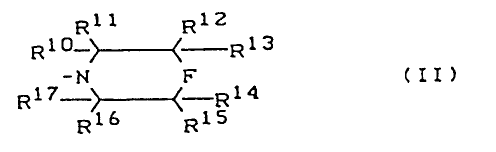 where
F denotes sulphur, oxygen, NR¹⁸ OR CR¹⁹R²⁰
and
R¹⁸ represents
- H, (C₁-C₁₀)-alkyl or (C₃-C₆)-cycloalkyl, optionally substituted by one or more substituents Hal, OH, CN, COOR or OR where R = (C₁-C₇)-alkyl,
- aryl (C₆-C₁₂), optionally substituted by halogen, -O-alkyl (C₁-C₂) or (C₁-C₄)-alkyl,
- CN,
- COOalkyl (C₁-C₄),
- C₁-C₄-acyl or
- (C₁-C₄)-sulphonyl,
R¹⁰, R¹¹, R¹², R¹³, R¹⁴, R¹⁵, R¹⁶, R¹⁷, R¹⁹ and R²⁰ are identical or different and represent
- hydrogen,
- C₁-C₃-alkyl or C₃-C₆-cycloalkyl, optionally substituted by one or more halogens, OH, methoxy, ethoxy, methyl, ethyl, cyano or CO₂Me,
- hydroxyl,
- or amino, alkylamino having 1-4 carbon atoms, dialkylamino having 1-3 carbon atoms per alkyl group or phenyl which is optionally substituted by halogen,
or
R³ and R⁵ represent a radical of the structures 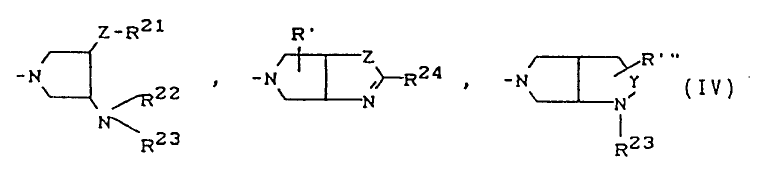 where
R²¹ can represent H, C₁-C₄-alkyl, aryl or C₁-C₄-acyl,
R²² can represent H, C₁-C₄-alkyl, OH or OCH₃, where R²¹ and R²² together can also denote a C₁-C₃-alkylene bridge which is optionally monosubstituted or disubstituted by methyl,
R²³ can represent H, C₁-C₄-alkyl, aryl, heteroaryl, benzyl, C₁-C₄-alkoxycarbonyl or C₁-C₄-acyl,
R²⁴ can represent H or C₁-C₄-alkyl,
Y can represent O, CH₂, CH₂CH₂ or CH₂-O, where the linkage of the CH₂-O group to the nitrogen can take place both via O and via CH₂,
Z can represent O or S,
ZR²¹ can represent hydrogen, and
R⁶, R⁷, R⁸ and R⁹ are identical or different and represent
- hydrogen
- methyl, ethyl, nitro, amino, monoalkylamino having 1-3 C atoms, dialkylamino having 1-4 C atoms, methoxy, ethoxy, thiomethyl, thioethyl, trifluoromethyl, cyano or halogen,
and the pharmaceutically acceptable derivatives of these compounds for the production of a medicament for use in the antiviral therapy of the human or animal body.

14. Use of compounds according to Claim 12 of the general formula (I) 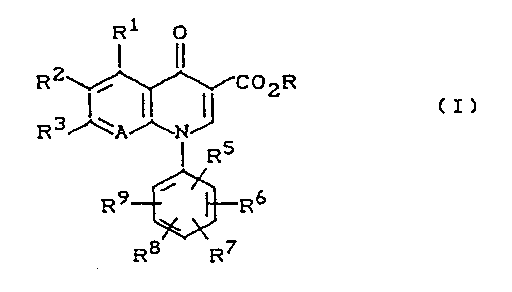 in which
R represents hydrogen, methyl or ethyl,
R¹ represents hydrogen or halogen,
R² represents fluorine, nitro or hydrogen,
A represents N or CR⁴, where
R⁴ represents hydrogen or halogen,
R³ and R⁵ are identical or different and represent NR²⁵R²⁶, in which R²⁵ and R²⁶ are identical or different and represent hydrogen, C₁-C₁₀-alkyl, optionally substituted by one or more halogens, O-alkyl (C₁-C₆), alkyl (C₁-C₄) or phenyl which is optionally substituted by halogen, or
R³ and R⁵ represent a group having the formula (II) 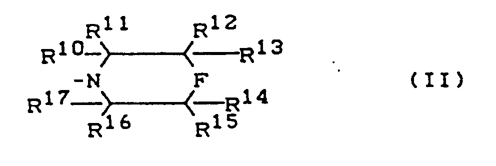 where
F denotes sulphur, oxygen, NR¹⁸ or CR¹⁹R²⁰
and
R¹⁸ represents
- hydrogen,
- methyl, ethyl, propyl,
- (C₃-C₆)-cycloalkyl,
- phenyl, optionally substituted by halogen,
- CN,
- COO-alkyl (C₁-C₄),
- C₁-C₄-acyl or
- (C₁-C₄)-sulphonyl, and
R¹⁰, R¹¹, R¹⁷ and R¹⁶ represent hydrogen,
R¹², R¹³, R¹⁴, R¹⁵, R¹⁹ and R²⁰ are identical or different and represent,
- hydrogen,
- C₁-C₃-alkyl or C₃-C₆-cycloalkyl, optionally substituted by one or more halogens, OH, methoxy, ethoxy, methyl, ethyl, cyano, CO₂Me,
- hydroxyl,
- or amino, alkylamino having 1-4 carbon atoms or dialkylamino having 1-3 carbon atoms,
R³ and R⁵ are identical or different and represent a group of the formula 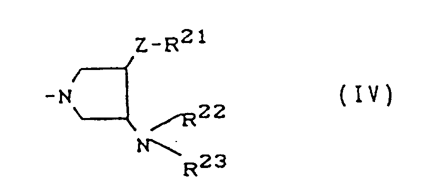 where
R²¹ can represent hydrogen, methyl, ethyl, phenyl or C₁-C₄-acyl,
R²² can represent hydrogen, methyl, ethyl, OH or OCH₃, where R²¹ and R²² may together also denote a C₁-C₃-alkylene bridge which is optionally monosubstituted or disubstituted by methyl,
R²³ can represent H, methyl, ethyl, aryl, benzyl, C₁-C₄-alkoxycarbonyl or C₁-C₄-acyl,
Z can represent O or S,
ZR²¹ can represent hydrogen,
and the pharmaceutically acceptable derivatives of these compounds for the production of a medicament for use in the antiviral therapy of the human or animal body.

## Claims (Claims for the following Contracting State(s): ES)

1. Process for the preparation of compounds of the general formula (I) 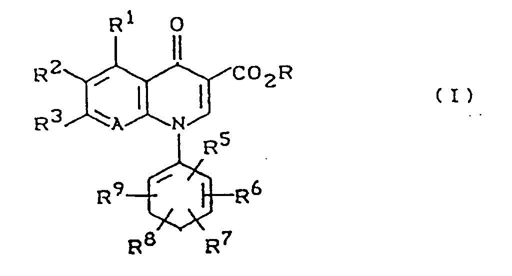 in which
R represents hydrogen or alkyl having 1-4 carbon atoms,
R¹ represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen, cyano or nitro,
R² represents hydrogen, nitro or halogen,
A represents N or CR⁴, where
R⁴ represents hydrogen, halogen, methyl, cyano, nitro, methoxy or amino or, alternatively, represents an O, S or CH₂ bridge which is connected to the ortho-position of the phenyl ring at N-1,
R³ and R⁵ are identical or different and represent NR²⁵R²⁶, in which R²⁵ and R²⁶ are identical or different and represent hydrogen, C₁-C₂₀-alkyl, optionally substituted by one or more halogens, OH, O-alkyl (C₁-C₆), alkyl (C₁-C₄), CN, or COOalkyl (C₁-C₆),
- hydroxyl,
- or amino, alkylamino having 1-4 carbon atoms, dialkylamino having 1-3 carbon atoms per alkyl group or phenyl which is optionally substituted by halogen, or
R³ and R⁵ represent a group having the formula (II) 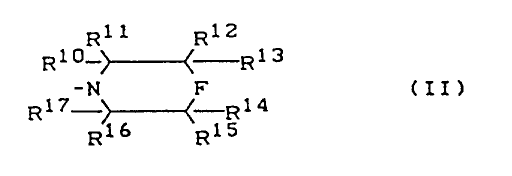 in which
F denotes O, S, NR¹⁸ or CR¹⁹R²⁰ and
R⁶, R⁷, R⁸ and R⁹ are identical or different and represent
- hydrogen,
- methyl, ethyl, nitro, amino, monoalkylamino having 1-3 C atoms, dialkylamino having 1-4 C atoms, methoxy, ethoxy, thiomethyl, thioethyl, trifluoromethyl, cyano or halogen,
R¹⁸ represents
- H, (C₁-C₁₀)-alkyl or (C₃-C₆)-cycloalkyl, optionally substituted by one or more substituents Hal, OH, CN, COOR or OR where R = (C₁-C₇)-alkyl,
- aryl (C₆-C₁₂), optionally substituted by halogen, -O-alkyl (C₁-C₂) or (C₁-C₄)-alkyl,
- CN,
- COOalkyl (C₁-C₄),
- C₁-C₄-acyl or
- (C₁-C₄)-sulphonyl,
R¹⁰, R¹¹, R¹², R¹³, R¹⁴, R¹⁵, R¹⁶, R¹⁷, R¹⁹ and R²⁰ are identical or different and represent
- hydrogen,
- C₁-C₃-alkyl or C₃-C₆-cycloalkyl, optionally substituted by one or more halogens, OH, O-alkyl (C₁-C₆), alkyl (C₁-C₄), CN or COO alkyl (C₁-C₆),
- hydroxyl,
- or amino, alkylamino having 1-4 carbon atoms, dialkylamino having 1-3 carbon atoms per alkyl group or phenyl which is optionally substituted by halogen, or
R³ and R⁵ represent a radical of the structure (IV) 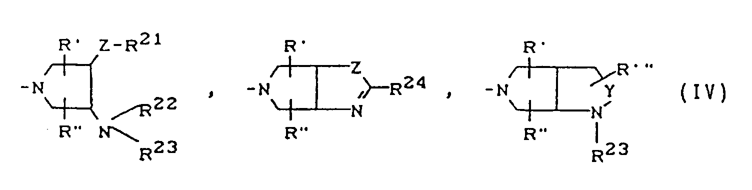 where
R²¹ can represent H, C₁-C₄-alkyl, aryl or C₁-C₄-acyl,
R²² can represent H, C₁-C₄-alkyl, OH or OCH₃, where R²¹ and R²² can together also denote a C₁-C₃-alkylene bridge which is optionally monosubstituted or disubstituted by methyl,
R²³ can represent H, C₁-C₄-alkyl, aryl, heteroaryl, benzyl, C₁-C₄-alkoxycarbonyl, C₁-C₄-acyl or (5-methyl-2-oxo-1,3-dioxol-4-yl)-methyl,
R²⁴ can represent H or C₁-C₄-alkyl,
R' can represent H, CH₃ or phenyl,
R'' can represent H, CH₃ or phenyl,
R''' can represent H or CH₃,
Y can represent O, CH₂, CH₂CH₂ or CH₂-O, where the linkage of the CH₂-O group to the nitrogen can take place both via O and via CH₂,
Z can represent O or S,
ZR²¹ can represent hydrogen, where
R⁶, R⁷, R⁸ and R⁹ are identical or different and represent
- hydrogen
- alkyl having 1 to 5 carbon atoms, nitro, amino, monoalkylamino having 1-3 C atoms, dialkylamino having 1-6 C atoms, O-alkyl having 1-5 carbon atoms, S-alkyl having 1-5 carbon atoms, trifluoromethyl, cyano or halogen,
with the exception of 1-(3-methylamino-4-fluorophenyl)-6-fluoro-7-(3-methylpiperazinyl)-1,4-dihydro-4-oxo-3-quinoline carboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid,
and the pharmaceutically acceptable derivatives of these compounds, characterized in that quinolines of the general formula (VI) 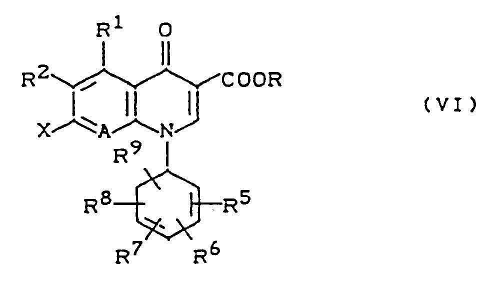 in which A, R, R¹, R², R⁵, R⁶, R⁷, R⁸ and R⁹ have the abovementioned meaning and X represents halogen, are reacted with amines of the general formulae (II) and (IV) 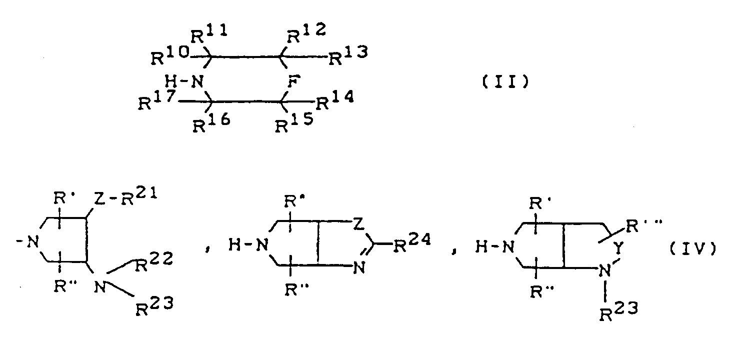 in which
R¹⁰ to R¹⁷, R²¹ to R²⁴, R', R'' and R''', Y, Z and F have the meaning indicated in Claim 1,
if appropriate in the presence of diluents and if appropriate in the presence of acid binders, and, if appropriate, the compounds thus obtained are converted into pharmaceutically acceptable derivatives in a manner known per se.

2. Process for the preparation of the compounds of the formula (I) according to Claim 1, with the exception of 1-(3-methylamino-4-fluorophenyl)-6-fluoro-7-(3-methylpiperazinyl)-1,4-dihydro-4-oxo-3-quinolinecarboxylic acid and 1-(4-aminophenyl)-6-fluoro-7-(4-methylpiperazinyl)-1,4-dihydro-4-oxo-3-quinolinecarboxylic acid, characterized in that compounds of the general formula (VII) 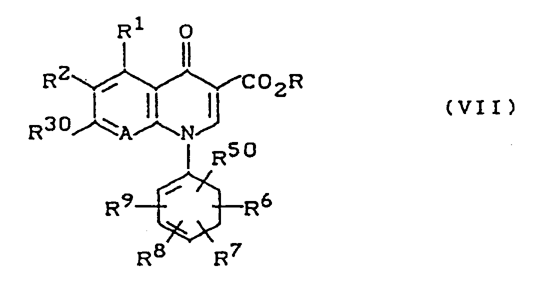 where R, R¹, R², A and R⁶-R⁹ have the meaning indicated in Claim 1, the radicals R³⁰ and R⁵⁰ are identical or different, where secondary or primary amines are present in R³⁰ and R⁵⁰ or only in R³⁰ or only in R⁵⁰ and the structures of R³⁰ and R⁵⁰ are determined by the substituents of the radicals R³ or R⁵,
are reacted with acylating agents or akylating agents of the general formula (VIII)
V - W (VIII)
in which
W = - CN
- (C₂-C₄)-acyl, optionally substituted
- (C₁-C₄)-sulphonyl
- (C₁-C₄)-alkoxycarbonyl
- (C₁-C₅)-alkyl
and V represents a leaving group, such as
V = - halogen
- trifluoromethanesulphonate
- mesylate
- tosylate
- acetate
- trihaloacetate
if appropriate in the presence of diluents and if appropriate in the presence of acid binders, and, if appropriate, the compounds thus obtained are converted into pharmaceutically acceptable derivatives in a manner known per se.

3. Process for the preparation of the compounds of the general formula (I) according to Claim 1, with the exception of 1-(3-methylamino-4-fluorophenyl)-6-fluoro-7-(3-methylpiperazinyl)-1,4-dihydro-4-oxo-3-quinolinecarboxylic acid, characterized in that compounds of the general formula (VII) according to Claim 2 are reacted with compounds of the general formula (IX)
CH₂=CH-U (IX)
in which U denotes an electron-attracting group such as COOB, COB, CONH₂, CN, SO₂B or SO₃B and B represents (C₁-C₄)-alkyl which is optionally substituted,
if appropriate in the presence of diluents.

4. Process for the preparation of the compounds of the formula (I) according to Claim 1, with the exception of 1-(3-methylamino-4-fluorophenyl)-6-fluoro-7-(3-methylpiperazinyl)-1,4-dihydro-4-oxo-3-quinolinecarboxylic acid, characterized in that compounds of the general formula (VII) according to Claim 2 are reacted with carboxylic acids of the general formula (X)
D-COOH (X)
in which D represents H or optionally substituted (C₁-C₄)-alkyl, if appropriate in the presence of diluents and if appropriate in the presence of dehydrating reagents and if appropriate in the presence of a catalyst.

## Claims (Claims for the following Contracting State(s): GR)

1. Compounds of the general formula (I) 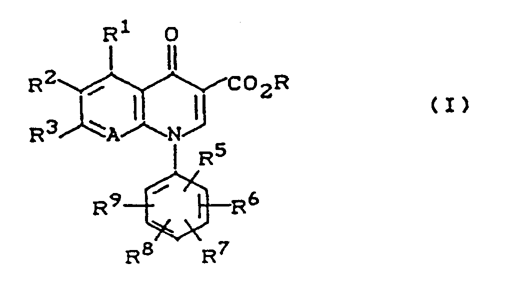 in which
R represents hydrogen or alkyl having 1-4 carbon atoms,
R¹ represents hydrogen, amino, alkylamino having 1 to 4 carbon atoms, dialkylamino having 1 to 3 carbon atoms per alkyl group, hydroxyl, alkoxy having 1 to 4 carbon atoms, mercapto, alkylthio having 1 to 4 carbon atoms, arylthio, halogen, cyano or nitro,
R² represents hydrogen, nitro or halogen,
A represents N or CR⁴, where
R⁴ represents hydrogen, halogen, methyl, cyano, nitro, methoxy or amino or, alternatively, represents an O, S or CH₂ bridge which is connected to the ortho-position of the phenyl ring at N-1,
R³ and R⁵ are identical or different and represent NR²⁵R²⁶, in which R²⁵ and R²⁶ are identical or different and represent hydrogen, C₁-C₂₀-alkyl, optionally substituted by one or more halogens, OH, O-alkyl (C₁-C₆), alkyl (C₁-C₄), CN, or COOalkyl (C₁-C₆),
- hydroxyl,
- or amino, alkylamino having 1-4 carbon atoms, dialkylamino having 1-3 carbon atoms per alkyl group or phenyl which is optionally substituted by halogen, or
R³ and R⁵ represent a group having the formula (II) 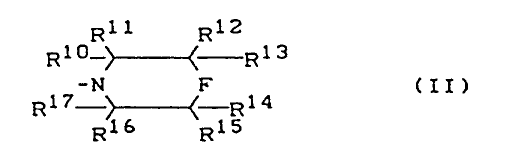 in which
F denotes O, S, NR¹⁸ or CR¹⁹R²⁰ and
R⁶, R⁷, R⁸ and R⁹ are identical or different and represent
- hydrogen,
- methyl, ethyl, nitro, amino, monoalkylamino having 1-3 C atoms, dialkylamino having 1-4 C atoms, methoxy, ethoxy, thiomethyl, thioethyl, trifluoromethyl, cyano or halogen,
R¹⁸ represents
- H, (C₁-C₁₀)-alkyl or (C₃-C₆)-cycloalkyl, optionally substituted by one or more substituents Hal, OH, CN, COOR or OR where R = (C₁-C₇)-alkyl,
- aryl (C₆-C₁₂), optionally substituted by halogen, -O-alkyl (C₁-C₂) or (C₁-C₄)-alkyl,
- CN,
- COOalkyl (C₁-C₄),
- C₁-C₄-acyl or
- (C₁-C₄)-sulphonyl,
R¹⁰, R¹¹, R¹², R¹³, R¹⁴, R¹⁵, R¹⁶, R¹⁷, R¹⁹ and R²⁰ are identical or different and represent
- hydrogen,
- C₁-C₃-alkyl or C₃-C₆-cycloalkyl, optionally substituted by one or more halogens, OH, O-alkyl (C₁-C₆), alkyl (C₁-C₄), CN or COO alkyl (C₁-C₆),
- hydroxyl,
- or amino, alkylamino having 1-4 carbon atoms, dialkylamino having 1-3 carbon atoms per alkyl group or phenyl which is optionally substituted by halogen, or
R³ and R⁵ represent a radical of the structure (IV) 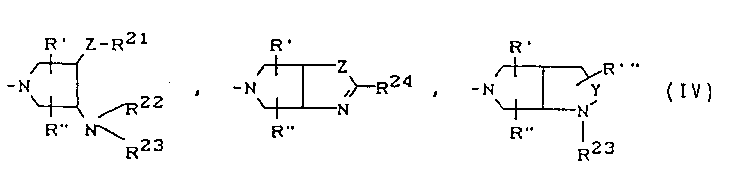 where
R²¹ can represent H, C₁-C₄-alkyl, aryl or C₁-C₄-acyl,
R²² can represent H, C₁-C₄-alkyl, OH or OCH₃, where R²¹ and R²² can together also denote a C₁-C₃-alkylene bridge which is optionally monosubstituted or disubstituted by methyl,
R²³ can represent H, C₁-C₄-alkyl, aryl, heteroaryl, benzyl, C₁-C₄-alkoxycarbonyl, C₁-C₄-acyl or (5-methyl-2-oxo-1,3-dioxol-4-yl)-methyl,
R²⁴ can represent H or C₁-C₄-alkyl,
R' can represent H, CH₃ or phenyl,
R'' can represent H, CH₃ or phenyl,
R''' can represent H or CH₃,
Y can represent O, CH₂, CH₂CH₂ or CH₂-O, where the linkage of the CH₂-O group to the nitrogen can take place both via O and via CH₂,
Z can represent O or S,
ZR²¹ can represent hydrogen, where
R⁶, R⁷, R⁸ and R⁹ are identical or different and represent
- hydrogen
- alkyl having 1 to 5 carbon atoms, nitro, amino, monoalkylamino having 1-3 C atoms, dialkylamino having 1-6 C atoms, O-alkyl having 1-5 carbon atoms, S-alkyl having 1-5 carbon atoms, trifluoromethyl, cyano or halogen,
with the exception of 1-(3-methylamino-4-fluorophenyl)-6-fluoro-7-(3-methylpiperazinyl)-1,4-dihydro-4-oxo-3-quinoline carboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid,
and the pharmaceutically acceptable derivatives of these compounds.

2. Compounds of the general formula (I) according to Claim 1, 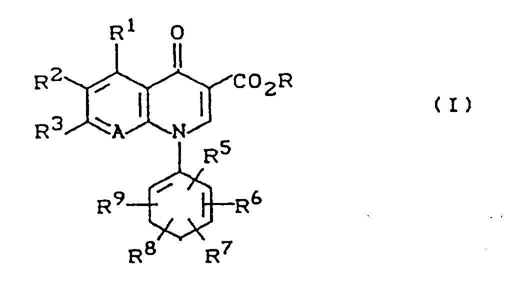 in which
R represents hydrogen, methyl or ethyl,
R¹ represents hydrogen, amino, nitro, methoxy, methylthio, cyano or halogen,
R² represents hydrogen, nitro or halogen,
A represents N or CR⁴, where
R⁴ represents hydrogen, halogen, methyl, cyano, nitro, methoxy or amino or, alternatively, represents an O, S or CH₂ bridge which is connected to the ortho-position of the phenyl ring at N-1,
R³ and R⁵ are identical or different and represent NR²⁵R²⁶, in which R²⁵ and R²⁶ are identical or different and represent hydrogen, C₁-C₂₀-alkyl, optionally substituted by one or more halogens, O-alkyl (C₁-C₆), alkyl (C₁-C₄) or phenyl which is optionally substituted by halogen, or
R³ and R⁵ represent a group having the formula (II) 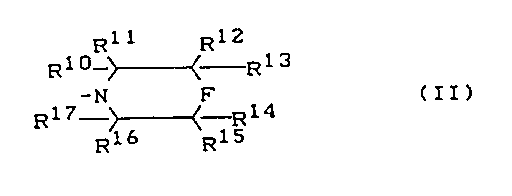 where
F denotes sulphur, oxygen, NR¹⁸ OR CR¹⁹R²⁰
and
R¹⁸ represents
- H, (C₁-C₁₀)-alkyl or (C₃-C₆)-cycloalkyl, optionally substituted by one or more substituents Hal, OH, CN, COOR or OR where R = (C₁-C₇)-alkyl,
- aryl (C₆-C₁₂), optionally substituted by halogen, -O-alkyl (C₁-C₂) or (C₁-C₄)-alkyl,
- CN,
- COOalkyl (C₁-C₄),
- C₁-C₄-acyl or
- (C₁-C₄)-sulphonyl,
R¹⁰, R¹¹, R¹², R¹³, R¹⁴, R¹⁵, R¹⁶, R¹⁷, R¹⁹ and R²⁰ are identical or different and represent
- hydrogen,
- C₁-C₃-alkyl or C₃-C₆-cycloalkyl, optionally substituted by one or more halogens, OH, methoxy, ethoxy, methyl, ethyl, cyano or CO₂Me,
- hydroxyl,
- or amino, alkylamino having 1-4 carbon atoms, dialkylamino having 1-3 carbon atoms per alkyl group or phenyl which is optionally substituted by halogen,
or
R³ and R⁵ represent a radical of the structures 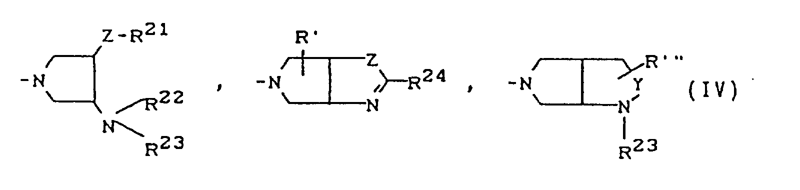 where
R²¹ can represent H, C₁-C₄-alkyl, aryl or C₁-C₄-acyl,
R²² can represent H, C₁-C₄-alkyl, OH or OCH₃, where R²¹ and R²² together can also denote a C₁-C₃-alkylene bridge which is optionally monosubstituted or disubstituted by methyl,
R²³ can represent H, C₁-C₄-alkyl, aryl, heteroaryl, benzyl, C₁-C₄-alkoxycarbonyl or C₁-C₄-acyl,
R²⁴ can represent H or C₁-C₄-alkyl,
Y can represent O, CH₂, CH₂CH₂ or CH₂-O, where the linkage of the CH₂-O group to the nitrogen can take place both via O and via CH₂,
Z can represent O or S,
ZR²¹ can represent hydrogen, and
R⁶, R⁷, R⁸ and R⁹ are identical or different and represent
- hydrogen
- methyl, ethyl, nitro, amino, monoalkylamino having 1-3 C atoms, dialkylamino having 1-4 C atoms, methoxy, ethoxy, thiomethyl, thioethyl, trifluoromethyl, cyano or halogen,
with the exception of 1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid,
and the pharmaceutically acceptable derivatives of these compounds.

3. Compounds of the general formula (I) according to Claim 1 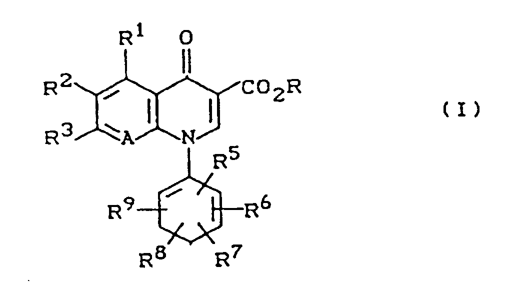 in which
R represents hydrogen, methyl or ethyl,
R¹ represents hydrogen or halogen,
R² represents fluorine, nitro or hydrogen,
A represents N or CR⁴, where
R⁴ represents hydrogen or halogen,
R³ and R⁵ are identical or different and represent NR²⁵R²⁶, in which R²⁵ and R²⁶ are identical or different and represent hydrogen, C₁-C₁₀-alkyl, optionally substituted by one or more halogens, O-alkyl (C₁-C₆), alkyl (C₁-C₄) or phenyl which is optionally substituted by halogen, or
R³ and R⁵ represent a group having the formula (II) 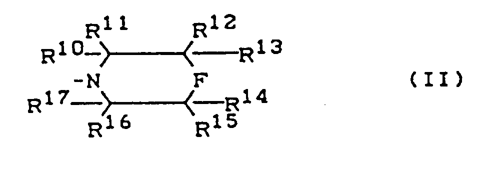 where
F denotes sulphur, oxygen, NR¹⁸ or CR¹⁹R²⁰
and
R¹⁸ represents
- hydrogen,
- methyl, ethyl, propyl,
- (C₃-C₆)-cycloalkyl,
- phenyl, optionally substituted by halogen,
- CN,
- COO-alkyl (C₁-C₄),
- C₁-C₄-acyl or
- (C₁-C₄)-sulphonyl, and
R¹⁰, R¹¹, R¹⁷ and R¹⁶ represent hydrogen,
R¹², R¹³, R¹⁴, R¹⁵, R¹⁹ and R²⁰ are identical or different and represent,
- hydrogen,
- C₁-C₃-alkyl or C₃-C₆-cycloalkyl, optionally substituted by one or more halogens, OH, methoxy, ethoxy, methyl, ethyl, cyano, CO₂Me,
- hydroxyl,
- or amino, alkylamino having 1-4 carbon atoms or dialkylamino having 1-3 carbon atoms,
R³ and R⁵ are identical or different and represent a group of the formula 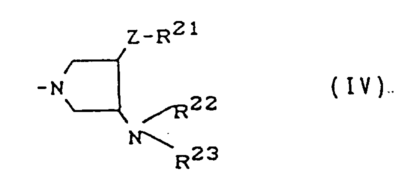 where
R²¹ can represent hydrogen, methyl, ethyl, phenyl or C₁-C₄-acyl,
R²² can represent hydrogen, methyl, ethyl, OH or OCH₃, where R²¹ and R²² may together also denote a C₁-C₃-alkylene bridge which is optionally monosubstituted or disubstituted by methyl,
R²³ can represent H, methyl, ethyl, aryl, benzyl, C₁-C₄-alkoxycarbonyl or C₁-C₄-acyl,
Z can represent O or S,
ZR²¹ can represent hydrogen,
with the exception of 1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl1,4-dihydro-4-oxo-3-quinolinecarboxylic acid,
and the pharmaceutically acceptable derivatives of these compounds.

4. Process for the preparation of compounds of the formula (I) according to Claim 1, in which R³ and R⁵ are identical, with the exception of 1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid, characterized in that quinolines of the general formula (III) 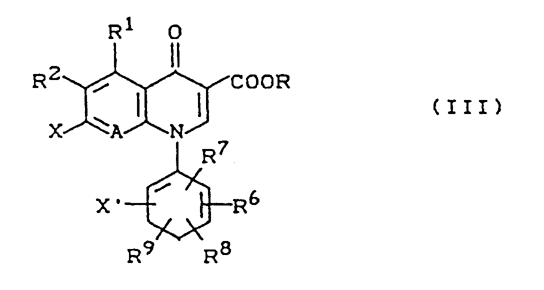 in which
A, R, R¹, R², R⁶, R⁷, R⁸ and R⁹ have the meaning intended in Claim 1 and X and X' are identical or different and represent a halogen atom, are reacted with amines of the general formulae (II) and (IV) 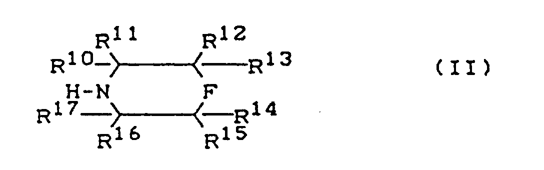 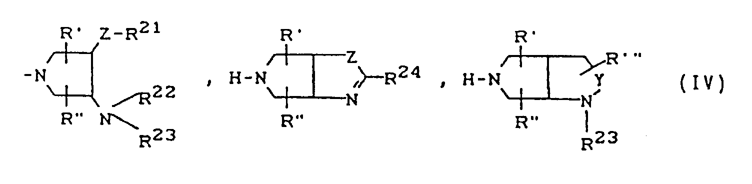 in which
R¹⁰ to R¹⁷, R²¹ to R²⁴, Y, Z, R', R'', R''' and F have the meaning indicated in Claim 1, if appropriate in the presence of diluents and if appropriate in the presence of acid binders.

5. Process for the preparation of the compounds of the formula (I) according to Claim 1, with the exception of
1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid, characterized in that compounds of the general formula (V) 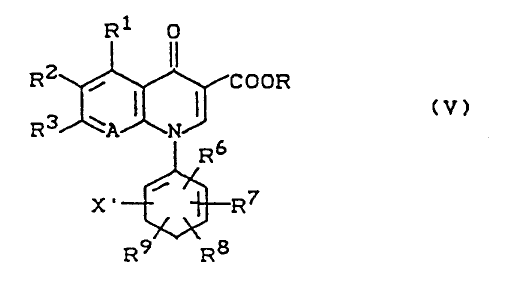 in which A, R, R¹ to R⁹ have the abovementioned meaning and X' represents a halogen atom, are reacted with amines of the general formulae (II) and (IV) 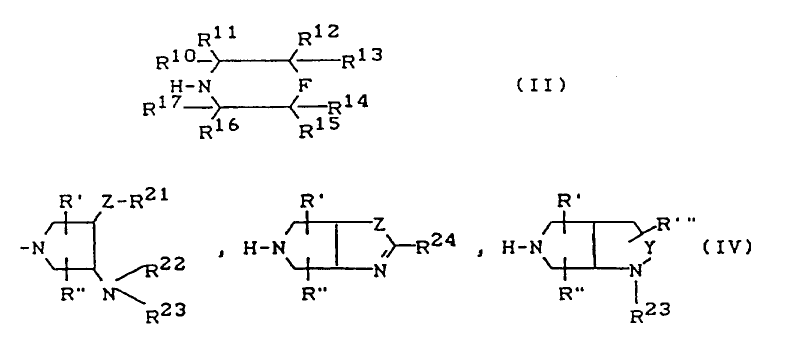 in which
R¹⁰ to R¹⁷, R²¹ to R²⁴, R', R'', and R''', Y, Z and F have the meaning indicated in Claim 1, if appropriate in the presence of diluents and if appropriate in the presence of acid binders.

6. Process for the preparation of the compounds of the formula (I) according to Claim 1, with the exception of
1-(3-methylamino-4-fluorophenyl)-6-fluoro-7-(3-methylpiperazinyl)-1,4-dihydro-4-oxo-3-quinolinecarboxylic acid,
1-(4-aminophenyl)-6-fluoro-7-piperazinyl-1,4-dihydro-4-oxo-3-quinolinecarboxylic acid,
1-(4-aminophenyl)-6-fluoro-7-(4-methylpiperazinyl)-1,4-dihydro-4-oxo-3-quinolinecarboxylic acid and
1-(4-aminophenyl)-6,8-difluoro-7-morpholinyl-1,4-dihydro-4-oxo-3-quinolinecarboxylic acid, characterized in that quinolines of the general formula (VI) 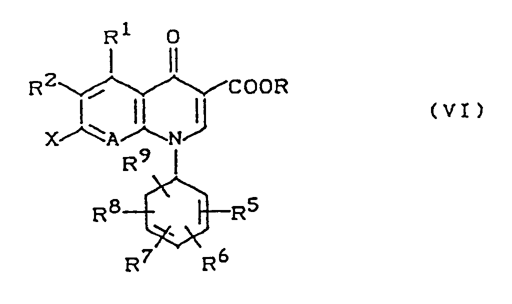 in which A, R, R¹, R², R⁵, R⁶, R⁷, R⁸ and R⁹ have the meaning indicated in Claim 1 and X represents halogen, are reacted with amines of the general formulae (II) and (IV) 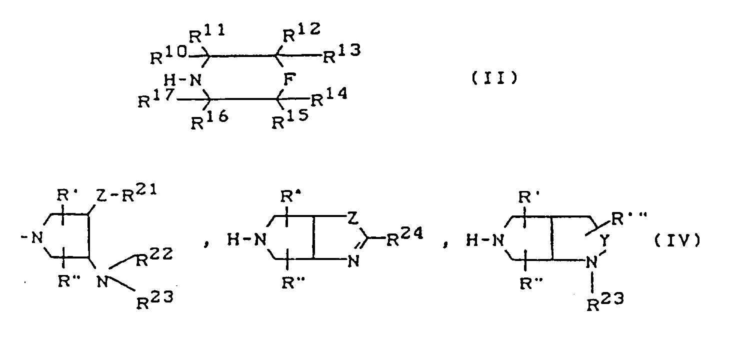 in which
R¹⁰ to R¹⁷, R²¹ to R²⁴, R', R'' and R''', Y, Z and F have the meaning indicated in Claim 1,
if appropriate in the presence of diluents and if appropriate in the presence of acid binders.

7. Process for the preparation of the compounds of the formula (I) according to Claim 1, with the exception of 1-(3-methylamino-4-fluorophenyl)-6-fluoro-7-(3-methylpiperazinyl)-1,4-dihydro-4-oxo-3-quinolinecarboxylic acid and 1-(4-aminophenyl)-6-fluoro-7-(4-methylpiperazinyl)-1,4-dihydro-4-oxo-3-quinolinecarboxylic acid, characterized in that compounds of the general formula (VII) 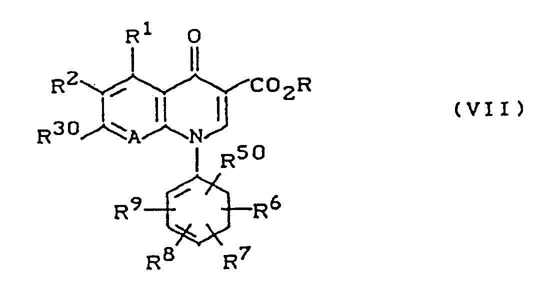 where R, R¹, R², A and R⁶-R⁹ have the meaning indicated in Claim 1, the radicals R³⁰ and R⁵⁰ are identical or different, where secondary or primary amines are present in R³⁰ and R⁵⁰ or only in R³⁰ or only in R⁵⁰ and the structures of R³⁰ and R⁵⁰ are determined by the substituents of the radicals R³ or R⁵,
are reacted with acylating agents or alkylating agents of the general formula (VIII)
V - W (VIII)
in which
W = - CN
- (C₂-C₄)-acyl, optionally substituted
- (C₁-C₄)-sulphonyl
- (C₁-C₄)-alkoxycarbonyl
- (C₁-C₅)-alkyl
and V represents a leaving group, such as
V = - halogen
- trifluoromethanesulphonate
- mesylate
- tosylate
- acetate
- trihaloacetate
if appropriate in the presence of diluents and if appropriate in the presence of acid binders.

8. Process for the preparation of the compounds of the general formula (I) according to Claim 1, with the exception of 1-(3-methylamino-4-fluorophenyl)-6-fluoro-7-(3-methylpiperazinyl)-1,4-dihydro-4-oxo-3-quinolinecarboxylic acid, characterized in that compounds of the general formula (VII) according to Claim 7 are reacted with compounds of the general formula (IX)
CH₂=CH-U (IX)
in which U denotes an electron-attracting group such as COOB, COB, CONH₂, CN, SO₂B or SO₃B and B represents (C₁-C₄)-alkyl which is optionally substituted,
if appropriate in the presence of diluents.

9. Process for the preparation of the compounds of the formula (I) according to Claim 1, with the exception of 1-(3-methylamino-4-fluorophenyl)-6-fluoro-7-(3-methylpiperazinyl)-1,4-dihydro-4-oxo-3-quinolinecarboxylic acid, characterized in that compounds of the general formula (VII) according to Claim 7 are reacted with carboxylic acids of the general formula (X)
D-COOH (X)
in which D represents H or optionally substituted (C₁-C₄)-alkyl, if appropriate in the presence of diluents and if appropriate in the presence of dehydrating reagents and if appropriate in the presence of a catalyst.

## Revendications (Revendications pour l'(les) Etat(s) contractant(s) suivant(s): AT, BE, CH, DE, DK, FR, GB, IT, LI, LU, NL, SE)

1. Composés de formule générale (I) 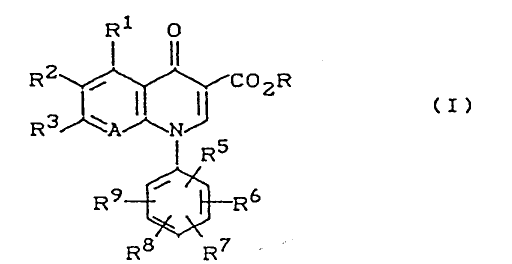 dans laquelle
R est de l'hydrogène ou un groupe alkyle ayant 1 à 4 atomes de carbone,
R¹ est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène, un groupe cyano ou nitro,
R² est de l'hydrogène, un groupe nitro ou un halogène,
A représente N ou un groupe CR₄, dans lequel
R⁴ est de l'hydrogène, un halogène, un radical méthyle, cyano, nitro, méthoxy ou amino, ou représente aussi un pont par l'intermédiaire de O, S ou CH₂ qui et lié en N-1 à la position ortho du noyau phényle,
R³ et R⁵ sont égaux ou différents et représentent un groupe NR²⁵R²⁶ dans lequel R²⁵ et R²⁶ sont égaux ou différents et représentent de l'hydrogène, un radical alkyle en C₁ à C₂₀, portant éventuellement un ou plusieurs substituants halogéno, OH, O-(alkyle en C₁ à C₆), alkyle en C₁ à C₄, CN, COO-(alkyle en C₁ à C₆),
- un groupe hydroxyle,
- un groupe amino, alkylamino ayant 1 à 4 atomes de carbone, dialkylamino ayant 1 à 3 atomes de carbone par groupe alkyle, ou phényle éventuellement substitué par un halogène, ou bien
R³ et R⁵ forment un groupe de formule (II) 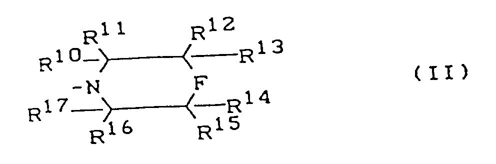
dans laquelle
F représente O, S, un groupe NR¹⁸ ou CR⁹R²⁰ et
R⁶, R⁷, R⁸ et R⁹ sont égaux ou différents et représentent
- de l'hydrogène,
- un groupe méthyle, éthyle, nitro, amino, monoalkylamino ayant 1 à 3 atomes de carbone, dialkylamino ayant 1 à 4 atomes de carbone, méthoxy, éthoxy, thiométhyle, thioéthyle,
trifluorométhyle, cyano, halogéno,
R¹⁸ représente
- H, un radical alkyle en C₁ à C₁₀ ou cycloalkyle en C₃ à C₆, portant éventuellement un ou plusieurs substituants Hal, OH, CN, COOR, OR dans lequel R est un radical alkyle en C₁ à C₇,
- un radical aryle en C₆ à C₁₂, portant éventuellement un substituant halogéno, -O-alkyle en C₁ à C₂ ou alkyle en C₁ à C₄,
- un radical CN
- un radical COO- (alkyle en C₁ à C₄),
- acyle en C₁ à C₄ ou
- sulfonyle en C₁ à C₄,
R¹⁰, R¹¹, R¹², R¹³, R¹⁴, R¹⁵, R¹⁶, R¹⁷, R¹⁹ et R²⁰ sont égaux ou différents et représentent :
- de l'hydrogène,
- un groupe alkyle en C₁ à C₃ ou cycloalkyle en C₃ à C₆ portant le cas échéant un ou plusieurs substituants halogéno, OH, O-alkyle en C₁ à C₆, alkyle en C₁ à C₄, CN, COO-(alkyle en C₁ à C₆),
- un groupe hydroxyle,
- un groupe amino, alkylamino ayant 1 à 4 atomes de carbone, dialkylamino ayant 1 à 3 atomes de carbone par groupe alkyle, ou phényle éventuellement substitué par un halogène, ou bien
R³ et R⁵ représentent un reste de structure (IV) 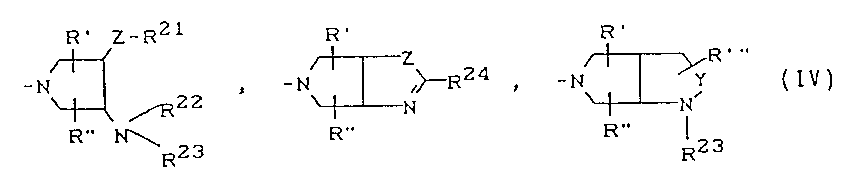
dans laquelle
R²¹ représente H, un groupe alkyle en C₁ à C₄, aryle, acyle en C₁ à C₄,
R²² représente H, un groupe alkyle en C₁ à C₄, OH, OCH₃, R²¹ et R²² pouvant aussi former ensemble un pont alkylène en C₁ à C₃ éventuellement substitué une ou deux fois par un radical alkyle,
R²³ peut être de l'hydrogène, un groupe alkyle en C₁ à C₄, aryle, hétéroaryle, benzyle, (alkoxy en C₁ à C₄)-carbonyle, acyle en C₁ à C₄ ou (5-méthyl-2-oxo-1,3-dioxol-4-yl)-méthyle,
R²⁴ peut représenter de l'hydrogène ou un groupe alkyle en C₁ à C₄,
R' peut représenter de l'hydrogène, un groupe CH₃ ou phényle,
R'' peut représenter de l'hydrogène, un groupe CH₃ ou phényle,
R''' peut représenter de l' hydrogène ou un groupe CH₃,
Y peut représenter de l'oxygène, un groupe CH₂, CH₂CH₂ ou CH₂-O, la liaison du groupe CH₂-O à l'azote pouvant être effectuée aussi bien par l'intermédiaire de O que par l'intermédiaire de CH₂,
Z peut représenter O ou S,
ZR²¹ peut être de l'hydrogène,
R⁶, R⁷, R⁸ et R⁹ étant égaux ou différents et représentant
- de l'hydrogène,
- un groupe alkyle ayant 1 à 5 atomes de carbone, nitro, amino, monoalkylamino ayant 1 à 3 atomes de carbone, dialkylamino ayant 1 à 6 atomes de carbone, O-alkyle ayant 1 à 5 atomes de carbone, S-alkyle ayant 1 à 5 atomes de carbone, trifluorométhyle, cyano, halogéno,
à l'exception :
de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique,
et les dérivés acceptables du point de vue pharmaceutique de ces composés.

2. Composés de formule générale (I) suivant la revendication 1, 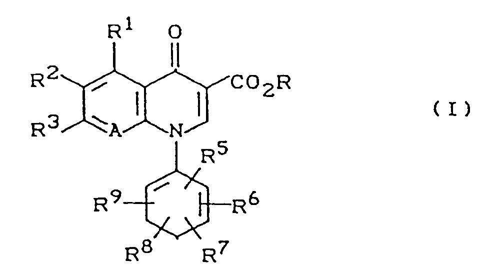 dans laquelle
R représente de l'hydrogène, un groupe méthyle ou éthyle,
R¹ est de l'hydrogène, un groupe amino, nitro, méthoxy, méthylthio, cyano ou un halogène,
R² est de l'hydrogène, un groupe nitro ou un halogène,
A représente N ou un groupe CR₄, dans lequel
R⁴ est de l'hydrogène, un halogène, un groupe méthyle, cyano, nitro, méthoxy ou amino ou aussi un pont par l'intermédiaire de O, S ou CH₂ qui est lié en N-1 à la position ortho du noyau phényle,
R³ et R⁵ sont égaux ou différents et représentent un groupe NR²⁵R²⁶, dans lequel R²⁵ et R²⁶ sont égaux ou différents et représentent de l'hydrogène, un radical alkyle en C₁ à C₂₀, portant le cas échéant un ou plusieurs substituants halogéno, O-alkyle en C₁ à C₆, alkyle en C₁ à C₄ par groupe alkyle ou phényle éventuellement substitué par un halogène, ou bien
R³ et R⁵ représentent un groupe de formule (II) 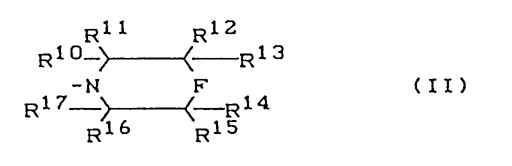
dans laquelle
F représente du soufre, de l'oxygène, un groupe NR¹⁸ ou CR¹⁹R²⁰ et
et
R¹⁸ représente
- de l'hydrogène, un groupe alkyle en C₁ à C₁₀ ou cycloalkyle en C₃ à C₁₀, portant éventuellement un ou plusieurs substituants Hal, OH, CN, COOR, OR dans lequel R est un radical alkyle en C₁ à C₇,
- un groupe aryle en C₆ à C₁₂, éventuellement substitué par un halogène, un radical -O-alkyle en C₁ ou C₂ ou alkyle en C₁ à C₄,
- un groupe CN,
- un groupe COO-(alkyle en C₁ à C₄),
- un groupe acyle en C₁ à C₄ ou
- un groupe sulfonyle en C₁ à C₄,
R¹⁰, R¹¹, R¹², R¹³, R¹⁴, R¹⁵, R¹⁶, R¹⁷, R¹⁹ et R²⁰ sont égaux ou différents et représentent :
- de l'hydrogène,
- un groupe alkyle en C₁ à C₃ ou cycloalkyle en C₃ à C₆ portant le cas échéant un ou plusieurs substituants halogéno, OH, méthoxy, éthoxy, méthyle, éthyle, cyano, CO₂Me,
- un groupe hydroxyle,
- un groupe amino, alkylamino ayant 1 à 4 atomes de carbone, dialkylamino ayant 1 à 3 atomes de carbone par groupe alkyle ou un groupe phényle éventuellement substitué par un halogène,
ou bien
R³ et R⁵ représentent un reste des structures 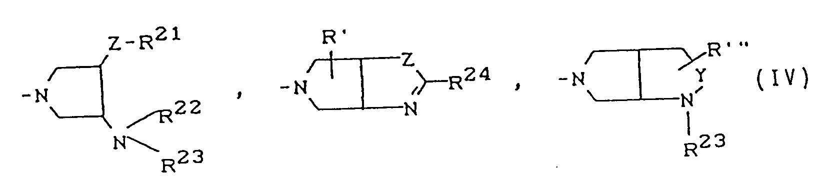
dans lesquelles
R²¹ représente H, un groupe alkyle en C₁ à C₄, aryle, acyle en C₁ à C₄,
R²² représente H, un groupe alkyle en C₁ à C₄, OH, OCH₃, R²¹ et R²² pouvant aussi former ensemble un pont alkylène en C₁ à C₃ portant éventuellement un ou deux substituants méthyle,
R²³ représente H, un groupe alkyle en C₁ à C₄, aryle, hétéroaryle, benzyle, (alkoxy en C₁ à C₄)carbonyle, acyle en C₁ à C₄,
R²⁴ représente H ou un groupe alkyle en C₁ à C₄,
Y peut représenter O, CH₂, CH₂CH₂ ou CH₂-O, la liaison du groupe CH₂-O à l'azote pouvant être effectuée aussi bien par l'intermédiaire de O que par l'intermédiaire de CH₂,
Z peut représenter O ou S,
ZR²¹ peut représenter de l'hydrogène,
R⁶, R⁷, R⁸ et R⁹ étant égaux ou différents et représentant
- de l'hydrogène,
- un groupe méthyle, éthyle, nitro, amino, monoalkylamino ayant 1 à 3 atomes de carbone, dialkylamino ayant 1 à 4 atomes de carbone, méthoxy, éthoxy, thiométhyle, thioéthyle, trifluorométhyle, cyano, halogéno,
à l'exception
de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique,
et les dérivés acceptables du point de vue pharmaceutique de ces composés.

3. Composés de formule générale (I) suivant la revendication 2 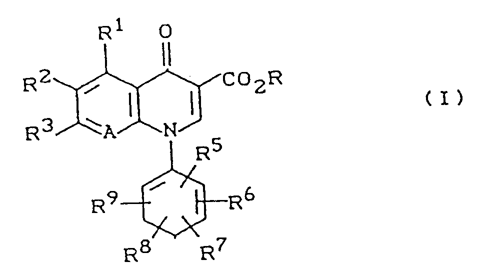 dans laquelle
R représente de l'hydrogène, un groupe méthyle ou éthyle,
R¹ est de l'hydrogène, un halogène,
R² est du fluor, un groupe nitro ou de l'hydrogène,
A représente N ou CR₄ où
R⁴ est de l'hydrogène ou un halogène,
R³ et R⁵ sont égaux ou différents et représentent un groupe NR²⁵R²⁶ dans lequel R²⁵ et R²⁶ sont égaux ou différents et représentent de l'hydrogène, un radical alkyle en C₁ à C₁₀, portant le cas échéant un ou plusieurs substituants halogéno, O-alkyle en C₁ à C₆, alkyle en C₁ à C₄ par groupe alkyle ou un groupe phényle éventuellement substitué par un halogène ou bien
R³ et R⁵ représentent un groupe de formule (II) 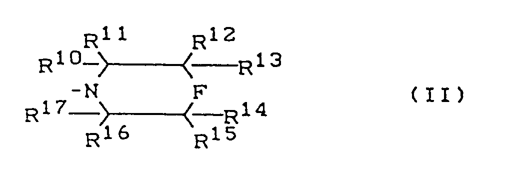
dans laquelle
F est du soufre, de l'oxygène, un groupe NR¹⁸ ou CR¹⁹R²⁰
et
R¹⁸ représente
- de l'hydrogène,
- un groupe méthyle, éthyle, propyle,
- un groupe cycloalkyle en C₃ à C₆,
- un groupe phényle éventuellement substitué par un halogène,
- un groupe CN,
- un groupe -COO-alkyle en C₁ à C₄,
- acyle en C₁ à C₄,
- sulfonyle en C₁ à C₄, et
R¹⁰, R¹¹, R¹⁷, R¹⁶ représentent de l'hydrogène, R¹², R¹³, R¹⁴, R¹⁵, R¹⁹, R²⁰ sont égaux ou différents et représentent
- de l'hydrogène,
- un groupe alkyle en C₁ à C₃ ou cycloalkyle en C₃ à C₆, portant éventuellement un ou plusieurs substituants halogéno, OH, méthoxy, éthoxy, méthyle, éthyle, cyano, CO₂Me
- un groupe hydroxyle,
- ou un groupe amino, alkylamino ayant 1 à 4 atomes de carbone, dialkylamino ayant 1 à 3 atomes de carbone,
R³ et R⁵ sont égaux ou différents et représentent un groupe de formule 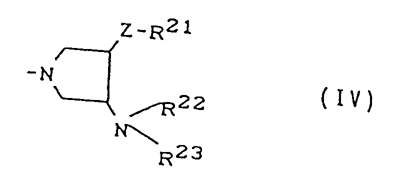
dans laquelle
R²¹ est de l'hydrogène, un groupe méthyle, éthyle, phényle, acyle en C₁ à C₄,
R²² est de l'hydrogène, un groupe méthyle, éthyle, OH, OCH₃, R²¹ et R²² pouvant aussi représenter un pont alkylène en C₁ à C₃ substitué une ou deux fois par un radical méthyle,
R²³ représente de l'hydrogène, un groupe méthyle, éthyle, aryle, benzyle, (alkoxy en C₁ à C₄)-carbonyle, acyle en C₁ à C₄,
Z représente O ou S,
ZR²¹ peut représenter de l'hydrogène,
à l'exception
de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique,
et les dérivés acceptables du point de vue pharmaceutique de ces composés.

4. Procédé de production de composés de formule (I) suivant la revendication 1, dans laquelle R³ et R⁵ sont égaux, à l'exception
de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique, caractérisé en ce qu'on fait réagir des quinoléines de formule générale (III) 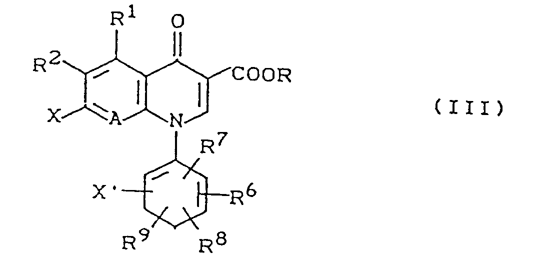 dans laquelle
A, R, R¹, R², R⁶, R⁷, R⁸ et R⁹ ont la définition indiquée dans la revendication 1 et X et X' sont égaux ou différents et représentent un atome d'halogène,
avec des amines de formules générales (II) et (IV) 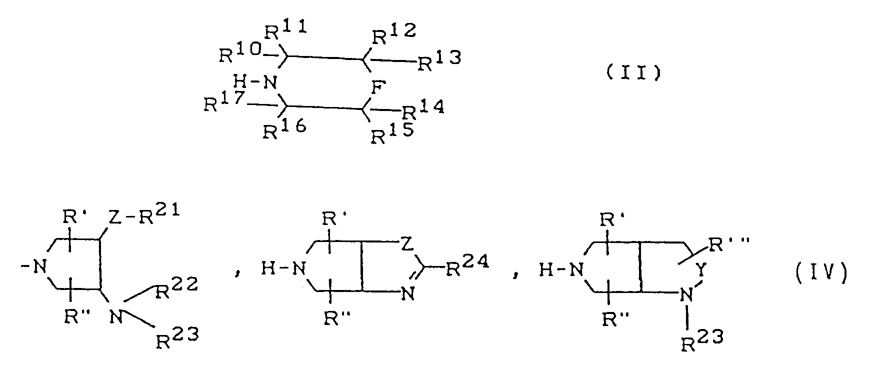 dans lesquelles
R¹⁰ à R¹⁷, R²¹ à R²⁴, Y, Z, R', R'', R''' et F ont la définition indiquée dans la revendication 1,
le cas échéant en présence de diluants et en la présence éventuelle d'accepteurs d'acides.

5. Procédé de production des composés de formule (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique, caractérisé en ce qu'on fait réagir des composés de formule générale (V) 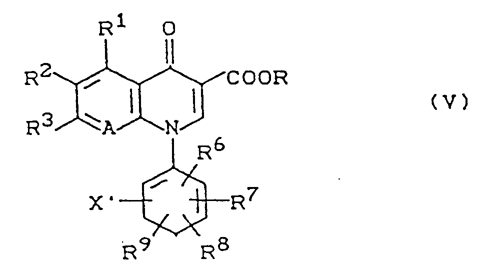 dans laquelle
A, R, R¹ à R⁹ ont la définition indiquée dans la revendication 1 et X' représente un atome d'halogène,
avec des amines de formules générales (II) et (IV) 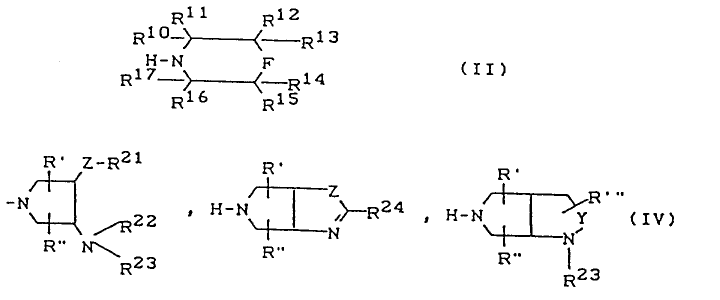 dans lesquelles
R¹⁰ à R¹⁷, R²¹ à R²⁴, R', R'' et R''', Y, Z et F ont la définition indiquée dans la revendication 1,
le cas échéant en présence de diluants et en la présence éventuelle d'accepteurs d'acides.

6. Procédé de production des composés de formule (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique, caractérisé en ce qu'on fait réagir des quinoléines de formule générale (VI) 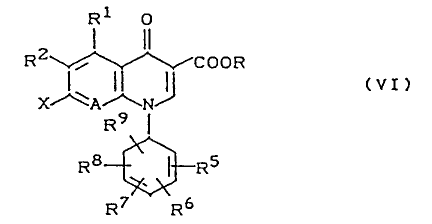 dans laquelle
A, R, R¹, R², R⁵, R⁶, R⁷, R⁸ et R⁹ ont la définition indiquée dans la revendication 1 et X représente un halogène, avec des amines de formules (II) et (IV), 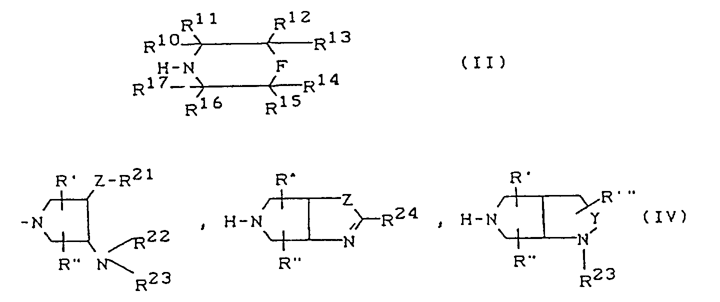 dans lesquelles
R¹⁰ à R¹⁷, R²¹ à R²⁴, R', R'' et R''', Y, Z et F ont la définition indiquée dans la revendication 1,
le cas échéant en présence de diluants et en la présence éventuelle d'accepteurs d'acides.

7. Procédé de production des composés de formule (I) suivant la revendication 1, à l'exception de l'acide 1-(3-méthylamino-4-fluorophényl)-6-fluoro-7-(3-méthylpipérazi nyl)-1,4-dihydro-4-oxo-3-quinoléinecarboxylique, et de l'acide 1-(4-aminophényl)-6-fluoro-7-(4-méthylpipérazinyl)-1,4-dihydro-4-oxo-3-quinoléinecarboxylique, caractérisé en ce qu'on fait réagir des composés de formule (VII) 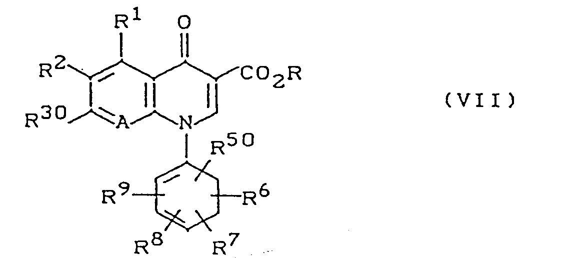 dans laquelle R, R¹, R², A, R⁶ à R⁹ ont la définition indiquée dans la revendication 1, les restes R³⁰ et R⁵⁰ sont égaux ou différents, les amines secondaires ou primaires étant présentes dans R³⁰ et R⁵⁰ ou seulement dans R³⁰ ou seulement dans R⁵⁰ et les structures de R³⁰ et R⁵⁰ étant déterminées par les représentants des restes R³ et respectivement R⁵,
avec des agents acylants ou des agents alkylants de formule générale (VIII)
V - W (VIII)
dans laquelle
W représente
- un groupe CN
- un groupe acyle en C₂ à C₄ éventuellement substitué,
- un groupe sulfonyle en C₂ à C₄,
- un groupe (alkoxy en C₁ à C₄)-carbonyle,
- un groupe alkyle en C₁ à C₅ et
V représente un groupe partant tel que :
V = - un halogène
- un groupe trifluorométhylsulfonate
- un groupe mésylate
- un groupe tosylate
- un groupe acétate
- un groupe trihalogénacétate
le cas échéant en présence de diluants et en la présence éventuelle d'accepteurs d'acides.

8. Procédé de production des composés de formule générale (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxylique, caractérisé en ce qu'on fait réagir des composés de formule générale (VII) suivant la revendication 7 avec des composés de formule générale (IX)
CH₂=CH-U (IX)
dans laquelle U est un groupe attirant les électrons, tel que COOB, COB, CONH₂, CN, SO₂B, SO₃B et B représente un groupe alkyle en C₁ à C₄ qui est éventuellement substitué, le cas échéant en présence de diluants.

9. Procédé de production des composés de formule (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxylique, caractérisé en ce qu'on fait réagir des composés de formule générale (VII) suivant la revendication 7 avec des acides carboxyliques de formule générale (X)
D-COOH (X)
dans laquelle D représente de l'hydrogène ou un groupe alkyle en C₁ à C₄ éventuellement substitué, le cas échéant en présence de diluants, et en la présence éventuelle de réactifs déshydratants et, le cas échéant, en présence d'un catalyseur.

10. Médicaments contenant des composés suivant les revendications 1 à 3.

11. Agents antiviraux, contenant des composés suivants les revendications 1 à 3.

12. Utilisation de composés de formule générale 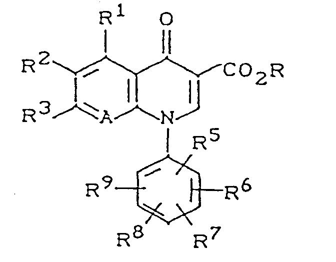 (I) dans laquelle
R est de l'hydrogène ou un groupe alkyle ayant 1 à 4 atomes de carbone,
R¹ est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène, un groupe cyano ou nitro,
R² est de l'hydrogène, un groupe nitro ou un halogène,
A représente N ou un groupe CR₄, dans lequel
R⁴ est de l'hydrogène, un halogène, un radical méthyle, cyano, nitro, méthoxy ou amino, ou représente aussi un pont par l'intermédiaire de O, S ou CH₂ qui et lié en N-1 à la position ortho du noyau phényle,
R³ et R⁵ sont égaux ou différents et représentent un groupe NR²⁵R²⁶ dans lequel R²⁵ et R²⁶ sont égaux ou différents et représentent de l'hydrogène, un radical alkyle en C₁ à C₂₀, portant éventuellement un ou plusieurs substituants halogéno, OH, O-(alkyle en C₁ à C₆), alkyle en C₁ à C₄, CN, COO-(alkyle en C₁ à C₆),
- un groupe hydroxyle,
- un groupe amino, alkylamino ayant 1 à 4 atomes de carbone, dialkylamino ayant 1 à 3 atomes de carbone par groupe alkyle, ou phényle éventuellement substitué par un halogène, ou bien
R³ et R⁵ forment un groupe de formule (II) 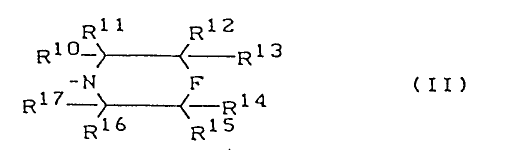
dans laquelle
F représente O, S, un groupe NR¹⁸ ou CR⁹R²⁰ et
R⁶, R⁷, R⁸ et R⁹ sont égaux ou différents et représentent
- de l'hydrogène,
- un groupe méthyle, éthyle, nitro, amino, monoalkylamino ayant 1 à 3 atomes de carbone, dialkylamino ayant 1 à 4 atomes de carbone, méthoxy, éthoxy, thiométhyle, thioéthyle, trifluorométhyle, cyano, halogéno,
R¹⁸ représente
- H, un radical alkyle en C₁ à C₁₀ ou cycloalkyle en C₃ à C₆, portant éventuellement un ou plusieurs substituants Hal, OH, CN, COOR, OR dans lequel R est un radical alkyle en C₁ à C₇,
- un radical aryle en C₆ à C₁₂, portant éventuellement un substituant halogéno, -O-alkyle en C₁ à C₂ ou alkyle en C₁ à C₄,
- un radical CN
- un radical COO- (alkyle en C₁ à C₄),
- acyle en C₁ à C₄ ou
- sulfonyle en C₁ à C₄,
R¹⁰, R¹¹, R¹², R¹³, R¹⁴, R¹⁵, R¹⁶, R¹⁷, R¹⁹ et R²⁰ sont égaux ou différents et représentent :
- de l'hydrogène,
- un groupe alkyle en C₁ à C₃ ou cycloalkyle en C₃ à C₆ portant le cas échéant un ou plusieurs substituants halogéno_{,} OH, O-alkyle en C₁ à C₆, alkyle en C₁ à C₄, CN, COO-(alkyle en C₁ à C₆),
- un groupe hydroxyle,
- un groupe amino, alkylamino ayant 1 à 4 atomes de carbone, diaikylamino ayant 1 à 3 atomes de carbone par groupe alkyle, ou phényle éventuellement substitué par un halogène, ou bien
R³ et R⁵ représentent un reste de structure (IV) 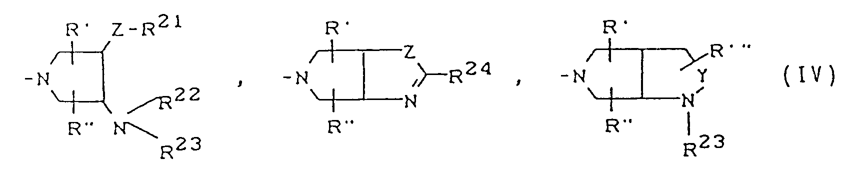
dans laquelle
R²¹ représente H, un groupe alkyle en C₁ à C₄, aryle, acyle en C₁ à C₄,
R²² représente H, un groupe alkyle en C₁ à C₄, OH, OCH₃, R²¹ et R²² pouvant aussi former ensemble un pont alkylène en C₁ à C₃ éventuellement substitué une ou deux fois par un radical alkyle,
R²³ peut être de l'hydrogène, un groupe alkyle en C₁ à C₄, aryle, hétéroaryle, benzyle, (alkoxy en C₁ à C₄)-carbonyle, acyle en C₁ à C₄ ou (5-méthyl-2-oxo-1,3-dioxol-4-yl)-méthyle,
R²⁴ peut représenter de l'hydrogène ou un groupe alkyle en C₁ à C₄,
R' peut représenter de l'hydrogène, un groupe CH₃ ou phényle,
R'' peut représente de l'hydrogène, un groupe CH₃ ou phényle,
R''' peut représenter de l'hydrogène ou un groupe CH₃,
Y peut représenter de l'oxygène, un groupe CH₂, CH₂CH₂ ou CH₂-O, la liaison du groupe CH₂-O à l'azote pouvant être effectuée aussi bien par l'intermédiaire de O que par l'intermédiaire de CH₂,
Z peut représente O ou S,
ZR²¹ peut être de l'hydrogène,
R⁶, R⁷, R⁸ et R⁹ étant égaux ou différents et représentant
- de l'hydrogène,
- un groupe alkyle ayant 1 à 5 atomes de carbone, nitro, amino, monoalkylamino ayant 1 à 3 atomes de carbone, dialkylamino ayant 1 à 6 atomes de carbone, O-alkyle ayant 1 à 5 atomes de carbone, S-alkyle ayant 1 à 5 atomes de carbone, trifluorométhyle, cyano, halogéno,
et les dérivés acceptables du point de vue pharmaceutique de ces composés pour la préparation d'un médicament destiné à être utilisé dans le traitement antiviral du corps humain ou d'un animal.

13. Utilisation de composés suivant la revendication 12, de formule générale (I) 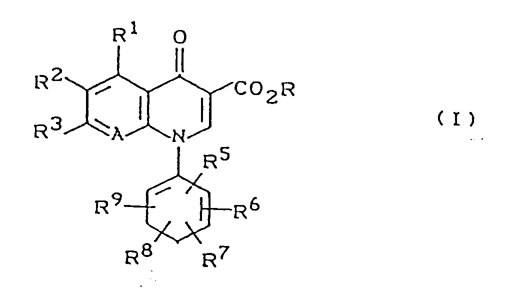 dans laquelle
R représente de l'hydrogène, un groupe méthyle ou éthyle,
R¹ est de l'hydrogène, un groupe amino, nitro, méthoxy, méthylthio, cyano ou un halogène,
R² est de l'hydrogène, un groupe nitro ou un halogène,
A représente N ou un groupe CR₄, dans lequel
R⁴ est de l'hydrogène, un halogène, un groupe méthyle, cyano, nitro, méthoxy ou amino ou aussi un pont par l'intermédiaire de O, S ou CH₂ qui est lié en N-1 à la position ortho du noyau phényle,
R³ et R⁵ sont égaux ou différents et représentent un groupe NR²⁵R²⁶, dans lequel R²⁵ et R²⁶ sont égaux ou différents et représentent de l'hydrogène, un radical alkyle en C₁ à C₂₀, portant le cas échéant un ou plusieurs substituants halogéno, O-alkyle en C₁ à C₆, alkyle en C₁ à C₄ par groupe alkyle ou phényle éventuellement substitué par un halogène, ou bien
R³ et R⁵ représentent un groupe de formule (II) 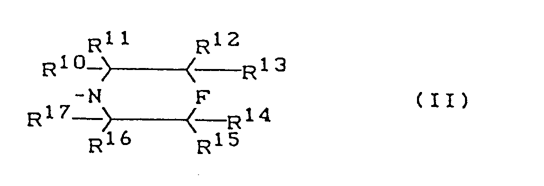
dans laquelle
F représente du soufre, de l'oxygène, un groupe NR¹⁸ ou CR¹⁹R²⁰ et
et
R¹⁸ représente
- de l'hydrogène, un groupe alkyle en C₁ à C₁₀ ou cycloalkyle en C₃ à C₁₀, portant éventuellement un ou plusieurs substituants Hal, OH, CN, COOR, OR dans lequel R est un radical alkyle en C₁ à C₇,
- un groupe aryle en C₆ à C₁₂, éventuellement substitué par un halogène, un radical -O-alkyle en C₁ ou C₂ ou alkyle en C₁ à C₄,
- un groupe CN,
- un groupe COO-(alkyle en C₁ à C₄),
- un groupe acyle en C₁ à C₄ ou
- un groupe sulfonyle en C₁ à C₄,
R¹⁰, R¹¹, R¹², R¹³, R¹⁴, R¹⁵, R¹⁶, R¹⁷_{,} R¹⁹ et R²⁰ sont égaux ou différents et représentent :
- de l'hydrogène,
- un groupe alkyle en C₁ à C₃ ou cycloalkyle en C₃ à C₆ portant le cas échéant un ou plusieurs substituants halogéno, OH, méthoxy, éthoxy, méthyle, éthyle, cyano, CO₂Me,
- un groupe hydroxyle,
- un groupe amino, alkylamino ayant 1 à 4 atomes de carbone, dialkylamino ayant 1 à 3 atomes de carbone par groupe alkyle ou un groupe phényle éventuellement substitué par un halogène, ou bien
R³ et R⁵ représentent un reste de structures 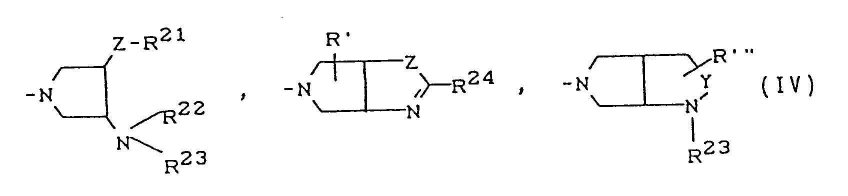
dans lesquelles
R²¹ représente H, un groupe alkyle en C₁ à C₄, aryle, acyle en C₁ à C₄,
R²² représente H, un groupe alkyle en C₁ à C₄, OH, OCH₃, R²¹ et R²² pouvant aussi former ensemble un pont alkylène en C₁ à C₃ portant éventuellement un ou deux substituants méthyle,
R²³ représente H, un groupe alkyle en C₁ à C₄, aryle, hétéroaryle, benzyle, (alkoxy en C₁ à C₄)carbonyle, acyle en C₁ à C₄,
R²⁴ représente H ou un groupe alkyle en C₁ à C₄,
Y peut représenter O, CH₂, CH₂CH₂ ou CH₂-O, la liaison du groupe CH₂-O à l'azote pouvant être effectuée aussi bien par l'intermédiaire de O que par l'intermédiaire de CH₂,
Z peut représente O ou S,
ZR²¹ peut représenter de l'hydrogène,
R⁶, R⁷, R⁸ et R⁹ étant égaux ou différents et représentant
- de l'hydrogène,
- un groupe méthyle, éthyle, nitro, amino, monoalkylamino ayant 1 à 3 atomes de carbone, dialkylamino ayant 1 à 4 atomes de carbone, méthoxy, éthoxy, thiométhyle, thioéthyle, trifluorométhyle, cyano, halogéno,
et les dérivés acceptables du point de vue pharmaceutique de ces composés pour la préparation d'un médicament destiné à être utilisé dans le traitement antiviral du corps humain et d'un animal.

14. Utilisation de composés suivant la revendication 12 de formule générale (I) 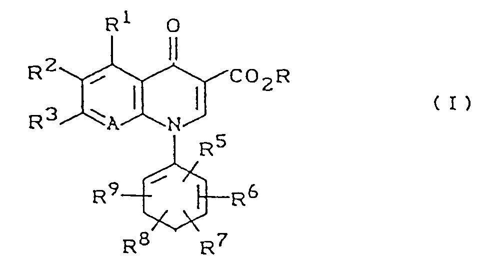 dans laquelle
R représente de l'hydrogène, un groupe méthyle ou éthyle,
R¹ est de l'hydrogène, un halogène,
R² est du fluor, un groupe nitro ou de l'hydrogène,
A représente N ou CR₄ où
R⁴ est de l'hydrogène ou un halogène,
R³ et R⁵ sont égaux ou différents et représentent un groupe NR²⁵R²⁶ dans lequel R²⁵ et R²⁶ sont égaux ou différents et représentent de l'hydrogène, un radical alkyle en C₁ à C₁₀, portant le cas échéant un ou plusieurs substituants halogéno, O-alkyle en C₁ à C₆, alkyle en C₁ à C₄ par groupe alkyle ou un groupe phényle éventuellement substitué par un halogène ou bien
R³ et R⁵ représentent un groupe de formule (II) 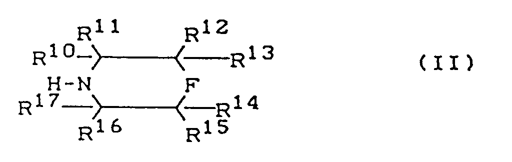
dans laquelle
F est du soufre, de l'oxygène, un groupe NR¹⁸ ou CR¹⁹R²⁰
et
R¹⁸ représente
- de l'hydrogène,
- un groupe méthyle, éthyle, propyle,
- un groupe cycloalkyle en C₃ à C₆,
- un groupe phényle éventuellement substitué par un halogène,
- un groupe CN,
- un groupe -COO-alkyle en C₁ à C₄,
- acyle en C₁ à C₄,
- sulfonyle en C₁ à C₄, et
R¹⁰, R¹¹, R¹⁷, R¹⁶ représente de l'hydrogène, R¹², R¹³, R¹⁴, R¹⁵, R¹⁹, R²⁰ sont égaux ou différents et représentent
- de l'hydrogène,
- un groupe alkyle en C₁ à C₃ ou cycloalkyle en C₃ à C₆, portant éventuellement un ou plusieurs substituants halogéno, OH, méthoxy, éthoxy, méthyle, éthyle, cyano, CO₂Me
- un groupe hydroxyle,
- ou un groupe amino, alkylamino ayant 1 à 4 atomes de carbone, dialkylamino ayant 1 à 3 atomes de carbone,
R³ et R⁵ sont égaux ou différents et représentent un groupe de formule 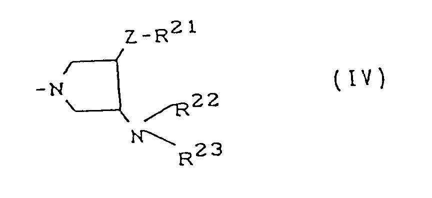
dans laquelle
R²¹ est de l'hydrogène, un groupe méthyle, éthyle, phényle, acyle en C₁ à C₄,
R²² est de l'hydrogène, un groupe méthyle, éthyle, OH, OCH₃, R²¹ et R²² pouvant aussi représenter un pont alkylène en C₁ à C₃ substitué une ou deux fois par un radical méthyle,
R²³ représente de l'hydrogène, un groupe méthyle, éthyle, aryle, benzyle, (alkoxy en C₁ à C₄)-carbonyle, acyle en C₁ à C₄,
Z représente O ou S,
ZR²¹ peut représenter de l'hydrogène,
et les dérivés pharmaceutiquement acceptables de ces composés pour la préparation d'un médicament destiné à être utilisé dans le traitement antiviral du corps humain ou d'un animal.

## Revendications (Revendications pour l'(les) Etat(s) contractant(s) suivant(s): ES)

1. Procédé de production de composés de formule générale (I) 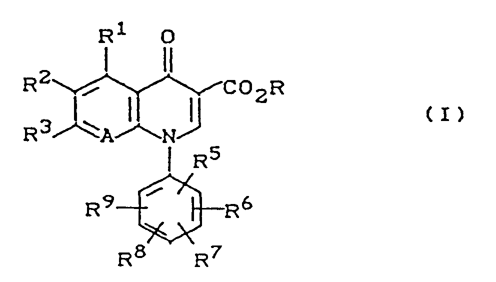 dans laquelle
R est de l'hydrogène ou un groupe alkyle ayant 1 à 4 atomes de carbone,
R¹ est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène, un groupe cyano ou nitro,
R² est de l'hydrogène, un groupe nitro ou un halogène,
A représente N ou un groupe CR₄, dans lequel
R⁴ est de l'hydrogène, un halogène, un radical méthyle, cyano, nitro, méthoxy ou amino, ou représente aussi un pont par l'intermédiaire de O, S ou CH₂ qui et lié en N-1 à la position ortho du noyau phényle,
R³ et R⁵ sont égaux ou différents et représentent un groupe NR²⁵R²⁶ dans lequel R²⁵ et R²⁶ sont égaux ou différents et représentent de l'hydrogène, un radical alkyle en C₁ à C₂₀, portant éventuellement un ou plusieurs substituants halogéno, OH, O-(alkyle en C₁ à C₆), alkyle en C₁ à C₄, CN, COO-(alkyle en C₁ à C₆),
- un groupe hydroxyle,
- un groupe amino, alkylamino ayant 1 à 4 atomes de carbone, dialkylamino ayant 1 à 3 atomes de carbone par groupe alkyle, ou phényle éventuellement substitué par un halogène, ou bien
R³ et R⁵ forment un groupe de formule (II) 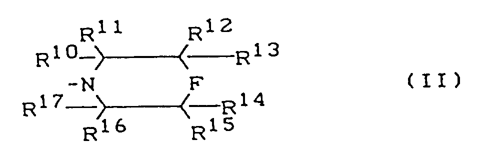
dans laquelle
F représente O, S, un groupe NR¹⁸ ou CR⁹R²⁰ et
R⁶, R⁷, R⁸ et R⁹ sont égaux ou différents et représentent
- de l'hydrogène,
- un groupe méthyle, éthyle, nitro, amino, monoalkylamino ayant 1 à 3 atomes de carbone, dialkylamino ayant 1 à 4 atomes de carbone, méthoxy, éthoxy, thiométhyle, thioéthyle,
trifluorométhyle, cyano, halogéno,
R¹⁸ représente
- H, un radical alkyle en C₁ à C₁₀ ou cycloalkyle en C₃ à C₆, portant éventuellement un ou plusieurs substituants Hal, OH, CN, COOR, OR dans lequel R est un radical alkyle en C₁ à C₇,
- un radical aryle en C₆ à C₁₂, portant éventuellement un substituant halogéno, -O-alkyle en C₁ à C₂ ou alkyle en C₁ à C₄,
- un radical CN
- un radical COO-(alkyle en C₁ à C₄),
- acyle en C₁ à C₄ ou
- sulfonyle en C₁ à C₄,
R¹⁰, R¹¹, R¹², R¹³, R¹⁴, R¹⁵, R¹⁶, R¹⁷, R¹⁹ et R²⁰ sont égaux ou différents et représentent :
- de l'hydrogène,
- un groupe alkyle en C₁ à C₃ ou cycloalkyle en C₃ à C₆ portant le cas échéant un ou plusieurs substituants halogéno, OH, O-alkyle en C₁ à C₆, alkyle en C₁ à C₄, CN, COO-(alkyle en C₁ à C₆),
- un groupe hydroxyle,
- un groupe amino, alkylamino ayant 1 à 4 atomes de carbone, dialkylamino ayant 1 à 3 atomes de carbone par groupe alkyle, ou phényle éventuellement substitué par un halogène, ou bien
R³ et R⁵ représentent un reste de structure (IV) 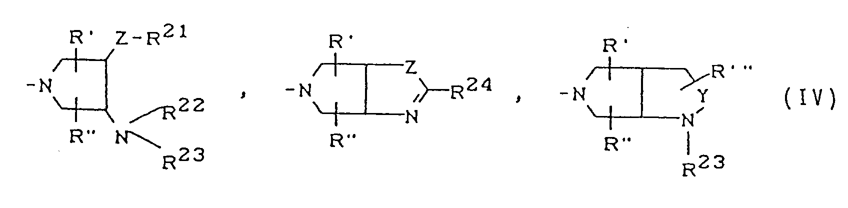
dans laquelle
R²¹ représente H, un groupe alkyle en C₁ à C₄, aryle, acyle en C₁ à C₄,
R²² représente H, un groupe alkyle en C₁ à C₄, OH, OCH₃, R²¹ et R²² pouvant aussi former ensemble un pont alkylène en C₁ à C₃ éventuellement substitué une ou deux fois par un radical alkyle,
R²³ peut être de l'hydrogène, un groupe alkyle en C₁ à C₄, aryle, hétéroaryle, benzyle, (alkoxy en C₁ à C₄)-carbonyle, acyle en C₁ à C₄ ou (5-méthyl-2-oxo-1,3-dioxol-4-yl)-méthyle,
R²⁴ peut représenter de l'hydrogène ou un groupe alkyle en C₁ à C₄,
R' peut représenter de l'hydrogène, un groupe CH₃ ou phényle,
R'' peut représente de l'hydrogène, un groupe CH₃ ou phényle,
R''' peut représenter de l'hydrogène ou un groupe CH₃,
Y peut représenter de l'oxygène, un groupe CH₂, CH₂CH₂ ou CH₂-O la liaison du groupe CH₂-O à l'azote pouvant être effectuée aussi bien par l'intermédiaire de O que par l'intermédiaire de CH₂,
Z peut représenter O ou S,
ZR²¹ peut être de l'hydrogène,
R⁶, R⁷, R⁸ et R⁹ étant égaux ou différents et représentant
- de l'hydrogène,
- un groupe alkyle ayant 1 à 5 atomes de carbone, nitro, amino, monoalkylamino ayant 1 à 3 atomes de carbone, dialkylamino ayant 1 à 6 atomes de carbone, O-alkyle ayant 1 à 5 atomes de carbone, S-alkyle ayant 1 à 5 atomes de carbone, trifluorométhyle, cyano, halogéno,
à l'exception :
de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique,
et les dérivés acceptables du point de vue pharmaceutique de ces composés,
caractérisé en ce qu'on fait réagir des quinoléines de formule générale (VI) 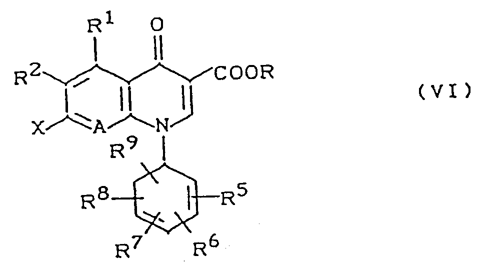 dans laquelle A, R, R¹, R², R⁵, R⁶, R⁷, R⁸, R⁹ ont la définition indiquée ci-dessus et X représente un halogène, avec des amines de formules générales (II) et (IV), 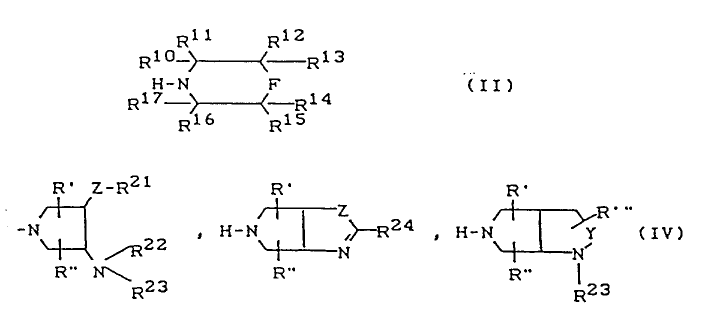 dans lesquelles
R¹⁰ à R¹⁷, R²¹ à R²⁴, R', R'' et R''', Y, Z et F ont la définition indiquée dans la revendication 1,
le cas échéant en présence de diluants et en la présence éventuelle d'accepteurs d'acides, et on transforme éventuellement les composés ainsi obtenus, d'une manière connue, en dérivés acceptables du point de vue pharmaceutique.

2. Procédé de production des composés de formule (1) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxylique et de l'acide 1-(4-aminophényl)-6-fluoro-7-(4-méthylpipérazinyl)-1,4-dihydro-4-oxo-3-quinoléinecarboxylique, caractérisé en ce qu'on fait réagir des composés de formule générale (VII) 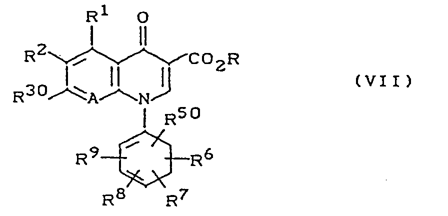 dans laquelle R, R¹, R², A, R⁶ à R⁹ ont la définition indiquée dans la revendication 1, les restes R³⁰ et R⁵⁰ sont égaux ou différents, des amines secondaires ou primaires étant présentes dans R³⁰ et dans R⁵⁰ ou seulement dans R³⁰ ou seulement dans R⁵⁰ et les structures de R³⁰ et R⁵⁰ étant déterminées par les représentants des restes R³ et respectivement R⁵,
avec des agents acylants ou des agents alkylants de formule générale (VIII)
v - w (VIII)
dans laquelle
w représente
- un groupe CN
- un groupe acyle en C₂ à C₄, éventuellement substitué,
- un groupe sulfonyle en C₁ à C₄,
- un groupe (alkoxy en C₁ à C₄)-carbonyle,
- un groupe alkyle en C₁ à C₅
et V représente un groupe partant tel que :
V = - un halogène
- un groupe trifluorométhanesulfonate
- un groupe mésylate
- un groupe tosylate
- un groupe acétate
- un groupe trihalogénacétate
le cas échéant en présence de diluants et la présence éventuelle d'accepteurs d'acides, et on transforme éventuellement les composés ainsi obtenus, d'une manière connue, en dérivés acceptables du point de vue pharmaceutique.

3. Procédé de production des composés de formule générale (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxylique, caractérisé en ce qu'on fait réagir un composé de formule générale (VII) suivant la revendication 2, avec des composés de formule générale (IX)
CH₂=CH-O (IX)
dans laquelle U est un groupe attirant les électrons, tel que COOB, COB, CONH₂n CN, SO₂B, SO₃B et B est un groupe alkyle en C₁ à C₄ qui est éventuellement substitué,
le cas échéant en présence de diluants.

4. Procédé de production des composés de formule (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxylique, caractérisé en ce qu'on fait réagir des composés de formule générale (VII) suivant la revendication 2 avec des acides carboxyliques de formule générale (X)
D-COOH (X)
dans laquelle D représente H ou un groupe alkyle en C₁ à C₄ éventuellement substitué, le cas échéant en présence de diluants, en la présence éventuelle de réactifs déshydratants et le cas échéant en présence d'un catalyseur.

## Revendications (Revendications pour l'(les) Etat(s) contractant(s) suivant(s): GR)

1. Composés de formule générale (I) 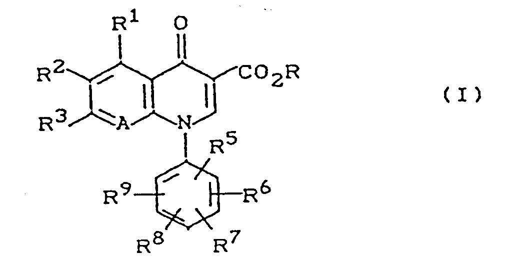 dans laquelle
R est de l'hydrogène ou un groupe alkyle ayant 1 à 4 atomes de carbone,
R¹ est de l'hydrogène, un groupe amino, alkylamino ayant 1 à 4 atomes de carbone, dialkylamino ayant 1 à 3 atomes de carbone par groupe alkyle, hydroxy, alkoxy ayant 1 à 4 atomes de carbone, mercapto, alkylthio ayant 1 à 4 atomes de carbone, arylthio, un halogène, un groupe cyano ou nitro,
R² est de l'hydrogène, un groupe nitro ou un halogène,
A représente N ou un groupe CR₄, dans lequel
R⁴ est de l'hydrogène, un halogène, un radical méthyle, cyano, nitro, méthoxy ou amino, ou représente aussi un pont par l'intermédiaire de O, S ou CH₂ qui et lié en N-1 à la position ortho du noyau phényle,
R³ et R⁵ sont égaux ou différents et représentent un groupe NR²⁵R²⁶ dans lequel R²⁵ et R²⁶ sont égaux ou différents et représentent de l'hydrogène, un radical alkyle en C₁ à C₂₀, portant éventuellement un ou plusieurs substituants halogéno, OH, O-(alkyle en C₁ à C₆), alkyle en C₁ à C₄, CN, COO-(alkyle en C₁ à C₆), - un groupe hydroxyle, - un groupe amino, alkylamino ayant 1 à 4 atomes de carbone, dialkylamino ayant 1 à 3 atomes de carbone par groupe alkyle, ou phényle éventuellement substitué par un halogène, ou bien
R³ et R⁵ forment un groupe de formule (II) 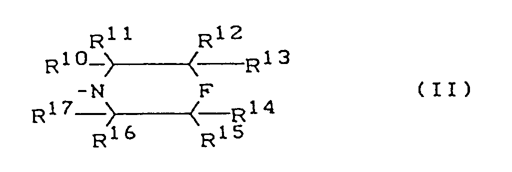
dans laquelle
F représente O, S, un groupe NR¹⁸ ou CR⁹R²⁰ et
R⁶, R⁷, R⁸ et R⁹ sont égaux ou différents et représentent
- de l'hydrogène,
- un groupe méthyle, éthyle, nitro, amino, monoalkylamino ayant 1 à 3 atomes de carbone, dialkylamino ayant 1 à 4 atomes de carbone, méthoxy, éthoxy, thiométhyle, thioéthyle, trifluorométhyle, cyano, halogéno,
R¹⁸ représente
- H, un radical alkyle en C₁ à C₁₀ ou cycloalkyle en C₃ à C₆, portant éventuellement un ou plusieurs substituants Hal, OH, CN, COOR, OR dans lequel R est un radical alkyle en C₁ à C₇,
- un radical aryle en C₆ à C₁₂, portant éventuellement un substituant halogéno, -O-alkyle en C₁ à C₂ ou alkyle en C₁ à C₄,
- un radical CN
- un radical COO- (alkyle en C₁ à C₄),
- acyle en C₁ à C₄ ou
- sulfonyle en C₁ à C₄,
R¹⁰, R¹¹, R¹², R¹³, R¹⁴, R¹⁵, R¹⁶, R¹⁷, R¹⁹ et R²⁰ sont égaux ou différents et représentent :
- de l'hydrogène,
- un groupe alkyle en C₁ à C₃ ou cycloalkyle en C₃ à C₆ portant le cas échéant un ou plusieurs substituants halogéno, OH, O-alkyle en C₁ à C₆, alkyle en C₁ à C₄, CN, COO-(alkyle en C₁ à C₆),
- un groupe hydroxyle,
- un groupe amino, alkylamino ayant 1 à 4 atomes de carbone, dialkylamino ayant 1 à 3 atomes de carbone par groupe alkyle, ou phényle éventuellement substitué par un halogène, ou bien
R³ et R⁵ représentent un reste de structure (IV) 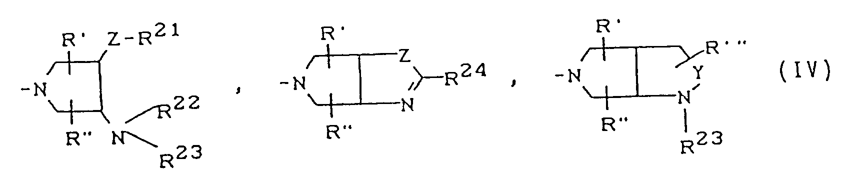
dans laquelle
R²¹ représente H, un groupe alkyle en C₁ à C₄, aryle, acyle en C₁ à C₄,
R²² représente H, un groupe alkyle en C₁ à C₄, OH, OCH₃, R²¹ et R²² pouvant aussi former ensemble un pont alkylène en C₁ à C₃ éventuellement substitué une ou deux fois par un radical alkyle,
R²³ peut être de l'hydrogène, un groupe alkyle en C₁ à C₄, aryle, hétéroaryle, benzyle, (alkoxy en C₁ à C₄)-carbonyle, acyle en C₁ à C₄ ou (5-méthyl-2-oxo-1,3-dioxol-4-yl)-méthyle,
R²⁴ peut représenter de l'hydrogène ou un groupe alkyle en C₁ à C₄,
R' peut représenter de l'hydrogène, un groupe CH₃ ou phényle,
R'' peut représente de l'hydrogène, un groupe CH₃ ou phényle,
R''' peut représenter de l'hydrogène ou un groupe CH₃,
Y peut représenter de l'oxygène, un groupe CH₂, CH₂CH₂ ou CH₂-O, la liaison du groupe CH₂-O à l'azote pouvant être effectuée aussi bien par l'intermédiaire de O que par l'intermédiaire de CH₂,
Z peut représenter O ou S,
ZR²¹ peut être de l'hydrogène,
R⁶, R⁷, R⁸ et R⁹ étant égaux ou différents et représentant
- de l'hydrogène,
- un groupe alkyle ayant 1 à 5 atomes de carbone, nitro, amino, monoalkylamino ayant 1 à 3 atomes de carbone, dialkylamino ayant 1 à 6 atomes de carbone, O-alkyle ayant 1 à 5 atomes de carbone, S-alkyle ayant 1 à 5 atomes de carbone, trifluorométhyle, cyano, halogéno,
à l'exception :
de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique,
et les dérivés acceptables du point de vue pharmaceutique de ces composés.

2. Composés de formule générale (I) suivant la revendication 1, 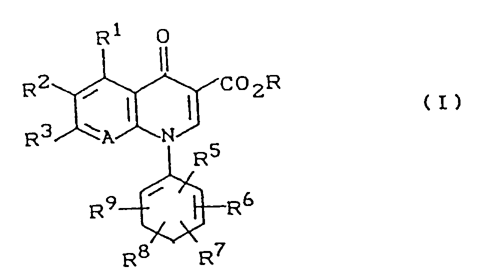 dans laquelle
R représente de l'hydrogène, un groupe méthyle ou éthyle,
R¹ est de l'hydrogène, un groupe amino, nitro, méthoxy, méthylthio, cyano ou un halogène,
R² est de l'hydrogène, un groupe nitro ou un halogène,
A représente N ou un groupe CR₄, dans lequel
R⁴ est de l'hydrogène, un halogène, un groupe méthyle, cyano, nitro, méthoxy ou amino ou aussi un pont par l'intermédiaire de O, S ou CH₂ qui est lié en N-1 à la position ortho du noyau phényle,
R³ et R⁵ sont égaux ou différents et représentent un groupe NR²⁵R²⁶, dans lequel R²⁵ et R²⁶ sont égaux ou différents et représentent de l'hydrogène, un radical alkyle en C₁ à C₂₀, portant le cas échéant un ou plusieurs substituants halogéno, O-alkyle en C₁ à C₆, alkyle en C₁ à C₄ par groupe alkyle ou phényle éventuellement substitué par un halogène, ou bien
R³ et R⁵ représentent un groupe de formule (II) 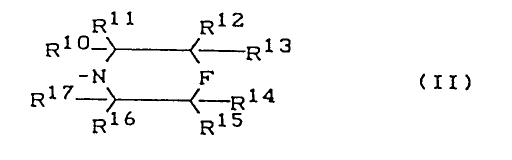
dans laquelle
F représente du soufre, de l'oxygène, un groupe NR¹⁸ ou CR¹⁹R²⁰ et
et
R¹⁸ représente
- de l'hydrogène, un groupe alkyle en C₁ a C₁₀ ou cycloalkyle en C₃ à C₁₀, portant éventuellement un ou plusieurs substituants Hal, OH, CN, COOR, OR dans lequel R est un radical alkyle en C₁ à C₇,
- un groupe aryle en C₆ à C₁₂, éventuellement substitué par un halogène, un radical -O-alkyle en C₁ ou C₂ ou alkyle en C₁ à C₄,
- un groupe CN,
- un groupe COO-(alkyle en C₁ à C₄),
- un groupe acyle en C₁ à C₄ ou
- un groupe sulfonyle en C₁ à C₄,
R¹⁰, R¹¹, R¹², R¹³, R¹⁴, R¹⁵, R¹⁶, R¹⁷, R¹⁹ et R²⁰ sont égaux ou différents et représentent :
- de l'hydrogène,
- un groupe alkyle en C₁ à C₃ ou cycloalkyle en C₃ à C₆ portant le cas échéant un ou plusieurs substituants halogéno, OH, méthoxy, éthoxy, méthyle, éthyle, cyano, CO₂Me,
- un groupe hydroxyle,
- un groupe amino, alkylamino ayant 1 à 4 atomes de carbone, dialkylamino ayant 1 à 3 atomes de carbone par groupe alkyle ou un groupe phényle éventuellement substitué par un halogène, ou bien
R³ et R⁵ représentent un reste des structures 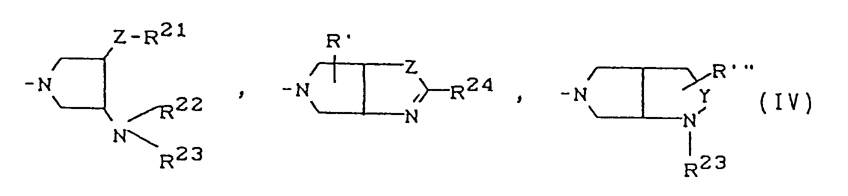
dans lesquelles
R²¹ représente H, un groupe alkyle en C₁ à C₄, aryle, acyle en C₁ à C₄,
R²² représente H, un groupe alkyle en C₁ à C₄, OH, OCH₃, R²¹ et R²² pouvant aussi former ensemble un pont alkylène en C₁ à C₃ portant éventuellement un ou deux substituants méthyle,
R²³ représente H, un groupe alkyle en C₁ à C₄, aryle, hétéroaryle, benzyle, (alkoxy en C₁ à C₄)carbonyle, acyle en C₁ à C₄,
R²⁴ représente H ou un groupe alkyle en C₁ à C₄,
Y peut représenter O, CH₂, CH₂CH₂ ou CH₂-O, la liaison du groupe CH₂-O à l'azote pouvant être effectuée aussi bien par l'intermédiaire de O que par l'intermédiaire de CH₂,
Z peut représenter O ou S,
ZR²¹ peut représenter de l'hydrogène,
R⁶, R⁷, R⁸ et R⁹ étant égaux ou différents et représentant
- de l'hydrogène,
- un groupe méthyle, éthyle, nitro, amino, monoalkylamino ayant 1 à 3 atomes de carbone, dialkylamino ayant 1 à 4 atomes de carbone, méthoxy, éthoxy, thiométhyle, thioéthyle, trifluorométhyle, cyano, halogéno,
à l'exception de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique,
et les dérivés acceptables du point de vue pharmaceutique de ces composés.

3. Composés de formule générale (I) suivant la revendication 2 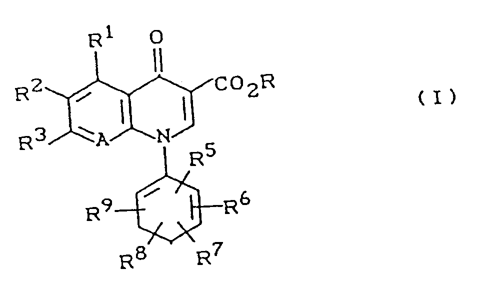 dans laquelle
R représente de l'hydrogène, un groupe méthyle ou éthyle,
R¹ est de l'hydrogène, un halogène,
R² est du fluor, un groupe nitro ou de l'hydrogène,
A représente N ou CR₄ où
R⁴ est de l'hydrogène ou un halogène,
R³ et R⁵ sont égaux ou différents et représentent un groupe NR²⁵R²⁶ dans lequel R²⁵ et R²⁶ sont égaux ou différents et représentent de l'hydrogène, un radical alkyle en C₁ à C₁₀, portant le cas échéant un ou plusieurs substituants halogéno, O-alkyle en C₁ à C₆, alkyle en C₁ à C₄ par groupe alkyle ou un groupe phényle éventuellement substitué par un halogène ou bien
R³ et R⁵ représentent un groupe de formule (II) 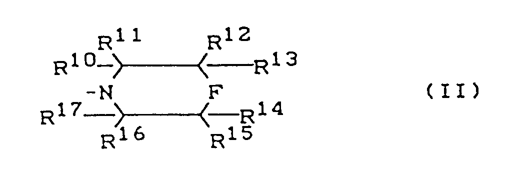
dans laquelle
F est du soufre, de l'oxygène, un groupe NR¹⁸ ou CR¹⁹R²⁰
et
R¹⁸ représente
- de l'hydrogène,
- un groupe méthyle, éthyle, propyle,
- un groupe cycloalkyle en C₃ à C₆,
- un groupe phényle éventuellement substitué par un halogène,
- un groupe CN,
- un groupe -COO-alkyle en C₁ à C₄,
- acyle en C₁ à C₄,
- sulfonyle en C₁ à C₄, et
R¹⁰, R¹¹, R¹⁷, R¹⁶ représentent de l'hydrogène, R¹², R¹³, R¹⁴, R¹⁵, R¹⁹, R²⁰ sont égaux ou différents et représentent
- de l'hydrogène,
- un groupe alkyle en C₁ à C₃ ou cycloalkyle en C₃ à C₆, portant éventuellement un ou plusieurs substituants halogéno, OH, méthoxy, éthoxy, méthyle, éthyle, cyano, CO₂Me
- un groupe hydroxyle,
- ou un groupe amino, alkylamino ayant 1 à 4 atomes de carbone, dialkylamino ayant 1 à 3 atomes de carbone,
R³ et R⁵ sont égaux ou différents et représentent un groupe de formule 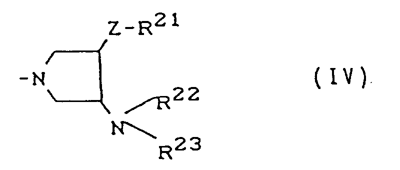 dans laquelle
R²¹ est de l'hydrogène, un groupe méthyle, éthyle, phényle, acyle en C₁ à C₄,
R²² est de l'hydrogène, un groupe méthyle, éthyle, OH, OCH₃, R²¹ et R²² pouvant aussi représenter un pont alkylène en C₁ à C₃ substitué une ou deux fois par un radical méthyle,
R²³ représente de l'hydrogène, un groupe méthyle, éthyle, aryle, benzyle, (alkoxy en C₁ à C₄)-carbonyle, acyle en C₁ à C₄,
Z représente O ou S,
ZR²¹ peut représenter de l'hydrogène,
à l'exception
de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique,
et les dérivés acceptables du point de vue pharmaceutique de ces composés.

4. Procédé de production de composés de formule (I) suivant la revendication 1, dans laquelle R³ et R⁶ sont égaux, à l'exception
de l'acide 1-(3-méthylamino-4-fluorophényl)-6-fluoro-7-(3-méthylpipérazinyl)-1,4-dihydro-4-oxo-3-quinoléinecarboxylique,
de l'acide 1-(4-aminophényl)-6-fluoro-7-pipérazinyl-1,4-dihydro-4-oxo-3-quinoléinecarboxylique, de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique, caractérisé en ce qu'on fait réagir des quinoléines de formule générale (III) 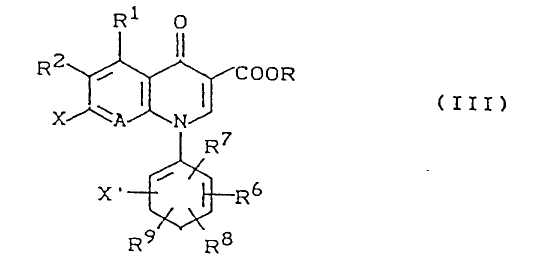 dans laquelle
A, R, R¹, R², R⁶, R⁷, R⁸ et R⁹ ont la définition indiquée dans la revendication 1 et X et X' sont égaux ou différents et représentent un atome d'halogène,
avec des amines de formules générales (II) et (IV) 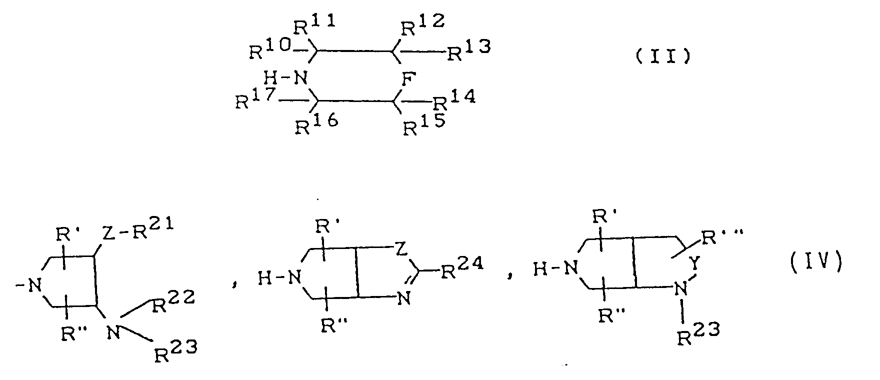 dans lesquelles
R¹⁰ à R¹⁷, R²¹ à R²⁴, Y, Z, R', R'', R''' et F ont la définition indiquée dans la revendication 1,
le cas échéant en présence de diluants et en la présence éventuelle d'accepteurs d'acides.

5. Procédé de production des composés de formule (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxyli que,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique, caractérisé en ce qu'on fait réagir des composés de formule générale (V) 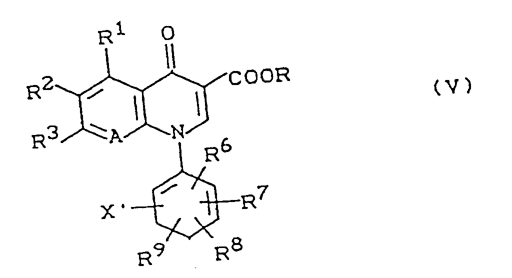 dans laquelle
A, R, R¹ à R⁹ ont la définition indiquée dans la revendication 1 et X' représente un atome d'halogène,
avec des amines de formules générales (II) et (IV) 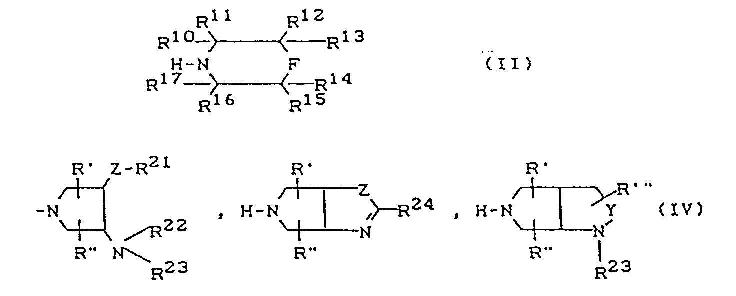 dans lesquelles
R¹⁰ à R¹⁷, R²¹ à R²⁴, R', R'' et R''', Y, Z et F ont la définition indiquée dans la revendication 1,
le cas échéant en présence de diluants et en la présence éventuelle d'accepteurs d'acides.

6. Procédé de production des composés de formule (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxyli que,
de l'acide 1-(4-aminophényl)-6-fluoro-7-pipérazinyl-1,4-dihydro-4-oxo-3-quinoléinecarboxylique,
de l'acide 1-(4-aminophényl)-6-fluoro-7-(4-méthylpipérazinyl)-1,4-dihydro-4-oxo-3-quinoléinecarboxylique et de l'acide 1-(4-aminophényl)-6,8-difluoro-7-morpholinyl-1,4-dihydro-4-oxo-3-quinoléinecarboxylique, caractérisé en ce qu'on fait réagir des quinoléines de formule générale (VI) 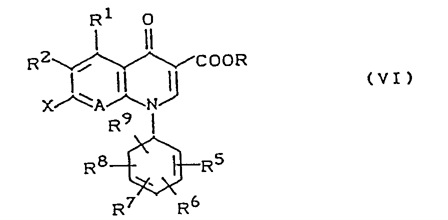 dans laquelle
A, R, R¹, R², R⁵, R⁶, R⁷, R⁸ et R⁹ ont la définition indiquée dans la revendication 1 et X représente un halogène, avec des amines de formules (II) et (IV), 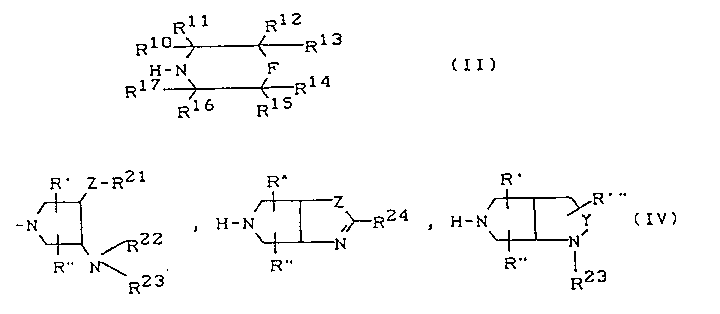 dans lesquelles
R¹⁰ à R¹⁷, R²¹ à R²⁴, R', R'' et R''', Y, Z et F ont la définition indiquée dans la revendication 1, le cas échéant en présence de diluants et en la présence éventuelle d'accepteurs d'acides.

7. Procédé de production des composés de formule (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxylique, et de l'acide 1-(4-aminophényl)-6-fluoro-7-(4-méthylpipérazinyl)-1,4-dihydro-4-oxo-3-quinoléinecarboxylique, caractérisé en ce qu'on fait réagir des composés de formule (VII) 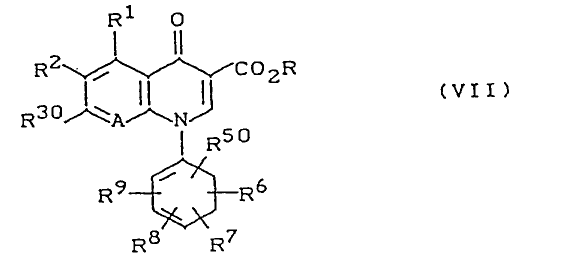 dans laquelle R, R¹, R², A, R⁶ à R⁹ ont la définition indiquée dans la revendication 1, les restes R³⁰ et R⁵⁰ sont égaux ou différents, les amines secondaires ou primaires étant présentes dans R³⁰ et R⁵⁰ ou seulement dans R³⁰ ou seulement dans R⁵⁰ et les structures de R³⁰ et R⁵⁰ étant déterminées par les représentants des restes R³ et respectivement R⁵,
avec des agents acylants ou des agents alkylants de formule générale (VIII)
V - W (VIII)
dans laquelle
W représente
- un groupe CN
- un groupe acyle en C₂ à C₄ éventuellement substitué,
- un groupe sulfonyle en C₂ à C₄,
- un groupe (alkoxy en C₁ à C₄)-carbonyle,
- un groupe alkyle en C₁ à C₅ et
V représente un groupe partant tel que :
V = - un halogène
- un groupe trifluorométhylsulfonate
- un groupe mésylate
- un groupe tosylate
- un groupe acétate
- un groupe trihalogénacétate
le cas échéant en présence de diluants et en la présence éventuelle d'accepteurs d'acides.

8. Procédé de production des composés de formule générale (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxylique, caractérisé en ce qu'on fait réagir des composés de formule générale (VII) suivant la revendication 7 avec des composés de formule générale (IX)
CH₂=CH-U (IX)
dans laquelle U est un groupe attirant les électrons, tel que COOB, COB, CONH₂, CN, SO₂B, SO₃B et B représente un groupe alkyle en C₁ à C₄ qui est éventuellement substitué,
le cas échéant en présence de diluants.

9. Procédé de production des composés de formule (I) suivant la revendication 1, à l'exception de l'acide 1-(3-méthylamino-4-fluorophényl)-6-fluoro-7-(3-méthylpipérazinyl)-1,4-dihydro-4-oxo-3-quinoléinecarboxylique, caractérisé en ce qu'on fait réagir des composés de formule générale (VII) suivant la revendication 7 avec des acides carboxyliques de formule générale (X)
D-COOH (X)
dans laquelle D représente de l'hydrogène ou un groupe alkyle en C₁ à C₄ éventuellement substitué, le cas échéant en présence de diluants, et en la présence éventuelle de réactifs déshydratants et, le cas échéant, en présence d'un catalyseur.
